(19) 

**Europäisches Patentamt**

**European Patent Office**

**Office européen des brevets**

(11) **EP 4 768 039 A1**

(12) **EUROPEAN PATENT APPLICATION**
published in accordance with Art. 153(4) EPC

(43) Date of publication:
01.07.2026 Bulletin 2026/27

(21) Application number: 24855887.6

(22) Date of filing: 23.08.2024

(51) International Patent Classification (IPC):
*A61K 38/17* (2006.01)   *C12N 15/86* (2006.01)
*C07K 19/00* (2006.01)   *C07K 16/22* (2006.01)
*C07K 14/005* (2006.01)   *A61P 25/28* (2006.01)

(52) Cooperative Patent Classification (CPC):
A61K 38/17; A61P 25/28; C07K 14/005;
C07K 16/22; C07K 19/00; C12N 15/86

(86) International application number:
PCT/CN2024/114158

(87) International publication number:
WO 2025/040166 (27.02.2025 Gazette 2025/09)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 23.08.2023   CN 202311073344
24.08.2023   CN 202311077883

(71) Applicant: Shanghai Regenelead Therapies Co.,
Ltd.
Shanghai 201206 (CN)

(72) Inventors:
• HAN, Longyu
  Shanghai 201206 (CN)
• LIAO, Cheng
  Shanghai 201206 (CN)

(74) Representative: Regimbeau
20, rue de Chazelles
75847 Paris Cedex 17 (FR)

Remarks:
The complete document including Reference
Table(s) and the Sequence Listing(s) can be
downloaded from the EPO website

(54) **PHARMACEUTICAL COMPOSITION AND USE THEREOF**

(57)    The present invention relates to a pharmaceutical composition and the use thereof. In particular, the present invention relates to a pharmaceutical composition comprising an rAAV particle and the use thereof.

EP 4 768 039 A1

## Description

The present disclosure claims priority to the following patent applications: Chinese Patent Application No. CN202311073344.0 filed on August 23, 2023 and Chinese Patent Application No. CN202311077883.1 filed on August 24, 2023, which are incorporated herein by reference in their entireties.

## TECHNICAL FIELD

The present disclosure belongs to the field of biopharmaceuticals and particularly relates to a pharmaceutical composition comprising recombinant adeno-associated virus (rAAV) particles and use thereof as a medicament.

## BACKGROUND

Adeno-associated virus (AAV) belongs to the genus *Dependovirus* of the family *Parvoviridae*. The AAV genome consists of a single-stranded DNA molecule, which is approximately 4.7 kilobases (kb) in length and is composed of two major open reading frames (encoding the non-structural Rep (replication) protein and the structural Cap (capsid) protein, respectively). The AAV encoding region is flanked by two cis-acting inverted terminal repeat (ITR) sequences, each approximately 145 nucleotides in length. These ITRs contain interrupted palindromic sequences (that can be folded into hairpin structures that serve as primers during the initiation of DNA replication).

As vectors, AAVs have great potential for the treatment of hereditary diseases and genetic diseases and are useful for gene supplementation therapy (also referred to as gene enhancement therapy). AAVs recover or restore the lost or dysregulated gene function resulting from mutations by supplying the missing gene function, thereby restoring the biological function of the target cell to a normal physiological state. The performance of AAV-based vectors in preclinical disease models and in human clinical trials has shown prospects for its application to the treatment of several diseases. For example, a gene can be efficiently transferred into retinal cells and constantly expressed for a long time (Boye et al. Mol Ther. 2013 Mar; 21(3):509-19. Trapani et al. Prog Retin Eye Res. 2014 Nov; 43:108-28). At present, the AAV vectors for clinical use have been engineered to be latent when no helper virus is present. Their safety and long-term expression of transgenes have been extensively tested in rodent models, non-human primates, and several human trials (MacLaren et al. Lancet. 2014 Mar 29; 383(9923):1129-37; Maguire et al. N Engl J Med. 2008 May 22; 358(21):2240-8; Simonelli et al. Mol Ther. 2010 Mar; 18(3):643-50; Nathwani et al. N Engl J Med. 2014 Nov 20; 371(21):1994-2004).

The recombinant adeno-associated virus (rAAV) is derived from a non-pathogenic wild-type adeno-associated virus and has characteristics of good safety, wide host cell range (dividing and non-dividing cells), low immunogenicity, and long duration for expressing exogenous genes *in vivo.* It is considered to be one of the most promising gene delivery vectors and is widely applied to gene therapy and vaccine research worldwide (Snyder 1999; Xiao, Lentz, and Samulski 2012).

To make AAV safe for clinical use, AAV has been genetically modified at several positions within its genome. For example, Rep genes required for viral replication and elements required for site-specific integration have been eliminated from the AAV genome in many viral vectors. This recombinant AAV (rAAV) exists in an extrachromosomal state and has an extremely low efficiency of integration into genomic DNA. If not completely eliminated, the probability of the rAAV inducing random mutagenesis in the host cell is thus reduced. Due to these properties and the lack of pathogenicity, rAAV has shown great promise as a gene therapy vector in multiple aspects of preclinical and clinical applications. New serotype vectors are also the focus of clinical development at present.

AAV research also focuses on the development of AAV formulations for administration to humans. An important problem that needs to be addressed at present is how to develop AAV formulations that maintain AAV long-term stability and strong potency and maintain infection efficiency and biological activity under various stress conditions.

## SUMMARY

The present disclosure provides a pharmaceutical composition comprising an adeno-associated virus (AAV) particle, a method for preparing the pharmaceutical composition, and a method for treating or preventing a disease using same, or related pharmaceutical use.

The present disclosure provides a pharmaceutical composition comprising recombinant adeno-associated virus (rAAV) particles and excipients. In some embodiments, the excipient comprises Hepes and/or BTP. Illustratively, the excipient comprises Hepes, BTP, or a combination of Hepes and BTP.

In some embodiments, the pharmaceutical composition comprises Hepes. Hepes is also known as N-2-hydroxyethylpiperazine-N'-2-ethanesulfonic acid. Hepes plays a role as a buffer, a stabilizer, etc. in the pharmaceutical composition, and can allow the pharmaceutical composition to maintain stability and high biological potency for a long period of time (e.g., 1W, 2W, 3W, 1M, 2M, 3M, etc.).

In some embodiments, the pharmaceutical composition comprises BTP. BTP is also known as bis-tris propane and can act as a buffer, a stabilizer, etc. In some embodiments, the pharmaceutical composition comprises Hepes and BTP.

In some embodiments, the Hepes or BTP is at a concentration of about 1 mM to about 100 mM, e.g., about 1 mM to about 50 mM, about 1 mM to about 55 mM, about 1 mM to about 60 mM, about 1 mM to about 70 mM, about 1 mM to about 80 mM, about 5 mM to about 100 mM, about 1 mM to about 80 mM, about 5 mM to about 70 mM, about 5 mM to about 50 mM, about 5 mM to about 45 mM, about 5 mM to about 40 mM, about 5 mM to about 35 mM, about 5 mM to about 30 mM, about 5 mM to about 25 mM, about 5 mM to about 20 mM, about 5 mM to about 10 mM, about 10 mM to about 20 mM, about 10 mM to about 30 mM, about 10 mM to about 35 mM, about 10 mM to about 40 mM, about 10 mM to about 50 mM, about 15 mM to about 20 mM, about 15 mM to about 25 mM, about 15 mM to about 35 mM, about 15 mM to about 50 mM, about 20 mM to about 35 mM, about 1 mM to about 10 mM, about 1 mM to about 20 mM, or any range between these point values. In some embodiments, the Hepes or BTP is at a concentration of about 5 mM to about 50 mM. In some embodiments, the Hepes or BTP is at a concentration of about 5 mM to about 25 mM. In some embodiments, the Hepes or BTP is at a concentration of about 10 mM.

In some embodiments, the Hepes or BTP is at a concentration of about 1 mM, about 5 mM, about 6 mM, about 7 mM, about 8 mM, about 9 mM, about 10 mM, about 11 mM, about 12 mM, about 13 mM, about 14 mM, about 15 mM, about 16 mM, about 17 mM, about 18 mM, about 19 mM, about 20 mM, about 22 mM, about 25 mM, about 26 mM, about 28 mM, about 30 mM, about 35 mM, about 40 mM, about 43 mM, about 45 mM, about 48 mM, about 50 mM, about 55 mM, about 57 mM, about 58 mM, about 60 mM, about 62 mM, about 65 mM, about 68 mM, about 70 mM, about 72 mM, about 75 mM, about 78 mM, about 80 mM, about 82 mM, about 85 mM, about 88 mM, about 90 mM, about 92 mM, about 95 mM, or about 100 mM.

In some embodiments, the pharmaceutical composition has a pH of about 4.0 to about 10.0, e.g., about 5.0 to about 10.0, about 6.0 to about 10.0, about 5.0 to about 9.0, about 5.5 to about 9.0, about 5.5 to about 8.5, about 6.5 to about 8.5, about 5.5 to about 8.0, about 5.5 to about 7.5, about 5.5 to about 7.0, about 6.0 to about 7.0, about 6.0 to about 7.5, about 6.0 to about 8.0, about 6.0 to about 8.5, about 6.0 to about 9.0, about 4.0 to about 6.0, about 4.0 to about 5.5, about 4.0 to about 5.0, about 4.0 to about 4.8, 4.0 to about 4.5, about 4.8 to about 5.0, about 5.0 to about 5.5, about 4.5 to about 4.8, or any range between these point values. In some embodiments, the pharmaceutical composition has a pH of about 6.0 to about 8.0. In some embodiments, the buffer or the pharmaceutical composition has a pH of about 6.0 to about 7.5.

In some embodiments, the buffer or the pharmaceutical composition has a pH of about 4.0, about 4.1, about 4.2, about 4.3, about 4.4, about 4.5, about 4.6, about 4.7, about 4.8, about 4.9, about 5.0, about 5.1, about 5.2, about 5.3, about 5.4, about 5.5, about 5.6, about 5.7, about 5.8, about 5.9, about 6.0, about 6.1, about 6.2, about 6.3, about 6.4, about 6.5, about 6.6, about 6.7, about 6.8, about 6.9, about 7.0, about 7.1, about 7.2, about 7.3, about 7.4, about 7.5, about 7.6, about 7.7, about 7.8, about 7.9, about 8.0, about 8.1, about 8.2, about 8.3, about 8.4, about 8.5, about 8.6, about 8.7, about 8.8, about 8.9, about 9.0, about 9.1, about 9.2, about 9.3, about 9.4, about 9.5, about 9.6, about 9.7, about 9.8, about 9.9, or about 10.0.

Generally, the pH of a pharmaceutical composition obtained by buffer exchange is almost consistent with the pH of the buffer. Also, it is well known to those skilled in the art that in the process of pharmaceutical formulation, there may sometimes be a pH drift, but the pH drift of the pharmaceutical formulation is generally small (e.g., within a range of ±0.8). In some embodiments, the pH drift of the pharmaceutical formulation is within a range of ±0.5.

In some embodiments, provided is the pharmaceutical composition according to any one of the above, which comprises a surfactant. In some embodiments, the surfactant is a nonionic surfactant. In some embodiments, the surfactant is selected from the group consisting of poloxamer (e.g., poloxamer 188), polysorbate (e.g., polysorbate 20 (i.e., PS20) or polysorbate 80 (i.e., PS80)), poloxamer, Triton, sodium dodecyl sulfonate, sodium lauryl sulfonate, sodium octyl glycoside, lauryl-sulfobetaine, myristyl-sulfobetaine, linoleyl-sulfobetaine, stearyl-sulfobetaine, lauryl-sarcosine, myristyl-sarcosine, linoleyl-sarcosine, stearyl-sarcosine, linoleyl-betaine, myristyl-betaine, cetyl-betaine, lauramido propyl-betaine, cocamidopropyl-betaine, linoleamidopropyl-betaine, myristamidopropyl betaine, palmitamidopropyl betaine, isostearamidopropyl-betaine, myristamidopropyl-dimethylamine, palmitamidopropyl-dimethylamine, isostearamidopropyl-dimethylamine, sodium methyl cocoyl, sodium methyl oleyl taurate, polyethylene glycol, polypropylene glycol, copolymers of ethylene and propylene glycol, and any combinations thereof. In some embodiments, the surfactant is sodium dodecyl sulfate (SDS) or polysorbate. In some embodiments, the surfactant is poloxamer 188 and/or polysorbate 80. In some embodiments, the surfactant is poloxamer 188.

In some embodiments, the surfactant is at a concentration of about 0.001% (w/v) to about 1.0% (w/v), e.g., about 0.001% (w/v) to about 0.8% (w/v), about 0.001% (w/v) to about 0.5% (w/v), about 0.001% (w/v) to about 0.2% (w/v), about 0.001% (w/v) to about 0.1% (w/v), about 0.001% (w/v) to about 0.08% (w/v), about 0.001% (w/v) to about 0.05% (w/v), about 0.001% (w/v) to about 0.03% (w/v), about 0.001% (w/v) to about 0.02% (w/v), about 0.001% (w/v) to about 0.01% (w/v), about 0.005% (w/v) to about 0.08% (w/v), about 0.005% (w/v) to about 0.05% (w/v), about 0.005% (w/v) to about 0.03% (w/v), about 0.005% (w/v) to about 0.02% (w/v), about 0.005% (w/v) to about 0.01% (w/v), about 0.005% (w/v) to about 0.1% (w/v), about 0.005% (w/v) to about 0.15% (w/v), about 0.005% (w/v) to about 0.18% (w/v), about 0.008% (w/v) to about 0.18% (w/v), about 0.008% (w/v) to about 0.15% (w/v), about 0.005% (w/v) to about 0.2% (w/v), about 0.005% (w/v) to about 0.3% (w/v), about 0.01% (w/v) to about 1.0% (w/v), about 0.01% (w/v) to about 0.8% (w/v), about 0.01% (w/v) to about 0.5% (w/v), about 0.01% (w/v) to about 0.2% (w/v), about 0.01% (w/v) to about 0.1% (w/v), about 0.01% (w/v) to about 0.18% (w/v), about 0.01% (w/v) to about 0.18% (w/v), about 0.01% (w/v) to about 0.15% (w/v), about 0.01% (w/v) to about 0.1% (w/v), about 0.01% (w/v) to about 0.08% (w/v), about 0.01% (w/v) to about 0.06% (w/v), about 0.01% (w/v) to about 0.04% (w/v), about 0.02% (w/v) to about 0.2% (w/v), about 0.02% (w/v) to about 0.15% (w/v), about 0.02% (w/v) to about 0.1% (w/v), about 0.02% (w/v) to about 0.08% (w/v), about 0.02% (w/v) to about 0.04% (w/v), about 0.04% (w/v) to about 0.2% (w/v), about 0.04% (w/v) to about 0.1% (w/v), about 0.04% (w/v) to about 0.08% (w/v), and any one range between these point values. In some embodiments, the surfactant is at a concentration of about 0.001% (w/v) to about 0.05% (w/v). In some embodiments, the surfactant is at a concentration of about 0.01% (w/v) to about 0.02% (w/v).

In some embodiments, the surfactant is at a concentration of about 0.001% (w/v), 0.002% (w/v), 0.003% (w/v), 0.004% (w/v), 0.005% (w/v), 0.006% (w/v), 0.007% (w/v), 0.008% (w/v), 0.009% (w/v), 0.01% (w/v), 0.011% (w/v), 0.012% (w/v), 0.013% (w/v), 0.015% (w/v), 0.018% (w/v), 0.019% (w/v), 0.02% (w/v), about 0.01% (w/v), about 0.02% (w/v), about 0.03% (w/v), about 0.04% (w/v), about 0.05% (w/v), about 0.06% (w/v), about 0.07% (w/v), about 0.08% (w/v), about 0.09% (w/v), about 0.1% (w/v), about 0.11% (w/v), about 0.12% (w/v), about 0.13% (w/v), about 0.14% (w/v), about 0.15% (w/v), about 0.16% (w/v), about 0.17% (w/v), about 0.18% (w/v), about 0.19% (w/v), about 0.2% (w/v), about 0.3% (w/v), about 0.4% (w/v), about 0.5% (w/v), about 0.6% (w/v), about 0.7% (w/v), or about 0.8% (w/v).

In some embodiments, provided is the pharmaceutical composition according to any one of the above, which comprises one or more of a saccharide, an alcohol, and a sugar alcohol. The "one or more" in the present disclosure includes one, two, or more than two.

Any saccharide, such as monosaccharides, disaccharides, or polysaccharides, or water-soluble glucans, including, for example, fructose, glucose, mannose, sorbose, xylose, maltose, lactose, sucrose, dextran, trehalose, pullulan, dextrin, cyclodextrin, soluble starch, hydroxyethyl starch, and carboxymethyl cellulose, may be used in the present disclosure. In particular embodiments, the saccharide may be sucrose, glucose, trehalose, or a combination thereof. In certain embodiments, the trehalose may be trehalose dihydrate. Sugar alcohols are defined as hydrocarbons with between about 4 and about 8 carbon atoms and a hydroxyl group. Non-limiting examples of alcohols or sugar alcohols in the present disclosure include mannitol, sorbitol, inositol, galactitol, dulcitol, xylitol, and arabitol. In certain embodiments, mannitol may be used as a sugar alcohol additive. In certain embodiments, the buffer solution preparation contains both a saccharide and a sugar alcohol additive. In certain embodiments, the saccharide may be sucrose. In certain embodiments, the saccharide is trehalose. In certain embodiments, the alcohol is glycerol. Saccharides, alcohols, and sugar alcohols may be used individually or in combination. In some embodiments, the pharmaceutical composition comprises a saccharide and an alcohol. In some embodiments, the pharmaceutical composition comprises sucrose and glycerol.

In some embodiments, the saccharide is at a concentration of about 0.1% (w/v) to about 10% (w/v), e.g., about 0.1% (w/v) to about 9% (w/v), about 0.1% (w/v) to about 8% (w/v), about 0.1% (w/v) to about 7% (w/v), about 0.1% (w/v) to about 6% (w/v), about 0.1% (w/v) to about 5% (w/v), about 0.1% (w/v) to about 3% (w/v), about 1% (w/v) to about 9% (w/v), about 1% (w/v) to about 8% (w/v), about 1% (w/v) to about 7% (w/v), about 1% (w/v) to about 6% (w/v), about 1% (w/v) to about 5% (w/v), about 1% (w/v) to about 3% (w/v), about 1.5% (w/v) to about 9% (w/v), about 1.5% (w/v) to about 8% (w/v), about 1.5% (w/v) to about 7% (w/v), about 1.5% (w/v) to about 6% (w/v), about 1.5% (w/v) to about 5% (w/v), about 1.5% (w/v) to about 3% (w/v), and any one range between these point values.

In some embodiments, the saccharide is at a concentration of about 0.1% (w/v), about 0.2% (w/v), about 0.5% (w/v), about 0.6% (w/v), about 0.7% (w/v), about 0.8% (w/v), about 0.9% (w/v), about 1.0% (w/v), about 1.1% (w/v), about 1.2% (w/v), about 1.3% (w/v), about 1.4% (w/v), about 1.5% (w/v), about 1.6% (w/v), about 1.7% (w/v), about 1.8% (w/v), about 1.9% (w/v), about 2.1% (w/v), about 2.2% (w/v), about 2.3% (w/v), about 2.4% (w/v), about 2.5% (w/v), about 2.6% (w/v), about 2.7% (w/v), about 2.8% (w/v), about 2.9% (w/v), about 3.0% (w/v), about 3.1% (w/v), about 3.2% (w/v), about 3.3% (w/v), about 3.4% (w/v), about 3.5% (w/v), about 3.6% (w/v), about 3.7% (w/v), about 3.8% (w/v), about 3.9% (w/v), about 4.0% (w/v), about 4.1% (w/v), about 4.2% (w/v), about 4.3% (w/v), about 4.4% (w/v), about 4.5% (w/v), about 4.6% (w/v), about 4.7% (w/v), about 4.8% (w/v), about 4.9% (w/v), about 5.0% (w/v), about 5.1% (w/v), about 5.2% (w/v), about 5.3% (w/v), about 5.5% (w/v), about 5.5% (w/v), about 5.6% (w/v), about 5.7% (w/v), about 5.8% (w/v), about 5.9% (w/v), about 6.0% (w/v), about 6.1% (w/v), about 6.3% (w/v), about 6.5% (w/v), about 6.7% (w/v), about 6.8% (w/v), about 7.1% (w/v), about 7.3% (w/v), about 7.5% (w/v), about 7.7% (w/v), about 7.8% (w/v), about 7.9% (w/v), about 8.0% (w/v), about 8.5% (w/v), about 9.0% (w/v), about 9.5% (w/v), or about 10.0% (w/v).

In some embodiments, the alcohol is at a concentration of about 0.01% (w/v) to about 10% (w/v), e.g., about 0.01% (w/v) to about 8% (w/v), about 0.01% (w/v) to about 9% (w/v), about 0.01% (w/v) to about 8% (w/v), about 0.01% (w/v) to about 7% (w/v), about 0.01% (w/v) to about 5% (w/v), about 0.01% (w/v) to about 4% (w/v), about 0.01% (w/v) to about 3% (w/v), about 0.01% (w/v) to about 1% (w/v), 0.05% (w/v) to about 1% (w/v), 0.05% (w/v) to about 1.5% (w/v). 0.05% (w/v) to about 2% (w/v), 0.05% (w/v) to about 3.5% (w/v), 0.05% (w/v) to about 5.0% (w/v), 0.05% (w/v) to about 6.0% (w/v), 0.05% (w/v) to about 7.0% (w/v), about 0.1% (w/v) to about 1% (w/v), about 0.1% (w/v) to about 0.8% (w/v), about 0.1% (w/v) to about 0.7% (w/v), about 0.1% (w/v) to about 0.6% (w/v), about 0.1% (w/v) to about 0.5% (w/v), about 0.1% (w/v) to about 0.4% (w/v), about 0.2% (w/v) to about 0.8% (w/v), about 0.1% (w/v) to about 0.3% (w/v), or about 0.2% (w/v) to about 2% (w/v).

In some embodiments, the alcohol is at a concentration of about 0.01% (w/v), about 0.05% (w/v), about 0.015% (w/v), about 0.02% (w/v), about 0.01% (w/v), about 0.08% (w/v), about 0.1% (w/v), about 0.15% (w/v), about 0.18% (w/v), about 0.2% (w/v), about 0.22% (w/v), about 0.25% (w/v), about 0.28% (w/v), about 0.3% (w/v), about 0.35% (w/v), about 0.4% (w/v), about 0.45% (w/v), about 0.5% (w/v), about 0.55% (w/v), about 0.6% (w/v), about 0.65% (w/v), about 0.7% (w/v), about 0.8% (w/v), about 0.9% (w/v), about 1% (w/v), about 2% (w/v), about 3% (w/v), about 4% (w/v), about 5% (w/v), about 6% (w/v), about 7% (w/v), about 8% (w/v), about 9% (w/v), or about 10.0% (w/v).

In some embodiments, provided is the pharmaceutical composition according to any one of the above, which comprises an amino acid. The amino acid includes, but is not limited to, arginine, glycine, cysteine, histidine, and the like. In some embodiments, the amino acid is histidine. For example, the amino acid is L-histidine.

In some embodiments, the amino acid is at a concentration of about 1 mM to about 50 mM, e.g., about 1 mM to about 40 mM, about 1 mM to about 35 mM, about 1 mM to about 30 mM, about 1 mM to about 25 mM, about 1 mM to about 15 mM, about 1 mM to about 20 mM, about 1 mM to about 10 mM, about 1 mM to about 5 mM, about 2 mM to about 40 mM, about 2 mM to about 35 mM, about 2 mM to about 30 mM, about 2 mM to about 25 mM, about 2 mM to about 25 mM, about 2 mM to about 20 mM, about 2 mM to about 20 mM, about 2 mM to about 5 mM, about 3 mM to about 40 mM, about 3 mM to about 35 mM, about 3 mM to about 30 mM, about 3 mM to about 35 mM, about 3 mM to about 35 mM, about 3 mM to about 30 mM, about 3 mM to about 30 mM, about 3 mM to about 5 mM, about 2 mM to about 8 mM, about 3 mM to about 10 mM, and any one range between these point values.

In some embodiments, the amino acid is at a concentration of about 1 mM, about 2 mM, about 2.5 mM, about 3 mM, about 3.5 mM, about 4 mM, about 4.5 mM, about 5 mM, about 6 mM, about 7 mM, about 8 mM, about 9 mM, about 10 mM, about 20 mM, about 30 mM, about 40 mM, or about 50 mM.

In some embodiments, provided is the pharmaceutical composition according to any one of the above, which comprises an inorganic salt. Illustratively, the inorganic salt includes, but is not limited to, a sodium salt and/or a magnesium salt. In some embodiments, the sodium salt is sodium chloride. In some embodiments, the magnesium salt is magnesium chloride. "Magnesium chloride" may be anhydrous magnesium chloride or a solvate of magnesium chloride formed with a solvent (e.g., magnesium chloride hexahydrate). In some specific embodiments, the "magnesium chloride" described herein refers to magnesium chloride hexahydrate.

In some embodiments, the sodium salt is at a concentration of about 10 mM to about 500 mM, e.g., about 20 mM to about 500 mM, about 30 mM to about 500 mM, about 50 mM to about 500 mM, about 60 mM to about 500 mM, about 70 mM to about 500 mM, about 80 mM to about 500 mM, about 20 mM to about 400 mM, about 30 mM to about 400 mM, about 50 mM to about 400 mM, about 60 mM to about 400 mM, about 70 mM to about 400 mM, about 80 mM to about 400 mM, about 20 mM to about 350 mM, about 30 mM to about 350 mM, about 50 mM to about 350 mM, about 60 mM to about 350 mM, about 70 mM to about 350 mM, about 80 mM to about 350 mM, about 20 mM to about 250 mM, about 30 mM to about 250 mM, about 50 mM to about 250 mM, about 60 mM to about 250 mM, about 70 mM to about 250 mM, about 80 mM to about 250 mM, about 20 mM to about 200 mM, about 30 mM to about 200 mM, about 50 mM to about 200 mM, about 60 mM to about 200 mM, about 70 mM to about 200 mM, about 80 mM to about 200 mM, about 20 mM to about 180 mM, about 30 mM to about 180 mM, about 50 mM to about 180 mM, about 60 mM to about 180 mM, about 70 mM to about 180 mM, about 80 mM to about 180 mM, about 20 mM to about 150 mM, about 30 mM to about 150 mM, about 50 mM to about 150 mM, about 60 mM to about 150 mM, about 70 mM to about 150 mM, about 80 mM to about 150 mM, about 100 mM to about 150 mM, or any range between any of the aforementioned point values.

In some embodiments, the sodium salt is at a concentration of about 10 mM, about 15 mM, about 20 mM, about 25 mM, about 30 mM, about 35 mM, about 40 mM, about 45 mM, about 50 mM, about 55 mM, about 60 mM, about 65 mM, about 70 mM, about 75 mM, about 80 mM, about 85 mM, about 90 mM, about 95 mM, about 100 mM, about 110 mM, about 120 mM, about 125 mM, about 130 mM, about 135 mM, about 140 mM, about 145 mM, about 150 mM, about 155 mM, about 160 mM, about 165 mM, about 170 mM, about 175 mM, about 180 mM, about 185 mM, about 190 mM, about 195 mM, about 200 mM, about 210 mM, about 220 mM, about 250 mM, about 260 mM, about 270 mM, about 280 mM, about 290 mM, about 300 mM, about 350 mM, about 400 mM, about 450 mM, about 500 mM, or any range between any of the aforementioned point values.

In some embodiments, the magnesium salt is at a concentration of about 0.1 mM to about 50 mM, e.g., about 0.5 mM to about 50 mM, about 0.5 mM to about 45 mM, about 0.5 mM to about 40 mM, about 0.5 mM to about 35 mM, about 0.5 mM to about 30 mM, about 0.5 mM to about 25 mM, about 0.5 mM to about 20 mM, about 0.5 mM to about 15 mM, about 0.5 mM to about 10 mM, about 0.1 mM to about 15 mM, about 0.1 mM to about 10 mM, about 0.5 mM to about 8 mM, about 0.5 mM to about 7 mM, about 0.5 mM to about 6 mM, about 0.5 mM to about 5 mM, about 1 mM to about 5 mM, about 1 mM to about 2 mM, about 1 mM to about 3 mM, about 1 mM to about 4 mM, about 2 mM to about 5 mM, about 2 mM to about 4 mM, about 2 mM to about 3 mM, or any range between any of the aforementioned point values.

In some embodiments, the magnesium salt is at a concentration of about 0.1 mM, about 0.2 mM, about 0.3 mM, about 0.4 mM, about 0.5 mM, about 0.6 mM, about 0.7 mM, about 0.8 mM, about 0.9 mM, about 1.0 mM, about 1.1 mM, about 1.2 mM, about 1.3 mM, about 1.4 mM, about 1.5 mM, about 1.6 mM, about 1.7 mM, about 1.8 mM, about 1.9 mM, about 2.0 mM, about 2.1 mM, about 2.2 mM, about 2.3 mM, about 2.4 mM, about 2.5 mM, about 2.6 mM, about 2.7 mM, about 2.8 mM, about 2.9 mM, about 3.0 mM, about 3.2 mM, about 3.5 mM, about 3.7 mM, about 4.0 mM, about 4.5 mM, about 4.8 mM, about 5.0 mM, about 5.5 mM, about 6.0 mM, about 6.5 mM, about 7.0 mM, about 7.5 mM, about 8.0 mM, about 8.5 mM, about 9.0 mM, about 9.5 mM, about 10.0 mM, about 12 mM, about 14 mM, about 15 mM, about 16 mM, about 18 mM, about 19 mM, about 20 mM, about 22 mM, about 25 mM, about 28 mM, about 30 mM, about 35 mM, about 40 mM, about 45 mM, about 50 mM, or any range between any of the aforementioned point values.

In some embodiments, in the pharmaceutical composition according to any one of the above, the recombinant adeno-associated virus (rAAV) particle has a titer of about $1 \times 10^8$ Vg/mL to about $1 \times 10^{14}$ Vg/mL, e.g., about $1 \times 10^9$ Vg/mL to about $1 \times 10^{14}$ Vg/mL, about $1 \times 10^{10}$ Vg/mL to about $1 \times 10^{14}$ Vg/mL, about $1 \times 10^{11}$ Vg/mL to about $1 \times 10^{14}$ Vg/mL, about $1 \times 10^{11}$ Vg/mL to about $9 \times 10^{13}$ Vg/mL; about $1 \times 10^{12}$ Vg/mL to about $9 \times 10^{13}$ Vg/mL, about $1 \times 10^{12}$ Vg/mL to about $8 \times 10^{13}$ Vg/mL, about $1 \times 10^{12}$ Vg/mL to about $7 \times 10^{13}$ Vg/mL, about $1 \times 10^{12}$ Vg/mL to about $6 \times 10^{13}$ Vg/mL, about $1 \times 10^{12}$ Vg/mL to about $5 \times 10^{13}$ Vg/mL, about $1 \times 10^{12}$ Vg/mL to about $2 \times 10^{13}$ Vg/mL, and any one range between these point values.

In certain embodiments, the saccharide, the alcohol, the surfactant, and the amino acid used in the present disclosure can further improve the physical stability, chemical stability, and biological activity of the pharmaceutical composition. The rAAV particles maintain high potency and are suitable for long-term stable storage and use.

In some embodiments, the pharmaceutical composition comprises recombinant adeno-associated virus (rAAV) particles and excipients, wherein the excipient may be any one of the following:

(a) about 1 mM to about 100 mM Hepes,

about 10 mM to about 500 mM sodium salt and about 1 mM to about 50 mM magnesium salt, 0.1% (w/v) to about 10% (w/v) saccharide,
about 0.01% (w/v) to about 10% (w/v) alcohol,
1 mM to about 50 mM amino acid, and
about 0.001% (w/v) to about 0.05% (w/v) surfactant selected from the group consisting of poloxamer 188 and/or polysorbate 80, the pharmaceutical composition having a pH of about 4.0 to about 10.0;

(b) about 1 mM to about 100 mM BTP,

about 10 mM to about 500 mM sodium salt and about 1 mM to about 50 mM magnesium salt, 0.1% (w/v) to about 10% (w/v) saccharide,
about 0.01% (w/v) to about 10% (w/v) alcohol,
1 mM to about 50 mM amino acid, and
about 0.001% (w/v) to about 0.05% (w/v) surfactant selected from the group consisting of poloxamer 188 and/or polysorbate 80, the pharmaceutical composition having a pH of about 4.0 to about 10.0;

(c) about 5 mM to about 50 mM Hepes,

about 10 mM to about 200 mM sodium chloride and about 1 mM to about 10 mM magnesium chloride hexahydrate,
about 1% (w/v) to about 10% (w/v) saccharide selected from the group consisting of sucrose and/or trehalose,
about 0.05% (w/v) to about 5% (w/v) glycerol,
about 1 mM to about 10 mM histidine, and
about 0.001% (w/v) to about 0.05% (w/v) surfactant selected from the group consisting of poloxamer 188 and/or polysorbate 80, the pharmaceutical composition having a pH of about 4.0 to about 10.0, e.g., about 6.0 to about 8.0;

(d) about 5 mM to about 50 mM BTP,

about 10 mM to about 200 mM sodium chloride and about 1 mM to about 10 mM magnesium chloride hexahydrate,
about 1% (w/v) to about 10% (w/v) saccharide selected from the group consisting of sucrose and/or trehalose,
about 0.05% (w/v) to about 5% (w/v) glycerol,
about 1 mM to about 10 mM histidine, and
about 0.001% (w/v) to about 0.05% (w/v) surfactant selected from the group consisting of poloxamer 188 and/or polysorbate 80, the pharmaceutical composition having a pH of about 4.0 to about 10.0, e.g., about 6.0 to about 8.0;

(e) about 10 mM to about 30 mM Hepes,

about 50 mM to about 200 mM sodium chloride and about 1 mM to about 5 mM magnesium chloride hexahydrate,
about 1% (w/v) to about 5% (w/v) sucrose,
about 0.1% (w/v) to about 0.5% (w/v) glycerol,
about 1 mM to about 5 mM histidine, and
about 0.001% (w/v) to about 0.02% (w/v) poloxamer 188, the pharmaceutical composition having a pH of about 6.0 to about 8.0, e.g., about 6.0 to about 7.5;

(f) about 10 mM to about 30 mM BTP,

about 50 mM to about 200 mM sodium chloride and about 1 mM to about 5 mM magnesium chloride hexahydrate,
about 1% (w/v) to about 5% (w/v) sucrose,
about 0.1% (w/v) to about 0.5% (w/v) glycerol,
about 1 mM to about 5 mM histidine, and
about 0.001% (w/v) to about 0.02% (w/v) poloxamer 188, the pharmaceutical composition having a pH of about 6.0 to about 8.0, e.g., about 6.0 to about 7.5;

(g) about 5 mM to about 20 mM Hepes,

about 100 mM to about 150 mM sodium chloride and about 1 mM to about 5 mM magnesium chloride hexahydrate,
about 1% (w/v) to about 5% (w/v) sucrose,
about 0.1% (w/v) to about 0.5% (w/v) glycerol,
about 1 mM to about 5 mM histidine, and
about 0.01% (w/v) to about 0.02% (w/v) poloxamer 188, the pharmaceutical composition having a pH of about 6.0 to about 8.0, e.g., about 6.0 to about 7.5;

(h) about 5 mM to about 20 mM Hepes,

about 80 mM to about 120 mM sodium chloride and about 1 mM to about 5 mM magnesium chloride hexahydrate,
about 1% (w/v) to about 5% (w/v) sucrose,
about 0.1% (w/v) to about 0.5% (w/v) glycerol,
about 1 mM to about 5 mM histidine, and
about 0.01% (w/v) to about 0.02% (w/v) poloxamer 188, the pharmaceutical composition having a pH of about 6.0 to about 8.0, e.g., about 6.0 to about 7.5;

(i) about 5 mM to about 20 mM Hepes,

about 120 mM to about 180 mM sodium chloride and about 1 mM to about 5 mM magnesium chloride hexahydrate,
about 1% (w/v) to about 5% (w/v) sucrose,
about 0.1% (w/v) to about 0.5% (w/v) glycerol,
about 1 mM to about 5 mM histidine, and
about 0.01% (w/v) to about 0.02% (w/v) poloxamer 188, the pharmaceutical composition having a pH of about 6.0 to about 8.0, e.g., about 6.0 to about 7.5;

In some embodiments, in the pharmaceutical composition according to any one of the above, the recombinant adeno-associated virus (rAAV) particle has a titer of about $1 \times 10^8$ Vg/mL to about $1 \times 10^{14}$ Vg/mL, e.g., about $1 \times 10^9$ Vg/mL to about $1 \times 10^{14}$ Vg/mL, about $1 \times 10^{10}$ Vg/mL to about $1 \times 10^{14}$ Vg/mL, about $1 \times 10^{11}$ Vg/mL to about $1 \times 10^{14}$ Vg/mL, about $1 \times 10^{11}$ Vg/mL to about $9 \times 10^{13}$ Vg/mL; about $1 \times 10^{12}$ Vg/mL to about $9 \times 10^{13}$ Vg/mL, about $1 \times 10^{12}$ Vg/mL to about $8 \times 10^{13}$ Vg/mL, about $1 \times 10^{12}$ Vg/mL to about $7 \times 10^{13}$ Vg/mL, about $1 \times 10^{12}$ Vg/mL to about $6 \times 10^{13}$ Vg/mL, about $1 \times 10^{12}$ Vg/mL to about $5 \times 10^{13}$ Vg/mL, about $1 \times 10^{12}$ Vg/mL to about $2 \times 10^{13}$ Vg/mL, and any one range between these point values.
In some embodiments, the pharmaceutical composition comprises any one of the following:

(1) about $1 \times 10^{11}$ Vg/mL to about $9 \times 10^{13}$ Vg/mL said recombinant adeno-associated virus (rAAV) particle,

about 10 mM Hepes,
about 100 mM sodium chloride and about 2 mM magnesium chloride hexahydrate,
about 2.5% (w/v) sucrose,
about 0.25% (w/v) glycerol,
about 5 mM histidine, and
about 0.01% (w/v) poloxamer 188, the pharmaceutical composition having a pH of about 6.5 to about 8.0, e.g., about 7.5;

(2) about $1 \times 10^{11}$ Vg/mL to about $9 \times 10^{13}$ Vg/mL said recombinant adeno-associated virus (rAAV) particle,

about 10 mM BTP,
about 100 mM sodium chloride and about 2 mM magnesium chloride hexahydrate,
about 2.5% (w/v) sucrose,
about 0.25% (w/v) glycerol,
about 5 mM histidine, and
about 0.01% (w/v) poloxamer 188, the pharmaceutical composition having a pH of about 6.5 to about 8.0, e.g., about 7.5;

(3) about $1 \times 10^{11}$ Vg/mL to about $9 \times 10^{13}$ Vg/mL said recombinant adeno-associated virus (rAAV) particle,

about 10 mM Hepes,
about 100 mM sodium chloride and about 2 mM magnesium chloride hexahydrate,
about 2.5% (w/v) sucrose,
about 0.25% (w/v) glycerol,
about 5 mM histidine, and
about 0.01% (w/v) polysorbate 80, the pharmaceutical composition having a pH of about 6.5 to about 8.0, e.g., about 7.5;

(4) about $1 \times 10^{11}$ Vg/mL to about $9 \times 10^{13}$ Vg/mL said recombinant adeno-associated virus (rAAV) particle,

about 10 mM Hepes,
about 150 mM sodium chloride and about 2 mM magnesium chloride hexahydrate,
about 2.5% (w/v) sucrose,
about 0.25% (w/v) glycerol,
about 5 mM histidine, and
about 0.01% (w/v) poloxamer 188, the pharmaceutical composition having a pH of about 5.0 to about 7.0, e.g., about 6.0;

(5) about $1 \times 10^{11}$ Vg/mL to about $9 \times 10^{13}$ Vg/mL said recombinant adeno-associated virus (rAAV) particle,

about 10 mM BTP,
about 150 mM sodium chloride and about 2 mM magnesium chloride hexahydrate,
about 2.5% (w/v) sucrose,
about 0.25% (w/v) glycerol,
about 5 mM histidine, and
about 0.01% (w/v) poloxamer 188, the pharmaceutical composition having a pH of about 5.0 to about 7.0, e.g., about 6.0;

(6) about $1 \times 10^{11}$ Vg/mL to about $9 \times 10^{13}$ Vg/mL said recombinant adeno-associated virus (rAAV) particle,

about 10 mM Hepes,
about 150 mM sodium chloride and about 2 mM magnesium chloride hexahydrate,
about 2.5% (w/v) sucrose,
about 0.25% (w/v) glycerol,
about 5 mM histidine, and
about 0.01% (w/v) polysorbate 80, the pharmaceutical composition having a pH of about 5.0 to about 7.0, e.g.,

about 6.0.

In some embodiments, in any one of the pharmaceutical compositions (1)-(3), the pH is about 6.5 to about 8.0, e.g., about 7.0 to about 8.0, about 7.0 to about 7.5, or about 7.5 to about 8.0. Illustratively, the pH is about 6.5, about 6.6, about 6.7, about 6.8, about 6.9, about 7.0, about 7.1, about 7.2, about 7.3, about 7.4, about 7.5, about 7.6, about 7.7, about 7.8, about 7.9, or about 8.0.

In some embodiments, in any one of the pharmaceutical compositions of (1)-(3), the pH is about 7.5.

In some embodiments, in any one of the pharmaceutical compositions (4)-(6), the pH is about 5.0 to about 7.0, e.g., about 5.5 to about 7.0, about 5.5 to about 6.5, about 5.5 to about 6.0, or about 6.0 to about 6.5. Illustratively, the pH is about 5.0, about 5.1, about 5.2, about 5.3, about 5.4, about 5.5, about 5.6, about 5.7, about 5.8, about 5.9, about 6.0, about 6.1, about 6.2, about 6.3, about 6.4, about 6.5, about 6.6, about 6.7, about 6.8, about 6.9, or about 7.0.

In some embodiments, in any one of the pharmaceutical compositions (4)-(6), the pH is about 6.0.

In some embodiments, the recombinant adeno-associated virus (rAAV) particle comprises a nucleic acid molecule and an AAV capsid.

AAV capsid and AAV particle

The AAV capsid comprises or is derived from any natural or recombinant AAV serotype, including but not limited to: PHP.B, PHP.A, AAV1, AAV2, AAV2G9, AAV3, AAV3a, AAV3b, AAV3-3, AAV4, AAV4-4, AAV5, AAV6, AAV6.1, AAV6.2, AAV6.1.2, AAV7, AAV7.2, AAV8, AAV9, AAV9.11, AAV9.13, AAV9.16, AAV9.24, AAV9.45, AAV9.47, AAV9.61, AAV9.68, AAV9.84, AAV9.9, AAV10, AAV11, AAV12, AAV16.3, AAV24.1, AAV27.3, AAV42.12, AAV42-1b, AAV42-2, AAV42-3a, AAV42-3b, AAV42-4, AAV42-5a, AAV42-5b, AAV42-6b, AAV42-8, AAV42-10, AAV42-11, AAV42-12, AAV42-13, AAV42-15, AAV42-aa, AAV43-1, AAV43-12, AAV43-20, AAV43-21, AAV43-23, AAV43-25, AAV43-5, AAV44.1, AAV44.2, AAV44.5, AAV223.1, AAV223.2, AAV223.4, AAV223.5, AAV223.6, AAV223.7, AAV1-7/rh.48, AAV1-8/rh.49, AAV2-15/rh.62, AAV2-3/rh.61, AAV2-4/rh.50, AAV2-5/rh.51, AAV3.1/hu.6, AAV3.1/hu.9, AAV3-9/rh.52, AAV3-11/rh.53, AAV4-8/r11.64, AAV4-9/rh.54, AAV4-19/rh.55, AAV5-3/rh.57, AAV5-22/rh.58, AAV7.3/hu.7, AAV16.8/hu.10, AAV16.12/hu.11, AAV29.3/bb.1, AAV29.5/bb.2, AAV106.1/hu.37, AAV114.3/hu.40, AAV127.2/hu.41, AAV127.5/hu.42, AAV128.3/hu.44, AAV130.4/hu.48, AAV145.1/hu.53, AAV145.5/hu.54, AAV145.6/hu.55, AAV161.10/hu.60, AAV161.6/hu.61, AAV33.12/hu.17, AAV33.4/hu.15, AAV33.8/hu.16, AAV52/hu.19, AAV52.1/hu.20, AAV58.2/hu.25, AAVA3.3, AAVA3.4, AAVA3.5, AAVA3.7, AAVC1, AAVC2, AAVC5, AAV-DJ, AAV-DJ8, AAVF3, AAVF5, AAVH2, AAVrh.72, AAVhu.8, AAVrh.68, AAVrh.70, AAVpi.1, AAVpi.3, AAVpi.2, AAVrh.60, AAVrh.44, AAVrh.65, AAVrh.55, AAVrh.47, AAVrh.69, AAVrh.45, AAVrh.59, AAVhu.12, AAVH6, AAVLK03, AAVH-1/hu.1, AAVH-5/hu.3, AAVLG-10/rh.40, AAVLG-4/rh.38, AAVLG-9/hu.39, AAVN721-8/rh.43, AAVCh.5, AAVCh.5R1, AAVcy.2, AAVcy.3, AAVcy.4, AAVcy.5, AAVCy.5R1, AAVCy.5R2, AAVCy.5R3, AAVCy.5R4, AAVcy.6, AAVhu.1, AAVhu.2, AAVhu.3, AAVhu.4, AAVhu.5, AAVhu.6, AAVhu.7, AAVhu.9, AAVhu.10, AAVhu.11, AAVhu.13, AAVhu.15, AAVhu.16, AAVhu.17, AAVhu.18, AAVhu.20, AAVhu.21, AAVhu.22, AAVhu.23.2, AAVhu.24, AAVhu.25, AAVhu.27, AAVhu.28, AAVhu.29, AAVhu.29R, AAVhu.31, AAVhu.32, AAVhu.34, AAVhu.35, AAVhu.37, AAVhu.39, AAVhu.40, AAVhu.41, AAVhu.42, AAVhu.43, AAVhu.44, AAVhu.44R1, AAVhu.44R2, AAVhu.44R3, AAVhu.45, AAVhu.46, AAVhu.47, AAVhu.48, AAVhu.48R1, AAVhu.48R2, AAVhu.48R3, AAVhu.49, AAVhu.51, AAVhu.52, AAVhu.54, AAVhu.55, AAVhu.56, AAVhu.57, AAVhu.58, AAVhu.60, AAVhu.61, AAVhu.63, AAVhu.64, AAVhu.66, AAVhu.67, AAVhu.68, AAVhu.14/9, AAVhu.t19, AAVrh.2, AAVrh.2R, AAVrh.8, AAVrh.8R, AAVrh.10, AAVrh.12, AAVrh.13, AAVrh.13R, AAVrh.14, AAVrh.17, AAVrh.18, AAVrh.19, AAVrh.20, AAVrh.21, AAVrh.22, AAVrh.23, AAVrh.24, AAVrh.25, AAVrh.31, AAVrh.32, AAVrh.33, AAVrh.34, AAVrh.35, AAVrh.36, AAVrh.37, AAVrh.37R2, AAVrh.38, AAVrh.39, AAVrh.40, AAVrh.46, AAVrh.48, AAVrh.48.1, AAVrh.48.1.2, AAVrh.48.2, AAVrh.49, AAVrh.51, AAVrh.52, AAVrh.53, AAVrh.54, AAVrh.56, AAVrh.57, AAVrh.58, AAVrh.61, AAVrh.64, AAVrh.64R1, AAVrh.64R2, AAVrh.67, AAVrh.73, AAVrh.74, AAVrh8R, AAVrh8R A586R mutant, AAVrh8R R533A mutant, AAAV, BAAV, goat AAV, bovine AAV, ovine AAV, AAVhE1.1, AAVhEr1.5, AAVhER1.14, AAVhEr1.8, AAVhEr1.16, AAVhEr1.18, AAVhEr1.35, AAVhEr1.7, AAVhEr1.36, AAVhEr2.29, AAVhEr2.4, AAVhEr2.16, AAVhEr2.30, AAVhEr2.31, AAVhEr2.36, AAVhER1.23, AAVhEr3.1, AAV2.5T, AAV-PAEC, AAV-LK01, AAV-LK02, AAV-LK03, AAV-LK04, AAV-LK05, AAV-LK06, AAV-LK07, AAV-LK08, AAV-LK09, AAV-LK10, AAV-LK11, AAV-LK12, AAV-LK13, AAV-LK14, AAV-LK15, AAV-LK16, AAV-LK17, AAV-LK18, AAV-LK19, AAV-PAEC2, AAV-PAEC4, AAV-PAEC6, AAV-PAEC7, AAV-PAEC8, AAV-PAEC11, AAV-PAEC12, AAV-2-pre-miRNA-101, AAV-8h, AAV-8b, AAV-h, AAV-b, AAV SM 10-2, AAVShuffle 100-1, AAV Shuffle 100-3, AAV Shuffle 100-7, AAV Shuffle 10-2, AAVShuffle 10-6, AAV Shuffle 10-8, AAV Shuffle 100-2, AAV SM 10-1, AAV SM 10-8, AAVSM 100-3, AAV SM 100-10, BNP61 AAV, BNP62 AAV, BNP63 AAV, AAVrh.50, AAVrh.43, AAVrh.62, AAVrh.48, AAVhu.19, AAVhu.11, AAVhu.53, AAV4-8/rh.64, AAVLG-9/hu.39, AAV54.5/hu.23, AAV54.2/hu.22, AAV54.7/hu.24, AAV54.1/hu.21, AAV54.4R/hu.27, AAV46.2/hu.28, AAV46.6/hu.29, AAV128.1/hu.43, true type AAV (ttAAV), UPENNAAV 10, Japanese AAV 10 serotype, AAV CBr-7.1, AAV CBr-7.10, AAV CBr-7.2, AAV CBr-7.3, AAVCBr-7.4, AAV CBr-7.5, AAV CBr-7.7, AAV CBr-7.8, AAV CBr-B7.3, AAV CBr-B7.4, AAVCBr-E1, AAV CBr-E2, AAV CBr-E3, AAV CBr-E4, AAV CBr-E5, AAV CBr-e5, AAV CBr-E6, AAVCBr-E7, AAV CBr-E8, AAV CHt-1, AAV CHt-2, AAV CHt-3, AAV CHt-6.1, AAV CHt-6.10, AAVCHt-6.5, AAV CHt-6.6, AAV CHt-6.7, AAV CHt-6.8, AAV CHt-P1, AAV CHt-P2, AAV CHt-P5, AAV CHt-P6, AAV CHt-P8, AAV CHt-P9, AAV CKd-1, AAV CKd-10, AAV CKd-2, AAV CKd-3, AAVCKd-4, AAV CKd-6, AAV CKd-7, AAV CKd-8, AAV CKd-B1, AAV CKd-B2, AAV CKd-B3, AAV CKd-B4, AAV CKd-B5, AAV CKd-B6, AAV CKd-B7, AAV CKd-B8, AAV CKd-H1, AAV CKd-H2, AAV CKd-H3, AAV CKd-H4, AAV CKd-H5, AAV CKd-H6, AAV CKd-N3, AAV CKd-N4, AAV CKd-N9, AAV CLg-F1, AAV CLg-F2, AAV CLg-F3, AAV CLG-F4, AAV CLg-F5, AAV CLg-F6, AAV CLg-F7, AAV CLg-F8, AAV CLv-1, AAV CLv1-1, AAV Clv-1-10, AAV CLv1-2, AAV CLv-12, AAV CLv1-3, AAV CLv-13, AAV CLv1-4, AAV Clv1-7, AAV Clv1-8, AAV Clv1-9, AAV CLv-2, AAV CLv-3, AAV CLv-4, AAV CLv-6, AAV CLv-8, AAV CLv-D1, AAV CLv-D2, AAV CLv-D3, AAV CLv-D4, AAV CLv-D5, AAV CLv-D6, AAV CLv-D7, AAV CLv-D8, AAV CLv-E1, AAV CLv-K1, AAV CLv-K3, AAV CLv-K6, AAV CLv-L4, AAV CLv-L5, AAV CLv-L6, AAV CLv-M1, AAV CLv-M11, AAV CLv-M2, AAV CLv-M5, AAV CLv-M6, AAV CLv-M7, AAV CLv-M8, AAV CLv-M9, AAV CLv-R1, AAV CLv-R2, AAV CLv-R3, AAV CLv-R4, AAV CLv-R5, AAV CLv-R6, AAV CLv-R7, AAV CLv-R8, AAV CLv-R9, AAV CSp-1, AAV CSp-10, AAV CSp-11, AAV CSp-2, AAV CSp-3, AAV CSp-4, AAV CSp-6, AAV CSp-7, AAVCSp-8, AAV CSp-8.10, AAV CSp-8.2, AAV CSp-8.4, AAV CSp-8.5, AAV CSp-8.6, AAV CSp-8.7, AAV CSp-8.8, AAV CSp-8.9, AAV CSp-9, AAVhu.48R3, AAV.VR-355, AAV3B, AAV4, AAV5, AAVF1/HSC1, AAVF11/HSC11, AAVF12/HSC12, AAVF13/HSC13, AAVF14/HSC14, AAVF15/HSC15, AAVF16/HSC16, AAVF17/HSC17, AAVF2/HSC2, AAVF3/HSC3, AAVF4/HSC4, AAVF5/HSC5, AAVF6/HSC6, AAVF7/HSC7, AAVF8/HSC8, AAVF9/HSC9, PHP.B(AAV-PHP.B), PHP.A(AAV.PHP.A), G2B-26, G2B-13, TH1.1-32, TH1.1-35, AAVPHP.B2, AAVPHP.B3, AAVPHP.N/PHP.B-DGT, AAVPHP.B-EST, AAVPHP.B-GGT, AAVPHP.B-ATP, AAVPHP.B-ATT-T, AAVPHP.B-DGT-T, AAVPHP.B-GGT-T, AAVPHP.B-SGS, AAVPHP.B-AQP, AAVPHP.B-QQP, AAVPHP.B-SNP(3), AAVPHP.B-SNP, AAVPHP.B-QGT, AAVPHP.B-NQT, AAVPHP.B-EGS, AAVPHP.B-SGN, AAVPHP.B-EGT, AAVPHP.B-DST, AAVPHP.B-DST, AAVPHP.B-STP, AAVPHP.B-PQP, AAVPHP.B-SQP, AAVPHP.B-QLP, AAVPHP.B-TMP, AAVPHP.B-TTP, AAVPHP.S/G2A12, AAVG2A15/G2A3, AAVG2B4, AAVG2B5, AAVDJ8, and variants thereof.

In some embodiments, the AAV capsid may be modified or mutated, for example, containing one or more mutations of Y252F, Y272F, Y444F, Y500F, Y700F, Y704F, Y730F, Y275F, Y281F, Y508F, Y576F, Y612G, Y673F, and Y720F; for example, containing one or more mutations of F129L, D418E, K531E, L584F, V598A, and H642N; for example, containing substitution of at least one of Tyr residues at positions 252, 272, 444, 500, 700, 704, and 730 of AAV2 with, for example, a Phe residue; for example, containing an N587A, E548A, or N708A mutation in the AAV2 capsid; for example, containing a T446F mutation in the AAV9 capsid; for example, containing a V708K mutation in the AAV capsid; for example, the AAV capsid is generated via an AAV9 capsid library having mutations in amino acids at positions 390-627 (VP1 numbering).

The AAV capsids in WO2022247917A, WO2018232055 (e.g. Table 1 thereof), WO2005033321, WO2015168666, WO2015121501, WO2015038958, WO2016065001, WO2016130589, WO2016049230, WO2016134375, WO2017100671, WO2017083722, WO2017015102, WO2017058892, WO2017066764, US9546112, US7198951, US9233131, US6156303, US9624274, US9475845, US8734809, US20130224836, US20140359799, US20150315612, US20150376240, US20150159173, US20150376607, US20150238550, US20160369298, US20160361439, US20170145405, and NPulicherla et al., (MolecularTherapy19(6):1070-1078(2011)) are incorporated in the present disclosure by reference in their entirety. In some specific embodiments, the AAV capsids used in the present disclosure are the AAV2 and AAV9 capsids in the prior art described above.

In some embodiments, the AAV capsid is engineered; for example, the AAV capsid is a hybrid AAV capsid from two or more parental serotypes. For example, the AAV capsid may be AAV2G9, which comprises sequences from AAV2 and AAV9. The sequence of AAV2G9 in US20160017005 is incorporated herein by reference in its entirety.

In some embodiments, the AAV capsid is an AAV2 or AAV9 capsid, e.g., comprising the amino acid sequence set forth in SEQ ID NO: 41 or 42.

In some embodiments, the AAV particle may comprise all or part of the vector genome of a nucleotide sequence or a variant of any naturally occurring and/or recombinant AAV capsid, in addition to the encoded heterologous payload.

In some embodiments, the AAV particle may be replication-deficient (e.g., it lacks sequences encoding functional Rep and Cap proteins in the vector genome). In some specific embodiments, the replication-deficient AAV particle may lack most or all of the parental coding sequences, and carry essentially only one or two AAV ITR sequences and a polynucleotide of interest for delivery to a cell, tissue, organ, or organism.

In some embodiments, the AAV particle may be a recombinant AAV (rAAV) particle.

In some embodiments, the AAV particle may be selected from the group consisting of a single-stranded AAV particle (e.g., ssAAV) and a self-complementary AAV particle (e.g., scAAV). By skipping the synthesis of a second strand, scAAV achieves rapid expression in cells.

In some embodiments, the pharmaceutical composition comprises recombinant adeno-associated virus (rAAV) particles and excipients; the recombinant adeno-associated virus (rAAV) particle comprises a nucleic acid molecule and an AAV9 capsid, and the excipient comprises any one of (a)-(f) and (h).

In some embodiments, the pharmaceutical composition comprises recombinant adeno-associated virus (rAAV) particles and excipients; the recombinant adeno-associated virus (rAAV) particle comprises a nucleic acid molecule and an AAV9 capsid, and the excipient comprises any one of (1)-(3).

In some embodiments, the pharmaceutical composition comprises recombinant adeno-associated virus (rAAV) particles and excipients; the recombinant adeno-associated virus (rAAV) particle comprises a nucleic acid molecule and an AAV2 capsid, and the excipient comprises any one of (a)-(f) and (i).

In some embodiments, the pharmaceutical composition comprises recombinant adeno-associated virus (rAAV) particles and excipients; the recombinant adeno-associated virus (rAAV) particle comprises a nucleic acid molecule and an AAV2 capsid, and the excipient comprises any one of (4)-(6).

In some embodiments, the nucleic acid molecule or the AAV particle is derived from any of the nucleic acid molecules or AAV particles in WO2023093905A, and the aforementioned patent is incorporated herein by reference. In some embodiments, the nucleic acid molecule or the AAV particle is derived from any of the VEGF-binding molecules or AAV particles in WO2023155918A, and the aforementioned patent is incorporated herein by reference.

Nucleic acid molecule I

The present disclosure provides a nucleic acid molecule comprising a polynucleotide encoding AADC protein (the encoded amino acid sequence of the AADC protein is set forth, for example, in SEQ ID NO: 1), wherein the polynucleotide has at least 60%, at least 70%, at least 75%, at least 80%, at least 90%, or at least 95% identity (e.g., has at least 80.83%, at least 81.21%, at least 79.18%, at least 79.63%, or higher identity) to SEQ ID NO: 3, or has at least 95% identity to any one of SEQ ID NOs: 4-7. In some embodiments, the polynucleotide encoding the AADC protein comprises the polynucleotide sequence set forth in any one of SEQ ID NOs: 3-7.

The present disclosure provides a nucleic acid molecule comprising a polynucleotide encoding GDNF protein (the encoded amino acid sequence of the AADC protein is set forth, for example, in SEQ ID NO: 2), wherein the polynucleotide has at least 60%, at least 70%, at least 80%, at least 90%, or at least 95% identity (e.g., has at least 75.13%, at least 73.99%, at least 77.52%, at least 77.83% or higher identity) to SEQ ID NO: 8, or has at least 95% identity to any one of SEQ ID NOs: 9-12. In some embodiments, the polynucleotide encoding the GDNF protein comprises the polynucleotide sequence set forth in any one of SEQ ID NOs: 8-12.

In some embodiments of the present disclosure, the AADC protein and the GDNF protein encompass analogs thereof.

The present disclosure provides a nucleic acid molecule comprising a first polynucleotide and a second polynucleotide, wherein the first polynucleotide comprises a polynucleotide encoding AADC protein (the encoded amino acid sequence of the AADC protein is set forth, for example, in SEQ ID NO: 1), and the second polynucleotide comprises a polynucleotide encoding GDNF protein (the encoded amino acid sequence of the AADC protein is set forth, for example, in SEQ ID NO: 2). In some embodiments, the first polynucleotide and the second polynucleotide are operably linked, and the first polynucleotide and the second polynucleotide may be located in the same polynucleotide, the same plasmid, or the same expression cassette. Alternatively, in some other embodiments, the first polynucleotide and the second polynucleotide are present independently of each other in two polynucleotides that are present independently of each other, in two different plasmids that are present independently of each other, or in two different expression cassettes.

In some embodiments, the amino acid sequence of the AADC protein comprises or is set forth in SEQ ID NO: 1, and the amino acid sequence of the GDNF protein comprises or is set forth in SEQ ID NO: 2.

In some embodiments, the first polynucleotide sequence encoding the AADC protein and/or the second polynucleotide sequence encoding the GDNF protein are/is codon-optimized. For example, the codon-optimized polynucleotide sequence encoding the AADC protein has at least 60%, at least 70%, at least 75%, at least 80%, at least 90%, or at least 95% identity to SEQ ID NO: 3; the codon-optimized polynucleotide sequence encoding the GDNF protein has at least 60%, at least 70%, at least 80%, at least 90%, or at least 95% identity to SEQ ID NO: 8.

In some embodiments, the polynucleotide sequence encoding the AADC protein comprises a sequence having at least 95% identity to any one of SEQ ID NOs: 4-7, and/or the polynucleotide sequence encoding the GDNF protein comprises a sequence having at least 95% identity to any one of SEQ ID NOs: 9-12. In some specific embodiments, the polynucleotide sequence encoding AADC comprises or is the polynucleotide sequence set forth in any one of SEQ ID NOs: 3-7; the polynucleotide sequence encoding GDNF comprises or is the polynucleotide sequence set forth in any one of SEQ ID NOs: 8-12.

In the present disclosure, "at least 95% identity" is meant to encompass at least 95%, at least 96%, at least 97%, at least 98%, at least 99%, or higher identity.

The codon-optimized polynucleotide expressing the AADC protein (e.g., set forth in any one of SEQ ID NOs: 4-7) results in an increased expression level of the AADC protein as compared to a wild-type or non-codon-optimized polynucleotide (e.g., SEQ ID NO: 3). The codon-optimized polynucleotide expressing the GDNF protein (e.g., set forth in any one of SEQ ID NOs: 9-12) results in an increased expression level of the GDNF protein as compared to a wild-type or non-codon-optimized polynucleotide (e.g., SEQ ID NO: 8). The increase is an increase in the protein expression level of at least 10%, at least 20%, at least 30%, at least 50%, at least 75%, at least 100%, or at least several times (including 2, 3, 4, 5, 6, 7, 8, 9, 10 or more) compared to the wild-type or non-codon-optimized polynucleotide.

In some embodiments, the nucleic acid molecule provided by the present disclosure further comprises the same expression control sequence operably linked to the first polynucleotide and the second polynucleotide, or two identical or different expression control sequences operably linked to the first polynucleotide and the second polynucleotide, respectively, wherein the expression control sequence comprises (c) a promoter and/or (d) an enhancer.

In some embodiments, the nucleic acid molecule provided by the present disclosure further comprises any one or any combination of the following:

(a) a 5' inverted terminal repeat (5' ITR);
(b) a 3' inverted terminal repeat (3' ITR);
(e) an intron;
(f) a post-transcriptional regulatory element;
(g) a polyadenylation signal (polyA); and
(h) a multiple cloning site (MCS).

In some specific embodiments, any combination of (a)-(h) is capable of fulfilling the function of allowing expression of a target gene (AADC and/or GDNF), e.g., expression in the brain (such as substantia nigra or striatum) of a subject.

In some specific embodiments, the polynucleotide of any one of (a)-(h), or any combination thereof, is operably linked to the polynucleotide encoding the AADC protein and/or the polynucleotide encoding the GDNF protein.

In some specific embodiments, the 5' ITR and/or 3' ITR are/is derived from AAV1, AAV2, AAV3, AAV4, AAV5, AAV6, AAV7, AAV8, AAV9, AAV9.47, AAV9(hu14), AAV10, AAV11, AAV12, AAVrh8, AAVrh10, AAV-DJ, or AAV-DJ8, for example, from AAV2 or AAV9.

In some specific embodiments, the promoter is selected from the group consisting of CMV, CAG, CBh, EFS, EF1 (e.g., EF-1α), PGK, SV40, Ubi, RSV, or any combination thereof.

In some specific embodiments, the enhancer is selected from the group consisting of Ubi, CMV and RSV enhancers, or any combination thereof.

In some specific embodiments, the intron is selected from the group consisting of MVM, SV40, β Globin, EF1 (e.g., EF-1α) and hybrid introns, or any combination thereof.

In some specific embodiments, the polyA is selected from the group consisting of PA75 polyA, SV40 polyA, hGH polyA, BGH polyA, rbGlob polyA, or any combination thereof.

In some specific embodiments, the post-transcriptional regulatory element is selected from the group consisting of WPRE, HPRE, or a combination thereof.

In some embodiments, the nucleic acid molecule comprises a polynucleotide encoding AADC protein, a CMV enhancer (e.g., CMV enhancer 1), and a CBA promoter, and optionally, the first polynucleotide further comprises a hybrid intron and a polyA, the polyA being selected from the group consisting of SV40 polyA and hGH polyA; and/or the nucleic acid molecule comprises a polynucleotide encoding GDNF protein, a CMV enhancer (e.g., CMV enhancer 2), and a CMV promoter, and optionally, the second polynucleotide further comprises a β globin intron and a polyA, the polyA being selected from the group consisting of PA75 polyA and SV40 polyA; wherein the CMV enhancer 1 and the CMV enhancer 2 are both CMV enhancers, and they may be identical or different.

In some embodiments, AADC and GDNF in the polynucleotide may use different expression frameworks independently or share one expression framework by linkage via a linker (e.g., P2A sequence).

The first polynucleotide and the second polynucleotide are operably linked, so that the AADC protein and the GDNF protein are expressed at the same target administration position of a subject, which can prevent the therapeutic effect of the combination of the two proteins from being affected due to inconsistent administration positions of the two proteins, and can reduce side effects caused by leakage of one of the proteins to other positions. Referring to the examples of the present disclosure, the first polynucleotide and the second polynucleotide are operably linked and then administered to a diseased model mouse, and the results show that a significant therapeutic effect can be exerted.

In some embodiments, the first polynucleotide and the second polynucleotide are linked by a third polynucleotide; for example, the third polynucleotide encodes an amino acid sequence with linker function. The linking order may be, from the 5' end to the 3' end, first polynucleotide-third polynucleotide-second polynucleotide, or from the 5' end to the 3' end, second polynucleotide-third polynucleotide-first polynucleotide. In some specific embodiments, the amino acid sequence encoded by the third polynucleotide is set forth in any one of SEQ ID NOs: 43-46; for example, the sequence of the third polynucleotide is set forth in any one of SEQ ID NOs: 47-50 or has at least 80%, at least 90%, at least 95%, or at least 98% identity thereto.

In some embodiments, the polynucleotide encoding the AADC protein and the polynucleotide encoding the GDNF protein are regulated and expressed by the same regulatory element, or regulated and expressed by two different regulatory elements, respectively. The regulatory element includes, but is not limited to, 5' **ITR, 3' ITR,** a promoter, an enhancer, an intron, a polyA, a post-transcriptional regulatory element, and the like.

In some embodiments, the nucleic acid molecule comprises, from the 5' end to the 3' end:

a) a CMV enhancer, a CBA promoter, a first polynucleotide encoding AADC protein, a second polynucleotide encoding GDNF protein, and a polyA;

in some optional embodiments, a hybrid intron is further comprised between the CBA promoter and the first polynucleotide encoding the AADC protein;

in some optional embodiments, a WPRE sequence is further comprised between the second polynucleotide encoding the GDNF protein and the polyA;

in some specific embodiments, the nucleic acid molecule comprises: a CMV enhancer, a CBA promoter, a hybrid intron, a first polynucleotide encoding AADC protein, a second polynucleotide encoding GDNF protein, a WPRE sequence, and a polyA;

b) a CMV enhancer, a CBA promoter, a second polynucleotide encoding GDNF protein, a first polynucleotide encoding AADC protein, and a polyA;

in some optional embodiments, a hybrid intron is further comprised between the CBA promoter and the second polynucleotide encoding the GDNF protein;

in some optional embodiments, a WPRE sequence is further comprised between the first polynucleotide encoding the AADC protein and the polyA;

in some specific embodiments, the nucleic acid molecule comprises: a CMV enhancer, a CBA promoter, a hybrid intron, a second polynucleotide encoding GDNF protein, a first polynucleotide encoding AADC protein, a WPRE sequence, and a polyA;

c) a CMV enhancer, a CBA promoter, a first polynucleotide encoding AADC protein, a polyA, a CMV enhancer, a CMV promoter, a second polynucleotide encoding GDNF protein, and a polyA;

in some optional embodiments, a hybrid intron is further comprised between the CBA promoter and the first polynucleotide encoding the AADC protein;

in some optional embodiments, a β globin intron is further comprised between the CMV promoter and the second polynucleotide encoding the GDNF protein;

in some optional embodiments, a WPRE sequence is further comprised between the second polynucleotide encoding the GDNF protein and the polyA;

in some specific embodiments, the nucleic acid molecule comprises:

CMV enhancer 1, a CBA promoter, a hybrid intron, a first polynucleotide encoding AADC protein, a polyA, CMV enhancer 2, a CMV promoter, a second polynucleotide encoding GDNF protein, and a polyA; or

CMV enhancer 1, a CBA promoter, a hybrid intron, a first polynucleotide encoding AADC protein, a polyA, CMV enhancer 2, a CMV promoter, a second polynucleotide encoding GDNF protein, a WPRE sequence, and a polyA; or

CMV enhancer 1, a CBA promoter, a hybrid intron, a first polynucleotide encoding AADC protein, a polyA, CMV enhancer 2, a CMV promoter, a β globin intron, a second polynucleotide encoding GDNF protein, and a polyA.

d) a CMV enhancer, a CMV promoter, a second polynucleotide encoding GDNF protein, a polyA, a CMV enhancer, a CBA promoter, a first polynucleotide encoding AADC protein, and a polyA;

in some optional embodiments, a β globin intron or an MVM intron is further comprised between the CMV promoter and the second polynucleotide encoding the GDNF protein;

in some optional embodiments, a hybrid intron is further comprised between the CBA promoter and the first polynucleotide encoding the AADC protein;

in some optional embodiments, a WPRE sequence is further comprised between the first polynucleotide encoding the AADC protein and the polyA;

in some specific embodiments, the nucleic acid molecule comprises:

CMV enhancer 2, a CMV promoter, a β globin intron, a second polynucleotide encoding GDNF protein, a polyA, CMV enhancer 1, a CBA promoter, a hybrid intron, a first polynucleotide encoding AADC protein, and a polyA;

CMV enhancer 2, a CMV promoter, a second polynucleotide encoding GDNF protein, a polyA, CMV enhancer 1, a CBA promoter, a hybrid intron, a first polynucleotide encoding AADC protein, a WPRE sequence, and a polyA; or

CMV enhancer 2, a CMV promoter, an MVM intron, a second polynucleotide encoding GDNF protein, a polyA, CMV enhancer 1, a CBA promoter, a hybrid intron, a first polynucleotide encoding AADC protein, a WPRE sequence, and a polyA;

e) a CMV enhancer, a CBA promoter, a first polynucleotide encoding AADC protein, a third polynucleotide, a second polynucleotide encoding GDNF protein, and a polyA;

in some optional embodiments, a hybrid intron is further comprised between the CBA promoter and the first polynucleotide encoding the AADC protein;
in some optional embodiments, a WPRE sequence is further comprised between the second polynucleotide encoding the GDNF protein and the polyA;
in some specific embodiments, the nucleic acid molecule comprises: a CMV enhancer, a CBA promoter, a hybrid intron, a first polynucleotide encoding AADC protein, a third polynucleotide, a second polynucleotide encoding GDNF protein, a WPRE sequence, and a polyA;

f) a CMV enhancer, a CBA promoter, a second polynucleotide encoding GDNF protein, a third polynucleotide, a first polynucleotide encoding AADC protein, and a polyA;

in some optional embodiments, a hybrid intron is further comprised between the CBA promoter and the second polynucleotide encoding the GDNF protein;
in some optional embodiments, a WPRE sequence is further comprised between the first polynucleotide encoding the AADC protein and the polyA;
in some specific embodiments, the nucleic acid molecule comprises: a CMV enhancer, a CBA promoter, a hybrid intron, a second polynucleotide encoding GDNF protein, a third polynucleotide, a first polynucleotide encoding AADC protein, a WPRE sequence, and a polyA;

g) a CMV enhancer, a CBA promoter, a first polynucleotide or a second polynucleotide, and a polyA;

in some optional embodiments, a hybrid intron is further comprised between the CBA promoter and the first polynucleotide, or a hybrid intron is further comprised between the CBA and the second polynucleotide;
in some optional embodiments, a WPRE sequence is further comprised between the first polynucleotide and the polyA, or a WPRE sequence is further comprised between the second polynucleotide and the polyA;
in some specific embodiments, the nucleic acid molecule comprises: a CMV enhancer, a CBA promoter, a hybrid intron, a first polynucleotide, and a polyA;
a CMV enhancer, a CBA promoter, a hybrid intron, a second polynucleotide, and a polyA;
a CMV enhancer, a CBA promoter, a hybrid intron, a first polynucleotide, a WPRE sequence, and a polyA; or
a CMV enhancer, a CBA promoter, a hybrid intron, a second polynucleotide, a WPRE sequence, and a polyA.

In some embodiments, the polyA is selected from the group consisting of hGH polyA, PA75 polyA, and SV40 polyA.

In some embodiments, provided is a polynucleotide, and the nucleic acid molecule comprises, from the 5' end to the 3' end:

(1) CMV enhancer-CBA promoter-hybrid intron-first polynucleotide encoding AADC-third polynucleotide-second polynucleotide encoding GDNF-WPRE-SV40 polyA;
(2) CMV enhancer-CBA promoter-hybrid intron-second polynucleotide encoding GDNF-third polynucleotide-first polynucleotide encoding AADC-WPRE-SV40 polyA;
(3) CMV enhancer-CBA promoter-hybrid intron-first polynucleotide encoding AADC-SV40 polyA-CMV enhancer-CMV promoter-second polynucleotide encoding GDNF-PA75 polyA;
(4) CMV enhancer-CBA promoter-hybrid intron-first polynucleotide encoding AADC-SV40 polyA-CMV enhancer-CMV promoter-second polynucleotide encoding GDNF-WPRE-PA75 polyA;
(5) CMV enhancer-CBA promoter-hybrid intron-first polynucleotide encoding AADC-SV40 polyA-CMV enhancer-CMV promoter-β globin intron-second polynucleotide encoding GDNF-PA75 polyA;
(6) CMV enhancer-CMV promoter-β globin intron-second polynucleotide encoding GDNF-PA75 polyA-CMV enhancer-CBA promoter-hybrid intron-first polynucleotide encoding AADC-SV40 polyA;
(7) CMV enhancer-CMV promoter-second polynucleotide encoding GDNF-PA75 polyA-CMV enhancer-CBA promoter-hybrid intron-first polynucleotide encoding AADC-WPRE-SV40 polyA; or
(8) CMV enhancer-CMV promoter-MVM intron-second polynucleotide encoding GDNF-PA75 polyA-CMV enhancer-CBA promoter-hybrid intron-first polynucleotide encoding AADC-WPRE-SV40 polyA.

In some embodiments, provided is a polynucleotide comprising, in the direction from 5' to 3', 1)-13) as follows or any combination thereof:

1) 5' ITR, CMV enhancer 1, a CBA promoter, a hybrid intron, a polynucleotide encoding AADC or a polynucleotide encoding GDNF, hGH polyA, and 3' ITR;

2) 5' ITR, CMV enhancer 1, a CBA promoter, a hybrid intron, a polynucleotide encoding AADC or a polynucleotide encoding GDNF, SV40 polyA, and 3' ITR;

3) 5' ITR, CMV enhancer 1, a CBA promoter, a hybrid intron, a polynucleotide encoding AADC or a polynucleotide encoding GDNF, WPRE, SV40 polyA, and 3' ITR;

4) 5' ITR, CMV enhancer 1, a CBA promoter, a hybrid intron, a polynucleotide encoding AADC, P2A, a polynucleotide encoding GDNF, WPRE, SV40 polyA, and 3' ITR;

5) 5' ITR, CMV enhancer 1, a CBA promoter, a hybrid intron, a polynucleotide encoding GDNF, P2A, a polynucleotide encoding AADC, WPRE, SV40 polyA, and 3' ITR;

6) 5' ITR, MCS, CMV enhancer 1, a CBA promoter, a hybrid intron, a polynucleotide encoding AADC, SV40 polyA, CMV enhancer 2, a CMV promoter, a polynucleotide encoding GDNF, PA75 polyA, and 3' ITR;

7) 5' ITR, MCS, CMV enhancer 1, a CBA promoter, a hybrid intron, a polynucleotide encoding AADC, SV40 polyA, CMV enhancer 2, a CMV promoter, a polynucleotide encoding GDNF, WPRE, PA75 polyA, and 3' ITR;

8) 5' ITR, MCS, CMV enhancer 1, a CBA promoter, a hybrid intron, a polynucleotide encoding AADC, SV40 polyA, CMV enhancer 2, a CMV promoter, a β globin intron, a polynucleotide encoding GDNF, PA75 polyA, and 3' ITR;

9) 5' ITR, CMV enhancer 2, a CMV promoter, a β globin intron, a polynucleotide encoding GDNF, PA75 polyA, CMV enhancer 1, a CBA promoter, a hybrid intron, a polynucleotide encoding AADC, SV40 polyA, and 3' ITR;

10) 5' ITR, CMV enhancer 2, a CMV promoter, a polynucleotide encoding GDNF, PA75 polyA, CMV enhancer 1, a CBA promoter, a hybrid intron, a polynucleotide encoding AADC, WPRE, SV40 polyA, and 3' ITR;

11) 5' ITR, CMV enhancer 2, a CMV promoter, an MVM intron, a polynucleotide encoding GDNF, PA75 polyA, CMV enhancer 1, a CBA promoter, a hybrid intron, a polynucleotide encoding AADC, WPRE, SV40 polyA, and 3' ITR;

12) 5' ITR, CMV enhancer 1, a CBA promoter, a hybrid intron, a polynucleotide encoding AADC, P2A, a polynucleotide encoding GDNF, WPRE, SV40 polyA, and 3' ITR; and

13) 5' ITR, CMV enhancer 2, a CMV promoter, a β globin intron, a polynucleotide encoding GDNF, PA75 polyA, CMV enhancer 1, a CBA promoter, a hybrid intron, a polynucleotide encoding AADC, SV40 polyA, and 3' ITR.

In some specific embodiments,

the CMV enhancer 1 comprises or is the sequence set forth in SEQ ID NO: 27;
the CMV enhancer 2 comprises or is the sequence set forth in SEQ ID NO: 37;
the CBA promoter comprises or is the sequence set forth in SEQ ID NO: 28;
the CMV promoter comprises or is the sequence set forth in SEQ ID NO: 35;
the β globin intron comprises or is the sequence set forth in SEQ ID NO: 38;
the hybrid intron comprises or is the sequence set forth in SEQ ID NO: 29;
the MVM intron comprises or is the sequence set forth in SEQ ID NO: 39;
the PA75 polyA comprises or is the sequence set forth in SEQ ID NO: 36;
the SV40 polyA comprises or is the sequence set forth in SEQ ID NO: 32;
the hGH polyA comprises or is the sequence set forth in SEQ ID NO: 30;
the HPRE comprises or is the sequence set forth in SEQ ID NO: 33;
the WPRE comprises or is the sequence set forth in SEQ ID NO: 34;
the 5' ITR comprises or is the sequence set forth in SEQ ID NO: 26;
the 3' ITR comprises or is the sequence set forth in SEQ ID NO: 31;
the MCS comprises or is the sequence set forth in SEQ ID NO: 40; and/or
the P2A comprises or is the sequence set forth in SEQ ID NO: 33.

In some embodiments, provided is a nucleic acid molecule comprising a polynucleotide, wherein the polynucleotide has at least 95% identity to the sequence set forth in any one of SEQ ID NOs: 13-25 and 140-141. In some embodiments, the polynucleotide comprises the sequence set forth in any one of SEQ ID NOs: 13-25 and 140-141.

In some embodiments, the AADC protein (e.g., AADC protein set forth in SEQ ID NO: 1) comprises a variant (e.g., the variant has at least 60%, at least 70%, at least 80%, at least 90%, at least 95% or higher identity to SEQ ID NO: 1, NP_000781.2, NP_001076440.2, NP_001229815.2, NP_001229816.2, NP_001229817.2, NP_001229818.2, or NP_001229819.2) or a fragment thereof, the variant or the fragment having the same or similar biological activity or functions as the AADC protein; the GDNF protein (e.g., GDNF protein set forth in SEQ ID NO: 2) comprises a variant (e.g., the variant has at least 60%, at least 70%, at least 80%, at least 90%, at least 95% or higher identity to SEQ ID NO: 2, NP_001177397.1, NP_001177398.1, NP_001265027.1, or NP_954701.1) or a fragment thereof, the variant or the fragment having the same or similar biological activity or functions as the GDNF protein.

In some embodiments, the polynucleotide comprises a precursor molecule that is processed inside a cell. The AADC and/or GDNF polynucleotides or processed forms thereof may be encoded in a plasmid, a vector, a genome, or other polynucleotide expression vectors for delivery to a cell.

In some embodiments, the vector comprises an intact replicon, such that, for example, upon transfection, infection, or transformation of a cell, the vector can be replicated in the cell. In some embodiments, the vector is or is derived from a retrovirus, adenovirus, herpesvirus, baculovirus, papillomavirus, or modified or engineered viruses thereof. In some embodiments, methods for delivering the vector include, but are not limited to, direct delivery of naked DNA or delivery via cationic or anionic liposomes, via complexation with cationic polymers, via forming complexes with proteins or polypeptides.

In some embodiments, the polynucleotide is designed as a component of an AAV vector genome (or viral genome) and packaged in rAAV particles that are processed in a cell to produce AADC and/or GDNF proteins, and the AADC and/or GDNF proteins may be wild-type proteins or variants thereof.

In some embodiments, the polynucleotide may be a payload of the rAAV particles. The AAV capsid and the AAV particle are as set forth in any one of the above.

In some embodiments, the AAV capsid is an AAV2 or AAV9 capsid, e.g., comprising the amino acid sequence set forth in SEQ ID NO: 41 or 42.

In some embodiments, the AAV particle may comprise all or part of the vector genome of a nucleotide sequence or a variant of any naturally occurring and/or recombinant AAV capsid, in addition to the encoded heterologous payload.

In some embodiments, the AAV particle may be replication-deficient (e.g., it lacks sequences encoding functional Rep and Cap proteins in the vector genome). In some specific embodiments, the replication-deficient AAV particle may lack most or all of the parental coding sequences, and carry essentially only one or two AAV ITR sequences and a polynucleotide of interest for delivery to a cell, tissue, organ, or organism.

In some embodiments, the AAV particle may be a recombinant AAV (rAAV) particle.

In some embodiments, the AAV particle may be selected from the group consisting of a single-stranded AAV particle (e.g., ssAAV) and a self-complementary AAV particle (e.g., scAAV). By skipping the synthesis of a second strand, scAAV achieves rapid expression in cells.

In some embodiments, the AAV particle wraps a polynucleotide having at least 95% identity to the sequence set forth in any one of SEQ ID NOs: 13-25 and 140-141.

In some embodiments, the AAV particle wraps a polynucleotide comprising the sequence set forth in any one of SEQ ID NOs: 13-25 and 140-141.

Regulatory element

The vector genome (or viral genome) of the polynucleotide or AAV particle according to any one of the foregoing of the present disclosure comprises at least one regulatory element, such that a target gene (e.g., AADC and GDNF) in a payload can be replicated, transcribed, and translated.

In some embodiments, the regulatory element includes, but is not limited to, sequences for transcription initiation and/or termination, promoter and/or enhancer sequences, efficient RNA processing signals (e.g., splicing and polyadenylation signals), sequences that stabilize cytoplasmic mRNA, sequences that enhance translation efficiency (e.g., Kozak consensus sequences), sequences that enhance protein stability, and/or sequences that enhance protein processing and/or secretion. Exemplary regulatory elements include, but are not limited to, promoters, enhancers, introns, endogenous miRNAs, post-transcriptional regulatory elements (PREs), polyadenylation (PolyA) signal sequences, and upstream enhancers (USEs).

In some embodiments, the regulatory elements described below (e.g., promoters) drive expression of the payload in a target tissue, e.g., the brain (e.g., striatum or substantia nigra) of a subject, for a period of time, for example, for 1 day, 2 days, 3 days, 4 days, 5 days, 6 days, 7 days, 1 week, 2 weeks, 3 weeks, 4 weeks, 1 month, 2 months, 3 months, 4 months, 5 months, 6 months, 7 months, 8 months, 9 months, 10 months, 11 months, 12 months, 1 year, 2 years, 3 years, 4 years, 5 years, 6 years, 7 years, 8 years, 9 years, 10 years, 20 years, 30 years, 40 years, 50 years, 60 years or more, as another example, for 1-2 weeks, 1-3 weeks, 1-4 weeks, 1-2 months, 1-4 months, 1-6 months, 2-6 months, 3-6 months, 3-9 months, 4-8 months, 6-12 months, 1-2 years, 1-5 years, 2-5 years, 3-6 years, 3-8 years, 4-8 years, 5-10 years, 10-20 years, 10-30 years, 20-40 years, 20-50 years, or the lifetime of the subject.

Inverted terminal repeat (ITR)

The vector genome (or viral genome) of the polynucleotide or AAV particle according to any one of the foregoing of the present disclosure comprises at least one ITR, e.g., has two ITRs flanking a payload at the 5' end and the 3' end, respectively.

In some embodiments, the ITRs have origin of replication function.

In some embodiments, the ITRs comprise sequence regions that may be complementary and symmetrically arranged.

In some embodiments, the ITRs may consist of naturally occurring polynucleotide sequences or recombinantly derived polynucleotide sequences.

In some embodiments, the ITRs may be derived from the same or different serotype as the capsid. In some embodiments, the 5' ITR and the 3' ITR may be derived from the same serotype or may be derived from different serotypes. For example, both 5' ITR and 3' ITR are from AAV2, or both are from AAV9. For another example, the 5' ITR comprises the sequence set forth in SEQ ID NO: 26, and/or the 3' ITR comprises the nucleotide sequence set forth in SEQ ID NO: 31.

In some embodiments, the length of each ITR may be about 100 to about 150 nucleotides, such as 100-105 nucleotides, 106-110 nucleotides, 111-115 nucleotides, 116-120 nucleotides, 121-125 nucleotides, 126-130 nucleotides, 131-135 nucleotides, 136-140 nucleotides, 141-145 nucleotides, or 146-150 nucleotides. In one embodiment, the length of the ITR is 140-142 nucleotides, such as 141 nucleotides. Non-limiting examples of the length of the ITR are 102 nucleotides, 140 nucleotides, 141 nucleotides, 142 nucleotides, 145 nucleotides, and nucleotides having at least 95% identity thereto.

Promoter

The vector genome (or viral genome) of the polynucleotide or AAV particle according to any one of the foregoing of the present disclosure comprises at least one promoter, including but not limited to, species-specific promoters, inducible promoters, tissue-specific promoters, or cell cycle-specific promoters.

In some embodiments, the promoter drives expression of a protein or polypeptide (e.g., AADC and/or GDNF) encoded in a payload of the vector genome of the AAV particle.

In some embodiments, the promoter is specific to or has tropism for a target tissue, such as a promoter capable of expressing a payload in a neural tissue.

In some embodiments, the promoter may be a viral promoter, a plant promoter, a mammalian promoter, or a human promoter.

In some embodiments, the promoter includes, but is not limited to, CMV, CBA (including derivatives CAG, CBh, etc.), EF-1$\alpha$, PGK, UBC, RSV, EFS, EF1, GUSB (hGBp), UCOE (the promoter of HNRPA2B1-CBX3), NSE, Synapsin, MeCP2, MeP418, MeP426, VMD2, MRHO, TRE, Ac5, Polyhedrin, CaMKIIa, Gall, TEF1, GDS, ADHI, Ubi, GFAP, or PKG promoters, and may be selected from the group consisting of neurofilament light (NFL) promoters, neurofilament heavy (NFH) promoters, SCN8A promoters, frataxin (FXN) promoters (or known as FRDA promoters), H1 promoters, RNA pol III promoters (e.g., U6 or H1), and small nuclear RNA (ULB or ULA) promoters. In some embodiments, the promoter is a liver or skeletal muscle promoter; the liver promoter is, for example, human $\alpha$-1-antitrypsin (hAAT) and thyroxine-binding globulin (TBG), and the skeletal muscle promoter is, for example, desmin, MCK, or synthetic C5-12.

In some embodiments, the AAV vector genome of the present disclosure comprises two promoters, for example, CMV and CBA promoters, the sequences of which are set forth in SEQ ID NOs: 35 and 28, respectively; for another example, an EF1$\alpha$ promoter and a CMV promoter, or a combination of any two promoters of the foregoing promoters.

In some embodiments, the promoter is a tissue-specific expression element that can limit expression to certain cell types, including but not limited to, a muscle-specific promoter, a B cell promoter, a monocyte promoter, a leukocyte promoter, a macrophage promoter, a pancreatic acinar cell promoter, an endothelial cell promoter, a lung tissue promoter, an astrocyte promoter, or a nervous system promoter, which can be used to limit expression to neurons, astrocytes, or oligodendrocytes.

In some embodiments, the promoter is a neuronal tissue-specific expression element, including but not limited to, neuron-specific enolase (NSE), platelet-derived growth factor (PDGF), platelet-derived growth factor B chain (PDGF-$\beta$), synapsin (Syn), methyl CpG binding protein 2 (MeCP2), Ca$^{2+}$/calmodulin-dependent protein kinase II (CaMKII), metabotropic glutamate receptor 2 (mGluR2), neurofilament light (NFL) or heavy (NFH), $\beta$-globin minigene n$\beta$2, preproenkephalin (PPE), enkephalin (Enk), and excitatory amino acid transporter 2 (EAAT2) promoters. Non-limiting examples of tissue-specific expression elements for astrocytes include glial fibrillary acidic protein (GFAP) and EAAT2 promoters. A non-limiting example of tissue-specific expression elements for oligodendrocytes includes a myelin basic protein (MBP) promoter.

In some embodiments, the promoter is a truncation or a variant of the aforementioned promoters and has a length of less than 1 kb, such as 200-300, 200-400, 300-400, 200-500, 200-600 or more.

In some embodiments, the promoter may be a combination of two or more components of the same or different initial or parental promoters, such as CMV and CBA.

<u>Enhancer</u>

The vector genome (or viral genome) of the polynucleotide or AAV particle according to any one of the foregoing of the present disclosure comprises at least one enhancer, including but not limited to, species-specific enhancers, inducible enhancers, tissue-specific enhancers, or cell cycle-specific enhancers.

In some embodiments, the enhancer is specific to or has tropism for a target tissue, such as an enhancer capable of regulating expression of a payload in a neural tissue.

In some embodiments, the enhancer may be or be derived from a viral enhancer, a plant enhancer, a mammalian enhancer, or a human enhancer.

In some embodiments, the enhancer may be located upstream or downstream of a promoter and operably linked to the promoter. Expression of a target gene (e.g., AADC and/or GDNF) is enhanced by at least 20%, at least 50%, at least 80%, at least 100%, or at least several times (including 1, 2, 3, 4, 5, 6, 7, 8, 9, 10 times or more) when the enhancer is present as compared to when the enhancer is not present.

In some embodiments, the enhancer includes, but is not limited to, EF-1$\alpha$, Ubc, humanb-actin, CAG, TRE, Ac5, Polyhedrin, CaMKIIa, GalI, TEF1, GDS, ADH1, Ubi, and $\alpha$-1-antitrypsin (hAAT), or variants or fragments of the above enhancers. In some embodiments, the enhancer is selected from the group consisting of IRBP, RSV, or CMV enhancers, or variants or fragments thereof.

In some embodiments, the enhancer comprises or is an enhancer set forth in SEQ ID NO: 27 or 37 or a variant or a fragment thereof.

<u>Intron</u>

An intron or a portion thereof may be comprised in the vector genome (or viral genome) of the polynucleotide or AAV particle according to any one of the foregoing of the present disclosure.

In some embodiments, the intron includes, but is not limited to, MVM (67-97 bp), F.IX truncated intron 1 (about 300 bp), $\beta$-globin SD/immunoglobulin heavy chain splice acceptor (about 250 bp), adenovirus splice donor/immunoglobulin splice acceptor (about 500 bp), SV40 late splice donor/splice acceptor (19S/16S) (180 bp) and hybrid adenovirus splice donor/IgG splice acceptor (about 230 bp), hemoglobin introns, hybrid introns, $\beta$-globin introns, or variants or fragments of the above introns.

In some embodiments, the length of the intron is 100-800 nucleotides, such as about 100 nucleotides, about 200 nucleotides, about 300 nucleotides, about 400 nucleotides, about 500 nucleotides, and about 600 nucleotides, and further such as 200-250 nucleotides, 200-300 nucleotides, 100-200 nucleotides, 300-500 nucleotides, 500-600 nucleotides, 400-600 nucleotides, and 100-200 nucleotides.

In some embodiments, the intron in the technical solutions of the present disclosure is selected from the group consisting of a hybrid intron and a $\beta$-globin intron, the sequences of which are set forth in SEQ ID NOs: 29 and 38, respectively.

<u>Untranslated</u> region (UTR)

An untranslated region (UTR) may be comprised in the vector genome (or viral genome) of the polynucleotide or AAV particle according to any one of the foregoing of the present disclosure, wherein the UTR is selected from the group consisting of a 5' UTR and/or a 3' UTR. The UTR is used to regulate (e.g., increase or decrease) polynucleotide stability and protein production. Typically, the 5' UTR starts at a transcription start site and terminates at a start codon, and the 3' UTR starts immediately after a stop codon and terminates at a transcription termination signal. The UTR may be a wild type, a variant thereof, or an artificial UTR.

In some embodiments, the 5' UTR comprises a Kozak sequence. In other embodiments, the 5' UTR does not comprise a Kozak sequence.

In some embodiments, the 3' UTR is rich in AU. In some embodiments, the 3' UTR is selected from the group consisting of: class I AREs, for example, but not limited to, c-Myc and MyoD, containing several dispersed copies of AUUUA motifs within the U-rich region; class II AREs, for example, but not limited to, GM-CSF and TNF-a, possessing two or more overlapping UUAUUUA(U/A)(U/A) nonamers; and class III ARES, for example, but not limited to, c-Jun and myogenin.

In some embodiments, the 3' UTR may comprise an oligomeric (dT) sequence for templated addition of a polyadenylation sequence (PolyA).

<u>Polyadenylation sequence (PolyA)</u>

A sequence encoding a PolyA may be comprised in the vector genome (or viral genome) of the polynucleotide or AAV particle according to any one of the foregoing of the present disclosure, wherein the PolyA may be a wild type, a variant thereof, or a modified PolyA, for protein translation.

In some embodiments, the sequence encoding the PolyA is located between the 3' end of a coding sequence of a payload and the 5' end of a 3' ITR.

In some embodiments, the length of the sequence encoding the PolyA is 0-500 nucleotides, such as about 75 nucleotides, about 100 nucleotides, about 110 nucleotides, about 120 nucleotides, about 150 nucleotides, about 160 nucleotides, about 200 nucleotides, or about 300 nucleotides, further such as 50-100, 50-150, 50-160, 50-200, 60-100, 60-150, 60-160, 60-200, 70-100, 70-150, 70-160, 70-200, 80-100, 80-150, 80-160, 80-200, 90-100, 90-150, 90-160, or 90-200.

In some embodiments, the encoding PolyA may encode continuous PolyA or discontinuous PolyA. When the discontinuous PolyA is encoded, the coding sequence may be interrupted or separated by other nucleotides. For example, the polyA has at least two 60-adenylate fragments that are separated by a sequence comprising 10-90 nucleotides.

In some embodiments, the sequence encoding the PolyA or polyA in the technical solutions of the present disclosure is selected from the group consisting of β-globin polyA, SV40 polyA, bGH polyA, PA75 polyA, MeCP2 polyA, RDH1 polyA, BGH polyA, SPA49 polyA, sNRP-TK65 polyA, sNRP polyA, TK65 polyA, or variants or fragments of the above PolyA. In some embodiments, the polyA set forth in SEQ ID NOs: 32 and 36, or variants or fragments thereof in the technical solutions of the present disclosure.

polyA or coding sequences thereof in WO2016005324, WO2016005004, WO2016091391, WO2019036513, and WO2020074642 are incorporated herein by reference in their entirety, all of which may be used in the technical solutions of the present disclosure.

Stuffer sequence

A stuffer sequence may be comprised in the vector genome (or viral genome) of the polynucleotide or AAV particle according to any one of the foregoing of the present disclosure, such that the length of the vector genome is the optimal size for packaging, for example, such that the length of the vector genome is about 2.3 kb, about 4.6 kb, about 4.7 kb, or about 5.1 kb.

In some embodiments, the vector genome is a single-stranded or double-stranded genome, and the packaged AAV particle is ssAAV or scAAV.

In some embodiments, one vector genome may comprise one or more (e.g., 1, 2, 3, 4, 5, 6, 7, 8, 9, or 10) stuffer sequences.

In some embodiments, the stuffer sequence may be located between or within a plurality of regulatory elements, e.g., between two introns, at 3' of a 5' ITR sequence, at 5' of a 5' ITR sequence, at 5' of a 3' ITR sequence, at 3' of a 3' ITR sequence, and before or after a promoter, an intron, an enhancer, a polyA, a multiple cloning site (MCS) region, an exon, or other regions.

Production or preparation method for rAAV particles

The present disclosure provides methods for producing, preparing, and/or modifying rAAV particles. The production, preparation, and/or modification methods for rAAV particles in WO200028004, WO200123001, WO2004112727, WO 2005005610, WO2005072364, WO2013123503, WO2015191508, and US20130195801 are incorporated herein by reference in their entirety. The rAAV particles may have enhanced delivery efficiency, may be efficiently packaged, and may successfully infect a target cell (e.g., a mammalian or human cell) with high frequency and minimum toxicity.

In some embodiments, provided is a production method for rAAV particles, which comprises packaging any of the polynucleotides or vector genomes (or viral genomes) or vectors of the present disclosure into an AAV capsid. In some specific embodiments, the method comprises the following steps: 1) co-transfecting competent bacterial cells with a baculovirus vector and a viral construct vector and/or an AAV payload construct vector, 2) isolating the resulting viral construct expression vector and AAV payload construct expression vector, and separately transfecting viral replication cells, 3) isolating and purifying the resulting payload and viral construct particles comprising the viral construct expression vector or AAV payload construct expression vector, 4) co-infecting the viral replication cells with both the AAV payload and the viral construct particles comprising the viral construct expression vector or AAV payload construct expression vector, and 5) harvesting and purifying AAV particles comprising a viral genome.

In some embodiments, provided is a production method for rAAV particles, which comprises the following steps:

1) co-transfecting a mammalian cell (e.g., an HEK293 cell) with any of the polynucleotides or vector genomes (or viral genomes) or vectors of the present disclosure, and a construct expressing Rep and Cap genes and a helper construct (to achieve helper functions) simultaneously; and

2) harvesting and purifying rAAV particles comprising a viral genome.

In some embodiments, the viral genome of the aforementioned rAAV particles optionally encodes a selection marker. The selection marker may include a cell surface marker, such as any protein expressed on the cell surface, including but not limited to, a receptor CD marker, a lectin, an integrin, or truncated forms thereof.

In some embodiments, provided is an AAV production system for producing the rAAV particles of the present disclosure, wherein the production system comprises:

1) a polynucleotide sequence encoding an AAV capsid;
2) any polynucleotide or vector genome (or viral genome) or vector of the present disclosure; and
3) sufficient AAV rep function and helper function to allow packaging of the polynucleotide or vector genome (or viral genome) or vector in (b) into the AAV capsid.

In the foregoing embodiments, the AAV capsid is selected from the group consisting of any of the AAV capsids of the present disclosure described above, such as AAV1, AAV2, AAV3, AAV4, AAV5, AAV6, AAV7, AAV8, AAV9, AAV9.47, AAV9(hu14), AAV10, AAV11, AAV12, AAVrh8, AAVrh10, AAV-DJ, or AAV-DJ8.

In some specific embodiments, the sufficient AAV rep function and helper function are provided by a packaging cell, wherein the packaging cell may comprise three plasmids pHelper, pRC9, and pGOI.

In some embodiments, the Rep genes encode the non-structural proteins that regulate functions such as the replication of the AAV genome, and may be selected from the group consisting of Rep78, Rep68, Rep52, and Rep40. Rep78 and Rep68 are generally transcribed from the p5 promoter, while Rep52 and Rep40 are generally transcribed from the p19 promoter. The Cap genes encode the structural proteins VP1, VP2, and/or VP3 that assemble to form the viral capsid. The Cap genes are generally transcribed from the p40 promoter.

In some embodiments, provided are rAAV particles produced by the AAV production system described above.

Nucleic acid molecule II

The present disclosure provides a VEGF-binding molecule comprising immunoglobulin-like domain 2 of VEGFR2 (R2D2) and/or immunoglobulin-like domain 3 of VEGFR2 (R2D3); or, the VEGF-binding molecule consists of R2D2 and/or R2D3.

In some embodiments, provided is a VEGF-binding molecule comprising: a first VEGF-binding domain and a second VEGF-binding domain, wherein the first VEGF-binding domain comprises immunoglobulin-like domain 2 of VEGFR2 (R2D2) and immunoglobulin-like domain 3 of VEGFR2 (R2D3) or consists of R2D2-R2D3; the second VEGF-binding domain comprises or consists of a VEGF Trap or an anti-VEGF antibody or an antigen-binding fragment thereof.

In some embodiments, the VEGF Trap in the second VEGF-binding domain is immunoglobulin-like domain 2 of VEGFR1 (R1D2) and/or immunoglobulin-like domain 3 of VEGFR2 (R2D3).

In some embodiments, the anti-VEGF antibody in the second VEGF-binding domain is bevacizumab or ranibizumab.

In some embodiments, provided is a VEGF-binding molecule comprising:

a first VEGF-binding domain and a second VEGF-binding domain, wherein the first VEGF-binding domain comprises or consists of R2D2-R2D3;
the second VEGF-binding domain comprises or consists of R1D2-R2D3, or comprises the heavy chain variable region (VH) and the light chain variable region (VL) of an anti-VEGF antibody; illustratively, the anti-VEGF antibody is bevacizumab or ranibizumab.

By way of non-limiting illustration, R2D2-R2D3 represents a VEGF-binding domain formed by linking R2D2 and R2D3, wherein R2D2 and R2D3 may be linked directly or indirectly (e.g., by a linker).

In some embodiments, provided is a VEGF-binding molecule comprising one or more selected from the group consisting of R1D2, R2D2, and R2D3. For example, the VEGF-binding molecule comprises or consists of 1 R1D2, 1 R2D2, and 2 R2D3s. For example, the VEGF-binding molecule comprises or consists of 1 R2D2 and 1 R2D3.

In some embodiments, the VEGF-binding molecule comprises an amino acid mutation that improves stability.

In some embodiments, the VEGF-binding molecule comprises an amino acid mutation that improves affinity for a ligand.

In some embodiments, the VEGF-binding molecule comprises an amino acid mutation that improves affinity for the ligand(s) VEGF-A and/or VEGF-C.

In some embodiments, the VEGF-binding molecule comprises both an amino acid mutation that improves stability and an amino acid mutation that improves affinity for the ligand(s) VEGF-A and/or VEGF-C.

In a first aspect, in some embodiments, the R2D2 and/or R2D3 of the VEGF-binding molecule comprise(s) an amino acid mutation at any one or more positions selected from the group consisting of the following: position 162, position 221, position 222, position 274, position 276, position 277, position 280, position 284, position 288, position 289, and position 313.

The position of the amino acid mutation described above is relative to a natural sequence position of the amino acid sequence set forth in SEQ ID NO: 51.

In some embodiments, the R2D2 and/or R2D3 comprise(s) an amino acid mutation at any one or more sets of positions selected from the group consisting of the following:

- position 162;
- position 162 and position 221;
- position 162 and position 222;
- position 162 and position 274;
- position 162 and position 276;
- position 162 and position 277;
- position 162 and position 289;
- position 162 and position 313;
- position 221 and position 222;
- position 277 and position 289;
- position 162, position 221, and position 222;
- position 162, position 277, and position 289; and
- position 162, position 221, position 222, and position 274.

In some embodiments, the R2D2 and/or R2D3 comprise(s) an amino acid mutation at any one set of positions selected from the group consisting of the following:

- position 162;
- position 162, position 221, position 222, and position 274.

In some embodiments, the R2D2 and/or R2D3 comprise(s) any one or more sets of amino acid mutations selected from the group consisting of the following:

- 162A, 162V, 162S, 162I, or 162L;
- 221S or 221N;
- 222K;
- 274F or 274I;
- 276Q;
- 277R;
- 280E;
- 284H;
- 288Y;
- 289Y; and
- 313R.

In some embodiments, the R2D2 and/or R2D3 comprise(s) any one or more sets of amino acid mutations selected from the group consisting of the following:

- C162A, C162V, C162S, C162I, or C162L;
- Y221S or Y221N;
- R222K;
- N274F or N274I;
- D276Q;
- L277R;
- Q280E;
- E284H;
- F288Y;
- L289Y; and
- L313R.

In some embodiments, the R2D2 and/or R2D3 comprise(s) any one or more sets of amino acid mutations selected from the group consisting of the following:

- 162V or 162I;
- 162V/274F; 162V/274I; 162I/274F; or 162I/274I;
- 221S/222K;
- 277R/289Y;
- 162V/221S/222K;
- 162V/276Q;
- 162V/289Y;
- 162V/277R/289Y;
- 162V/313R; and
- 162V/221S/222K/274F.

In some embodiments, the R2D2 and/or R2D3 comprise(s) any one or more sets of amino acid mutations selected from the group consisting of the following:

- C162V or C162I;
- C162V/N274F;C162V/N274I;C162I/N274F; or C162I/N274I;
- Y221S/R222K;
- L277R/L289Y;
- C162V/Y221S/R222K;
- C162V/D276Q;
- C162V/L289Y;
- C162V/L277R/L289Y;
- C162V/L313R;
- C162V/Y221S/R222K/N274F.

In some embodiments, the R2D2 and/or R2D3 comprise(s) any one or more sets of amino acid mutations selected from the group consisting of the following:

- 162V; and
- 162V/221S/222K/274F.

In some embodiments, the R2D2 and/or R2D3 comprise(s) any one or more sets of amino acid mutations selected from the group consisting of the following:

- C162V; and
- C162V/Y221S/R222K1N274F.

In some embodiments, VEGF-binding molecules comprising the aforementioned amino acid mutations have improved stability as compared to a wild type having the same molecular format.

In a second aspect, in some embodiments, the R2D2 and/or R2D3 of the VEGF-binding molecule comprise(s) an amino acid mutation at one or more positions selected from the group consisting of the following: position 135, position 197, position 199, position 213, position 215, position 219, position 257, position 275, position 277, position 278, position 283, position 289, position 313, and position 314.

The position of the amino acid mutation described above is relative to a natural sequence position of the amino acid sequence set forth in SEQ ID NO: 51.

In some embodiments, the R2D2 and/or R2D3 comprise(s) an amino acid mutation at position 283.

In some embodiments, the R2D2 and/or R2D3 comprise(s) any one or more sets of amino acid mutations selected from the group consisting of the following:

- 135Y;
- 197Y;
- 199Q or 199T;
- 213N;
- 215F or 215H;
- 219I;

- 257Q or 257T;
- 275L or 275E;
- 277R;
- 278D, 278S, or 278E;
- 283T;
- 289Y;
- 313Y; and
- 314S or 314Q.

In some embodiments, the R2D2 and/or R2D3 comprise(s) any one or more sets of amino acid mutations selected from the group consisting of the following:

- V135Y;
- M197Y;
- F199Q or F199T;
- M213N;
- I215F or I215H;
- V219I;
- D257Q or D257T;
- R275L or R275E;
- L277R;
- K278D, K278S, or K278E;
- S283T;
- L289Y;
- L313Y; and
- M314S or M314Q.

In some embodiments, the R2D2 and/or R2D3 comprise(s) 283T.
In some embodiments, the R2D2 and/or R2D3 comprise(s) the amino acid mutation S283T. In some embodiments, VEGF-binding molecules comprising the aforementioned amino acid mutations have improved ligand binding activity as compared to a wild type having the same molecular format.
In a third aspect, in some embodiments, the R2D2 and/or R2D3 of the VEGF-binding molecule comprise(s) an amino acid mutation at any one or more positions selected from the group consisting of the following: position 162, position 221, position 222, position 274, position 276, position 277, position 280, position 284, position 288, position 289, and position 313; and
comprise(s) an amino acid mutation at any one or more positions selected from the group consisting of the following: position 135, position 197, position 199, position 213, position 215, position 219, position 257, position 275, position 277, position 278, position 283, position 289, position 313, and position 314.
In some embodiments, the R2D2 and/or R2D3 of the VEGF-binding molecule comprise(s) an amino acid mutation at any one set of positions selected from the group consisting of the following:

- position 162 and position 275;
- position 162 and position 283;
- position 162 and position 313;
- position 162, position 274, and position 283;
- position 162, position 221, position 222, and position 283; and
- position 162, position 221, position 222, position 274, and position 283.

In some embodiments, the R2D2 and/or R2D3 comprise(s) an amino acid mutation at any one set of positions selected from the group consisting of the following:

- position 162 and position 283; and
- position 162, position 221, position 222, position 274, and position 283.

In some embodiments, the R2D2 and/or R2D3 comprise(s):

(i) any one or more sets of amino acid mutations selected from the group consisting of the following:

- 162A, 162V, 162S, 162I, or 162L;
- 221S;
- 222K;
- 274F or 274I;
- 276Q;
- 277R;
- 280E;
- 284H;
- 288Y;
- 289Y; and
- 313R; and

(ii) any one or more sets of amino acid mutations selected from the group consisting of the following:

- 135Y;
- 197Y;
- 199Q or 199T;
- 213N;
- 215F or 215H;
- 219I;
- 257Q or 257T;
- 275L or 275E;
- 277R;
- 278D, 278S, or 278E;
- 283T;
- 289Y;
- 313Y; and
- 314S or 314Q.

In some embodiments, the R2D2 and/or R2D3 comprise(s):

(i) any one or more sets of amino acid mutations selected from the group consisting of the following:

- C162A, C162V, C162S, C1621, or C162L;
- Y221S;
- R222K;
- N274F or N274I;
- D276Q;
- L277R;
- Q280E;
- E284H;
- F288Y;
- L289Y; and
- L313R; and

(ii) any one or more sets of amino acid mutations selected from the group consisting of the following:

- V135Y;
- M197Y;
- F199Q or F199T;
- M213N;
- I215F or I215H;
- V219I;
- D257Q or D257T;
- R275L or R275E;
- L277R;
- K278D, K278S, or K278E;
- S283T;

- L289Y;
- L313Y; and
- M314S or M314Q.

In some embodiments, the R2D2 and/or R2D3 comprise(s) any one or more sets of amino acid mutations selected from the group consisting of the following:

- 162V/275L;
- 162V/283T;
- 162V/313Y;
- 162V/274F/283T;
- 162V/221S/222K/283T; and
- 162V/221S/222K/274F/283T.

In some embodiments, the R2D2 and/or R2D3 comprise(s) any one or more sets of amino acid mutations selected from the group consisting of the following:

- C162V/R275L;
- C162V/S283T;
- C162V/L313Y;
- C162V/N274F/S283T;
- C162V/Y221S/R222K/S283T; and
- C162V/Y221S/R222K/N274F/S283T.

In some embodiments, the R2D2 and/or R2D3 comprise(s) any one or more sets of amino acid mutations selected from the group consisting of the following:

- 162V /283 T; and
- 162V/221S/222K/274F/283T.

In some embodiments, the R2D2 and/or R2D3 comprise(s) any one or more sets of amino acid mutations selected from the group consisting of the following:

- C162V/S283T;
- C162V/Y221S/R222K/N274F/S283T.

In the context of the present disclosure, "/" indicates that the amino acid mutations are present simultaneously in the same VEGF-binding molecule.

In the context of the present disclosure, ordinal numbers such as "first" and "second" are not to be understood as defining the number, level, or order of technical features; they are used only for distinguishing different technical features, steps, and elements.

In the embodiments described above, the mutations in the R2D2 and/or R2D3 of the VEGF-binding molecule may comprise the mutations described above or consist only of the mutations described above.

In some embodiments, the R2D2 and/or R2D3 of VEGFR2 are/is wild-type one(s) or functional variant(s) having at least 80%, at least 85%, at least 90%, at least 95%, at least 96%, at least 97%, at least 98%, or at least 99% sequence identity to the wild type one(s).

In some embodiments, immunoglobulin-like domain 2 and immunoglobulin-like domain 3 of wild-type VEGFR2 have the same amino acid sequences as the R2D2 and R2D3 in the sequence set forth in SEQ ID NO: 51.

In some embodiments, immunoglobulin-like domain 2 of VEGFR2 (R2D2) and immunoglobulin-like domain 3 of VEGFR2 (R2D3) comprise one or more amino acid differences (e.g., 1, 2, 3, 4, 5, 6, 7, 8, 9, 10, or more amino acid differences) as compared to wild-type ones; the amino acid differences include amino acid substitutions, insertions, or deletions.

In some embodiments, the amino acid mutations are relative to immunoglobulin-like domain 2 and immunoglobulin-like domain 3 of wild-type VEGFR2, wherein the amino acid sequence of wild-type VEGFR2 is set forth in SEQ ID NO: 51.

In some embodiments, immunoglobulin-like domain 2 and immunoglobulin-like domain 3 of wild-type VEGFR2 have the amino acid sequence set forth at positions 123-327 of SEQ ID NO: 51.

In some embodiments, R2D2-R2D3 has the amino acid sequence set forth at positions 123-327 of SEQ ID NO:

51.

In some embodiments, immunoglobulin-like domain 2 of wild-type VEGFR2 (R2D2) has the amino acid sequence set forth at positions 123-225 (single-underlined) of SEQ ID NO: 51, and immunoglobulin-like domain 3 of wild-type VEGFR2 (R2D3) has the amino acid sequence set forth at positions 226-327 (double-underlined) of SEQ ID NO: 51.

In the context of the present disclosure, the numbering of amino acid positions related to R2D2 and R2D3 uses the natural numbering of the amino acid sequence set forth in SEQ ID NO: 51; that is, the numbering starts at the methionine (M) at position 1 of SEQ ID NO: 51. The amino acid sequence of wild-type VEGFR2:

MQSKVLLAVALWLCVETRAASVGLPSVSLDLPRLSIQKDILTIKANTTLQITCRGQRDL
DWLWPNNQSGSEQRVEVTECSDGLFCKTLTIPKVIGNDTGAYKCFYRETDLASVIYVY
VQDYRSPFIASVSDQHGVVYITENKNKTVVIPCLGSISNLNVSLCARYPEKRFVPDGNR
ISWDSKKGFTIPSYMISYAGMVFCEAKINDESYQSIMYIVVVVGYRIYDVVLSPSHGIE

LSVGEKLVLNCTARTELNVGIDFNWEYPSSKHQHKKLVNRDLKTQSGSEMKKFLSTL
TIDGVTRSDQGLYTCAASSGLMTKKNSTFVRVHEKPFVAFGSGMESLVEATVGERVRI
PAKYLGYPPPEIKWYKNGIPLESNHTIKAGHVLTIMEVSERDTGNYTVILTNPISKEKQ
SHVVSLVVYVPPQIGEKSLISPVDSYQYGTTQTLTCTVYAIPPPHHIHWYWQLEEECA
NEPSQAVSVTNPYPCEEWRSVEDFQGGNKIEVNKNQFALIEGKNKTVSTLVIQAANVS
ALYKCEAVNKVGRGERVISFHVTRGPEITLQPDMQPTEQESVSLWCTADRSTFENLTW
YKLGPQPLPIHVGELPTPVCKNLDTLWKLNATMFSNSTNDILIMELKNASLQDQGDY
VCLAQDRKTKKRHCVVRQLTVLERVAPTITGNLENQTTSIGESIEVSCTASGNPPPQIM
WFKDNETLVEDSGIVLKDGNRNLTIRRVRKEDEGLYTCQACSVLGCAKVEAFFIIEGA
QEKTNLEIIILVGTAVIAMFFWLLLVIILRTVKRANGGELKTGYLSIVMDPDELPLDEHC
ERLPYDASKWEFPRDRLKLGKPLGRGAFGQVIEADAFGIDKTATCRTVAVKMLKEGA
THSEHRALMSELKILIHIGHHLNVVNLLGACTKPGGPLMVIVEFCKFGNLSTYLRSKR
NEFVPYKTKGARFRQGKDYVGAIPVDLKRRLDSITSSQSSASSGFVEEKSLSDVEEEE
APEDLYKDFLTLEHLICYSFQVAKGMEFLASRKCIHRDLAARNILLSEKNVVKICDFGL
ARDIYKDPDYVRKGDARLPLKWMAPETIFDRVYTIQSDVWSFGVLLWEIFSLGASPY
PGVKIDEEFCRRLKEGTRMRAPDYTTPEMYQTMLDCWHGEPSQRPTFSELVEHLGNL
LQANAQQDGKDYIVLPISETLSMEEDSGLSLPTSPVSCMEEEEVCDPKFHYDNTAGIS
QYLQNSKRKSRPVSVKTFEDIPLEEPEVKVIPDDNQTDSGMVLASEELKTLEDRTKLS
PSFGGMVPSKSRESVASEGSNQTSGYQSGYHSDDTDTTVYSSEEAELLKLIEIGVQTGS
TAQILQPDSGTTLSSPPV

SEQ ID NO: 51.

In some embodiments, the first VEGF-binding domain consists of R2D2-R2D3.

In some embodiments, the first VEGF-binding domain has the capacity to bind to VEGF-C. In some embodiments, the first VEGF-binding domain has the capacity to bind to VEGF-C and the capacity to bind to VEGF-A.

In some embodiments, the VEGF-binding molecule has increased stability and/or ligand binding activity.

In some embodiments, the second VEGF-binding domain has the capacity to bind to VEGF-A.

In some embodiments, the second VEGF-binding domain has the capacity to bind to VEGF-A and does not have the capacity to bind to VEGF-C or has a weak capacity to bind to VEGF-C. In some embodiments, the second VEGF-binding domain comprises or consists of a VEGF Trap.

In some embodiments, the VEGF Trap comprises immunoglobulin-like domain 2 of VEGFR1 (R1D2) and immunoglobulin-like domain 3 of VEGFR2 (R2D3).

In some embodiments, immunoglobulin-like domain 2 of VEGFR1 (R1D2) is a wild-type one or a functional variant of R1D2 having at least 80%, at least 85%, at least 90%, at least 95%, at least 96%, at least 97%, at least 98%, or at least 99% sequence identity to the wild type one.

In some embodiments, wild-type R1D2 has the same amino acid sequence as the immunoglobulin-like domain 2 in the wild-type VEGFR1 set forth in SEQ ID NO: 52.

In some embodiments, wild-type R1D2 has the amino acid sequence set forth at positions 129-231 of SEQ ID NO: 52.

In some embodiments, the functional variant of R1D2 comprises one or more amino acid differences (e.g., 1, 2, 3, 4, 5, 6, 7, 8, 9, 10, or more amino acid differences) as compared to a wild-type one; the amino acid differences include amino acid substitutions, insertions, or deletions.

In the context of the present disclosure, the numbering of amino acid positions related to R1D2 uses the natural numbering of the amino acid sequence set forth in SEQ ID NO: 52; that is, the numbering starts at the methionine (M) at position 1 of SEQ ID NO: 52.

The amino acid sequence of wild-type VEGFR1:

MVSYWDTGVLLCALLSCLLLTGSSSGSKLKDPELSLKGTQHIMQAGQTLHLQCRGEA

AHKWSLPEMVSKESERLSITKSACGRNGKQFCSTLTLNTAQANHTGFYSCKYLAVPTS

KKKETESAIYIFISDTGRPFVEMYSEIPEIIHMTEGRELVIPCRVTSPNITVTLKKFPLDTL

IPDGKRIIWDSRKGFIISNATYKEIGLLTCEATVNGHLYKTNYLTHRQTNTIIDVQISTPR

PVKLLRGHTLVLNCTATTPLNTRVQMTWSYPDEKNKRASVRRRIDQSNSHANIFYSVL

TIDKMQNKDKGLYTCRVRSGPSFKSVNTSVHIYDKAFITVKHRKQQVLETVAGKRSY

RLSMKVKAFPSPEVVWLKDGLPATEKSARYLTRGYSLIIKDVTEEDAGNYTILLSIKQS

NVFKNLTATLIVNVKPQIYEKAVSSFPDPALYPLGSRQILTCTAYGIPQPTIKWFWHPCN

HNHSEARCDFCSNNEESFILDADSNMGNRIESITQRMAIIEGKNKMASTLVVADSRISG

IYICIASNKVGTVGRNISFYITDVPNGFHVNLEKMPTEGEDLKLSCTVNKFLYRDVTWI

LLRTVNNRTMHYSISKQKMAITKEHSITLNLTIMNVSLQDSGTYACRARNVYTGEEIL

QKKEITIRDQEAPYLLRNLSDHTVAISSSTTLDCHANGVPEPQITWFKNNHKIQQEPGII

LGPGSSTLFIERVTEEDEGVYHCKATNQKGSVESSAYLTVQGTSDKSNLELITLTCTCV

AATLFWLLLTLFIRKMKRSSSEIKTDYLSIIMDPDEVPLDEQCERLPYDASKWEFARER

LKLGKSLGRGAFGKVVQASAFGIKKSPTCRTVAVKMLKEGATASEYKALMTELKILT

HIGHHLNVVNLLGACTKQGGPLMVIVEYCKYGNLSNYLKSKRDLFFLNKDAALHME

PKKEKMEPGLEQGKKPRLDSVTSSESFASSGFQEDKSLSDVEEEEDSDGFYKEPITME

DLISYSFQVARGMEFLSSRKCIHRDLAARNILLSENNVVKICDFGLARDIYKNPDYVR

KGDTRLPLKWMAPESIFDKIYSTKSDVWSYGVLLWEIFSLGGSPYPGVQMDEDFCSR

LREGMRMRAPEYSTPEIYQIMLDCWHRDPKERPRFAELVEKLGDLLQANVQQDGKD

YIPINAILTGNSGFTYSTPAFSEDFFKESISAPKFNSGSSDDVRYVNAFKFMSLERIKTFE

ELLPNATSMFDDYQGDSSTLLASPMLKRFTWTDSKPKASLKIDLRVTSKSKESGLSDV

SRPSFCHSSCGHVSEGKRRFTYDHAELERKIACCSPPPDYNSVVLYSTPPI

SEQ ID NO: 52.

In some embodiments, the VEGF Trap is aflibercept or conbercept. In some embodiments, the VEGF Trap is a VEGFR portion of aflibercept or a VEGFR portion of conbercept.

In some embodiments, provided is an anti-VEGF-A antibody or an antigen-binding fragment thereof. In some embodiments, the antigen-binding fragment is an scFv, Fv, Fab, or Fab' fragment.

In some embodiments, the anti-VEGF-A antibody is bevacizumab or ranibizumab. In some embodiments, the anti-VEGF-A antibody is a Fab of bevacizumab.

The present disclosure also provides a VEGF-binding molecule comprising immunoglobulin-like domain 2 of VEGFR2 (R2D2) and immunoglobulin-like domain 3 of VEGFR2 (R2D3), wherein the immunoglobulin-like domain 2 of VEGFR2 (R2D2) and/or the immunoglobulin-like domain 3 of VEGFR2 (R2D3) comprise(s) an amino acid mutation at any one or more positions selected from the group consisting of the following: position 162, position 221, position 222, position 274, position 276, position 277, position 280, position 284, position 288, position 289, and position 313. The position of the amino acid mutation is relative to a natural sequence position of the amino acid sequence set forth in SEQ ID NO: 51. In some embodiments, the R2D2 and/or R2D3 comprise(s) an amino acid mutation at any one or more sets of positions selected from the group consisting of the following:

- position 162;
- position 162 and position 221;
- position 162 and position 222;
- position 162 and position 274;
- position 162 and position 276;
- position 162 and position 277;
- position 162 and position 289;
- position 162 and position 313;
- position 221 and position 222;
- position 277 and position 289;
- position 162, position 221, and position 222;
- position 162, position 277, and position 289; and
- position 162, position 221, position 222, and position 274.

In some embodiments, the R2D2 and/or R2D3 comprise(s) an amino acid mutation at any one set of positions selected from the group consisting of the following:

- position 162;
- position 162, position 221, position 222, and position 274.

In some embodiments, the R2D2 and/or R2D3 comprise(s) any one or more sets of amino acid mutations selected from the group consisting of the following:

- 162A, 162V, 162S, 162I, or 162L;
- 221S or 221N;
- 222K;
- 274F or 274I;
- 276Q;
- 277R;
- 280E;
- 284H;
- 288Y;
- 289Y; and
- 313R.

In some embodiments, the R2D2 and/or R2D3 comprise(s) any one or more sets of amino acid mutations selected from the group consisting of the following:

- C162A, C162V, C162S, C1621, or C162L;
- Y221S;
- R222K;
- N274F or N274I;
- D276Q;
- L277R;
- Q280E;
- E284H;
- F288Y;
- L289Y; and
- L313R.

In some embodiments, the R2D2 and/or R2D3 comprise(s) any one or more sets of amino acid mutations selected from the group consisting of the following:

- 162V or 162I;
- 162V/274F; 162V/274I; 162I/274F; or 162I/274I;
- 221S/222K;
- 277R/289Y;
- 162V/221S/222K;
- 162V/276Q;
- 162V/289Y;
- 162V/277/289Y;
- 162V/313R; and
- 162V/221S/222K/274F.

In some embodiments, the R2D2 and/or R2D3 comprise(s) any one or more sets of amino acid mutations selected from the group consisting of the following:

- C162V or C1621;
- C162V/N274F;C162V/N274I;C162I/N274F; or C162I/N274I;
- Y221S/R222K;
- L277R/L289Y;
- C162V/Y221 S/R222K;
- C162V/D276Q;
- C162V/L289Y;
- C162V/L277R/L289Y;
- C162V/L313R;
- C162V/Y221 S/R222K1N274F.

In some embodiments, the R2D2 and/or R2D3 comprise(s) any one or more sets of amino acid mutations selected from the group consisting of the following:

- 162V; and
- 162V/221S/222K/274F.

In some embodiments, the R2D2 and/or R2D3 comprise(s) any one or more sets of amino acid mutations selected from the group consisting of the following:

- C162V; and
- C162V/Y221S/R222K/N274F.

In some embodiments, the R2D2 and/or R2D3 comprise(s) an amino acid mutation at any one or more positions selected from the group consisting of the following: position 135, position 197, position 199, position 213, position 215, position 219, position 257, position 275, position 277, position 278, position 283, position 289, position 313, and position 314. The position of the amino acid mutation is relative to a natural sequence position of the amino acid sequence set forth in SEQ ID NO: 51.

In some embodiments, the R2D2 and/or R2D3 comprise(s) an amino acid mutation at position 283.

In some embodiments, the R2D2 and/or R2D3 comprise(s) any one or more sets of amino acid mutations selected from the group consisting of the following:

- 135Y;
- 197Y;
- 199Q or 199T;
- 213N;
- 215F or 215H;
- 219I;
- 257Q or 257T;
- 275L or 275E;
- 277R;
- 278D, 278S, or 278E;
- 283T;
- 289Y;

- 313Y; and
- 314S or 314Q.

In some embodiments, the R2D2 and/or R2D3 comprise(s) any one or more sets of amino acid mutations selected from the group consisting of the following:

- V135Y;
- M197Y;
- F199Q or F199T;
- M213N;
- I215F or I215H;
- V219I;
- D257Q or D257T;
- R275L or R275E;
- L277R;
- K278D, K278S, or K278E;
- S283T;
- L289Y;
- L313Y; and
- M314S or M314Q.

In some embodiments, the R2D2 and/or R2D3 comprise(s) an amino acid mutation 283T.

In some embodiments, the R2D2 and/or R2D3 comprise(s) the amino acid mutation S283T.

In some embodiments, the R2D2 and/or R2D3 comprise(s) an amino acid mutation at any one or more positions selected from the group consisting of the following: position 162, position 221, position 222, position 274, position 276, position 277, position 280, position 284, position 288, position 289, and position 313; and comprise(s) an amino acid mutation at any one or more positions selected from the group consisting of the following: position 135, position 197, position 199, position 213, position 215, position 219, position 257, position 275, position 277, position 278, position 283, position 289, position 313, and position 314.

In some embodiments, the R2D2 and/or R2D3 comprise(s) an amino acid mutation at any one or more sets of positions selected from the group consisting of the following:

- position 162 and position 275;
- position 162 and position 283;
- position 162 and position 313;
- position 162, position 274, and position 283;
- position 162, position 221, position 222, and position 283; and
- position 162, position 221, position 222, position 274, and position 283. In some embodiments, the R2D2 and/or R2D3 comprise(s) an amino acid mutation at any one or more sets of positions selected from the group consisting of the following:
- position 162 and position 283; and
- position 162, position 221, position 222, position 274, and position 283.

In some embodiments, the R2D2 and/or R2D3 comprise(s):

(i) any one or more sets of amino acid mutations selected from the group consisting of the following:

- 162A, 162V, 162S, 162I, or 162L;
- 221S;
- 222K;
- 274F or 274I;
- 276Q;
- 277R;
- 280E;
- 284H;
- 288Y;
- 289Y; and
- 313R; and

(ii) any one or more sets of amino acid mutations selected from the group consisting of the following:

- 135Y;
- 197Y;
- 199Q or 199T;
- 213N;
- 215F or 215H;
- 219I;
- 257Q or 257T;
- 275L or 275E;
- 277R;
- 278D, 278S, or 278E;
- 283T;
- 289Y;
- 313Y; and
- 314S or 314Q.

In some embodiments, the R2D2 and/or R2D3 comprise(s):

(i) any one or more sets of amino acid mutations selected from the group consisting of the following:

- C162A, C162V, C162S, C1621, or C162L;
- Y221S;
- R222K;
- N274F or N274I;
- D276Q;
- L277R;
- Q280E;
- E284H;
- F288Y;
- L289Y; and
- L313R; and

(ii) any one or more sets of amino acid mutations selected from the group consisting of the following:

- V135Y;
- M197Y;
- F199Q or F199T;
- M213N;
- I215F or I215H;
- V219I;
- D257Q or D257T;
- R275L or R275E;
- L277R;
- K278D, K278S, or K278E;
- S283T;
- L289Y;
- L313Y; and
- M314S or M314Q.

In some embodiments, the R2D2 and/or R2D3 comprise(s) any one or more sets of amino acid mutations selected from the group consisting of the following:

- 162V/275L;
- 162V/283T;
- 162V/313Y;
- 162V/274F/283T;
- 162V/221S/222K/283T; and

- 162V/221S/222K/274F/283T.

In some embodiments, the R2D2 and/or R2D3 comprise(s) any one or more sets of amino acid mutations selected from the group consisting of the following:

- C162V/R275L;
- C162V/S283T;
- C162V/L313Y;
- C162V/N274F/S283T;
- C162V/Y221S/R222K/S283T; and
- C162V/Y221S/R222K/N274F/S283T.

In some embodiments, the R2D2 and/or R2D3 comprise(s) any one set of amino acid mutations selected from the group consisting of the following:

- 162V /283 T; and
- 162V/221S/222K/274F/283T.

In some embodiments, the R2D2 and/or R2D3 comprise(s) any one set of amino acid mutations selected from the group consisting of the following:

- C162V/S283T;
- C162V/Y221S/R222K/N274F/S283T.

In some embodiments, the VEGF-binding molecule consists of R2D2-R2D3.
In some embodiments, the VEGF-binding molecule comprises the amino acid sequence set forth in any one of SEQ ID NOs: 55-56 and SEQ ID NOs: 112-115, or an amino acid sequence having at least 80% sequence identity thereto.
In some embodiments, the VEGF-binding molecule consists of the amino acid sequence set forth in any one of SEQ ID NOs: 55-56 and SEQ ID NOs: 112-115, or an amino acid sequence having at least 80% sequence identity thereto.
In some embodiments, the VEGF-binding molecule has the capacity to bind to VEGF-C.
In some embodiments, the VEGF-binding molecule has increased stability and/or ligand binding activity.
In some embodiments, VEGF-binding molecules comprising amino acid mutations have increased stability and/or ligand binding activity as compared to wild-type ones.
In some embodiments, any one of the aforementioned VEGF-binding molecules also optionally comprises: a first VEGF-binding domain, a second VEGF-binding domain, and a multimerizing component. In some embodiments, the multimerizing component includes, but is not limited to, an immunoglobulin Fc or a variant thereof.
In some embodiments, the Fc comprises any one of the hinge regions (e.g., upper, middle, and/or lower hinge regions) and CH2 and CH3 domains or any combination thereof. In some embodiments, the Fc comprises at least a CH2 domain and a CH3 domain. In some embodiments, the Fc comprises a hinge region, a CH2 domain, and a CH3 domain.
In some embodiments, the Fc is derived from an immunoglobulin of any species. In some embodiments, the Fc is derived from a human immunoglobulin. In some embodiments, the Fc is derived from immunoglobulins of other mammalian species, including but not limited to rodents (e.g., mice, rats, rabbits, and guinea pigs) or non-human primate (e.g., chimpanzee and macaque) species.
In some embodiments, the Fc is derived from an immunoglobulin of any subtype, including but not limited to: a human IgG1, IgG2, IgG3, IgG4, IgD, IgA, IgE, or IgM antibody. In some embodiments, the Fc is from human IgG1.
In some embodiments, the multimerizing component is an Fc variant whose amino acid sequence differs from a naturally occurring immunoglobulin Fc by no more than 20 amino acids, e.g., no more than 15, no more than 10, or no more than 5 amino acids. In some embodiments, the amino acid differences include amino acid substitutions, insertions, or deletions. In some embodiments, the Fc variant has reduced effector function (e.g., Fc$\gamma$R binding).
In some embodiments, the multimerizing component causes the VEGF-binding molecule to form a dimer, trimer, tetramer, pentamer, hexamer, heptamer, octamer, nonamer, or decamer.
In some embodiments, the VEGF-binding molecule is a dimer (including homodimers and heterodimers).
In some embodiments, the Fc comprises an IgG1 hinge region, a CH2 domain, and a CH3 domain.
In some embodiments, the VEGF-binding domain is fused with a multimerizing component (e.g., a human immunoglobulin Fc) to obtain a fusion protein that has an excellent VEGF-binding effect, good stability, and a long half-life and is capable of multivalently binding to VEGF molecules.
In some embodiments, the VEGF-binding molecule comprises, from N-terminus to C-terminus, any one selected from the group consisting of the following:

R2D2-R2D3-Fc;
R1D2-R2D3-R2D2-R2D3-Fc;
R1D2-R2D3-linker-R2D2-R2D3-Fc;
R2D2-R2D3-R1D2-R2D3-Fc; and
R2D2-R2D3-linker-R1D2-R2D3-Fc.

In some embodiments, the VEGF-binding molecule comprises a heavy chain and a light chain, wherein the heavy chain sequentially comprises, from N-terminus to C-terminus: R2D2-R2D3-Fc-linker-VH-CH1, and the light chain sequentially comprises, from N-terminus to C-terminus: VL-CL, wherein "-" indicates that the two domains are directly or indirectly linked, but their relative positions from N-terminus to C-terminus are unchanged.

In some embodiments, any two adjacent domains in the VEGF-binding molecule of the present disclosure may be linked directly or by a linker.

In some embodiments, the linker is a peptide linker.

In some embodiments, the linker is represented by the following formula: $(GS)_a(GGS)_b(GGGS)_c(GGGGS)_d(GGGGG)_e$, wherein a, b, c, d, and e are independently integers greater than or equal to 0; or the linker is selected from the group consisting of: $(EAAAK)_3$, $(EAAAR)_3$, $(EGGGK)_3$, $(EGGGR)_3$, $(DAAAR)_3$, $(DAAAK)_3$, $(DGGGR)_3$, and $(DGGGK)_3$; or the linker is $(G_xS)_y$, wherein x is selected from the group consisting of integers of 1-5, and y is selected from the group consisting of integers of 1-6.

In some embodiments, the linker is GGGGS. In some embodiments, in some embodiments, from N-terminus to C-terminus, the VEGF-binding molecule is selected from the group consisting of:

R2D2-R2D3-Fc;
R1D2-R2D3-R2D2-R2D3-Fc;
R1D2-R2D3-linker-R2D2-R2D3-Fc;
R2D2-R2D3-R1D2-R2D3-Fc; and
R2D2-R2D3-linker-R1D2-R2D3-Fc.

In some embodiments, the VEGF-binding molecule comprises a heavy chain and a light chain, wherein the heavy chain is, from N-terminus to C-terminus: R2D2-R2D3-Fc-linker-VH-CH1, and the light chain is, from N-terminus to C-terminus: VL-CL.

In some embodiments, the VEGF-binding molecule comprises the amino acid sequence set forth in any one of SEQ ID NOs: 53-54, SEQ ID NOs: 57-99, SEQ ID NOs: 102-111, and SEQ ID NO: 116, or an amino acid sequence having at least 80% sequence identity thereto.

In some embodiments, the VEGF-binding molecule consists of the amino acid sequence set forth in any one of SEQ ID NOs: 53-54, SEQ ID NOs: 57-99, SEQ ID NOs: 102-111, and SEQ ID NO: 116, or an amino acid sequence having at least 80% sequence identity thereto.

In some embodiments, the heavy chain of the VEGF-binding molecule comprises the amino acid sequence set forth in SEQ ID NO: 100 or an amino acid sequence having at least 80% sequence identity thereto; and the heavy chain of the VEGF-binding molecule comprises a light chain set forth in SEQ ID NO: 101 or an amino acid sequence having at least 80% sequence identity thereto.

In some embodiments, the heavy chain of the VEGF-binding molecule comprises the amino acid sequence set forth in SEQ ID NO: 117 or an amino acid sequence having at least 80% sequence identity thereto; and the light chain of the VEGF-binding molecule comprises the amino acid sequence set forth in SEQ ID NO: 118 or an amino acid sequence having at least 80% sequence identity thereto.

In some embodiments, the VEGF-binding molecule comprises the amino acid sequence set forth in SEQ ID NO: 94 or an amino acid sequence having at least 80% sequence identity thereto, or consists of the amino acid sequence set forth in SEQ ID NO: 94.

In some embodiments, the VEGF-binding molecule comprises the amino acid sequence set forth in SEQ ID NO: 111 or an amino acid sequence having at least 80% sequence identity thereto, or consists of the amino acid sequence set forth in SEQ ID NO: 111.

In some embodiments, the VEGF-binding molecule of the present disclosure includes full-length or partial proteins of any one of the aforementioned VEGF-binding molecules, or muteins, functional derivatives, functional fragments, bioactive peptides, fusion proteins, isotypes, or salts thereof obtained by further mutation on the basis of any one of the aforementioned VEGF-binding molecules, for example, fusion proteins comprising the VEGF-binding molecule, monomers or dimers or trimers or multimers of the VEGF-binding molecule, various modified forms of the VEGF-binding molecule (e.g., PEGylated, PEG-modified, glycosylated, albumin-conjugated or -fused, albumin antibody-conjugated or -fused, hydroxyethylated, and de-O-glycosylated), and homologs of the VEGF-binding molecule in various species. The modifications to the VEGF-binding molecule do not result in adverse effects on protein activity associated with treatment.

In some embodiments, the VEGF-binding molecule has increased stability and/or ligand binding activity.

In some embodiments, VEGF-binding molecules comprising amino acid mutations have increased stability and/or ligand binding activity as compared to wild-type ones.

In some embodiments, the VEGF-binding molecule of the present disclosure has an HPLC purity of at least 70%, at least 75%, at least 80%, at least 85%, at least 90%, or at least 95% when stored at 40 °C with 75% humidity for two weeks.

In some embodiments, the VEGF-binding molecule of the present disclosure has an HPLC purity of at least 70%, at least 75%, at least 80%, at least 85%, at least 90%, or at least 95% when stored at 40 °C with 75% humidity for four weeks.

In some embodiments, the VEGF-binding molecule of the present disclosure has an HPLC purity of at least 70%, at least 75%, at least 80%, at least 85%, at least 90%, or at least 95% when stored at room temperature with 75% humidity for two weeks.

In some embodiments, the VEGF-binding molecule of the present disclosure has an HPLC purity of at least 70%, at least 75%, at least 80%, at least 85%, at least 90%, or at least 95% when stored at room temperature with 75% humidity for four weeks.

In some embodiments, HPLC purity may be measured using the method in the examples.

In some embodiments, the binding activity IC50 of the VEGF-binding molecule of the present disclosure for VEGF-A is $\leq 10$ nM, $\leq 5$ nM, or $\leq 2$ nM; for example, the IC50 is 0.01 nM to 10 nM, 0.05 nM to 5 nM, 0.05 nM to 2 nM, 0.05 nM to 1.5 nM, 0.05 nM to 1.2 nM, or 0.05 nM to 1 nM; and/or

the binding activity IC50 of the VEGF-binding molecule of the present disclosure for VEGF-C is $\leq 10$ nM, $\leq 5$ nM, or $\leq 2$ nM; for example, the binding activity IC50 of the VEGF-binding molecule of the present disclosure for VEGF-C is 0.01 nM to 10 nM, 0.05 nM to 5 nM, 0.05 nM to 2 nM, 0.05 nM to 1.5 nM, 0.05 nM to 1.2 nM, or 0.05 nM to 1 nM.

In some embodiments, the binding kinetics $K_D$ of the VEGF-binding molecule of the present disclosure for VEGF-A is $\leq 1 \times 10^{-9}$; for example, $K_D \leq 1 \times 10^{-10}$, $K_D \leq 9 \times 10^{-11}$, $K_D \leq 8 \times 10^{-11}$, $K_D \leq 7 \times 10^{-11}$, $K_D \leq 6 \times 10^{-11}$, $K_D \leq 5 \times 10^{-11}$, or $K_D \leq 4 \times 10^{-11}$;

the binding kinetics $K_D$ of the VEGF-binding molecule of the present disclosure for VEGF-B is $\leq 1 \times 10^{-9}$; for example, $K_D \leq 4 \times 10^{-10}$, $K_D \leq 3 \times 10^{-10}$, $K_D \leq 2 \times 10^{-10}$, $K_D \leq 1 \times 10^{-10}$, $K_D \leq 9 \times 10^{-11}$, $K_D \leq 8 \times 10^{-11}$, $K_D \leq 7 \times 10^{-11}$, $K_D \leq 6 \times 10^{-11}$, $K_D \leq 5 \times 10^{-11}$, $K_D \leq 4 \times 10^{-11}$, $K_D \leq 3 \times 10^{-11}$, or $K_D \leq 2 \times 10^{-11}$; and/or

the binding kinetics $K_D$ of the VEGF-binding molecule of the present disclosure for VEGF-C is $\leq 1 \times 10^{-9}$; for example, $K_D \leq 1 \times 10^{-10}$, $K_D \leq 9 \times 10^{-11}$, $K_D \leq 8 \times 10^{-11}$, $K_D \leq 7 \times 10^{-11}$, $K_D \leq 6 \times 10^{-11}$, $K_D \leq 5 \times 10^{-11}$, $K_D \leq 4 \times 10^{-11}$, $K_D \leq 3 \times 10^{-11}$, or $K_D \leq 2 \times 10^{-11}$.

In some embodiments, the binding activity of the VEGF-binding molecule of the present disclosure for a ligand can be measured using the method described in Example 3 or Example 5.

Nucleic acid and vector

The present disclosure also provides a nucleic acid molecule encoding the VEGF-binding molecule of the present disclosure. In some embodiments, the nucleic acid of the present disclosure may be an RNA, DNA, or cDNA. According to some embodiments of the present disclosure, the nucleic acid of the present disclosure is an isolated nucleic acid.

The present disclosure also provides a DNA molecule encoding any of the aforementioned VEGF-binding molecules of the present disclosure.

The nucleic acid of the present disclosure may also be in the form of a vector and may be present in the vector and/or may be part of the vector. The vector is, for example, a plasmid, cosmid, YAC, or viral vector. The vector may especially be an expression vector, i.e., a vector that provides for the *in-vitro* and/or *in-vivo* (i.e., in suitable host cells, host organisms and/or expression systems) expression of the VEGF-binding molecule. The expression vector generally comprises at least one of the nucleic acids of the present disclosure, which is operably linked to one or more suitable expression regulatory elements (e.g., promoters, enhancers, and terminators). The selection of the elements and sequences thereof for expression in a particular host is common knowledge of those skilled in the art. Regulatory elements and other elements useful or necessary for expression of the VEGF-binding molecule of the present disclosure include, for example, promoters, enhancers, terminators, integration factors, selection labels, leader sequences, and reporter genes.

The nucleic acid of the present disclosure may be prepared or obtained by known means (e.g., by automatic DNA synthesis and/or recombinant DNA techniques) based on information about the amino acid sequences of the polypeptides of the present disclosure, and/or may be isolated from a suitable natural source.

In some embodiments, the nucleic acid molecule comprises a transgene or polynucleotide encoding any one of the VEGF-binding molecules of the present disclosure.

In some embodiments, the transgene comprises a nucleic acid sequence having at least 50%, at least 60%, at least 70%, at least 80%, at least 90%, at least 99%, or 100% identity to the nucleic acid sequence of any one of SEQ ID NOs: 128-136, or a codon-optimized variant of the nucleic acid sequences of SEQ ID NOs: 128-136.

In some embodiments, the nucleic acid sequence of the transgene is operably linked to a constitutive promoter. In other embodiments, the nucleic acid sequence of the transgene is operably linked to an inducible promoter. In some cases, the nucleic acid sequence of the transgene is operably linked to a tissue-specific or cell type-specific regulatory element.

In the context of the present disclosure, the transgene refers to the target gene, and a nucleic acid encoding any one of the VEGF-binding molecules of the present disclosure.

In some embodiments, the isolated nucleic acid further comprises a promoter operably linked to the transgene.

In some embodiments, the promoter is selected from the group consisting of a cytomegalovirus (CMV) promoter, a Rous sarcoma virus (RSV) promoter, an MMT promoter, an EF-1alpha promoter, a UB6 promoter, a chicken beta-actin promoter, a CAG promoter, an RPE65 promoter, and an opsin promoter, e.g., a CMV promoter.

In some embodiments, the isolated nucleic acid further comprises an enhancer; for example, the enhancer is a CMV enhancer.

In some embodiments, the isolated nucleic acid further comprises an intron.

In some embodiments, the isolated nucleic acid further comprises a Kozak sequence. In some embodiments, the Kozak sequence is located between the intron and the transgene encoding the VEGF-binding molecule.

In some embodiments, the isolated nucleic acid further comprises a 5'-UTR and a 3'-UTR.

In some embodiments, the isolated nucleic acid further comprises a WPRE sequence.

In some embodiments, the isolated nucleic acid further comprises a polyA sequence, e.g., an sv40polyA sequence.

The present disclosure also provides a vector comprising any one of the isolated nucleic acids described above. In some embodiments, the vector is a plasmid or a viral vector. In some embodiments, the virus is selected from the group consisting of an adeno-associated virus (AAV), a helper-dependent adenovirus, a retrovirus, a herpes simplex virus, a lentivirus, an adenovirus, an adeno-associated viral vector, a poxvirus, a hemagglutinating virus of Japan-liposome (HVJ) complex, a Moloney murine leukemia virus, and an HIV-based virus.

In some embodiments, the vector is a recombinant adeno-associated viral vector.

The present disclosure also provides a recombinant adeno-associated viral vector comprising:

(i) the transgene encoding the VEGF-binding molecule defined in any one of the foregoing of the present disclosure;
(ii) an enhancer (e.g., a CMV enhancer);
(iii) a promoter (e.g., a CMV promoter);
(iv) a 5'-UTR and a 3'-UTR;
(v) a post-transcriptional regulatory element (e.g., WPRE); and
(vi) a polyadenylation signal (polyA, e.g., SV40 polyA);

optionally, (vii) a Kozak sequence, and/or, (viii) an intron;
any one or any combination of (ii)-(viii) is operably linked to the (i).

In some embodiments, the recombinant adeno-associated viral vector comprises a 5' ITR and/or a 3' ITR. The 5' ITR and/or 3' ITR are/is derived from AAV1, AAV2, AAV3, AAV4, AAV5, AAV6, AAV7, AAV8, AAV9, AAV9.47, AAV9 (hu14), AAV10, AAV11, AAV12, AAVrh8, AAVrh10, AAV-DJ, or AAV-DJ8; for example, from AAV2 or AAV9.

In some specific embodiments, the promoter is selected from the group consisting of CMV, CAG, CBh, EFS, EF1 (e.g., EF-1α), PGK, SV40, Ubi, RSV, or any combination thereof.

In some specific embodiments, the enhancer is selected from the group consisting of Ubi, CMV, and RSV enhancers, or any combination thereof.

In some specific embodiments, the intron is selected from the group consisting of MVM, SV40, β Globin, EF1 (e.g., EF-1α), a hybrid intron, or any combination thereof.

In some specific embodiments, the polyA is selected from the group consisting of PA75 polyA, SV40 polyA, hGH polyA, BGH polyA, rbGlob polyA, or any combination thereof.

In some specific embodiments, the post-transcriptional regulatory element is selected from the group consisting of WPRE, HPRE or a combination thereof.

In some embodiments, the recombinant adeno-associated viral vector comprises any one of the aforementioned isolated nucleic acids and a capsid protein.

In some embodiments, the capsid protein is selected from the group consisting of the following serotypes: AAV1, AAV2, AAV3, AAV4, AAV5, AAV6, AAV7, AAV8, AAV9, AAV10, or variants thereof.

In some embodiments, the capsid protein has and comprises the amino acid sequence set forth in SEQ ID NO: 127 or an amino acid sequence having at least 70%, at least 75%, at least 80%, at least 85%, at least 90%, at least 95%, or at least 99% sequence identity to SEQ ID NO: 127.

The new-structure VEGF-binding molecule provided by the present disclosure is used as a novel VEGF inhibitor that may be administered in the form of a protein drug or nucleic acid drug. In terms of function, it is capable of binding to both VEGF-A and VEGF-C and thus has higher VEGF inhibition efficiency. In terms of efficacy, the VEGF-binding molecule has higher stability; in particular, the nucleic acid drug form can effectively reduce administration frequency; it is a drug with good clinical development prospects.

AAV capsid and AAV particle

The polynucleotide according to any one of the foregoing of the present disclosure may be wrapped using an AAV capsid to form an AAV particle. The AAV capsid is as set forth in any one of the above. In some specific embodiments, the AAV capsids used in the present disclosure are the AAV2 and AAV9 capsids in the prior art described above.

In some embodiments, the AAV capsid is engineered; for example, the AAV capsid is a hybrid AAV capsid from two or more parental serotypes. For example, the AAV capsid may be AAV2G9, which comprises sequences from AAV2 and AAV9. The sequence of AAV2G9 in US20160017005 is incorporated herein by reference in its entirety.

In some embodiments, the AAV capsid is an AAV2 or AAV9 capsid.

In some embodiments, the AAV particle may comprise all or part of the vector genome of a nucleotide sequence or a variant of any naturally occurring and/or recombinant AAV capsid, in addition to the encoded heterologous payload.

In some embodiments, the AAV particle may be replication-deficient (e.g., it lacks sequences encoding functional Rep and Cap proteins in the vector genome). In some specific embodiments, the replication-deficient AAV particle may lack most or all of the parental coding sequences, and carry essentially only one or two AAV ITR sequences and a nucleic acid of interest for delivery to a cell, tissue, organ, or organism.

In some embodiments, the AAV particle may be a recombinant AAV (rAAV) particle.

In some embodiments, the AAV particle may be selected from the group consisting of a single-stranded AAV particle (e.g., ssAAV) and a self-complementary AAV particle (e.g., scAAV). By skipping the synthesis of a second strand, scAAV achieves rapid expression in cells.

In some embodiments, the AAV particle has a nucleic acid wrapped therein, wherein a VEGF-binding molecule encoded by the nucleic acid comprises the amino acid sequence set forth in any one of SEQ ID NOs: 53-54, 57-99, 102-61, and 116; the VEGF-binding molecule comprises a heavy chain set forth in SEQ ID NO: 100 and a light chain set forth in SEQ ID NO: 101; or the VEGF-binding molecule comprises a heavy chain set forth in SEQ ID NO: 117 and a light chain set forth in SEQ ID NO: 118.

In some embodiments, the AAV particle has a nucleic acid wrapped therein, wherein the nucleic acid has at least 95% identity to the sequence set forth in any one of SEQ ID NOs: 128-136.

In some embodiments, the AAV particle has a nucleic acid wrapped therein, wherein the nucleic acid comprises the sequence set forth in any one of SEQ ID NOs: 128-136.

In some embodiments, the capsid protein of the AAV particle has and comprises the amino acid sequence set forth in SEQ ID NO: 127 or an amino acid sequence having at least 70%, at least 75%, at least 80%, at least 85%, at least 90%, at least 95%, or at least 99% sequence identity to SEQ ID NO: 127.

Regulatory element

The vector genome (or viral genome) of the nucleic acid or AAV particle according to any one of the foregoing of the present disclosure comprises at least one regulatory element, such that a target gene (e.g., a VEGF-binding molecule) in a payload can be replicated, transcribed, and translated.

In some embodiments, the regulatory element includes, but is not limited to, sequences for transcription initiation and/or termination, promoter and/or enhancer sequences, efficient RNA processing signals (e.g., splicing and polyadenylation signals), sequences that stabilize cytoplasmic mRNA, sequences that enhance translation efficiency (e.g., Kozak consensus sequences), sequences that enhance protein stability, and/or sequences that enhance protein processing and/or secretion. Exemplary regulatory elements include, but are not limited to, promoters, enhancers, introns, endogenous miRNAs, post-transcriptional regulatory elements (PREs), polyadenylation (PolyA) signal sequences, and upstream enhancers (USEs).

In some embodiments, the regulatory elements described below (e.g., promoters) drive expression of the payload in a target tissue (e.g., in an eye of a subject) for a period of time, for example, for 1 day, 2 days, 3 days, 4 days, 5 days, 6 days, 7 days, 1 week, 2 weeks, 3 weeks, 4 weeks, 1 month, 2 months, 3 months, 4 months, 5 months, 6 months, 7 months, 8 months, 9 months, 10 months, 11 months, 12 months, 1 year, 2 years, 3 years, 4 years, 5 years, 6 years, 7 years, 8 years, 9 years, 10 years, 20 years, 30 years, 40 years, 50 years, 60 years or more, as another example, for 1-2 weeks, 1-3 weeks, 1-4 weeks, 1-2 months, 1-4 months, 1-6 months, 2-6 months, 3-6 months, 3-9 months, 4-8 months, 6-12 months, 1-2 years, 1-5 years, 2-5 years, 3-6 years, 3-8 years, 4-8 years, 5-10 years, 10-20 years, 10-30 years, 20-40 years, 20-50 years, or the lifetime of the subject.

Inverted terminal repeat (ITR)

The vector genome (or viral genome) of the polynucleotide or AAV particle according to any one of the foregoing of the present disclosure comprises at least one ITR, e.g., has two ITRs flanking a payload at the 5' end and the 3' end, respectively.

In some embodiments, the ITRs have origin of replication function.

In some embodiments, the ITRs comprise sequence regions that may be complementary and symmetrically arranged.

In some embodiments, the ITRs may consist of naturally occurring polynucleotide sequences or recombinantly derived polynucleotide sequences.

In some embodiments, the ITRs may be derived from the same or different serotype as the capsid. In some embodiments, the 5' ITR and the 3' ITR may be derived from the same serotype or may be derived from different serotypes. For example, both 5' ITR and 3' ITR are from AAV2, or both are from AAV9. For another example, the 5' ITR comprises the sequence set forth in SEQ ID NO: 126, and/or the 3' ITR comprises the nucleotide sequence set forth in SEQ ID NO: 137.

In some embodiments, the length of each ITR may be about 100 to about 150 nucleotides, such as 100-105 nucleotides, 106-110 nucleotides, 111-115 nucleotides, 116-120 nucleotides, 121-125 nucleotides, 126-130 nucleotides, 131-135 nucleotides, 136-140 nucleotides, 141-145 nucleotides, or 146-150 nucleotides. In one embodiment, the length of the ITR is 140-142 nucleotides, such as 141 nucleotides. Non-limiting examples of the length of the ITR are 102 nucleotides, 140 nucleotides, 141 nucleotides, 142 nucleotides, 145 nucleotides, and nucleotides having at least 95% identity thereto.

Promoter

The vector genome (or viral genome) of the polynucleotide or AAV particle according to any one of the foregoing of the present disclosure comprises at least one promoter, including but not limited to, species-specific promoters, inducible promoters, tissue-specific promoters, or cell cycle-specific promoters.

In some embodiments, the promoter drives expression of a protein or polypeptide (e.g., a VEGF-binding molecule) encoded in a payload of the vector genome of the AAV particle.

In some embodiments, the promoter is specific to or has tropism for a target tissue, such as a promoter capable of expressing a payload in an eye tissue.

In some embodiments, the promoter may be a viral promoter, a plant promoter, a mammalian promoter, or a human promoter.

In some embodiments, the promoter includes, but is not limited to, CMV, CBA (including derivatives CAG, CBh, etc.), EF-1α, PGK, UBC, RSV, EFS, EF1, GUSB (hGBp), UCOE (the promoter of HNRPA2B1-CBX3), NSE, Synapsin, MeCP2, MeP418, MeP426, VMD2, MRHO, TRE, Ac5, Polyhedrin, CaMKIIa, Gall, TEF1, GDS, ADHI, Ubi, GFAP, or PKG promoters, and may be selected from the group consisting of neurofilament light (NFL) promoters, neurofilament heavy (NFH) promoters, SCN8A promoters, frataxin (FXN) promoters (or known as FRDA promoters), H1 promoters, RNA pol III promoters (e.g., U6 or H1), and small nuclear RNA (ULB or ULA) promoters. In some embodiments, the promoter is a liver or skeletal muscle promoter; the liver promoter is, for example, human α-1-antitrypsin (hAAT) and thyroxine-binding globulin (TBG), and the skeletal muscle promoter is, for example, desmin, MCK, or synthetic C5-12.

In some embodiments, the AAV vector genome of the present disclosure comprises two promoters, for example, a CMV promoter and a CBA promoter, the CMV sequence being set forth in SEQ ID NO: 125; as another example, an EF1α promoter and a CMV promoter, or a combination of any two of the aforementioned promoters.

In some embodiments, the promoter is a tissue-specific expression element that can limit expression to certain cell types, including but not limited to, a muscle-specific promoter, a B cell promoter, a monocyte promoter, a leukocyte promoter, a macrophage promoter, a pancreatic acinar cell promoter, an endothelial cell promoter, a lung tissue promoter, an astrocyte promoter, or a nervous system promoter, which can be used to limit expression to neurons, astrocytes, or oligodendrocytes.

In some embodiments, the promoter is a truncation or a variant of the aforementioned promoters and has a length of less than 1 kb, such as 200-300, 200-400, 300-400, 200-500, 200-600 or more.

In some embodiments, the promoter may be a combination of two or more components of the same or different initial or parental promoters, such as CMV and CBA.

Enhancer

The vector genome (or viral genome) of the polynucleotide or AAV particle according to any one of the foregoing of the present disclosure comprises at least one enhancer, including but not limited to, species-specific enhancers, inducible enhancers, tissue-specific enhancers, or cell cycle-specific enhancers.

In some embodiments, the enhancer is specific to or has tropism for a target tissue, such as an enhancer capable of regulating expression of a payload in a neural tissue.

In some embodiments, the enhancer may be or be derived from a viral enhancer, a plant enhancer, a mammalian enhancer, or a human enhancer.

In some embodiments, the enhancer may be located upstream or downstream of a promoter and operably linked to the promoter. Expression of a target gene (e.g., a VEGF-binding molecule) is enhanced by at least 20%, at least 50%, at least 80%, at least 100%, or at least several times (including 1, 2, 3, 4, 5, 6, 7, 8, 9, 10 times or more) when the enhancer is present as compared to when the enhancer is not present.

In some embodiments, the enhancer includes, but is not limited to, EF-1$\alpha$, Ubc, humanb-actin, CAG, TRE, Ac5, Polyhedrin, CaMKIIa, GalI, TEF1, GDS, ADH1, Ubi, and $\alpha$-1-antitrypsin (hAAT), or variants or fragments of the above enhancers. In some embodiments, the enhancer is selected from the group consisting of IRBP, RSV, or CMV enhancers, or variants or fragments thereof.

In some embodiments, the enhancer comprises or is an enhancer set forth in SEQ ID NO: 124 or a variant or fragment thereof.

Intron

An intron or a portion thereof may be comprised in the vector genome (or viral genome) of the polynucleotide or AAV particle according to any one of the foregoing of the present disclosure.

In some embodiments, the intron includes, but is not limited to, MVM (67-97 bp), F.IX truncated intron 1 (about 300 bp), $\beta$-globin SD/immunoglobulin heavy chain splice acceptor (about 250 bp), adenovirus splice donor/immunoglobulin splice acceptor (about 500 bp), SV40 late splice donor/splice acceptor (19S/16S) (180 bp) and hybrid adenovirus splice donor/IgG splice acceptor (about 230 bp), hemoglobin introns, hybrid introns, $\beta$-globin introns, or variants or fragments of the above introns.

In some embodiments, the length of the intron is 100-800 nucleotides, such as about 100 nucleotides, about 200 nucleotides, about 300 nucleotides, about 400 nucleotides, about 500 nucleotides, and about 600 nucleotides, and further such as 200-250 nucleotides, 200-300 nucleotides, 100-200 nucleotides, 300-500 nucleotides, 500-600 nucleotides, 400-600 nucleotides, and 100-200 nucleotides.

In some embodiments, the intron in the technical solutions of the present disclosure is selected from the group consisting of a hybrid intron and a $\beta$-globin intron.

Untranslated region (UTR)

An untranslated region (UTR) may be comprised in the vector genome (or viral genome) of the polynucleotide or AAV particle according to any one of the foregoing of the present disclosure, wherein the UTR is selected from the group consisting of a 5' UTR and/or a 3' UTR. The UTR is used to regulate (e.g., increase or decrease) polynucleotide stability and protein production. Typically, the 5' UTR starts at a transcription start site and terminates at a start codon, and the 3' UTR starts immediately after a stop codon and terminates at a transcription termination signal. The UTR may be a wild type, a variant thereof, or an artificial UTR.

In some embodiments, the 5' UTR comprises a Kozak sequence. In other embodiments, the 5' UTR does not comprise a Kozak sequence.

In some embodiments, the 3' UTR is rich in AU. In some embodiments, the 3' UTR is selected from the group consisting of: class I AREs, for example, but not limited to, c-Myc and MyoD, containing several dispersed copies of AUUUA motifs within the U-rich region; class II AREs, for example, but not limited to, GM-CSF and TNF-a, possessing two or more overlapping UUAUUUA(U/A)(U/A) nonamers; and class III ARES, for example, but not limited to, c-Jun and myogenin.

In some embodiments, the 3' UTR may comprise an oligomeric (dT) sequence for templated addition of a polyadenylation sequence (PolyA).

Polyadenylation sequence (PolyA)

A sequence encoding a PolyA may be comprised in the vector genome (or viral genome) of the polynucleotide or AAV particle according to any one of the foregoing of the present disclosure, wherein the PolyA may be a wild type, a variant thereof, or a modified PolyA, for protein translation.

In some embodiments, the sequence encoding the PolyA is located between the 3' end of a coding sequence of a payload and the 5' end of a 3' ITR.

In some embodiments, the length of the sequence encoding the PolyA is 0-500 nucleotides, such as about 75 nucleotides, about 100 nucleotides, about 110 nucleotides, about 120 nucleotides, about 150 nucleotides, about 160 nucleotides, about 200 nucleotides, or about 300 nucleotides, further such as 50-100, 50-150, 50-160, 50-200, 60-100, 60-150, 60-160, 60-200, 70-100, 70-150, 70-160, 70-200, 80-100, 80-150, 80-160, 80-200, 90-100, 90-150, 90-160, or 90-200.

In some embodiments, the encoding PolyA may encode continuous PolyA or discontinuous PolyA. When the discontinuous PolyA is encoded, the coding sequence may be interrupted or separated by other nucleotides. For example, the polyA has at least two 60-adenylate fragments that are separated by a sequence comprising 10-90 nucleotides.

In some embodiments, the sequence encoding the PolyA or polyA in the technical solutions of the present disclosure is selected from the group consisting of β-globin polyA, SV40 polyA, bGH polyA, PA75 polyA, MeCP2 polyA, RDH1 polyA, BGH polyA, SPA49 polyA, sNRP-TK65 polyA, sNRP polyA, TK65 polyA, or variants or fragments of the above PolyA. In some embodiments, the polyA set forth in SEQ ID NO: 139 or a variant or fragment thereof in the technical solutions of the present disclosure.

polyA or coding sequences thereof in WO2016005324, WO2016005004, WO2016091391, WO2019036513, and WO2020074642 are incorporated herein by reference in their entirety, all of which may be used in the technical solutions of the present disclosure.

Stuffer sequence

A stuffer sequence may be comprised in the vector genome (or viral genome) of the polynucleotide or AAV particle according to any one of the foregoing of the present disclosure, such that the length of the vector genome is the optimal size for packaging, for example, such that the length of the vector genome is about 2.3 kb, about 4.6 kb, about 4.7 kb, or about 5.1 kb.

In some embodiments, the vector genome is a single-stranded or double-stranded genome, and the packaged AAV particle is ssAAV or scAAV.

In some embodiments, one vector genome may comprise one or more (e.g., 1, 2, 3, 4, 5, 6, 7, 8, 9, or 10) stuffer sequences.

In some embodiments, the stuffer sequence may be located between or within a plurality of regulatory elements, e.g., between two introns, at 3' of a 5' ITR sequence, at 5' of a 5' ITR sequence, at 5' of a 3' ITR sequence, at 3' of a 3' ITR sequence, and before or after a promoter, an intron, an enhancer, a polyA, a multiple cloning site (MCS) region, an exon, or other regions.

Production or preparation method for recombinant adeno-associated virus (rAAV) particles

The present disclosure provides methods for producing, preparing, and/or modifying rAAV particles. The production, preparation, and/or modification methods for rAAV particles in WO200028004, WO200123001, WO2004112727, WO 2005005610, WO2005072364, WO2013123503, WO2015191508, and US20130195801 are incorporated herein by reference in their entirety. The rAAV particles may have enhanced delivery efficiency, may be efficiently packaged, and may successfully infect a target cell (e.g., a mammalian or human cell) with high frequency and minimum toxicity.

In some embodiments, provided is a production method for rAAV particles, which comprises packaging any of the nucleic acids or vector genomes (or viral genomes) or vectors of the present disclosure into an AAV capsid. In some specific embodiments, the method comprises the following steps:

   1) co-transfecting competent bacterial cells with a baculovirus vector and a viral construct vector and/or an AAV payload construct vector,
   2) isolating the resulting viral construct expression vector and AAV payload construct expression vector and separately transfecting viral replication cells,
   3) isolating and purifying the resulting payload and viral construct particles comprising the viral construct expression vector or AAV payload construct expression vector,
   4) co-infecting viral replication cells with both the AAV payload and the viral construct particles comprising the viral construct expression vector or AAV payload construct expression vector, and
   5) harvesting and purifying AAV particles comprising a viral genome.

In some embodiments, provided is a production method for rAAV particles, which comprises the following steps:

1) co-transfecting a mammalian cell (e.g., an HEK293 cell) with any of the nucleic acids or vector genomes (or viral genomes) or vectors of the present disclosure, and a construct expressing Rep and Cap genes and a helper construct (to achieve helper functions) simultaneously; and
2) harvesting and purifying rAAV particles comprising a viral genome.

In some embodiments, the viral genome of the aforementioned rAAV particles optionally encodes a selection marker. The selection marker may include a cell surface marker, such as any protein expressed on the cell surface, including but not limited to, a receptor CD marker, a lectin, an integrin, or truncated forms thereof.

In some embodiments, provided is an AAV production system for producing the rAAV particles of the present disclosure, wherein the production system comprises:

1) a nucleic acid sequence encoding an AAV capsid;
2) any of the nucleic acids or vector genomes (or viral genomes) or vectors of the present disclosure; and
3) a helper packaging element having sufficient AAV rep function and helper function that allow packaging of the nucleic acid or vector genome (or viral genome) or vector in (b) into an AAV capsid.

The nucleic acid or vector genome (or viral genome) or vector comprises any of the aforementioned nucleic acids encoding the VEGF-binding molecule of the present disclosure. The AAV capsid is selected from the group consisting of any of the aforementioned AAV capsids of the present disclosure, e.g., AAV1, AAV2, AAV3, AAV4, AAV5, AAV6, AAV7, AAV8, AAV9, AAV9.47, AAV9 (hu14), AAV10, AAV11, AAV12, AAVrh8, AAVrh10, AAV-DJ, AAV-DJ8, or a variant of any one of the foregoing.

In some specific embodiments, the sufficient AAV rep function and helper function of the helper packaging plasmid including element are provided by a packaging cell, wherein the packaging cell may comprise any one or more of three plasmids pHelper, pRC9, and pGOI.

Illustratively, "helper function" or "helper virus function" refers to a function encoded in a helper virus genome which allows AAV replication and packaging (in conjunction with other requirements for replication and packaging described herein). In the present disclosure, "helper virus function" can be provided in a number of ways, including by providing a helper virus or providing, for example, polynucleotide sequences encoding the requisite functions to a producer cell in transport.

Illustratively, "AAV rep function" refers to functions provided by Rep genes and/or Cap genes.

In some embodiments, the Rep genes encode the non-structural proteins that regulate functions such as the replication of the AAV genome, and may be selected from the group consisting of Rep78, Rep68, Rep52, and Rep40. Rep78 and Rep68 are generally transcribed from the p5 promoter, while Rep52 and Rep40 are generally transcribed from the p19 promoter. The Cap genes encode the structural proteins VP1, VP2, and/or VP3 that assemble to form the viral capsid. The Cap genes are generally transcribed from the p40 promoter.

In some embodiments, provided are rAAV particles produced by the AAV production system described above.

In some embodiments, the pharmaceutical composition comprises the nucleic acid molecule according to any one of the foregoing.

In some embodiments, the pharmaceutical composition comprises recombinant adeno-associated virus (rAAV) particles and excipients; the recombinant adeno-associated virus (rAAV) particle comprises a nucleic acid molecule I and an AAV9 capsid, and the excipient comprises any one of (a)-(f) and (h).

In some embodiments, the pharmaceutical composition comprises recombinant adeno-associated virus (rAAV) particles and excipients; the recombinant adeno-associated virus (rAAV) particle comprises a nucleic acid molecule I and an AAV9 capsid, and the excipient comprises any one of (1)-(3).

In some embodiments, the pharmaceutical composition comprises recombinant adeno-associated virus (rAAV) particles and excipients; the recombinant adeno-associated virus (rAAV) particle comprises a nucleic acid molecule II and an AAV9 capsid, and the excipient comprises any one of (a)-(f) and (i).

In some embodiments, the pharmaceutical composition comprises recombinant adeno-associated virus (rAAV) particles and excipients; the recombinant adeno-associated virus (rAAV) particle comprises a nucleic acid molecule II and an AAV9 capsid, and the excipient comprises any one of (4)-(6).

The pharmaceutical composition of the present disclosure further comprises a solvent. The solvent in the pharmaceutical composition is selected from, without limitation, non-toxic physiologically acceptable liquid carriers, such as normal saline, water for injection, or a glucose solution (e.g. 5% glucose injection or glucose-sodium chloride injection).

The present disclosure also provides a freeze-dried formulation comprising a pharmaceutical composition, which is obtained by lyophilizing any one of the aforementioned pharmaceutical compositions.

In the present disclosure, a reconstituted solution with an increased protein concentration can be prepared by means of dilution-freezing-concentration-dissolution, and the reconstituted solution has a relatively good appearance and good stability.

In some embodiments, the composition is substantially stable over a relatively long period of time. "Stable" or "storage-stable" or "substantially stable" means that the composition does not significantly degrade and/or lose activity over a relatively long period of time. For example, a storage-stable composition may not have significant impurities caused by degradation of the composition over a relatively long period of time. For example, the composition has no more than 10% impurity, or no more than 9%, or no more than 8%, or no more than 7%, or no more than 6%, or no more than 5%, or no more than 4%, or no more than 3%, or no more than 2%, or no more than 1% degradation product over a relatively long period of time. In some cases, the storage-stable composition has no more than 5% impurity over a relatively long period of time. In some cases, the storage-stable composition substantially retains its activity for a relatively long period of time. For example, the composition retains 100%, or no less than 99%, or no less than 98%, or no less than 97%, or no less than 96%, or no less than 95%, or no less than 94%, or no less than 93%, or no less than 92%, or no less than 91%, or no less than 90%, or no less than 85%, or no less than 80%, or no less than 75% of the activity over a relatively long period of time. For example, a storage-stable composition may retain no less than 90% activity over a relatively long period of time. In some cases, the storage-stable composition retains no less than 95% activity over a relatively long period of time. The relatively long period of time is a period of time, e.g. 1 week or more, or 2 weeks or more, or 3 weeks or more, or 1 month or more, or 2 months or more, or 3 months or more, or 4 months or more, or 6 months or more, or 9 months or more, or 1 year or more, or 1.5 years (e.g., 18 months) or more, or 2 years or more, or 2.5 years (e.g., 30 months) or more, or 3 years or more, or 3.5 years (e.g., 42 months) or more, or 4 years or more, or 4.5 years (e.g., 54 months) or more, or 5 years or more. For example, the relatively long period of time may be 6 months or more. In some cases, the relatively long period of time is a period of time that is no less than 9 months. In some cases, the relatively long period of time is no less than 1 year (e.g., 12 months). In some cases, the relatively long period of time is no less than 1.5 years (e.g., 18 months). In some cases, the relatively long period of time is no less than 2 years (e.g., 24 months). In some embodiments, the storage-stable composition is substantially stable at ambient temperature over a relatively long period of time, and the ambient temperature is, e.g., 20-40 °C, or 25-35 °C, or 25-30 °C. In some cases, the storage-stable composition is substantially stable over a relatively long period of time at a temperature lower than ambient temperature, and the temperature lower than ambient temperature is, e.g., 0-20 °C, or 0-15 °C, or 0-10 °C, or 2-8 °C.

In certain embodiments, the rAAV particles in the pharmaceutical composition remain stable or substantially stable (e.g., stable Vg, purity, IU, and/or biological activity) for at least 6 months when stored at a temperature ranging from about -80 °C to about 40 °C. In certain embodiments, the viral particle remains stable for at least 6 months when stored at a temperature ranging from about -80 °C to about 25 °C. In certain embodiments, the viral vector remains stable for at least 6 months when stored at a temperature ranging from about -20 °C to about 18 °C. In certain embodiments, the viral vector remains stable for at least 6 months when stored at a temperature ranging from about -20 °C to about 4 °C. In certain embodiments, the viral vector remains stable for at least 6 months when stored at a temperature ranging from about 4 °C to about 40 °C. In certain embodiments, the viral vector remains stable for at least 6 months when stored at a temperature ranging from about 4 °C to about 25 °C. In certain embodiments, the viral vector remains stable for at least 6 months when stored at a temperature ranging from about 4 °C to about 18 °C.

The present disclosure further provides an article of manufacture, which comprises a container containing the pharmaceutical composition, the liquid formulation, the freeze-dried formulation, or the reconstituted solution according to any one of the above. In some embodiments, the container may be, but is not limited to, a tubular injection vial made of neutral borosilicate glass.

The present disclosure further provides use of the pharmaceutical composition according to any one of the above, the liquid formulation according to any one of the above, the freeze-dried formulation according to any one of the above, the reconstituted solution according to any one of the above, or the article of manufacture according to any one of the above in the preparation of a medicament for treating a disease.

The present disclosure further provides a method for treating or preventing a disease, which comprises administering to a subject in need thereof an effective amount of the pharmaceutical composition according to any one of the above, the liquid formulation according to any one of the above, the freeze-dried formulation according to any one of the above, the reconstituted solution according to any one of the above, or the article of manufacture according to any one of the above.

The present disclosure further provides the pharmaceutical composition according to any one of the above, the liquid formulation according to any one of the above, the freeze-dried formulation according to any one of the above, the reconstituted solution according to any one of the above, or the article of manufacture according to any one of the above for use in treating a disease.

In some embodiments, the disease is selected from the group consisting of a disease associated with abnormal angiogenesis and a central nervous system disease or symptom.

In some embodiments, the disease is a disease associated with abnormal angiogenesis; in some embodiments, the disease associated with abnormal angiogenesis is an angiogenic ocular disease or cancer.

In some embodiments, the disease is a central nervous system disease or symptom; preferably, the central nervous system disease or symptom is a neurodegenerative disease or symptom, more preferably dyskinesia and sleep disorder, and most preferably Parkinson's disease.

## BRIEF DESCRIPTION OF THE DRAWINGS

FIG. 1A shows the amount of AADC protein expressed by each AAV plasmid after AADC codon optimization; FIG. 1B shows the amount of GDNF protein expressed by each AAV plasmid after GDNF codon optimization.

FIG. 2A shows immunofluorescence signals of AADC in the mouse striatum after transfection with AAV2 and AAV9 serotypes; FIG. 2B shows immunofluorescence coverage areas of AADC in the mouse striatum after transfection with AAV2 and AAV9 serotypes.

FIG. 3A shows the expression level of AADC in the striatum after injection of AAV2-02A and AAV9-02A; FIG. 3B shows the expression level of GDNF in the striatum after injection of AAV2-02A and AAV9-02A.

FIG. 4A shows the response of mice to L-DOPA (counterclockwise rotation) after injection of HRPDAAV02-AI (i.e., 02AI, the same applies hereinafter) and HRPDAAV03-DI (i.e., 03DI, the same applies hereinafter) drugs; FIG. 4B shows the response of mice to L-DOPA (contralateral forelimb utilization rate) after the injection of HRPDAAV02-AI and HRPDAAV03-DI drugs.

FIG. 5A shows the response of mice to L-DOPA (DOPA concentration at the non-administration side) after the injection of HRPDAAV02-AI and HRPDAAV03-DI drugs;

FIG. 5B shows the response of mice to L-DOPA (DOPA concentration at the administration side) after the injection of HRPDAAV02-AI and HRPDAAV03-DI drugs; FIG. 5C shows the response of mice to L-DOPA (DOPA concentration in blood) after the injection of HRPDAAV02-AI and HRPDAAV03-DI drugs; FIG. 5D shows the protection of dopamine neurons in mice after the injection of HRPDAAV02-AI and HRPDAAV03-DI drugs, in which the immunofluorescence signal density of dopamine nerve terminals is statistically analyzed.

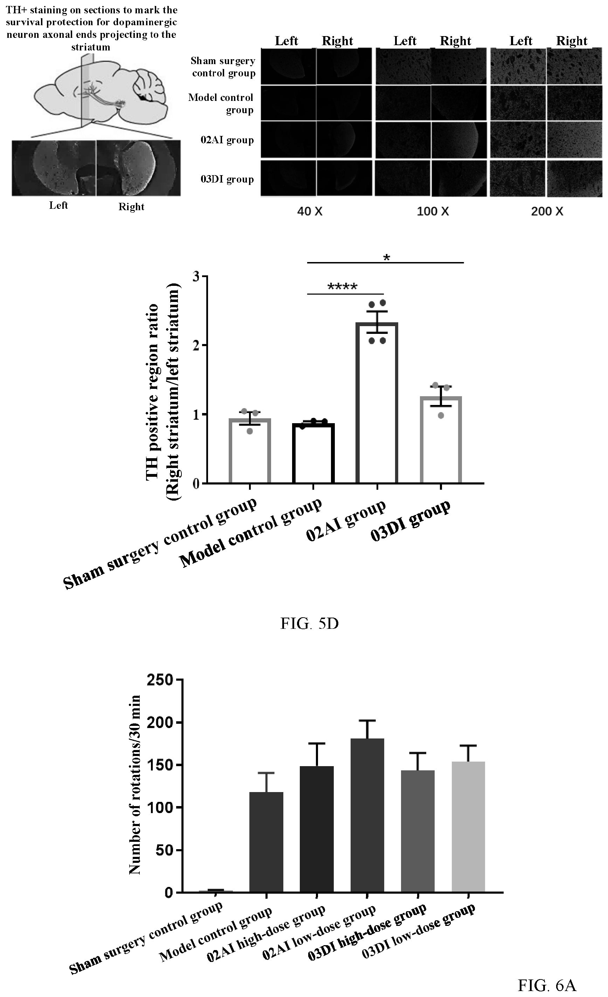

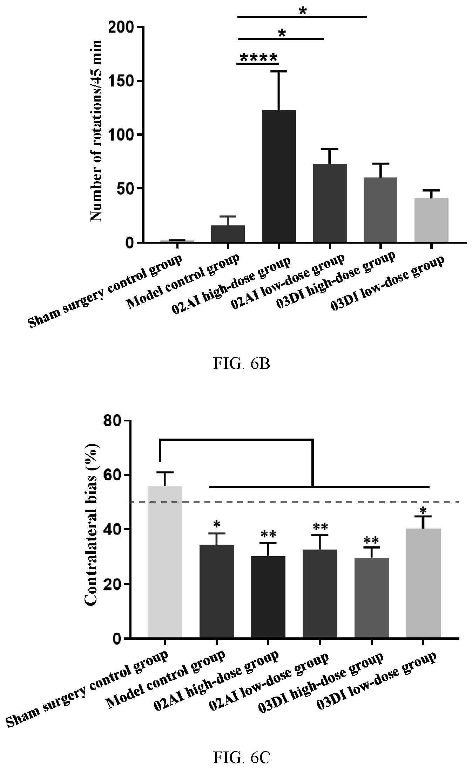

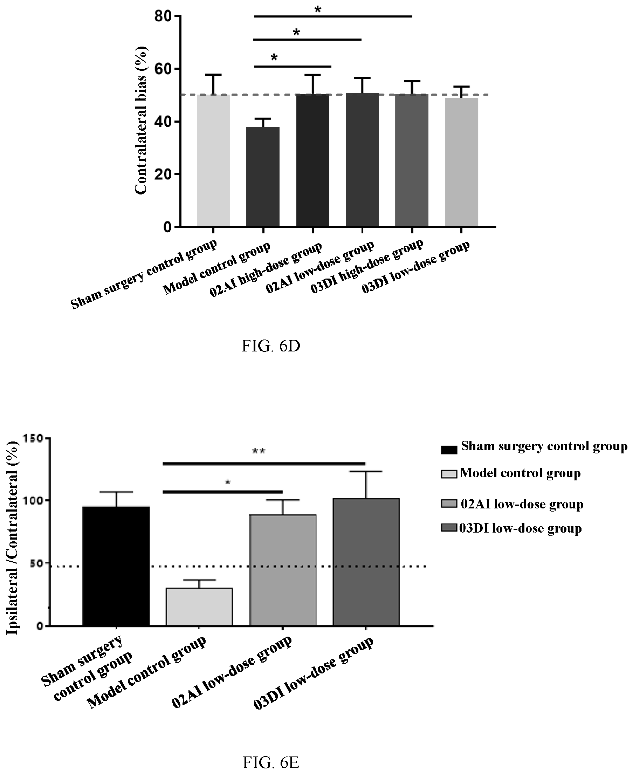

FIG. 6A shows the response of animals in each group to apomorphine after 6-OHDA modeling; FIG. 6B shows the response of rats to L-DOPA after the injection of HRPDAAV02-AI and HRPDAAV03-DI drugs (rotation test); FIG. 6C shows the test results of a rat model for which a cylinder test was established; FIG. 6D shows the response of rats to L-DOPA (contralateral forelimb utilization rate) after the injection of HRPDAAV02-IA and HRPDAAV03-DI drugs; 6E shows the test result of the metabolic capacity of striatum to L-DOPA (DOPA ratio of affected side/healthy side) after the administration of HRPDAAV02-IA and HRPDAAV03-DI; 6F shows the protection effect of HRPDAAV-03DI on DA neurons after administration.

In the present disclosure, **** represents $p < 0.0001$, *** represents $p$ between 0.0001 and 0.001, both representing extremely significant statistical differences; ** represents p between 0.001 and 0.01, indicating a very significant statistical difference; * represents p between 0.01 and 0.05, indicating a significant statistical difference.

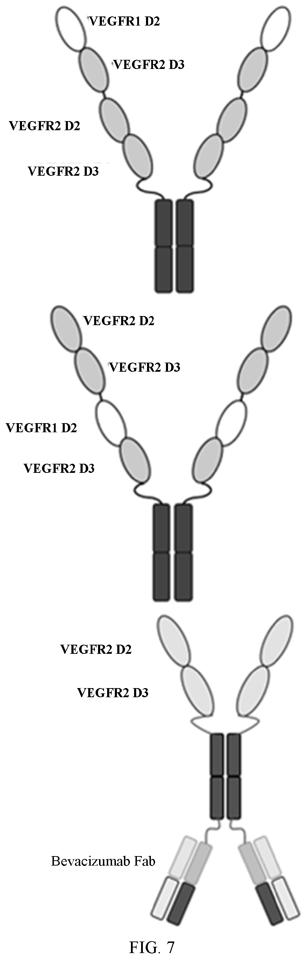

FIG. 7 shows a schematic diagram of molecular formats of VEGF-binding molecules.

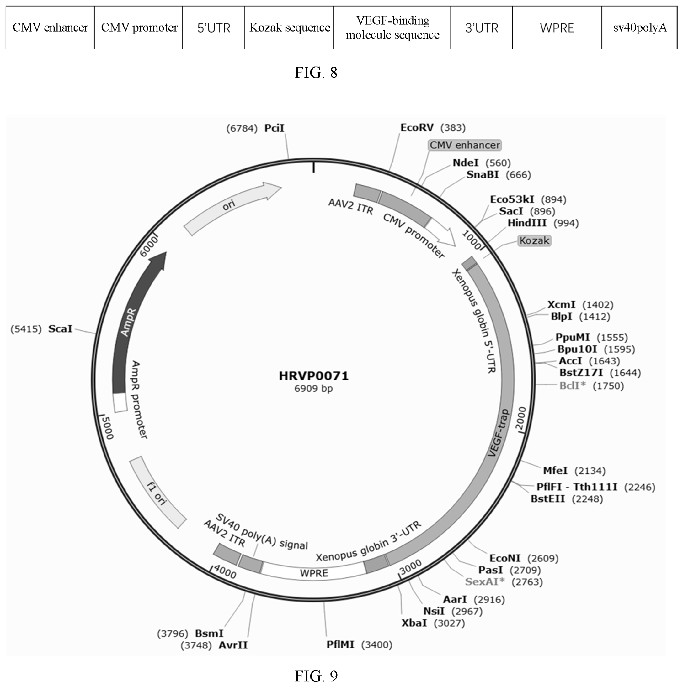

FIG. 8 shows a schematic diagram of the structure of a vector comprising a nucleic acid molecule expressing a VEGF-binding molecule.

FIG. 9 shows a schematic diagram of an AAV packaging plasmid.

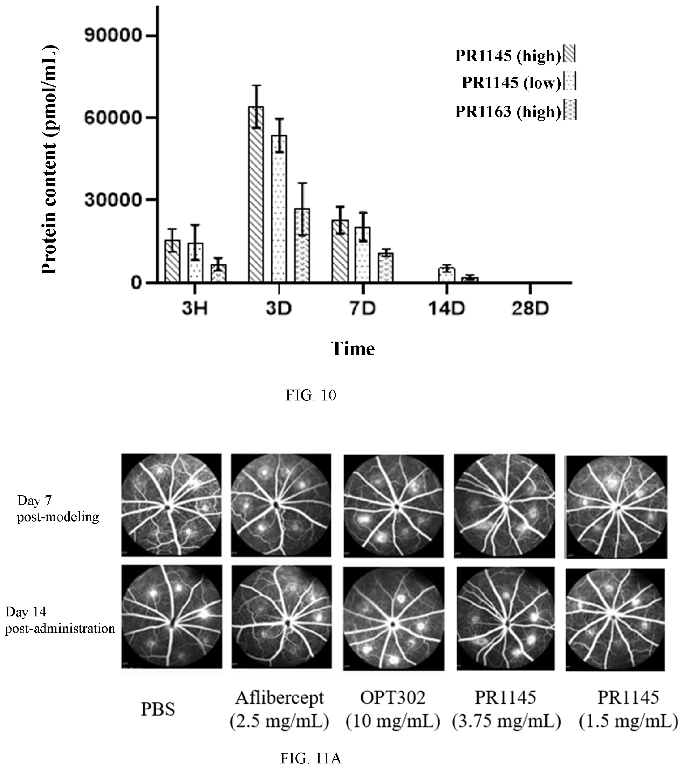

FIG. 10 shows the stability test results for VEGF-binding molecules in the vitreous humor of both eyes of rabbits.

FIG. 11A shows the fluorescein fundus angiography (FFA) results 7 days after rat CNV modeling and 14 days after rat CNV model administration.

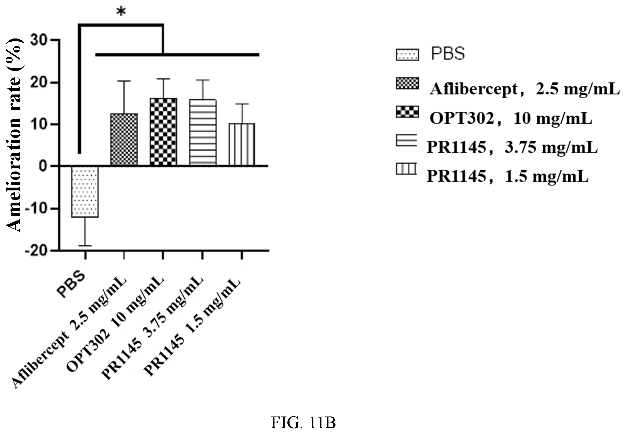

FIG. 11B shows the inhibition of choroidal neovascularization fluorescein sodium leakage 14 days after rat CNV model administration.

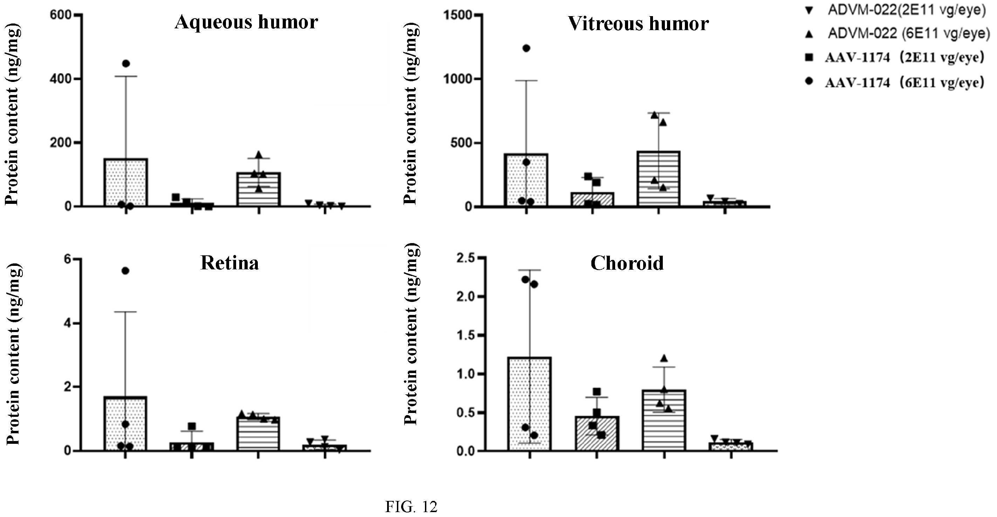

FIG. 12 shows the expression of AAV-1174 and ADVM-022 in the aqueous humor, the vitreous humor, the retina, and the choroid of rabbit eyes.

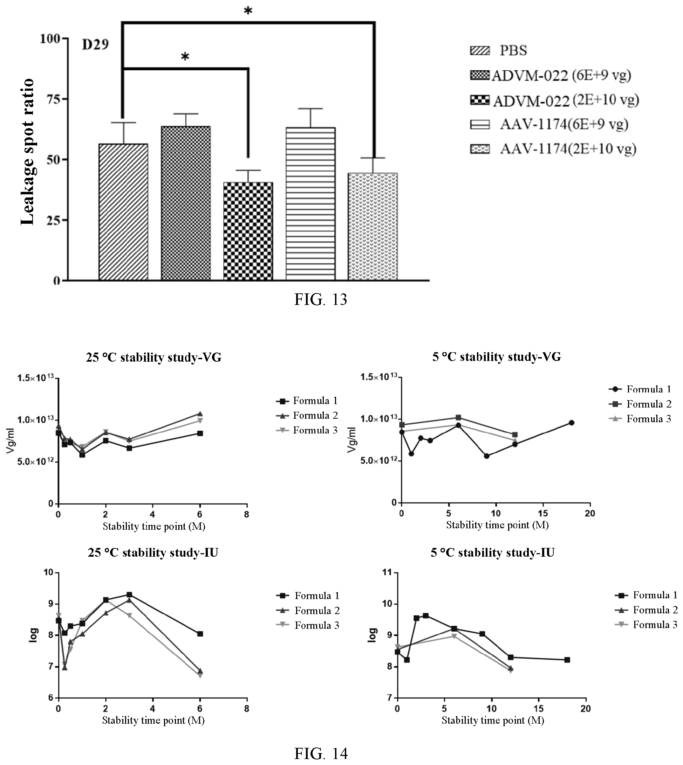

FIG. 13 shows the inhibition of choroidal neovascularization fluorescein sodium leakage after administration of AAV-1174 and ADVM-022 to a mouse CNV model.

FIG. 14 shows the results of the study on the effect of different formulations on virus stability.

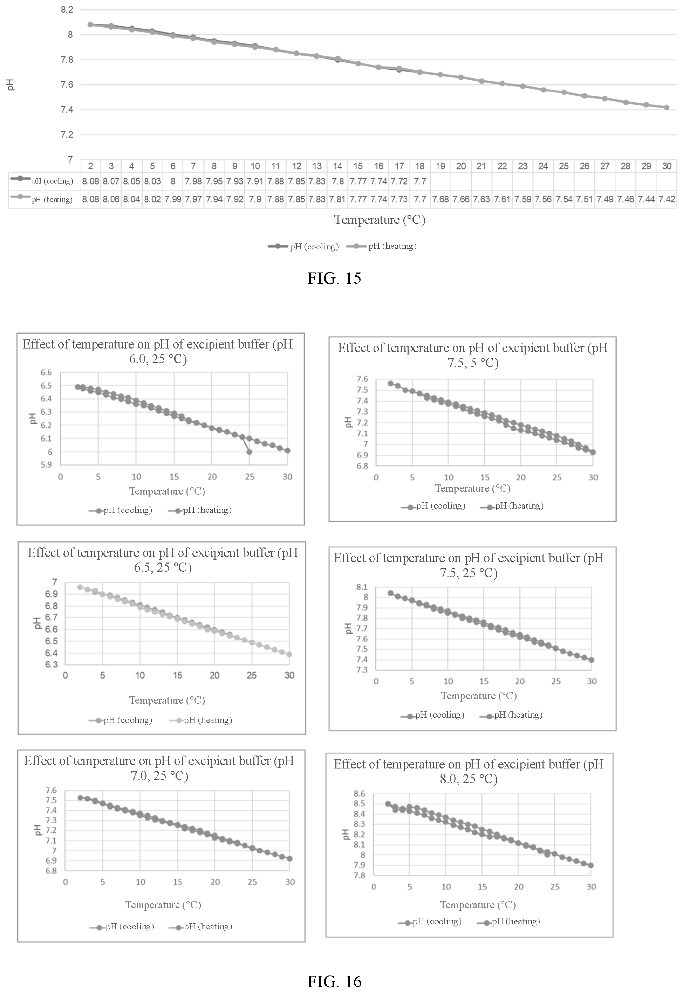

FIG. 15 shows the results of the study on the effect of temperature on the pH of a buffer working solution (pH 7.5, 27.2 °C).

FIG. 16 shows the results of the study on the effect of temperature on the pH of buffer working solutions at different pH values.

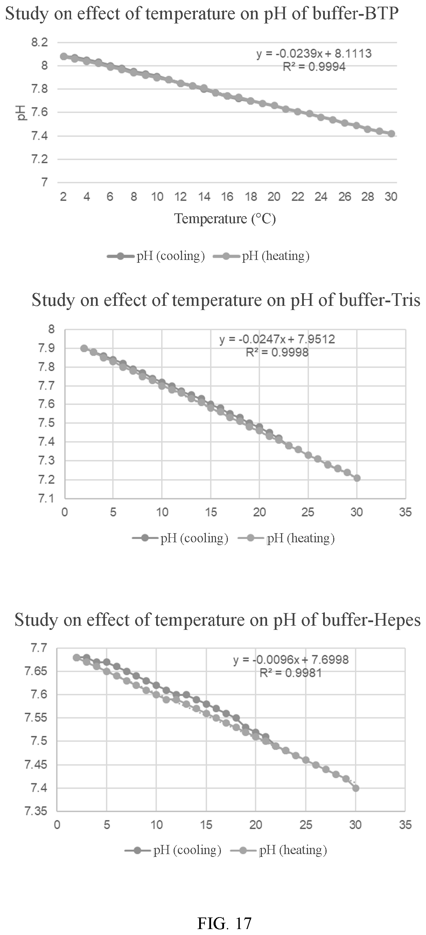

FIG. 17 shows the results of the study on the temperature sensitivity of pH of different buffer systems.

## DETAILED DESCRIPTION

**Terminology**

In order to facilitate the understanding of the present disclosure, certain technical and scientific terms are specifically defined below. Unless otherwise specifically defined herein, all other technical and scientific terms used herein have the meanings generally understood by those of ordinary skill in the art to which the present disclosure pertains.

"Buffer" refers to a buffer that resists changes in pH by the action of its acid-base conjugate components. Examples of buffers that maintain the pH within an appropriate range include acetate, succinate, gluconate, histidine, oxalate, lactate, phosphate, citrate, tartrate, fumarate, glycylglycine, and other organic acid buffers.

"Histidine buffer" is a buffer comprising histidine ions. Examples of histidine buffers include acetic acid-histidine, succinic acid-histidine, histidine-histidine hydrochloride, hydrochloric acid-histidine, sulfuric acid-histidine, and the like. Illustratively, the histidine-hydrochloride buffer is prepared with histidine and hydrochloric acid, or with histidine and histidine hydrochloride.

"Pharmaceutical composition" refers to a mixture containing one or more of the compounds described herein or physiologically/pharmaceutically acceptable salts or prodrugs thereof, and other chemical components, wherein the other components are, for example, physiologically/pharmaceutically acceptable carriers and excipients. The pharmaceutical composition is intended to promote administration to an organism and facilitate the absorption of the active ingredient so that it can exert its biological activity. As used herein, "pharmaceutical composition" and "formulation" are not mutually exclusive.

Unless otherwise specified, the solvent in the pharmaceutical composition described in the present disclosure in solution form is water.

The term "about" as used herein means that a numerical value is within an acceptable error range for the particular value determined by one of ordinary skill in the art, and the numerical value depends in part on how the value is measured or determined (i.e., the limits of the measurement system). For example, "about" may mean a standard deviation within 1 or more than 1 in each practice in the art. Alternatively, "about" or "substantially comprising" may mean a range of up to 20%. Furthermore, particularly for biological systems or processes, the term may mean up to an order of magnitude or up to 5-fold of a numerical value. Unless otherwise specified, when a specific value is provided in the present application and claims, the meaning of "about" or "substantially comprising" should be assumed to be within an acceptable error range for that specific value.

The pharmaceutical composition described in the present disclosure can achieve the effect of being stable: the antibody in the pharmaceutical composition substantially retains its physical and/or chemical stability and/or biological activity after storage; preferably, the pharmaceutical composition substantially retains its physical and chemical stability as well as its biological activity after storage. The storage period is generally selected based on a predetermined shelf life of the pharmaceutical composition. There are a variety of analytical techniques currently available for measuring protein stability, and the stability after storage for a selected period of time at a selected temperature can be measured.

A stable pharmaceutical antibody formulation is one in which no significant change is observed under the following conditions: stored at refrigeration temperature (2-8 °C) for at least 3 months, preferably 6 months, more preferably 1 year, and even more preferably at most 2 years. In addition, stable liquid formulations include liquid formulations that exhibit desirable features after storage at 25 °C for periods including 1 month, 3 months, and 6 months, or storage at 40 °C for periods including 1 month. Typical acceptable criteria for stability are as follows: typically, no more than about 10%, preferably no more than about 5%, of antibody monomer is degraded as measured by SEC-HPLC. The pharmaceutical antibody formulation is colorless or yellow, or clear to slightly opalescent, by visual analysis. The concentration, pH, and osmolality of the formulation have a change of no more than $\pm 10\%$. Typically, clippings of no more than about 10%, preferably no more than about 5%, are observed. Typically, aggregation of no more than about 10%, preferably no more than about 5%, is formed.

An antibody "retains its physical stability" in a pharmaceutical formulation if it shows no significant increase in aggregation, precipitation and/or denaturation upon visual inspection of color and/or clarity, or as determined by UV light scattering, size exclusion chromatography (SEC), and dynamic light scattering (DLS). Changes in protein conformation can be assessed by fluorescence spectroscopy (which determines the protein tertiary structure) and by FTIR spectroscopy (which determines the protein secondary structure).

An antibody "retains its chemical stability" in a pharmaceutical formulation if it shows no significant chemical change. Chemical stability can be evaluated by detecting and quantifying chemically changed proteins. Degradation processes that often change the chemical structure of proteins include hydrolysis or clipping (assessed by methods such as size exclusion chromatography and SDS-PAGE), oxidation (assessed by methods such as peptide mapping in combination with mass spectroscopy or MALDI/TOF/MS), deamidation (assessed by methods such as ion-exchange chromatography, capillary isoelectric focusing, peptide mapping, and isoaspartic acid determination), and isomerization (assessed by isoaspartic acid content determination, peptide mapping, etc.).

"AAV" is an abbreviation for adeno-associated virus, and can be used to refer to the virus itself or derivatives thereof. Unless otherwise indicated, this encompasses all AAV subtypes as well as naturally occurring and recombinant forms. "AAV" includes, but is not limited to, AAV type 1 (AAV1), AAV type 2 (AAV2), AAV type 3 (AAV3), AAV type 4 (AAV4), AAV type 5 (AAV5), AAV type 6 (AAV6), AAV type 7 (AAV7), AAV type 8 (AAV8), AAV type 9 (AAV9), AAV type 10 (AAV10), AAVrh type 10 (AAVrh10), as well as avian AAV, bovine AAV, canine AAV, equine AAV, primate AAV, non-primate AAV and ovine AAV of different species.

AAV is a nonpathogenic parvovirus composed of a 4.7 kb single-stranded DNA genome within a non-enveloped icosahedral capsid. The genome contains three open reading frames (ORFs) flanked by inverted terminal repeats (ITRs) that function as the viral origin of replication and packaging signal. The Rep ORF encodes four nonstructural proteins which play roles in viral replication, transcriptional regulation, site-specific integration, and virion assembly. The Cap ORF encodes three structural proteins (VP1-3) which assemble to form a 60-mer viral capsid. Finally, an ORF present as an alternate reading frame within the cap gene produces the assembly-activating protein (AAP), a viral protein that localizes AAV capsid proteins to the nucleus and functions in the capsid assembly process.

The genomic sequences of various serotypes of AAV, as well as the sequences of the native terminal repeats (ITRs), Rep proteins, and capsid subunits are known in the art. Such sequences can be found in the literature or in public databases such as GenBank. See, e.g., GenBank accession numbers NC_002077.1 (AAV1), AF063497.1 (AAV1), NC_001401.2 (AAV2), AF043303.1 (AAV2), J01901.1 (AAV2), U48704.1 (AAV3), NC_001729.1 (AAV3), NC_001829.1 (AAV4), U89790.1 (AAV4), NC_006152.1 (AAV5), AF085716.1 (AAV5), AF028704.1 (AAV6), NC_006260.1 (AAV7), AF513851.1 (AAV7), AF513852.1 (AAV8), NC_006261.1 (AAV8), and AY530579.1 (AAV9). See also Srivistava et al. (1983) J. Virology 45:555; Chiorini et al. (1998) J. Virology 71:6823; Chiorini et al. (1999) J. Virology 73:1309; Bantel-Schaal et al. (1999) J. Virology 73:939; Xiao et al. (1999) J. Virology 73:3994; Muramatsu et al. (1996) Virology 221:208; Shade et al., (1986) J. Virol. 58:921; Gao et al.

(2002) Proc. Nat. Acad. Sci. USA 99:11854; Moris et al. (2004) Virology 33:375-383; and WO00/28061, WO99/61601, WO98/11244, US6156303, WO2012145601A, WO2017197355A, and WO2018022905A. The above are incorporated in the present disclosure by reference in their entirety.

For the GH loop or loop IV of the AAV capsid, see, e.g., van Vliet et al. (2006) Mol. Ther. 14:809; Padron et al. (2005) J. Virol. 79:5047; and Shen et al. (2007) Mol. Ther. 15:1955. "AAV viral particle" or "AAV virion" refers to a viral particle composed of at least one AAV capsid protein and an encapsidated AAV polynucleotide.

"rAAV" refers to recombinant adeno-associated virus. "Recombinant" as applied to a polynucleotide means that the polynucleotide is the product of various combinations of cloning, restriction or ligation steps, and other processes that result in a construct that is different from a polynucleotide found in nature. A recombinant virus is a viral particle comprising a recombinant polynucleotide.

If an "AAV virion" comprises a heterologous polynucleotide (i.e., a polynucleotide other than a wild-type AAV genome, e.g., a gene product to be delivered to a target cell (such as a transgene or RNAi), it is typically referred to as a "recombinant AAV (rAAV) virion" or an "rAAV viral particle" or an "rAAV vector virus". In general, the heterologous polynucleotide is flanked by at least one, and generally by two AAV inverted terminal repeats (ITRs).

"rAAV vector" encompasses rAAV virions, which include rAAV polynucleotides; and also encompasses polynucleotides encoding rAAV (e.g., a single-stranded polynucleotide encoding rAAV (ss-rAAV); a double-stranded polynucleotide encoding rAAV (ds-rAAV), e.g., plasmids encoding rAAV; and the like).

"AAV variant", "AAV mutant" or "capsid-variant rAAV" refers to a viral particle composed of: (a) a variant AAV capsid protein, wherein the variant AAV capsid protein comprises at least one amino acid difference (e.g., amino acid substitution, insertion, or deletion) relative to a corresponding parental AAV capsid protein, wherein the AAV capsid protein differs from or does not correspond to the amino acid sequence of a naturally occurring AAV capsid protein; and optionally, (b) comprising a heterologous polynucleotide encoding a heterologous gene product, wherein the variant AAV capsid protein confers increased binding to heparan or heparan sulfate proteoglycan as compared to the binding by an AAV virion comprising a corresponding parental AAV capsid protein.

"Packaging" refers to a series of intracellular events that result in the assembly and encapsidation of an AAV particle.

The "rep" and "cap" genes refer to polynucleotide sequences encoding replication and encapsidation proteins of adeno-associated viruses. AAV rep and cap are referred to herein as AAV "packaging genes".

"Helper virus" refers to a virus that allows an AAV (e.g., wild-type AAV) to be replicated and packaged by a mammalian cell. A variety of helper viruses for AAVs are known in the art, including adenoviruses, herpesviruses and poxviruses such as vaccinia. Adenoviruses encompass a number of different subgroups, although adenovirus type 5 of subgroup C is most commonly used. Viruses of the herpes family include, for example, herpes simplex viruses (HSV) and Epstein-Barr viruses (EBV), as well as cytomegaloviruses (CMV) and pseudorabies viruses (PRV). Many adenoviruses, herpesviruses and the like of human, non-human mammalian and avian origin are known and available from institutions such as the ATCC.

"Helper virus function" or "helper function" refers to a function encoded in a helper virus genome which allows AAV replication and packaging (in conjunction with other requirements for replication and packaging described herein). In the present disclosure, "helper virus function" can be provided in a number of ways, including by providing a helper virus or providing, for example, polynucleotide sequences encoding the requisite functions to a producer cell in transport.

An "infectious" virus or viral particle is one that comprises a suitably assembled viral capsid and is capable of delivering a polynucleotide component into a cell for which the viral species is tropic. The term does not necessarily imply that the virus has any replication capacity. Assays for counting infectious viral particles are well-known in the art. Viral infectivity can be expressed as the ratio of infectious viral particles to total viral particles. Methods of determining the ratio of infectious viral particles to total viral particles are known in the art. See, e.g., Grainger et al. (2005) Mol. Ther. 11:S337 (describing a $TCID_{50}$ infectious titer assay); and Zolotukhin et al. (1999) Gene Ther. 6:973.

"Tropism" or "specificity" refers to the preferential targeting of cells of a particular host species or a particular type of cells within a host species by a virus (e.g., an AAV). For example, a virus that can infect cells of the heart, lung, liver, and muscle has a broader (i.e., increased) tropism relative to a virus that can infect only lung and muscle cells. Tropism can also include the dependence of a virus on particular types of cell surface molecules of the host. For example, some viruses can only infect cells with surface glycosaminoglycans, while other viruses can only infect cells with sialic acid (such dependence can be tested by using various cell lines lacking particular classes of molecules as potential host cells for viral infection). In some cases, the tropism of a virus describes the relative preferences of the virus. For example, a first virus may be able to infect all cell types but is more successful in infecting those cells with surface glycosaminoglycans. A second virus can be considered to have similar (or identical) tropism as the first virus if the second virus also prefers the same characteristics (for example, the second virus is also more successful in infecting those cells with surface glycosaminoglycans), even if the absolute transduction efficiencies are not similar. For example, the second virus may be more effective than the first virus in infecting every given cell type tested, but if the relative preferences are similar (or identical), the second virus can still be considered to have similar (or identical) tropism as the first virus. In some embodiments, the tropism of a virion comprising the variant AAV capsid protein of the present disclosure is not altered relative to a naturally occurring virion. In some embodiments, the tropism of a virion comprising the variant AAV capsid protein of the present disclosure is expanded (i.e., broadened) relative to a naturally occurring virion. In some embodiments, the tropism of a virion comprising the variant AAV capsid protein of the present disclosure is reduced relative to a naturally occurring virion.

"Polynucleotide" refers to a polymeric form of nucleotides of any length, or an analog thereof, including deoxyribonucleotides or ribonucleotides. A polynucleotide may comprise modified nucleotides, such as methylated nucleotides and nucleotide analogs, and may be interrupted by non-nucleotide components. Modifications to the nucleotide structure may be made before or after the assembly of the polymer. Polynucleotides can refer interchangeably to double-stranded and single-stranded molecules. Unless otherwise specified, the polynucleotide of the present disclosure encompasses the double-stranded form and two complementary single-stranded forms that constitute the double-stranded form.

"Homology" or "identity" refers to the sequence similarity between two polynucleotide sequences or two polypeptides. When positions in two compared sequences are occupied by identical nucleotides or amino acid monomer subunits, e.g., if the position of each of two DNA molecules is occupied by an identical nucleotide, the molecules are homologous at that position. The percent homology between two sequences is a function of the number of matching or homologous positions shared by the two sequences divided by the number of compared positions $\times$ 100%. For example, if 6 out of 10 positions are matched or homologous when two sequences are optimally aligned, the two sequences are 60% homologous. Sequence similarity can be determined in a number of different manners. To determine sequence identity, sequences can be aligned using the methods and computer programs, including BLAST, available at the Internet website ncbi.nlm.nih.gov/BLAST/. Another alignment algorithm is FASTA, which is available in the Genetics Computing Group (GCG) package, from Madison, Wisconsin, USA, a wholly owned subsidiary of Oxford Molecular Group, Inc. Other alignment techniques are described in Methods in Enzymology, vol. 266: Computer Methods for Macromolecular Sequence Analysis (1996), ed. Doolittle, Academic Press, Inc., a division of Harcourt Brace & Co., SanDiego, California, USA. Of particular interest are alignment programs that permit gaps in the sequence. The Smith-Waterman is one type of algorithm that permits gaps in sequence alignments. See Meth. Mol. Biol. 70:173-187 (1997). The GAP program using the Needleman and Wunsch alignment method can also be used to align sequences. See J. Mol. Biol. 48:443-453 (1970).

"Gene" refers to a polynucleotide comprising at least one open reading frame that is capable of encoding a particular gene product during transcription and sometimes after translation. "Gene" or "coding sequence" refers to an *in vitro* or *in vivo* nucleotide sequence that encodes a gene product. In some cases, a gene consists of or consists essentially of a coding sequence, i.e., a sequence that encodes a gene product. In other cases, a gene includes additional non-coding sequences. For example, a gene may or may not comprise regions preceding and following the coding region (e.g., 5' UTR, 3' UTR, and intervening sequences (introns) between individual coding segments (exons)).

"Gene product" is a molecule resulting from expression of a particular gene, e.g., a polypeptide, an aptamer, an interfering RNA, or an mRNA. In some embodiments, the "gene product" is a polypeptide, a peptide, a protein, or an interfering RNA, including short interfering RNA (siRNA), miRNA or small-hairpin RNA (shRNA). In some specific embodiments, the gene product is a therapeutic gene product, such as a therapeutic polypeptide. The therapeutic gene product confers beneficial effects on the cell, tissue, or mammal in which it is present. The beneficial effects include amelioration of a sign or symptom of a condition or disease, prevention or inhibition of a condition or disease, or conferral of a desired characteristic. When a gene encodes a polypeptide, "gene product" and "product expressed from the target gene" are used interchangeably.

"Polypeptide", "peptide", and "protein" refer to polymers of amino acids of any length. The terms also encompass modified amino acid polymers, for example, disulfide bond formation, glycosylation, lipidation, phosphorylation, or conjugation with a labeling component.

"Regulatory element" or "regulatory sequence" is a nucleotide sequence involved in an interaction of molecules that contributes to the functional regulation of a polynucleotide, including replication, duplication, transcription, splicing, translation, or degradation of the polynucleotide. The regulation may affect the frequency, speed, or specificity of the process, and may be enhancing or inhibitory in nature. Known control elements include, for example, transcriptional regulatory sequences such as promoters and enhancers. A promoter is a DNA region capable of binding to RNA polymerase and initiating transcription of a coding region usually located downstream (in the 3' direction) of the promoter under certain conditions.

"Expression vector" is a vector comprising a region encoding a target gene product, and is used for achieving the expression of the gene product in an intended target cell. The vector includes a polynucleotide encoding the gene product of interest. An expression vector also comprises control elements operatively linked to the coding region to facilitate the expression of the gene product in the target. The combination of control elements, such as promoters, enhancers, UTRs and miRNA targeting sequences, and one or more genes to which they are operably linked for expression is sometimes referred to as an "expression cassette". Many expression cassettes are known and available in the art, or can be readily constructed from components available in the art.

"Operatively linked" or "operably linked" refers to a juxtaposition of genetic elements, wherein the elements are in a relationship permitting them to operate in an intended manner. For example, a promoter is operatively linked to a coding polynucleotide sequence if the promoter helps initiate the transcription of the coding polynucleotide sequence. There may be intervening nucleic acid residues between the promoter and the coding polynucleotide sequence so long as this functional relationship is maintained.

"Administering" or "introducing" refers to delivering a vector for recombinant gene or protein expression to a cell, to cells and/or organs of a subject, or to a subject. Such administering or introducing may take place *in vivo, in vitro* or *ex vivo.* A vector for expressing a gene product can be introduced into a cell by:

transfection, which typically means inserting heterologous DNA into a cell by physical means (e.g., calcium phosphate transfection, electroporation, microinjection, or lipofection); infection, which typically refers to introduction by an infectious agent, i.e., a virus; or transduction, which typically means stably infecting a cell with a cell or transferring genetic material from one microorganism to another by a viral agent (e.g., a bacteriophage). "Transformation" is typically used to refer to bacteria comprising heterologous DNA or cells that express an oncogene and have been switched to a continuous growth mode, such as tumor cells. A vector used to "transform" a cell may be a plasmid, a virus, or other vehicles. A cell is generally referred to as "transduced", "infected", "transfected", or "transformed" according to the means used for administering, introducing, or inserting heterologous DNA (i.e., the vector) into the cell. "Transduce", "transfect", and "transform" are used interchangeably herein regardless of the method of introducing heterologous DNA.

"Host cell" refers to a cell which has been transduced, infected, transfected, or transformed with a vector. The term encompasses the original transduced, infected, transfected, or transformed cell and progeny thereof. A vector may be a plasmid, a viral particle, a phage, or the like. Culture conditions such as temperature and pH are apparent to those skilled in the art. "Treat", "treating", or "treatment" is generally used to mean obtaining a desired pharmacological and/or physiological effect. The effect may be prophylactic in terms of completely or partially preventing a disease or a symptom thereof, e.g., reducing the possibility that the disease or symptom thereof occurs in the subject, and/or may be therapeutic in terms of partially or completely curing a disease and/or an adverse effect arising from the disease. "Treat", "treating", or "treatment" covers any treatment of a disease in a mammal, and includes: (a) preventing the disease from occurring in a subject who may be predisposed to the disease but has not yet been diagnosed as having it; (b) inhibiting or arresting the progression of the disease; or (c) relieving the disease (or a symptom arising therefrom) or causing regression of the disease. The therapeutic agent may be administered before, during or after the occurring of the disease or injury. The treatment of ongoing disease, where the treatment stabilizes or reduces the undesirable clinical symptoms of the patient, is of particular interest. Some preferred protocols are to perform such treatment prior to complete loss of function in the affected tissue. Some preferred protocols are to administer the treatment of the present disclosure during the symptomatic stage of the disease and, in some cases, after the symptomatic stage of the disease.

"Effective amount" comprises an amount sufficient to ameliorate or prevent a symptom or sign of a medical disorder. An effective amount also means an amount sufficient to allow or facilitate diagnosis. The effective amount for a particular subject may vary depending on factors such as the disorder to be treated, the general health of the subject, the method and dose of administration, and the severity of side effects. An effective amount may be the maximum dose or administration regimen to avoid significant side effects or toxic effects. "Retinal cell" in the present disclosure may refer to any one of the cell types that comprise the retina, such as retinal ganglion cells; amacrine cells; horizontal cells; bipolar cells; photoreceptor cells including rod and cone cells; Miiller glial cells; astrocytes (such as retinal astrocytes); and retinal pigment epithelial cells.

"Individual", "subject", and "patient" are used interchangeably herein, and include, but are not limited to, humans and non-human primates, e.g., simians, humans, and other mammalian animals (such as horses, sheep, goats, dogs, cats and rodents (such as mice and rats)); humans are preferred.

In the present disclosure, "polypeptide" and "protein" are used interchangeably.

Examples

The present disclosure is further described below with reference to examples, but these examples are not intended to limit the scope of the present disclosure. In the examples of the present disclosure, experimental methods without specific conditions specified were generally conducted under conventional conditions, such as those outlined in Antibodies: A Laboratory Manual and Molecular Cloning: A Laboratory Manual by Cold Spring Harbor Laboratory, or under conditions recommended by the manufacturers of the starting materials or commercial products. Reagents without specific sources indicated were commercially available conventional reagents.

**Example 1. Design of AADC and GDNF Polynucleotide Structures**

In this example, sequence design and engineering of polynucleotides encoding AADC and GDNF were performed for transduction of cells or for *in vivo* transduction in humans and animals. The sequences could be inserted into plasmid, lentivirus, adenovirus, mRNA-LNP, and adeno-associated virus (AAV) vectors, for example, to achieve *in vivo* and *in vitro* transduction by a recombinant adeno-associated virus (rAAV). The specific sequences between the ITRs of the rAAV plasmid structure are shown in Table 1, the enhancer used may include, for example, a CMV enhancer, and the promoter may include, for example, a CMV promoter and a CBA promoter. The sequences in Table 2 were all non-codon-optimized.

Table 1. Engineered AADC/GDNF polynucleotide sequences

| rAAV plasmid name | Name in drawings | Insertion of target gene | Sequence No. | Structural characteristic |
|---|---|---|---|---|
| HRPDAAV01-A | 01A | AADC | SEQ ID NO:13 | Single-gene expression framework |
| HRPDAAV01-B | 01B | AADC | SEQ ID NO:14 | Single-gene expression framework |
| HRPDAAV01-C | 01C | AADC | SEQ ID NO:15 | Single-gene expression framework |
| HRPDAAV02-A | 02A | AADC/GDNF | SEQ ID NO:16 | Double-gene single-promoter framework |
| HRPDAAV02-B | 02B | AADC/GDNF | SEQ ID NO:17 | Double-gene single-promoter framework |
| HRPDAAV03-A | 03A | AADC/GDNF | SEQ ID NO:18 | Double-gene double-promoter framework |
| HRPDAAV03-B | 03B | AADC/GDNF | SEQ ID NO:19 | Double-gene double-promoter framework |
| HRPDAAV03-C | 03C | AADC/GDNF | SEQ ID NO:20 | Double-gene double-promoter framework |
| HRPDAAV03-D | 03D | AADC/GDNF | SEQ ID NO:21 | Double-gene double-promoter framework |
| HRPDAAV03-E | 03E | AADC/GDNF | SEQ ID NO:22 | Double-gene double-promoter framework |
| HRPDAAV03-F | 03F | AADC/GDNF | SEQ ID NO:23 | Double-gene double-promoter framework |
| (Note: AADC/GDNF indicates both AADC and GDNF are comprised, and the same applies hereinafter) | | | | |

**Example 2. Codon Optimization of AADC and GDNF**

1. In this example, 4 codon-optimized polynucleotide sequences were separately designed for AADC and GDNF. The wild-type polynucleotide sequence of AADC is set forth in SEQ ID NO: 3, the codon-optimized sequences AADC01, AADC02, AADC03, and AADC04 are set forth in SEQ ID NOs: 4-7, respectively, and the sequence identities of SEQ ID NOs: 4-7 to SEQ ID NO: 3 are 80.83%, 81.21%, 79.18%, and 79.63%, respectively. The wild-type polynucleotide sequence of GDNF is set forth in SEQ ID NO: 8, the codon-optimized sequences GDNF01, GDNF02, GDNF03, and GDNF04 are set forth in SEQ ID NOs: 9-12, respectively, and the sequence identities of SEQ ID NOs: 9-12 to SEQ ID NO: 8 are 75.13%, 73.99%, 77.52%, and 77.83%, respectively.

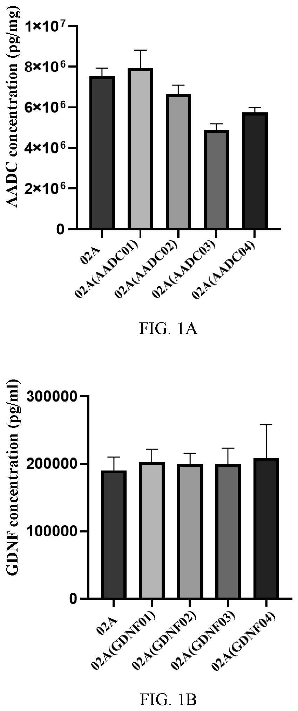

The AADC sequence in HRPDAAV02-A was replaced by AADC01, AADC02, AADC03, and AADC04 separately to obtain HRPDAAV02-A(AADC01), HRPDAAV02-A(AADC02), HRPDAAV02-A(AADC03), and HRPDAAV02-A(AADC04), respectively. The GDNF sequence in HRPDAAV02-A was replaced by GDNF 01, GDNF 02, GDNF 03, and GDNF 04 separately to obtain HRPDAAV02-A(GDNF01), HRPDAAV02-A (GDNF02), HRPDAAV02-A(GDNF03), and HRPDAAV02-A(GDNF04), respectively. Screening was then performed to obtain AADC and GDNF codon-optimized sequences with the strongest expression and function, AADC01 and GDNF04. The results are shown in FIGs. 1A and 1B.

AADC and GDNF in HRPDAAV02-A and HRPDAAV03-D were replaced by AADC01 (SEQ ID NO: 3) and GDNF04 (SEQ ID NO: 12) to obtain HRPDAAV02-AI (SEQ ID NO: 24) and HRPDAAV03-DI (SEQ ID NO: 25). HRPDAAV02-AI and HRPDAAV03-DI corresponded to 02AI and 03DI, respectively. The structures of the two are shown in Table 2. The codon-optimized sequence of HRPDAAV02-AI may also be SEQ ID NO: 140, and the codon-optimized sequence of HRPDAAV03-DI may also be SEQ ID NO: 141.

Table 2. HRPDAAV02-AI and HRPDAAV03-DI sequence structures

| HRPDAAV02-AI (i.e., 02AI) | | | | |
|---|---|---|---|---|
| Region | Starting position | Terminating position | Length of region | Sequence No. |
| 5'ITR | 1 | 141 | 141 | SEQ ID NO:26 |
| CMV enhancer | 157 | 442 | 286 | SEQ ID NO:27 |
| CBA promoter | 444 | 721 | 278 | SEQ ID NO:28 |
| Hybrid intron | 722 | 949 | 228 | SEQ ID NO:29 |
| AADC01 | 979 | 2418 | 1440 | SEQ ID NO:4 |
| P2A | 2419 | 2475 | 57 | SEQ ID NO:48 |
| GDNF04 | 2476 | 3111 | 636 | SEQ ID NO:12 |
| WPRE | 3118 | 3706 | 589 | SEQ ID NO:34 |
| SV40 poly(A) | 3707 | 3828 | 122 | SEQ ID NO:32 |
| 3'ITR | 3848 | 3988 | 141 | SEQ ID NO:31 |
| HRPDAAV03-DI (i.e., 03DI) | | | | |
| Region | Starting position | Terminating position | Length of region | Sequence No. |
| 5'ITR | 1 | 141 | 141 | SEQ ID NO:26 |
| CMV enhancer 2 | 151 | 454 | 286 | SEQ ID NO:37 |
| CMV promoter | 455 | 657 | 203 | SEQ ID NO:35 |
| β-globin intron | 658 | 1230 | 573 | SEQ ID NO:38 |
| GDNF04 | 1237 | 1872 | 636 | SEQ ID NO:12 |
| PA75 polyA | 1873 | 1947 | 75 | SEQ ID NO:36 |
| CMV enhancer | 1594 | 2239 | 304 | SEQ ID NO:27 |
| CBA promoter | 2241 | 2518 | 278 | SEQ ID NO:28 |
| Hybrid intron | 2519 | 2746 | 228 | SEQ ID NO:29 |
| AADC01 | 2746 | 4218 | 1443 | SEQ ID NO:4 |
| SV40 PolyA | 4225 | 4346 | 122 | SEQ ID NO:32 |
| 3'ITR | 4366 | 4506 | 141 | SEQ ID NO:31 |

**2.** HRPDAAV-02A was packaged and purified by the AAV2 serotype (with the amino acid sequence set forth in SEQ ID NO: 41) or AAV9 serotype (with the amino acid sequence set forth in SEQ ID NO: 42) to obtain AAV2-02A and AAV9-02A.

SD male rats were first anesthetized with 50 mg/kg Zoletil (i.p.), and the animals were bound to a cerebral stereotaxic apparatus; the skin was incised along the median sagittal direction of the cranium to expose the bregma; position 1: AP-0.0 mm, ML-2.6 mm and DV-4.7 mm and position 2: AP0.0 mm, ML-2.6 mm and DV-4.7 mm in the right striatal region were positioned. After the skull was opened by a dental drill, a micro-syringe was inserted into each site, and 2 μL of a test drug was automatically injected using a micro-peristaltic pump (0.2 μL/min). After the injection was completed, the needle was retained for 10 min and then slowly withdrawn, and the wound was sutured. Each rat was injected subcutaneously with 0.3 mL of levofloxacin injection to prevent infections. The rats were fed for another 8 weeks after the operation.

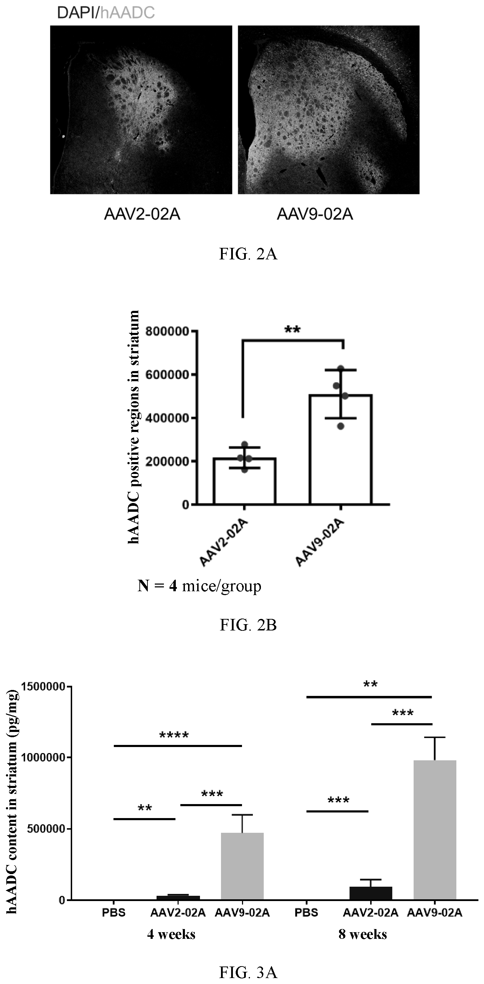

1) Cardiac perfusion was performed on the rats with 4% paraformaldehyde, the brain was collected and fixed for no less than 24 h, and the sample was transferred into a 30% sucrose solution for full sediment and dehydration. The striatum coronal was continuously sectioned with a section thickness of 30 $\mu$m using a freezing microtome, and the sections were placed into a cryopreservation solution and stored in a refrigerator at 4 °C. The brain sections were washed three times in PBS, treated with 0.3% TritonX100 for 10 min, and blocked in PBS containing 10% goat serum for 1 h, the primary antibody was a rabbit anti-AADC antibody (Absin, abs110350) (1:1000), and the mixture was incubated overnight at 4 °C. The next day, the sections were washed 3 times with PBS for 5 min each. The sections were incubated with a donkey anti-rabbit IgGAlexaFluor488 antibody (1:1500) and a nuclear dye DAPI (1:15000) at room temperature for 1 h, and then the sections were washed 3 times with PBS and mounted with a Dako antifade mountant. The sections were observed under an Olympus fluorescent microscope and photographed, as shown in FIG. 2A and FIG. 2B. The staining results show that the fluorescence area of AADC was significantly larger after the injection of AAV9-02A compared to AAV2-02A (P < 0.01, AAV2-02A vs AAV9-02A), demonstrating that AAV9 has higher expression efficiency in the striatal region than that of AAV2.

2) The rats were fed for 4 weeks or 8 weeks after the brain positioning operation, and then the striatum tissue at the injection side was directly extracted by decapitation. The tissue was weighed and cryopreserved in a refrigerator at -80 °C. Pre-cooled PBS (1 mg of tissue:10 $\mu$L of PBS) was added during tissue homogenization, and after a sample was thoroughly homogenized using a pre-cooled tissue homogenizer, 100 $\mu$L of homogenate was collected and used for HPLC experiments, 40 $\mu$L of homogenate was collected and used for tissue DNA extraction and qPCR experiments, and the remaining volume of homogenate was used for ELISA experiments.

The concentrations of AADC and GDNF were measured by ELISA. ELISA experiments were performed strictly according to the kit (RayBiotech, ELH-DDC) instructions, and the specific experimental steps are as follows:

The method for AADC analysis was as follows: 100 $\mu$L of dissolved standard substance or sample was added to each well of the kit (RayBiotech, ELH-DDC), and the mixture was incubated at room temperature for 2.5 h, spun to dryness, and washed; 100 $\mu$L of biotin antibody was added to each well, and the mixture was incubated at room temperature for 1 h, spun to dryness, and washed; 100 $\mu$L of Streptavidin solution was added to each well, and the mixture was incubated at room temperature for 45 min, spun to dryness, and washed; 100 $\mu$L of TMB was added to each well, and the mixture was incubated at room temperature for 30 min, spun to dryness, and washed. 50 $\mu$L of stop solution was added to each well. Spectral reading was immediately performed at 450 nm.

The method for GDNF analysis was as follows: 100 $\mu$L of dissolved standard substance or sample was added to each well of the kit (R&D, DY212), and the mixture was incubated at room temperature for 2 h, spun to dryness, and washed; 100 $\mu$L of test antibody was added to each well, and the mixture was incubated at room temperature for 2 h, spun to dryness, and washed; 100 $\mu$L of Streptavidin-HRP solution was added to each well, and the mixture was incubated at room temperature for 20 min, spun to dryness, and washed; 100 $\mu$L of substrate solution was added to each well, and the mixture was incubated at room temperature for 20 min. 50 $\mu$L of stop solution was added to each well. Spectral reading was immediately performed at 450 nm.

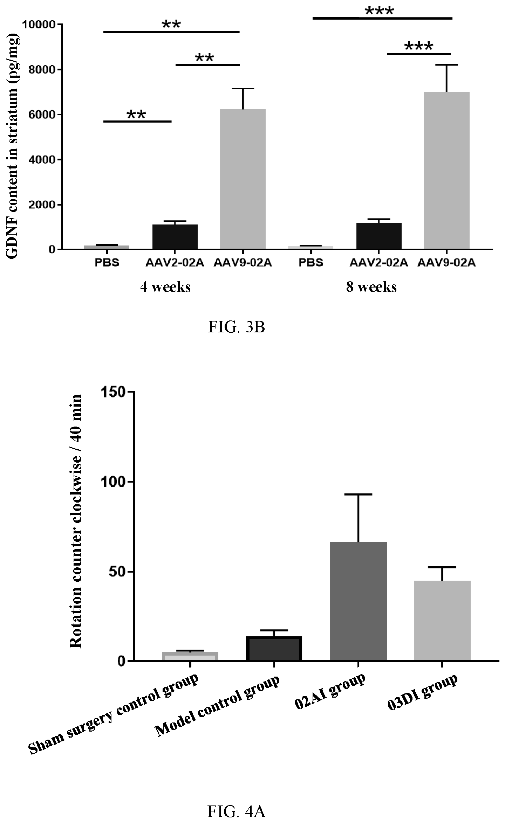

As shown in FIGs. 3A and 3B, the analysis results of the expression level of the target gene show that the expression levels of AADC and GDNF in the striatum of the AAV9-02A group were significantly higher than those in the AAV2-02A group, demonstrating that AAV9 has higher transduction efficiency in the striatal region than that of AAV2.

**Example 3. Pharmacodynamic Verification of Expressed Target Protein Following Delivery by AAV**

**1. Efficacy verification in MPTP-induced mouse PD model**

In this example, 1-methyl-4-phenyl-1,2,3,6-tetrahydropyridine (MPTP) was selected to build a mouse Parkinson's disease model. MPTP has high lipid solubility, easily permeates the blood-cerebrospinal fluid barrier, and can be converted into methyl-phenylpyridine ions (MPP+) under the action of glial cell monoamine oxidase B after entering the brain, and MPP+ is actively taken into the mitochondria of dopaminergic (DA) neurons by DA transporters, causing the degeneration and death of DAergic neurons. This process well simulates the symptoms and pathogenesis of PD. Adult male mice were intraperitoneally injected with MPTP for 5 consecutive days, which can induce the apoptosis of DA neurons and result in Parkinson's disease-like symptoms in the mice.

The method was as follows: Mice were divided into four groups, i.e., a sham surgery control group (i.e., normal control), a model control group (i.e., MPTP model control, the same applies hereinafter), a 02AI group (i.e., HRPDAAV-02AI), and a 03DI group (i.e., HRPDAAV-03DI). HRPDAAV-02AI and HRPDAAV-03DI were injected into the unilateral striatum of 8-week male mice, both using AAV9 capsids. Mice in the sham surgery control group and the model control group were subjected to sham surgery. C57BL/6N male mice were taken and anesthetized by intraperitoneal injection of 1% pentobarbital sodium (at a dose of 35-50 mg/kg); after the mice were confirmed to be in an anesthetic state, the mice were fixed to a stereotaxic apparatus (RWD, model: 68018) in the prone position; the scalp was incised after the instruments were sterilized conventionally, and soft tissues and periosteum were stripped bluntly to expose the right parietal bone; a bone window was drilled at a position 0.8 mm anterior to the bregma (AP = +0.8 mm) and 1.8 mm lateral to the midline (ML = 1.8 mm). AAV drugs were injected using a micro-syringe (specification: 1 μL; outer diameter: 0.55 mm); the needle was advanced at a needle insertion speed of 1 mm/min to a depth of 3 mm below the dura mater (DV = -3 mm), and then withdrawn to 2.8 mm (DV = -2.8 mm); the liquid was injected at a constant speed of 0.2 μL/min; after the injection was completed, the needle was retained for 3 min, and the needle withdrawing speed was 1 mm/min.

Twenty-eight days after the striatum injection, the mice in the model control group and the treatment groups were intraperitoneally injected with 30 mg/kg of MPTP with an injection volume of 10 mL/kg, and the mice in the sham surgery group were intraperitoneally injected with 10 mL/kg of normal saline. Five days after molding, the mice were allowed to rest for 3 days and subjected to the behavioral test.

1) Rotation test

To verify the response of AADC delivered by the HRPDAAV-02AI and HRPDAAV-03DI drugs to L-DOPA after unilateral striatum expression, a rotation test was performed. Levodopa (L-DOPA) (20 mg/kg) + benserazide (6 mg/kg) were intragastrically administered to the experimental mice in each group. After levodopa was administered to the mice, the mice were placed in a test environment for adaptation; 45 min after administration, the number of rotations of the mice began to be recorded; the rotation at the healthy side (rotation at the left side) of the mice within 30 min was tested.

The results are shown in FIG. 4A and Table 3. Compared to the sham surgery control group and the model control group, the HRPDAAV-02AI and HRPDAAV-03DI treatment groups showed significantly increased number of rotations after L-DOPA was administered ("02AI group vs. model control group", $p$ = 0.07; "03DI group vs. model control group", $p$ = 0.01), demonstrating that AADC delivered and expressed by AAV has a very good response to L-DOPA.

Table 3. Rotation test results in mice

| Group | Number of animals | Mean ± SEM |
|---|---|---|
| Sham surgery control group | 6 | 5.00±0.86 |
| Model control group | 13 | 14.08±3.19 |
| 02AI group | 11 | 66.45±26.59 |
| 03DI group | 12 | 44.92±7.72 |

2) Cylinder test

To analyze the forelimb use preference of mice in the background state, a cylinder test was performed. Four days after the last injection of MPTP in the mice, L-DOPA (10 mg/kg) + benserazide (3 mg/kg) were intragastrically administered; the mouse was placed in a transparent cylinder (with a diameter of 10 cm and a height of 45 cm), and the number of times the mouse touched the wall with the left forelimb, the right forelimb, and the bilateral forelimbs within 5 min was observed and recorded; if the mouse, supported by its hind limbs, touched the wall with one or both of the forelimbs and then returned to the bottom of the cylinder, this was recorded as one touching (if one limb of the mouse touched the wall first, and the other limb touched the wall later, this was recorded as bilateral touching). The cylinder was wiped with alcohol after each mouse test. Left forelimb utilization of the mice was analyzed: left forelimb usage rate (%) = (left + 0.5 bilateral)/(right + left + bilateral) × 100.

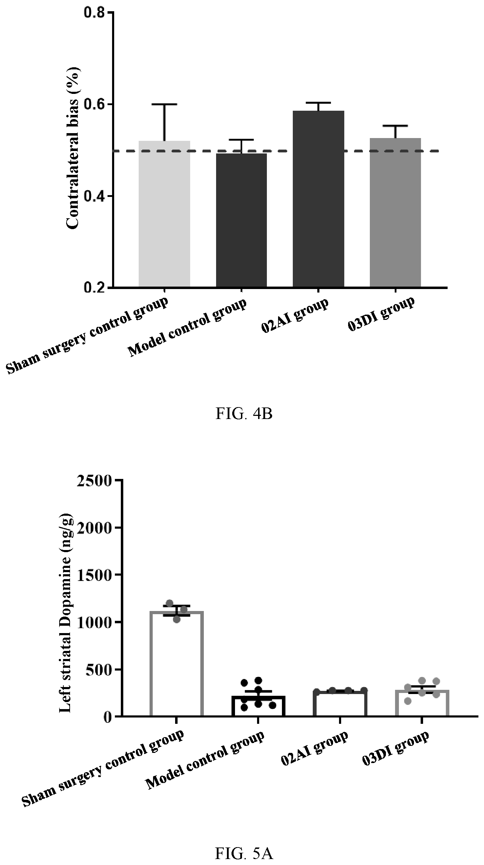

The results are shown in FIG. 4B and Table 4 and show that after administration of L-DOPA, the contralateral forelimb utilization rates of the HRPDAAV-02AI and HRPDAAV-03DI treatment groups were increased compared to the model control group, with 20.4% increase in the 02AI group and 8.2% increase in the 03DI group compared to the model control group, further demonstrating the response effect of AADC delivered and expressed by AAV on L-DOPA.

Table 4. Cylinder test results for mice

| Group | Number of animals | Mean ± SEM | Change value compared to model control group (%) |
|---|---|---|---|
| Sham surgery control group | 6 | 0.52±0.08 | 6.1% |

(continued)

| Group | Number of animals | Mean ± SEM | Change value compared to model control group (%) |
|---|---|---|---|
| Model control group | 13 | 0.49±0.03 | 0 |
| 02AI group | 11 | 0.59±0.02 | 20.4% |
| 03DI group | 12 | 0.53±0.03 | 8.2% |

3) Dopamine determination in striatum and blood

After the behavioral tests, the mice in each group were taken, and L-DOPA (20 mg/kg, 5 mL/kg) and benserazide (5 mg/kg, 5 mL/kg) were intragastrically administered. The mice were sacrificed 1 h after administration; peripheral blood was collected before sacrifice, and dopamine in the blood was detected. The striata at the healthy side and the affected side were separately collected after sacrifice. DOPA was determined by using a UPLC-MS/MS method. Referring to FIG. 5A and Table 5-1, the DOPA determination in the left striatum (non-administration side) showed a significant decrease in DOPA for both the model control group and the treatment groups compared to the sham surgery control group, demonstrating the success of MPTP molding.

Table 6-1. DOPA determination results in mouse left striatum (non-administration side)

| Group | Number of animals | Mean ± SEM |
|---|---|---|
| Sham surgery control group | 3 | 1122.0±49.7 |
| Model control group | 7 | 224.9±44.6 |
| 02AI group | 4 | 274.7±4.2 |
| 03DI group | 6 | 287.9±34.2 |

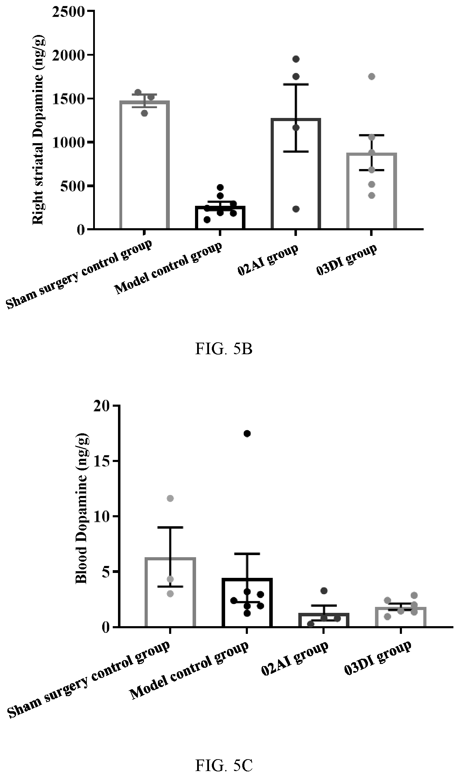

Referring to FIG. 5B and Table 5-2, the DOPA determination in the right striatum (administration side) showed a significant increase in DOPA for both HRPDAAV-02AI and HRPDAAV-03DI treatment groups compared to the model control group. The DOPA levels returned to the level of the sham surgery control group.

Table 5-2. DOPA determination results in mouse right striatum (administration side)

| Group | Number of animals | Mean ± SEM | Fold change compared to model control group |
|---|---|---|---|
| Sham surgery control group | 3 | 1473.6±72.6 | 4.43 |
| Model control group | 7 | 271.3±48.1 | 0 |
| 02AI group | 4 | 1277.1 ±385.1 | 3.71 |
| 03DI group | 6 | 880.8±200.2 | 2.25 |

Referring to FIG. 5C and Table 6-3, the plasma DOPA determination found that the groups differed little, demonstrating that the effects of HRPDAAV-02AI and HRPDAAV-03DI are primarily localized to the striatum of the brain, with no significant leakage.

Table 5-3. DOPA determination results in mouse plasma

| Group | Number of animals | Mean ± SEM |
|---|---|---|
| Sham surgery control group | 3 | 6.33±2.67 |
| Model control group | 7 | 4.44+2.19 |
| 02AI group | 4 | 1.29+0.68 |
| 03DI group | 6 | 1.85+0.29 |

4) Immunohistochemistry of brain tissue

Cardiac perfusion was performed on mice in each group, and the brain tissue was collected for fluorescence immunohistochemistry. Specifically, after general observation was performed on the experimental mice, the heart tissue in the thoracic cavity was exposed, a venous transfusion needle was inserted from the left ventricle and fixed, and the right auricle was cut off; perfusion was performed by using pre-cooled PBS until a transparent liquid flowed out from the right auricle, and then perfusion was performed on the heart by using 4% paraformaldehyde; the brain tissue was cut off, collected, and placed into a fixing agent (4% paraformaldehyde solution); the fixed tissue was subjected to paraffin sectioning and stained with tyrosine dehydrogenase TH.

Referring to FIG. 5D and Table 6, the fluorescence immunohistochemical analysis found that TH fluorescence intensities were comparable on both sides in the sham surgery control group and the model control group, while TH fluorescence intensities were significantly increased in the right striatum (administration side) compared to the left striatum (non-administration side) in the HRPDAAV-02AI and HRPDAAV-03DI treatment groups, demonstrating that the HRPDAAV-02AI and HRPDAAV-03DI drugs have good protection effects on mouse dopamine neurons.

Table 6. Immunohistochemistry results of mouse brain tissue

| Group | Number of animals | Right side average/left side average |
|---|---|---|
| Sham surgery control group | 3 | 0.94 |
| Model control group | 3 | 0.88 |
| 02AI group | 4 | 2.34 |
| 03DI group | 3 | 1.26 |

## 2. Efficacy verification in 6-OHDA-induced rat PD model

In this example, 6-OHDA was used to establish a rat PD model. 6-OHDA is a neurotoxin. Due to its structural similarity to dopamine (DA), it is often mistakenly taken up by DAergic neurons as a neurotransmitter. Through the formation of hydroxyl radicals and inhibition of mitochondrial oxidative respiratory chain complexes, it interferes with ATP synthesis and selectively causes the death of DAergic neurons. The DA content in the damaged striatum is decreased, causing rats to develop symptoms similar to human PD. The model has the characteristics of reliability, stability and irreversibility, in which the behavioral defects can be intuitively and quantitatively analyzed, and the model can be widely used for evaluating drugs against Parkinson's disease, especially drugs that have effects on DA and receptors thereof. Therefore, a single-side MFB injection 6-OHDA-induced Parkinson's disease model was adopted in this example to evaluate the treatment effects of HRPDAAV-02AI and HRPDAAV-03DI on Parkinson's disease.

The method was as follows: SD rats were divided into 6 experimental groups, i.e., a sham surgery control group (i.e., normal control group), a model control group, a 02AI high-dose group (1E+10 vg/dose), a 02AI low-dose group (2E+9 vg/dose), a 03DI high-dose group (1E+10 vg/dose), and a 03DI low-dose group (2E+9 vg/dose), and AAV9 capsids were used in all of them. The rat was anesthetized by intraperitoneal injection of 3% pentobarbital sodium (at a dose of 50-60 mg/kg), and then fixed in the prone position; the scalp was incised after the instruments were sterilized conventionally, and soft tissues and periosteum were stripped bluntly to expose the right parietal bone; connective tissues near the midline were removed, and a sagittal suture, a bregma, and a herringbone point were determined; a micro-syringe (specification: 10 μL; outer diameter: 0.5 mm) with a modeling reagent pumped in was fixed on a stereotaxic apparatus for coordinate positioning; the striatum was positioned on the right side of the rat by taking the bregma as a positioning central point; and a bone window was drilled at a position 0.0 mm anterior to the bregma (AP = +0.0 mm) and 3 mm lateral to the midline (ML = 3 mm). The needle was advanced at a needle insertion speed of 1 mm/min to a depth of 6 mm below the dura mater (DV = -6 mm), and then withdrawn to 5.8 mm (DV = -5.8 mm); 2 μL of liquid was injected at a constant speed of 0.6 μL/min; after the injection was completed, the needle was withdrawn to 3.8 mm (DV = -3.8 mm), and then 2 μL of liquid was injected at a constant speed of 0.6 μL/min; the needle was retained for 3 min after the two-point injection was completed, and the needle withdrawing speed was 2 mm/min. The scalp was sutured and disinfected, and the rat was placed on a heating pad at 37 °C until the rat was awake; penicillin sodium (80,000 units/day) was injected and an ibuprofen oral liquid was intragastrically administered for three consecutive days after the operation.

Three weeks after the striatum was injected with the drug, modeling was performed, and 3% pentobarbital sodium was adopted for anesthesia by intraperitoneal injection at an injection dose of 60 mg/kg with an injection volume of 2 mL/kg. After the anesthetic induction, the rat was transferred to an operation table and fixed to the cerebral stereotaxic apparatus in the prone position to ensure that the brain was on the horizontal plane; eyelid reflexes and nociceptive responses of the rat were observed, and the operation could be started after the eyelid reflex reactions and the nociceptive responses of limbs and the tail disappeared. A minimal-size drill bit was used to vertically drill a hole, taking care not to deflect and injure the brain tissue. The hole was positioned on the right side of the rat (administrating side), and a bone window was drilled at a position 2.0 mm posterior to the bregma (AP = -2.0 mm) and 2 mm lateral to the midline (ML = 2 mm). The needle was advanced at a needle insertion speed of 6 mm/min to a depth of 8.8 mm below the dura mater (DV = -8.8 mm), and then withdrawn to 8.5 mm (-8.5 mm); 6-OHDA was injected at a constant speed of 0.6 $\mu$L/min; 4 $\mu$L of 6-OHDA (5 $\mu$g/$\mu$L) was injected into each rat; after the injection was completed, the needle was retained for 3 min, and the needle withdrawing speed was 2 mm/min. The scalp was sutured and disinfected, and the rat was placed on the heating pad at 37 °C until the rat was awake; penicillin sodium (80,000 units/day) was injected and the ibuprofen oral liquid was intragastrically administered for three consecutive days after the operation.

Three weeks after molding, the response of the rats to the apomorphine drug was tested. The rats were intraperitoneally injected with apomorphine (0.5 mg/kg). 10 min later, the rats were placed in the test environment, and the number of rotations of the rats within 30 min was recorded.

Referring to FIG. 6A, the 6-OHDA-modeled rats all had significantly increased number of rotations compared to the sham surgery control group, demonstrating that the modeling experiment was successful.

1) Rotation test

To assess the response of exogenously expressed AADC in the striatum to exogenous L-DOPA, the rotation at the healthy side (rotation at the left side) of the rats after intragastric administration of L-DOPA was measured. The rats were orally and intragastrically administered with L-DOPA (20 mg/kg, 5 mL/kg) + benserazide (5 mg/kg, 5 mL/kg). 45 min later, the rats were placed in the test environment, and the number of rotations of the rats within 30 min was recorded.

Referring to FIG. 6B, the number of rotations of the model control group was not significantly increased compared to the sham surgery control group, while the HRPDAAV-02AI and HRPDAAV-03DI high and low dose treatment groups all showed significantly increased number of rotations compared to the model control group, indicating that AADC expressed in the striatum and delivered by AAV has a good response effect to exogenous L-DOPA drugs.

2) Cylinder test

To analyze the forelimb use preference of rats in the background state, a cylinder test was performed. The rat in each group was placed in a transparent cylinder with a diameter of 20 cm, and the number of times the rat touched the wall with the left forelimb, the right forelimb, and the bilateral forelimbs within 10 min was observed and recorded. If the rat, supported by its hind limbs, touched the wall with one or both of the forelimbs and then returned to the bottom of the cylinder, this was recorded as one touching (if one limb of the rat touched the wall first, and the other limb touched the wall later, this was recorded as bilateral touching). The left forelimb usage rate of the rat was calculated: left forelimb usage rate (%) = (left + 0.5 bilateral)/(right + left + bilateral) $\times$ 100.

Referring to FIG. 6C, the left forelimb utilization rates were all decreased in the model rat and the treatment groups compared to the blank control group, further verifying the validity of the model.

To assess the utilization of L-DOPA by rats treated with AAV drugs, the rats were orally and intragastrically administered with L-DOPA (5 mg/kg, 5 mL/kg) + benserazide (2.5 mg/kg, 5 mL/kg). 30 min later, the rat was placed in the transparent cylinder with the diameter of 20 cm, and the number of times the rat touched the wall with the left forelimb, the right forelimb, and the bilateral forelimbs within 10 min was observed and recorded.

Referring to FIG. 8D, the left forelimb utilization rate of the model control group after L-DOPA supplementation was not significantly improved, while the left forelimb utilization rates of the HRPDAAV-02AI and HRPDAAV-03DI groups were significantly improved compared to the model control group, demonstrating that the utilization rate of L-DOPA by rats after administration is significantly improved, and further demonstrating that AADC expressed in the striatum and delivered by AAV has a very good response effect to exogenous L-DOPA drugs.

3) Assessment of L-DOPA metabolizing ability of striatum after administration of HRPDAAV-02AI and HRPDAAV-03DI

After the behavioral tests, the rats in each group were taken, and L-DOPA (20 mg/kg, 5 mL/kg) and benserazide (5 mg/kg, 5 mL/kg) were intragastrically administered. The rats were sacrificed 1 h after the end of the administration. The striata at the healthy side and the affected side were separately collected after sacrifice. DOPA was determined by using a UPLC-MS/MS method.

Referring to FIG. 6E and Table 7, the DOPA ratio of affected side/healthy side was determined, and it was found that DOPA at the affected side of the model control group was significantly decreased compared to the treatment groups, demonstrating the success of 6-OHDA molding.

Table 7. DOPA determination results in rat bilateral striata

| Group | Number of animals | Affected side (mean ± SEM) | Healthy side (mean ± SEM) | Affected side/healthy side (%) |
|---|---|---|---|---|
| Sham surgery control group | 3 | 27729.03±8121.90 | 28687.57±3329.29 | 95.49±20.20 |
| Model control group | 9 | 7299.01±3113.39 | 25872.46±5398.04 | 30.05±19.39* |
| 02AI low-dose group | 9 | 17323.06±5465.61 | 25432.89±12362.06 | 77.76±33.49 |
| 03DI low-dose group | 9 | 19029.10±6859.95 | 20756.06±5501.87 | 101.99±63.33 |

The DOPA determination in the striatum at the administration side (affected side) showed a significant increase in DOPA for both HRPDAAV-02AI and HRPDAAV-03DI treatment groups compared to the model control group. Compared to the model control group, the DOPA ratio of affected side/healthy side of each treatment group was significantly improved, demonstrating that the L-DOPA drug metabolizing ability of the striatum after administration of HRPDAAV-02AI and HRPDAAV-03DI is significantly improved.

4) Assessment of protection effect of HRPDAAV-03DI on DA neurons after administration

Cardiac perfusion was performed on rats in each group, and the brain tissue was collected for fluorescence immunohistochemistry. Specifically, after general observation was performed on the experimental rats, the heart tissue in the thoracic cavity was exposed, a venous transfusion needle was inserted from the left ventricle and fixed, and the right auricle was cut off; perfusion was performed by using pre-cooled PBS until a transparent liquid flowed out from the right auricle, and then perfusion was performed on the heart by using 4% paraformaldehyde; the brain tissue was cut off, collected, and placed into a fixing agent (4% paraformaldehyde solution); the fixed tissue was subjected to paraffin sectioning and stained with tyrosine dehydrogenase TH.

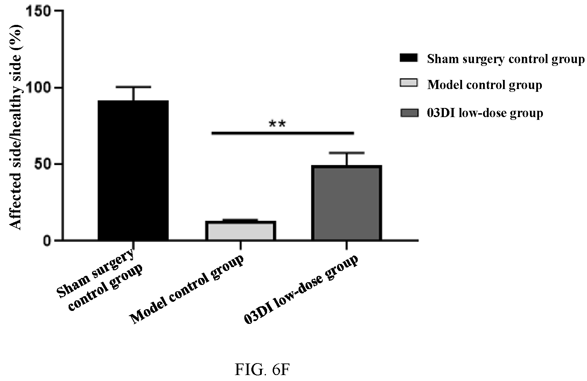

Referring to FIG. 6F, the TH positive cell ratio of affected side/contralateral side was analyzed by fluorescence immunohistochemistry, and it was found that the TH cell viability at the affected side was less than 15% in the experimental model control group compared to the sham surgery group, indicating that 6-OHDA modeling was successful.

The TH fluorescence intensity ratio of the affected side to the healthy side of the HRPDAAV-03DI low-dose treatment group was significantly increased compared to that of the model control group, demonstrating that the HRPDAAV-03DI drug has a very good protection effect on rat dopamine neurons and improves the TH cell viability.

**Example 4: VEGF-Binding Molecule and Point Mutation Design**

In this example, three molecular formats of VEGF-binding molecules were designed (FIG. 7), and they were, from N-terminus to C-terminus:

molecular format 1: VEGFR1 D2 + VEGFR2 D3 + VEGFR2 D2D3 + hinge region and CH2CH3 constant region of human IgG1;
molecular format 2: VEGFR2 D2D3 + VEGFR1 D2 + VEGFR2 D3 + hinge region and CH2CH3 constant region of human IgG1;
molecular format 3: heavy chain: VEGFR2 D2D3 + hinge region and CH2CH3 constant region of human IgG1 + bevacizumab VH + CH1; light chain: bevacizumab VL + CL.

To improve the stability and ligand affinity of fusion proteins, a series of point mutations were designed in the VEGF trap backbone molecule (i.e., the VEGFR2 D2D3 domain, underlined above) in the VEGF-binding molecules.

1. Based on the VEGFR2 D2D3 sequence (SEQ ID NO: 69), 5 mutants were designed by mutating the cysteine at position 162 (C162) into A/V/S/I/L, respectively.
2. Based on the VEGFR2 D2D3 sequence (SEQ ID NO: 69), 6 single point mutations and 2 combination mutations were designed (Table 1).
3. Based on the VEGFR2 D2D3 sequence (SEQ ID NO: 69), single point mutations were designed in the D2 or D3 domain (Table 2 and Table 3).

Table 8. VEGF trap backbone molecule VEGFR2 D3 mutations

| Residue No. | Wild type | Residue after mutation |
|---|---|---|
| 274 | N | F or I |
| 276 | D | Q |
| 280 | Q | E |
| 284 | E | H |
| 288 | F | Y |
| 313 | L | R |
| 221&222 | Y&R | S or N & K |
| 277&289 | L&L | R&Y |

Table 9. VEGF trap backbone molecule VEGFR2 D2 mutations

| Residue No. | Wild type | Residue after mutation |
|---|---|---|
| 135 | V | Y |
| 197 | M | Y |
| 199 | F | Q or T |
| 213 | M | N |
| 215 | I | F or H |
| 219 | V | I |

Table 10. VEGF trap backbone molecule VEGFR2 D3 mutations

| Residue No. | Wild type | Residue after mutation |
|---|---|---|
| 257 | D | Q or T |
| 275 | R | L or E |
| 277 | L | R |
| 278 | K | D or S or E |
| 283 | S | T |
| 289 | L | Y |
| 313 | L | Y |
| 314 | M | S or Q |

**Example 5: Construction of Plasmids Expressing VEGF-Binding Molecules and Protein Expression and Purification**

Using whole-gene synthesis, the VEGF-binding molecules designed in Example 6 were synthesized. The antagonistic activity against VEGF-C is mainly provided by VEGFR2 D2D3, and the antagonistic activity against VEGF-A is mainly provided by VEGFR1D2-VEGFR2D3 (aflibercept) or the Fab of bevacizumab.

After the amino acid sequences were optimized for the expression host, the genes encoding the nucleotide sequences described above were inserted into the pTT5 vector. The expression vectors were synthesized according to the sequence design described above and verified by sequencing.

Expression and purification of VEGF-binding molecules: The qualified plasmids were transfected into human 293F cells using PEI. The 293F cells were continuously cultured in serum-free culture medium (Shanghai OPM Biosciences, OPM-293 CD03) until they reached the logarithmic growth phase for cell transfection. 45 $\mu$ of plasmid was dissolved in 10 mL of Opti-MEM® I Reduced Serum Medium (GIBCO, 31985-070) and thoroughly mixed, and 200 $\mu$g of PEI was added and thoroughly mixed. The mixture was incubated at room temperature for 15 min and added to 50 mL of cells. The cell culture conditions were: 5% $CO_2$, 37 °C, 125 rpm/min. During the culture, a feeding was added at day 1 and day 3. When the cell viability was less than 70%, the cell supernatant was collected and filtered by centrifugation. The filtered cell culture supernatant was loaded onto a protein A column. The column was washed with phosphate buffer, and elution was performed with glycine hydrochloride buffer (pH 2.7, 0.1 M Gly-HCl). The eluate was neutralized with 1 M Tris-hydrochloride pH 9.0 and dialyzed against phosphate buffer to obtain finally the purified protein. SDS-PAGE and SEC-HPLC analyses confirmed the obtaining of the target fusion proteins.

## Example 6: Assay for Binding Activity of VEGF-Binding Molecules for Ligands VEGF-A and VEGF-C

The inhibition of KDR signaling activation by the VEGF-binding molecules was evaluated by measuring the VEGF-A-induced NFAT-RE Luc2P KDR cell reporter gene expression level. The experimental steps were as follows:

3A.1 Preparation of culture media: Complete culture medium: high-glucose DMEM (90%) + FBS (10%); complete culture medium: high-glucose DMEM (90%) + FBS (10%), 100 $\mu$g/mL hygromycin B, 250 $\mu$g/mL G-418. Analysis culture medium: high-glucose DMEM (99%) + FBS (1%).

3A.2 Dilution of VEGF-binding molecule samples: The test samples were serially diluted 3-fold, with the highest concentration being 300 nM. VEGFA dilution: the final concentration of VEGFA was 250 ng/mL.

3A.3 Preparation of reporter gene cell strain: Glo-ResponseTM NFAT-RE-luc2P/KDR HEK293 cells were digested, washed with 5 mL of PBS, and resuspended in the analysis culture medium, and the density was adjusted to $8 \times 10^5$ cells/mL. The cell suspension was added to a 96-well cell culture plate at 50 $\mu$L/well, i.e., $4 \times 10^4$ cells/well.

3A.4 Treatment with VEGF-binding molecules: The prepared different concentrations of VEGF-binding molecules were added to the cell wells at 50 $\mu$L/well, and the plate was incubated in a 5% $CO_2$ incubator at 37 °C for 6 h. Each concentration was tested in triplicate. Meanwhile, a VEGF-A-only control group and a VEGF-A-free control group were set.

3A.5 Color development: Bright-Glo color-developing solution was equilibrated to room temperature. After the incubation, 50 $\mu$L of Bright-Glo color-developing solution was added to the 96-well plate, thoroughly mixed by shaking at room temperature for 10 min, and left to stand for 2 min. The culture plate was placed on a multi-mode microplate reader, and chemiluminescence values were measured.

3A.6 Data processing:

$$\text{Top value} = \text{fluorescence value of VEGF-A} + 0 \text{ nM VEGF-binding molecule group}$$

$$\text{Bottom value} = \text{fluorescence value of VEGF-A-free group}$$

$$\text{Inhibition rate} = (\text{fluorescence value} - \text{bottom value})/(\text{top value} - \text{bottom value}) \times 100\%$$

Graphpad plotting: The results were analyzed using a four-parameter fit to obtain dose-response curves. The four-parameter fit formula was:

$$Y = (A - D)/(1 + (X/C)^{\wedge}B) + D$$

A: the estimated value of the lower asymptote; B: the slope of the curve; C: $IC_{50}$; D: the estimated value of the upper asymptote.

For the evaluation of the binding activity of the VEGF-binding molecules for VEGF-C, a BAF3-VEGFR2/R3 cell proliferation experiment was used in this example. BAF3-VEGFR2/R3 is a BA-F3 cell line overexpressing human VEGFR2/R3, and the growth of the cells depends on the exogenous addition of the growth factor VEGF-C. The basic experimental steps were as follows:

3B.1 Cell culture: The cell line described above was cultured in RPMI1640 with 10% FBS (supplemented with 10 ng/mL mouse IL3, 1 $\mu$g/mL puromycin, and 250 $\mu$g/mL G418) at $1 \times 105$ to $1.5 \times 10^5$ cells/mL. The culture was plated in a 5% $CO_2$ incubator at 37 °C, with the cell density not exceeding $1 \times 10^6$ cells/mL.

3B.2 Cell proliferation experiment: Cells in the logarithmic growth phase were washed three times with PBS and centrifuged at 800 r/min for 3 min. After the cell density was adjusted with RPMI1640 (FBS: 2%, 143 ng/mL VEFG-C), the cells were added to a 96-well plate at $2 \times 10^4$ cells/well/50 $\mu$L. Then, 50 $\mu$L of serially diluted VEGF-binding molecule (highest concentration 300 nM, 3-fold dilution) was added. Meanwhile, a VEGF-C-free control group and a VEGF-C-only control group were set. After 3 days of culture, 30 $\mu$L of cell titer-glo was added and thoroughly mixed, and cell counting was performed.

3B.3 Data processing:

$$\text{Top value} = \text{fluorescence value of VEGF-C} + \text{VEGF-binding molecule concentration 0 group}$$

$$\text{Bottom value} = \text{fluorescence value of VEGF-C-free group}$$

$$\text{Inhibition rate} = (\text{fluorescence value} - \text{bottom value})/(\text{top value} - \text{bottom value}) \times 100\%$$

Graphpad plotting: The results were analyzed using a four-parameter fit to obtain dose-response curves. The four-parameter fit formula was:

$$Y = (A - D)/(1 + (X/C)^B) + D$$

where A: the estimated value of the lower asymptote; B: the slope of the curve; C: $IC_{50}$; D: the estimated value of the upper asymptote.

In this example, the binding activity of VEGF-A and VEGF-C for the VEGF-binding molecules was evaluated through the two *in vitro* experiments described above, respectively. The specific data are shown in Table 11:

Table 11. Data on the activity of molecules based on molecular format 1 with single point mutations

| Structure | Protein name | VEGF-A $IC_{50}$ (nM) | VEGF-C $IC_{50}$ (nM) |
|---|---|---|---|
| R1D2-R2D3-Fc(SEQ ID NO:120) | Aflibercept | 0.76 | - |
| SEQ ID NO:121 | OPT-302 | - | 0.97 |
| R1D2-R2D3-linker-R2D2-R2D3-Fc | PR1010 | 2.71 | 9.45 |
| R1D2-R2D3-linker-R2D2(C162V)-R2D3-Fc | PR1094 | 0.34 | 2.57 |
| R1D2-R2D3-linker-R2D2(C162I)-R2D3-Fc | PR1165 | 0.92 | 2.89 |
| R1D2-R2D3-linker-R2D2(C162L)-R2D3-Fc | PR1166 | 1.12 | 2.69 |
| R1D2-R2D3-linker-R2D2(M197Y)-R2D3-Fc | PR1101 | 0.54 | 2.00 |
| R1D2-R2D3-linker-R2D2(V219I)-R2D3-Fc | PR1109 | 0.18 | 2.70 |
| R1D2-R2D3-linker-R2D2-R2D3(N274F)-Fc | PR1113 | 0.49 | 1.12 |
| R1D2-R2D3-linker-R2D2-R2D3(N274I)-Fc | PR1114 | 0.36 | 1.06 |
| R1D2-R2D3-linker-R2D2-R2D3(R275L)-Fc | PR1115 | 0.28 | 0.90 |
| R1D2-R2D3-linker-R2D2-R2D3(R275E)-Fc | PR1116 | 0.54 | 1.23 |
| R1D2-R2D3-linker-R2D2-R2D3(D276Q)-Fc | PR1118 | 0.22 | 0.92 |
| R1D2-R2D3-linker-R2D2-R2D3(L277R)-Fc | PR1119 | 0.14 | 1.84 |
| R1D2-R2D3-linker-R2D2-R2D3(L278D)-Fc | PR1120 | 0.38 | 2.20 |
| R1D2-R2D3-linker-R2D2-R2D3(K278E)-Fc | PR1122 | 0.36 | 2.26 |
| R1D2-R2D3-linker-R2D2-R2D3(Q280E)-Fc | PR1123 | 0.21 | 1.53 |
| R1D2-R2D3-linker-R2D2-R2D3(S283T)-Fc | PR1124 | 0.29 | 1.04 |
| R1D2-R2D3-linker-R2D2-R2D3(E284H)-Fc | PR1125 | 0.23 | 1.62 |
| R1D2-R2D3-linker-R2D2-R2D3(F288Y)-Fc | PR1126 | 0.70 | 2.36 |
| R1D2-R2D3-linker-R2D2-R2D3(L289Y)-Fc | PR1127 | 0.52 | 1.16 |
| R1D2-R2D3-linker-R2D2-R2D3(L313Y)-Fc | PR1128 | 0.42 | 1.06 |
| R1D2-R2D3-linker-R2D2-R2D3(M314S)-Fc | PR1129 | 0.67 | 2.62 |

(continued)

| Structure | Protein name | VEGF-A IC$_{50}$ (nM) | VEGF-C IC$_{50}$ (nM) |
|---|---|---|---|
| R1D2-R2D3-linker-R2D2-R2D3(M314Q)-Fc | PR1130 | 0.45 | 1.99 |
| R1D2-R2D3-linker-R2D2-R2D3(L277R/L289Y)-Fc | PR1132 | 0.42 | 1.06 |
| R1D2-R2D3-linker-R2D2(Y221S/R222K)-R2D3-Fc | PR1135 | 0.50 | 1.90 |
| R1D2-R2D3-linker-R2D2-R2D3(L313R)-Fc | PR1136 | 0.14 | 0.98 |

The *in vitro* activity of the VEGF-binding molecules of the present disclosure for VEGF-A and VEGF-C was comparable to that of the positive controls aflibercept and OPT-302.

Table 12. Data on the activity of proteins containing combinations of some of the single point mutations and C162V produced by the CHO-K1 system

| Protein name | Structure | VEGF-A IC$_{50}$ (nM) | VEGF-C IC$_{50}$ (nM) |
|---|---|---|---|
| PR1160 | R2D2(C162V)-R2D3-Fc | 28.36 | 0.80 |
| PR1141 | R1D2-R2D3-linker-R2D2(C162V)-R2D3(N274F)-Fc | 1.14 | 1.78 |
| PR1142 | R1D2-R2D3-linker-R2D2(C162V)-R2D3(N274I)-Fc | 0.99 | 1.92 |
| PR1143 | R1D2-R2D3-linker-R2D2(C162V)-R2D3(R275L)-Fc | 0.84 | 1.63 |
| PR1144 | R1D2-R2D3-linker-R2D2(C162V)-R2D3(D276Q)-Fc | 0.51 | 0.97 |
| PR1145 | R1D2-R2D3-linker-R2D2(C162V)-R2D3(S283T)-Fc | 0.92 | 1.05 |
| PR1146 | R1D2-R2D3-linker-R2D2(C162V)-R2D3(L289Y)-Fc | 0.98 | 2.01 |
| PR1147 | R1D2-R2D3-linker-R2D2(C162V)-R2D3(L313Y)-Fc | 1.16 | 1.26 |
| PR1148 | R1D2-R2D3-linker-R2D2(C162V)-R2D3(L277R/L289Y)-Fc | 1.49 | 2.53 |
| PR1149 | R1D2-R2D3-linker-R2D2(C162V/Y221S/R222K)-R2D3-Fc | 0.82 | 1.66 |
| PR1150 | R1D2-R2D3-linker-R2D2(C162V)-R2D3(L313R)-Fc | 0.81 | 1.07 |
| Aflibercept | SEQ ID NO:120 | 0.50 | - |
| OPT-302 | SEQ ID NO:121 | - | 0.42 |
| "-" indicates that the activity was unmeasurable. | | | |

Table 13. Data on the activity of proteins containing combination mutations produced by the CHO-K1 system

| Protein name | Structure | VEGF-A IC$_{50}$ (nM) | VEGF-C IC$_{50}$ (nM) |
|---|---|---|---|
| PR1167 | R1D2-R2D3-R2D2(C162V)-R2D3(N274F/S283T)-Fc | 1.10 | 2.91 |
| PR1168 | R1D2-R2D3-R2D2(C162V/Y221S/R222K)-R2D3(N274F)-Fc | 1.15 | 5.43 |
| PR1169 | R1D2-R2D3-R2D2(C162V/Y221S/R222K)-R2D3(S283T)-Fc | 0.90 | 3.49 |
| PR1170 | R1D2-R2D3-R2D2(C162V/Y221S/R222K)-R2D3(N274F/S283T)-Fc | 0.89 | 5.95 |
| PR1171 | R1D2-R2D3-linker-R2D2(C162V)-R2D3(N274F/S283T)-Fc | 1.09 | 3.37 |
| PR1172 | R1D2-R2D3-linker-R2D2(C162V/Y221S/R222K)-R2D3(N274F)-Fc | 0.92 | 7.57 |
| PR1173 | R1D2-R2D3-linker-R2D2(C162V/Y221S/R222K)-R2D3(S283T)-Fc | 1.08 | 4.30 |
| PR1174 | R1D2-R2D3-linker-R2D2(C162V/Y221S/R222K)-R2D3(N274F/S283T)-Fc | 1.02 | 5.56 |
| PR1179 | R1D2-R2D3-R2D2(C162V/Y221S/R222K)-R2D3-Fc | 0.97 | 3.63 |
| Aflibercept | SEQ ID NO:120 | 0.72 | - |

(continued)

| Protein name | Structure | VEGF-A $IC_{50}$ (nM) | VEGF-C $IC_{50}$ (nM) |
|---|---|---|---|
| OPT-302 | SEQ ID NO:121 | - | 1.03 |

As shown in Table 12 and Table 13, although PR1160, a VEGFR2 D2D3 (C162V)-Fc fusion protein, exhibited some *in vitro* inhibitory activity against VEGF-A, its activity was weaker than the positive control aflibercept, while the inhibitory activity of the other fusion proteins against VEGF-A and VEGF-C was comparable to that of the two positive control molecules. As shown in Table 14, the inhibitory activity of the VEGF-binding molecules PR1145, PR1174, PR1163, and PR1186 against VEGF-A and VEGF-C was comparable to that of the two positive control molecules (aflibercept and OPT-302) and stronger than the inhibitory activity of PR1010 and bevacizumab against VEGF-A.

## Example 8: Analysis of Thermal Stability of Different VEGF-Binding Molecules

The thermal stability of fusion proteins is an important criterion for evaluating the quality of molecules. Developing a fusion protein molecule with relatively good thermal stability has positive significance for process production, drug transportation, etc. In this example, accelerated thermal stability evaluation was performed on candidate molecules.

The specific experimental steps were as follows: The candidate molecules were diluted to 1 mg/mL and aliquoted into three 1.5 mL EP tubes, with 100 $\mu$L in each tube. One of the tubes was taken as the time-0 sample and stored frozen. The remaining samples were placed in a constant-temperature, constant-humidity incubator at 40 °C with 75% humidity. One tube of sample was taken at two weeks, and one tube of sample was taken at four weeks. The samples were stored frozen. After samples at three time points (0, 2, and 4 weeks) were taken, they were thawed at 4 °C and centrifuged at 12,000 rpm for 10 min to remove precipitates, and the supernatant was taken for HPLC analysis. In this example, the HPLC used was the ACQUITY Arc model produced by Waters, the column was XBridge BEH 200Å SEC 3.5 $\mu$m, the flow rate was 0.5 mL/min, and 30 $\mu$L of sample was injected. The final HPLC chromatograms of the samples are shown in the table below.

Although the parent molecule PR1010 of molecular format 1 had an initial purity of up to 90%, the purity dropped to 30% or less after storage at 40°C for two weeks, suggesting that the stability of this molecule needs to be improved. The purity of the PR1094 molecule obtained by introducing the VEGFR2 C162V mutation into PR1010 increased to 95% or more in HEK 293F, and the SEC purity was still as high as about 90% after storage at 40 °C for four weeks.

According to the HPLC chromatogram results, the thermal stability of the molecules was significantly improved after the C162V mutation, both on the VEGFR2 D2D3-Fc fusion protein (PR1160 vs. PR1088) and on molecular format 1 (PR1094 vs. RP1010).

Additionally, point mutations such as N274F (PR1113), N274I (PR1114), D276Q (PR1118), Q280E (PR1123), E284H (PR1125), Y221S/R222K (PR1135), and L313R (PR1136) can also significantly improve the stability of fusion proteins by stabilizing the VEGFR2 D2D3 domain.

To further study the effects of C162V/I-based combination mutations on protein expression and purification, CHO-K1 was subsequently used to express combination-mutation molecules: in most cases, combination mutations can further improve the stability of molecules (e.g., PR1145/PR1149 vs. PR1094).

The results are shown in Table 14 and Table 15, where PR1088 to PR1136 (Table 8-1) were obtained from HEK 293F expression, and PR1094 to RP1179 (Table 8-2) were obtained from CHO-K1 expression.

Table 14. The results of the analysis of the thermal stability of different VEGF-binding molecules

| Protein name | Structure | SEC (%)/ 0 weeks | SEC (%)/ 2 weeks | SEC (%)/ 4 weeks |
|---|---|---|---|---|
| PR1088 | R2D2-R2D3-Fc | 70.38 | - | - |
| PR1010 | R1D2-R2D3-linker-R2D2-R2D3-Fc | 90.02 | <30 | - |
| PR1011 | R2D2-R2D3-linker-R1D2-R2D3-Fc | 81.92 | <30 | - |
| PR1160 | R2D2(C162V)-R2D3-Fc | 93.24 | 82.20 | 86.16 |
| PR1094 | R1D2-R2D3-linker-R2D2(C162V)-R2D3-Fc | 95.23 | 91.75 | 89.45 |
| PR1101 | R1D2-R2D3-linker-R2D2(M197Y)-R2D3-Fc | 82.77 | 20.33 | - |
| PR1109 | R1D2-R2D3-linker-R2D2(V219I)-R2D3-Fc | 83.34 | 22.73 | - |

(continued)

| Protein name | Structure | SEC (%)/ 0 weeks | SEC (%)/ 2 weeks | SEC (%)/ 4 weeks |
|---|---|---|---|---|
| PR1113 | R1D2-R2D3-linker-R2D2-R2D3(N274F)-Fc | 83.18 | 51.64 | 16.36 |
| PR1114 | R1D2-R2D3-linker-R2D2-R2D3(N274I)-Fc | 85.40 | 56.41 | 26.69 |
| PR1115 | R1D2-R2D3-linker-R2D2-R2D3(R275L)-Fc | 80.85 | 27.12 | - |
| PR1116 | R1D2-R2D3-linker-R2D2-R2D3(R275E)-Fc | 83.99 | 19.72 | - |
| PR1118 | R1D2-R2D3-linker-R2D2-R2D3(D276Q)-Fc | 83.93 | 54.95 | 49.24 |
| PR1119 | R1D2-R2D3-linker-R2D2-R2D3(L277R)-Fc | 78.78 | 35.87 | - |
| PR1120 | R1D2-R2D3-linker-R2D2-R2D3(K278D)-Fc | 86.00 | 27.33 | - |
| PR1122 | R1D2-R2D3-linker-R2D2-R2D3(K278E)-Fc | 87.19 | 29.04 | - |
| PR1123 | R1D2-R2D3-linker-R2D2-R2D3(Q280E)-Fc | 87.73 | 58.97 | 52.72 |
| PR1124 | R1D2-R2D3-linker-R2D2-R2D3(S283T)-Fc | 88.46 | 23.73 | - |
| PR1125 | R1D2-R2D3-linker-R2D2-R2D3(E284H)-Fc | 87.25 | 53.93 | 47.11 |
| PR1126 | R1D2-R2D3-linker-R2D2-R2D3(F288Y)-Fc | 87.12 | 55.38 | - |
| PR1127 | R1D2-R2D3-linker-R2D2-R2D3(L289Y)-Fc | 86.60 | 18.59 | - |
| PR1128 | R1D2-R2D3-linker-R2D2-R2D3(L313Y)-Fc | 88.84 | 31.48 | - |
| PR1129 | R1D2-R2D3-linker-R2D2-R2D3(M314S)-Fc | 81.61 | 8.80 | - |
| PR1130 | R1D2-R2D3-linker-R2D2-R2D3(M314Q)-Fc | 84.85 | 25.17 | - |
| PR1132 | R1D2-R2D3-linker-R2D2-R2D3(L277R/L289Y)-Fc | 83.03 | 47.18 | - |
| PR1135 | R1D2-R2D3-linker-R2D2(Y221S/R222K)-R2D3-Fc | 91.76 | 72.49 | 65.05 |
| PR1136 | R1D2-R2D3-linker-R2D2-R2D3(L313R)-Fc | 92.13 | 67.75 | 60.39 |

Table 15. The results of the analysis of the thermal stability of different VEGF-binding molecules

| Protein name | Structure | SEC (%)/ 0 weeks | SEC (%)/ 2 weeks | SEC (%)/ 4 weeks |
|---|---|---|---|---|
| PR1094 | R1D2-R2D3-linker-R2D2(C162V)-R2D3-Fc | 85.56 | 81.43 | 77.17 |
| PR1141 | R1D2-R2D3-linker-R2D2(C162V)-R2D3(N274F)-Fc | 88.95 | 84.63 | 76.10 |
| PR1142 | R1D2-R2D3-linker-R2D2(C162V)-R2D3(R275L)-Fc | 87.09 | 81.39 | 77.13 |
| PR1143 | R1D2-R2D3-linker-R2D2(C162V)-R2D3(R275L)-Fc | 87.42 | 81.73 | 76.92 |
| PR1144 | R1D2-R2D3-linker-R2D2(C162V)-R2D3(D276Q)-Fc | 87.00 | 79.21 | 74.21 |
| PR1145 | R1D2-R2D3-linker-R2D2(C162V)-R2D3(S283T)-Fc | 90.10 | 83.81 | 78.61 |
| PR1146 | R1D2-R2D3-linker-R2D2(C162V)-R2D3(L289Y)-Fc | 87.04 | 72.54 | 65.91 |
| PR1147 | R1D2-R2D3-linker-R2D2(C162V)-R2D3(L313Y)-Fc | 88.25 | 81.73 | 75.98 |
| PR1148 | R1D2-R2D3-linker-R2D2(C162V)-R2D3(L277R/L289-Y)-Fc | 81.40 | 56.77 | 50.52 |

(continued)

| Protein name | Structure | SEC (%)/ 0 weeks | SEC (%)/ 2 weeks | SEC (%)/ 4 weeks |
|---|---|---|---|---|
| PR1149 | R1D2-R2D3-linker-R2D2(C162V/Y221S/R222K)-R2D3-Fc | 90.21 | 83.81 | 80.36 |
| PR1150 | R1D2-R2D3-linker-R2D2(C162V)-R2D3(L313R)-Fc | 87.08 | 71.75 | 66.13 |
| PR1163 | Heavy chain H: R2D2 (C162V)-R2D3-Fc-linker-VH-CH1; Light chain L: VL-CL | 98.92 | 92.31 | 83.80 |
| PR1167 | R1D2-R2D3-R2D2(C162V)-R2D3(N274F/S283-T)-Fc | 84.38 | 72.05 | 67.58 |
| PR1168 | R1D2-R2D3-R2D2(C162V/Y221S/R222K)-R2D3(-N274F)-Fc | 95.19 | 89.21 | 84.45 |
| PR1169 | R1D2-R2D3-R2D2(C162V/Y221S/R222K)-R2D3(-S283T)-Fc | 88.96 | 80.78 | 75.48 |
| PR1170 | R1D2-R2D3-R2D2(C162V/Y221S/R222K)-R2D3(-N274F/S283T)-Fc | 89.10 | 79.57 | 77.22 |
| PR1171 | R1D2-R2D3-linker-R2D2(C162V)-R2D3(N274F/S283-T)-Fc | 82.88 | 70.43 | 67.74 |
| PR1172 | R1D2-R2D3-linker-R2D2(C162V/Y221S/R222K)-R2D3(-N274F)-Fc | 90.34 | 81.77 | 76.05 |
| PR1173 | R1D2-R2D3-linker-R2D2(C162V/Y221S/R222K)-R2D3(-S283T)-Fc | 91.35 | 81.96 | 77.37 |
| PR1174 | R1D2-R2D3-linker-R2D2(C162V/Y221S/R222K)-R2D3(-N274F/S283T)-Fc | 96.86 | 91.58 | 88.10 |
| PR1179 | R1D2-R2D3-R2D2(C162V/Y221S/R222K)-R2D3-Fc | 90.08 | 80.65 | 76.93 |

Example 9: SPR Assay for Affinity Between VEGF-Binding Molecules and Different Ligands

Surface plasmon resonance (SPR) was used to measure the affinity between different VEGF-binding molecules and VEGF-A/B/C proteins (A Protein A chip was used, and the VEGF-binding molecules were captured onto the chip). The coupling level was set to 100 RU, and measurement was performed using a Biacore instrument (T200, GE Healthcare, BIAC-B20-03). The running buffer was HBS-EP + (10 mM HEPES, 150 mM NaCl, 3 mM EDTA, 0.05% surfactant P20). Diluted antibodies (6.25, 12.5, 25, 50, 100 nM) were flowed through the experimental and reference channels (100 nM VEGF-A/B/C) at a flow rate of 30 $\mu$L/min for 3 min, followed by 5 min of dissociation. Then the regeneration buffer, 10 mM glycine pH 1.5 (GE Healthcare, BR-1003-54), was then run at a flow rate of 30 $\mu$L/min for 30 s. Data were analyzed using Biacore 8K evaluation software.

The affinity constants of the VEGF-binding molecules for the human VEGF-A, VEGF-B, and VEGF-C proteins were calculated using the MuLti-Cycle method. The kinetic parameters Ka, Kd, and KD values obtained from the calculations are shown in the table below.

Table 16. The affinity of VEGF-binding molecules for different ligands

| Protein name | Analyte | Ka (1/Ms) | Kd (1/s) | $K_D$ (M) |
|---|---|---|---|---|
| PR1145 | Human VEGF-A protein | 2.19E+06 | 9.87E-05 | 4.50E-11 |
| PR1174 | | 2.68E+06 | 1.15E-04 | 4.29E-11 |
| PR1163 | | 2.45E+06 | 1.26E-04 | 5.14E-11 |
| PR1186 | | 1.33E+06 | 4.05E-05 | 3.04E-11 |
| Aflibercept | | 1.03E+07 | 8.46E-05 | 8.18E-12 |
| PR1145 | Human VEGF-B protein | 2.05E+07 | 2.71E-04 | 1.32E-11 |
| PR1174 | | 5.01E+05 | 1.61E-04 | 3.21E-10 |
| Aflibercept | | 1.51E+06 | 1.14E-04 | 7.54E-11 |
| PR1145 | Human VEGF-C protein | 2.81E+06 | 7.98E-05 | 2.84E-11 |
| PR1174 | | 2.21E+06 | 5.17E-05 | 2.34E-11 |
| PR1163 | | 6.56E+06 | 8.79E-05 | 1.34E-11 |
| PR1186 | | 3.36E+06 | 4.13E-05 | 1.23E-11 |
| OPT-302 | | 3.21E+06 | 4.85E-05 | 1.51E-11 |

## Example 10: Evaluation of *In Vivo* Stability of VEGF-Binding Molecules

To determine the *in vivo* stability of VEGF-binding molecules, changes in the concentration of these molecules in eye tissues were measured after intravitreal injection of these molecules. The specific experiment was as follows: Fourteen New Zealand rabbits, with an equal number of males and females, weighing 1.5-2.5 kg, were purchased. The experimental animals were divided into four groups: a PR1145 high-dose group, a PR1145 low-dose group, a PR1163 high-dose group, and a blank control group. All procedures were conducted on both eyes. Vitreous and aqueous humor samples were collected from 12 animals in the treatment groups 3 h, 3 d, 7 d, 14 d, and 28 d after bilateral intravitreal administration. After the samples were taken, the content of the target protein in the samples was measured using an enzyme-linked immunosorbent assay. The experiment was completed by Chengdu Bio-HT Company Limited. The doses are shown in Table 17.

Table 17. The administration regimen for the evaluation of the *in vivo* stability of VEGF-binding molecules

| Protein name | Number of animals | Protein dose | Injection volume |
|---|---|---|---|
| PR1145 (high) | 4 | 400 µg/eye | 50 µL |
| PR1145 (low) | 4 | 200 µg/eye | 50 µL |
| PR1163 (high) | 4 | 542 µg/eye | 50 µL |

FIG. 10 shows the VEGF-binding molecule content in rabbits measured 3 h, 3 d, 7 d, 14 d, and 28 d after bilateral intravitreal administration. The results show that in the PR1145 high-dose group, the PR1145 low-dose group, and the PR1163 high-dose group, the drug concentration in the animals peaked 3 days after injection administration. PR1145 and PR1163 were detectable in the animals for 14 consecutive days, indicating that the VEGF-binding molecules have relatively good *in vivo* stability.

## Example 11: Evaluation of *In Vivo* Efficacy of VEGF-Binding Molecules

To evaluate the inhibitory effects of VEGF-binding molecules on fundus choroidal neovascularization, a laser-induced fundus choroidal neovascularization (CNV) model was used for efficacy validation. This model is currently the classic model commonly used internationally for validating anti-choroidal neovascularization drugs. The specific experiment was as follows:

Forty-two rats, with an equal number of males and females, 8-10 weeks old, were purchased, and the experimental animals were divided into seven groups: a PR1145 high-dose group, a PR1145 low-dose group, a control group 1 (aflibercept), a control group 2 (OPT302), and a negative control group (PBS). The administration regimen is shown in Table 11. Each group consisted of 6 animals, and both eyes underwent laser CNV modeling. One week later, 4 μL of protein molecule was injected into both eyes. Two weeks post-administration, fundus angiography was performed to measure efficacy. PR1145 (3.75 mg/mL) and aflibercept (2.5 mg/mL) were administered at equimolar doses, and the molar ratio of aflibercept (2.5 mg/mL) to OPT302 (10 mg/mL) was 1:4.

Table 18. The administration regimen for the evaluation of the *in vivo* efficacy of VEGF-binding molecules

| Protein name | Group | Number of animals | Protein concentration | Injection volume |
|---|---|---|---|---|
| PR1145 (high) | Experimental group | 6 | 3.75 mg/mL | 4 μL |
| PR1145 (low) | Experimental group | 6 | 1.5 mg/mL | 4 μL |
| Aflibercept | Control group 1 | 6 | 2.5 mg/mL | 4 μL |
| OPT302 | Control group 2 | 6 | 10 mg/mL | 4 μL |
| PBS | Negative control | 6 | - | 4 μL |

Fundus photography was performed on the rats before modeling, and no abnormalities were observed in the fundi of the rats. As shown in FIG. 11A, significant laser spot fluorescein sodium leakage was observed in the PBS group 7 days post-modeling, indicating successful model establishment. All the rats were dosed 7 days after CNV modeling, and fluorescein fundus angiography (FFA) analysis was performed 14 days post-administration. In the PBS group, increased fluorescent spot fluorescein sodium leakage was observed, suggesting that within 21 days post-modeling, the degree of laser-induced choroidal neovascularization continued to progress and had a tendency to worsen. The results of FFA analysis of the inhibition of choroidal neovascularization by the VEGF-binding molecules 14 days post-administration are shown in FIG. 11B. Compared to the PBS group, the PR1145 high-dose group, the PR1145 low-dose group, aflibercept, and OPT302 all significantly ameliorated laser-induced choroidal neovascularization fluorescein sodium leakage. The amelioration rate of PR1145 (3.75 mg/mL) was 15.8%, higher than the amelioration rate of aflibercept (2.5 mg/mL) (12.5%). PR1145 (3.75 mg/mL) and OPT302 (10 mg/mL) exhibited comparable ameliorating effects, but the dose of PR1145 was significantly lower.

**Example 12: Structure of Gene Expression Cassette Delivered by AAV**

The expression vector provided in this example comprises a nucleic acid molecule that expresses a VEGF-binding molecule. Its structure is shown in FIG. 8. From the 5' end to the 3' end, it comprises: a CMV enhancer, a promoter, a 5' UTR, a Kozak sequence (gccacc), a VEGFR-binding molecule coding sequence, a 3' UTR, a WPRE, and an SV40polyA. The nucleic acid sequence of the CMV enhancer is set forth in SEQ ID NO: 124, the nucleic acid sequence of the CMV promoter is set forth in SEQ ID NO: 125, the 5' UTR is set forth in SEQ ID NO: 126, the Kozak sequence (gccacc) and the VEGF-binding molecule coding sequence are set forth in any one of SEQ ID NOs: 128-136 (SEQ ID NOs: 128-130 correspond to codon-optimized sequences 1, 2, and 3 of PR1145; SEQ ID NOs: 131-133 correspond to codon-optimized sequences 1, 2, and 3 of PR1174; SEQ ID NOs: 134-136 correspond to codon-optimized sequences 1, 2, and 3 of PR1163), the 3' UTR sequence is set forth in SEQ ID NO: 137, the WPRE sequence is set forth in SEQ ID NO: 138, and the SV40polyA sequence is set forth in SEQ ID NO: 139.

**Example 13: Construction of Expression Vector**

An expression cassette was constructed. An AAV packaging plasmid expressing a VEGF-binding molecule protein gene (see FIG. 9) was constructed by conventional molecular biology procedures such as enzyme digestion, ligation, transformation, and clone screening identification, and it sequentially comprises: a CMV enhancer, a promoter, a 5' UTR, a Kozak sequence (gccacc), a VEGFR-binding molecule, a 3' UTR, a WPRE, and an SV40polyA. The expression cassette was flanked by inverted terminal repeats (ITRs). EcoRV and BSMI were enzymatic digestion sites.

**Example 14: AAV Virus Preparation and Purification**

The amino acid sequence of the AAV capsid used for delivering the gene expression cassette in this example is set forth in SEQ ID NO: 127. Referring to the method for packaging and purifying recombinant AAV viruses reported by Martin Lock et al., PEI was used to co-transfect HEK293 cells with plasmids expressing AAV Rep and Cap proteins, a helper plasmid, and the expression vector of Example 13 to package and prepare a recombinant adeno-associated virus. After 48 h of transfection, cells and the culture supernatant were harvested, and the AAV virus was purified using iodixanol gradient ultracentrifugation (see Martin Lock, et al. Rapid, Simple, and Versatile Manufacturing of Recombinant Adeno-Associated Viral Vectors at Scale. HUMAN GENE THERAPY, 2010. 21:1259-1271) to obtain a recombinant adeno-associated viral vector. The viral titer was measured using real-time fluorescence quantitative PCR.

**Example 15: Intraocular Expression Experiment**

To determine the expression levels of the efficacy proteins in vitreous humor, aqueous humor, retina, and choroid tissues in the rabbit eye and changes in the expression levels after AAV-1174 (expressing the PR1174 (the nucleic acid sequence is SEQ ID NO: 131) efficacy protein) and ADVM-022 (see rAAV2.7m8-aflibercept in WO2017218974) were injected into the rabbit eye, the following experiment was conducted. Thirty-two New Zealand rabbits, 2 to 5 months old, were purchased, divided into 4 groups of 8, and intravitreally injected (both eyes) with the doses shown in Table 12 (each sample was injected at a high dose and at a low dose). At day 58 post-injection, vitreous humor, aqueous humor, retina, and choroid tissues were collected from both eyes. At each time point, the sampling number corresponding to each treatment group was 2 rabbits, i.e., 4 eyes. The samples were sent back to our laboratory, where the efficacy protein content in the samples was measured using an enzyme-linked immunosorbent assay.

Table 19. Dose grouping for the intraocular expression experiment

| Sample | Number (rabbit/eye) | Viral titer | Injection volume | VG/eye |
|---|---|---|---|---|
| AAV-1174 (low dose) | 2/4 | 2E12 vg/mL | 100 μL | 2E11 vg/eye |
| AAV-1174 (high dose) | 2/4 | 6E12 vg/mL | 100 μL | 6E11 vg/eye |
| ADVM-022 (low dose) | 2/4 | 2E12 vg/mL | 100 μL | 2E11 vg/eye |
| ADVM-022 (high dose) | 2/4 | 6E12 vg/mL | 100 μL | 6E11 vg/eye |

FIG. 12 shows the intraocular expression results. At D58, both the AAV-1174 high-dose group and the AAV-1174 low-dose group exhibited stable expression of PR1174 protein in the aqueous humor, vitreous humor, retina, and choroid of the rabbit eye, and the protein expression levels were higher than those of ADVM-022.

**Example 12: *In Vivo* Efficacy Experiment**

The laser-induced fundus choroidal neovascularization model is currently the classic model commonly used internationally for validating anti-choroidal neovascularization drugs. To validate the inhibitory effect of AAV-1174 on fundus choroidal neovascularization, this model was used for efficacy validation.

Thirty-six rats, with an equal number of males and females, 8-10 weeks old, were purchased, divided into 6 groups of 6, and intravitreally injected (both eyes) with the doses shown in Table 13. Three weeks after AAV-1174 (expressing the PR1174 (the nucleic acid sequence is SEQ ID NO: 131) efficacy protein) was administered, both eyes underwent laser modeling. One week post-modeling, fundus angiography was performed to measure efficacy.

Table 20. Dose grouping for the *in vivo* efficacy experiment

| Sample | Number | Viral dose | Injection volume |
|---|---|---|---|
| AAV-1174 (high dose) | 6 | 2E10 vg/mL | 1 μL |
| AAV-1174 (low dose) | 6 | 6E9 vg/mL | 1 μL |
| ADVM-022 (high dose) | 6 | 2E10 vg/mL | 1 μL |
| ADVM-022 (low dose) | 6 | 6E9 vg/mL | 1 μL |
| PBS | 6 | NA | 1 μL |

Table 21. The inhibition of fundus choroidal neovascularization

| Group | D29: grade 3 leakage spot ratio (%) (mean ± SD) | D29 spot leakage average score (mean ± SD) |
|---|---|---|
| 1-PBS | 56.7±27.5 | 2.33±0.50 |
| AAV-1174 (6E+9 vg) | 63.3±24.6 | 2.33±0.42 |
| AAV-1174 (2E+10 vg) | 44.4+21.7 | 2.05+0.40 |

(continued)

| Group | D29: grade 3 leakage spot ratio (%) (mean $\pm$ SD) | D29 spot leakage average score (mean $\pm$ SD) |
|---|---|---|
| ADVM-022 (6E+9 vg) | 63.9+17.2 | 2.42+0.35 |
| ADVM-022 (2E+10 vg) | 40.7+14.7 | 1.81+0.38 |

Fundus photography was performed on the rats before modeling, and no abnormalities were observed in the fundi of the rats. All the rats underwent CNV modeling 21 days post-administration, and FFA analysis was performed 28 days post-administration. Significant laser spot fluorescein sodium leakage was observed in the PBS group 7 days post-modeling, indicating successful model establishment; the fluorescent spot grading score was determined by FFA 28 days post-administration, and the grade 3 leakage spot ratio was determined. The results are shown in Table 21 and FIG. 13. The differences between the AAV-1174 (high dose) group and the PBS group were statistically significant. AAV-1174 exhibited a significant inhibitory effect on laser-induced choroidal neovascularization fluorescein sodium leakage.

**Formulation Component Screening and Stability Evaluation**

In this example, a pharmaceutical composition comprising viral (recombinant adeno-associated virus, rAAV) particles was prepared. The exemplary steps are as follows:

1. Preparation of a formulation buffer comprising rAAV particles and sterilization and filtration treatment of the formulation buffer: a) Filtration was performed using a 0.2 $\mu$m PES filter membrane (syringe filter), with the filter membrane model being KM2EKVS.
2. Preparation of stock solution: After sterilization, the virus suspension was subjected to ultrafiltration using the sterile formulation buffer to complete solvent exchange: a) before buffer exchange by ultrafiltration, a virus suspension sample was first sampled and tested for its physicochemical properties as an initial value; b) an ultrafiltration centrifuge tube was subjected to initial centrifugal washing using purified water, with the centrifugation parameter being 1500 g $\times$ 2 min; c) the ultrafiltration centrifuge tube was subjected to two centrifugal washings using the sterile formulation buffer, with the centrifugation parameter being 1500 g $\times$ 2 min; d) the virus suspension sample was subjected to four centrifugal ultrafiltration buffer exchanges using the sterile formulation buffer, with the volume of the concentrated solution of virus being 1/6 of the volume of the sterile formulation buffer added during each buffer exchange, and the centrifugation parameter being 1500 g $\times$ 1 min (about 2-3 centrifugations).
3. Sterilization and filtration of the virus stock solution obtained after ultrafiltration.
4. Preparation of virus solution (formulation): The virus solution obtained after ultrafiltration was subjected to Vg determination, and the virus solution was diluted based on the determination result.
5. Testing of virus thermal stability and freeze-thaw stability of the virus solution after concentration adjustment. Several indexes such as virus titer, IU, appearance, viral biological activity were mainly observed.

The rAAV particles in Test Example 1 to Test Example 6 described below comprised a nucleic acid molecule I; for example, the rAAV particles were HRPDAAV-02AI or HRPDAAV-03DI. The rAAV particles in Test Example 7 to Test Example 11 comprised a nucleic acid molecule I; for example, the rAAV particle was AAV-1145.

**Test Example 1: Component Screening of Formulas**

1) A formulation buffer was prepared and subjected to sterilization and filtration treatment, with the excipients in the buffer shown in Table 22. 2) After sterilization, the virus suspension was subjected to ultrafiltration using the sterile formulation buffer to complete solvent exchange. 3) The virus stock solution obtained after ultrafiltration was sterilized and filtered.

4) The virus solution obtained after ultrafiltration was subjected to Vg determination, and the virus solution was diluted to 8.5E12 vg/mL (8.5 $\times$ 10$^{12}$ vg/mL) based on the determination result.

V. The virus thermal stability and freeze-thaw stability testing was performed on the virus solution after concentration adjustment. The genome titer Vg was determined by qPCR, and several indexes such as log (IU), appearance, and viral biological activity were also studied. After storage at 25 °C for 1-6 months, storage at 5 °C for 1-18 months, and repeated freeze-thaw treatment, the appearance was examined, the genome titer Vg was determined by qPCR, and the infectious titer IU was determined by TCID50/qPCR.

The stability results are shown in Table 23 and FIG. 14. Under the accelerated (25 °C) condition and the long-term (5 °C) condition, the Vg levels of the viruses in the 3 formulas fluctuated, but there was no downward trend overall. In terms of the long-term condition, under the 6M accelerated condition, the IU of two formulas F2 and F3 was significantly reduced, and histidine was retained in the formula.

Table 22. Formulas for screening

| Excipient | F1 | F2 | F3 |
|---|---|---|---|
| L-histidine | 5 mM | N/A | N/A |
| Sodium chloride | 100 mM | 100 mM | 100 mM |
| Magnesium chloride hexahydrate | 2 mM | 2 mM | 2 mM |
| Glycerol | 0.25%(w/v) | 0.25%(w/v) | 0.25%(w/v) |
| BTP | 10 mM | 10 mM | 10 mM |
| Polysorbate 80 | 0.01% | N/A | N/A |
| Sucrose | 2.50% | 2.50% | N/A |
| Poloxamer 188 | 0.001%(w/v) | 0.01%(w/v) | 0.01%(w/v) |
| Trehalose | N/A | N/A | 2.50%(w/v) |
| pH | pH adjusted to 7.5 | pH adjusted to 7.5 | pH adjusted to 7.5 |

Table 23. Results of stability screening

| Condition | | F1 | | | F2 | | | F3 | | |
| | Time point | Vg (vg/mL) | Log (IU) | Appearance | Vg (vg/mL) | Log (IU) | Appearance | Vg | Log (IU) | Appearance |
|---|---|---|---|---|---|---|---|---|---|---|
| T0 | | 8.49E+12 | 8.47 | Colorless clear liquid | 9.36E+12 | 8.55 | Colorless clear liquid | 8.58E+12 | 8.63 | Colorless clear liquid |
| 25 °C | 1W | 7.13E+12 | 8.08 | Colorless clear liquid | 7.84E+12 | 6.98 | Colorless clear liquid | 7.17E+12 | 7.08 | Colorless clear liquid |
| | 2W | 7.37E+12 | 8.3 | Colorless clear liquid | 7.72E+12 | 7.8 | Colorless clear liquid | 7.27E+12 | 7.55 | Colorless clear liquid |
| | 1M | 5.88E+12 | 8.38 | Colorless clear liquid | 6.55E+12 | 8.05 | Colorless clear liquid | 6.85E+12 | 8.47 | Colorless clear liquid |
| | 2M | 7.58E+12 | 9.13 | Colorless clear liquid | 8.56E+12 | 8.72 | Colorless clear liquid | 8.62E+12 | 9.13 | Colorless clear liquid |
| | 3M | 6.69E+12 | 9.3 | Colorless clear liquid | 7.74E+12 | 9.13 | Colorless clear liquid | 7.52E+12 | 8.63 | Colorless clear liquid |
| | 6M | 8.45E+12 | 8.05 | Colorless clear liquid | 1.08E+13 | 6.88 | Colorless clear liquid | 9.96E+12 | 6.72 | Colorless clear liquid |
| 5 °C | 1M | 5.89E+12 | 8.22 | Colorless clear liquid | N/A | N/A | N/A | N/A | N/A | N/A |
| | 2M | 7.75E+12 | 9.55 | Colorless clear liquid | N/A | N/A | N/A | N/A | N/A | N/A |
| | 3M | 7.47E+12 | 9.63 | Colorless clear liquid | N/A | N/A | N/A | N/A | N/A | N/A |
| | 6M | 9.30E+12 | 9.22 | Colorless clear liquid | 1.02E+13 | 9.22 | Colorless clear liquid | 9.36E+12 | 8.97 | Colorless clear liquid |
| | 9M | 5.60E+12 | 9.05 | Colorless clear liquid | N/A | N/A | N/A | N/A | N/A | N/A |
| | 12M | 7.02E+12 | 8.3 | Colorless clear liquid | 8.18E+13 | 7.97 | Colorless clear liquid | 7.48E+12 | 7.88 | Colorless clear liquid |
| | 18M | 9.60E+12 | 8.22 | Colorless clear liquid | N/A | N/A | N/A | N/A | N/A | N/A |

EP 4 768 039 A1

| Condition | Time point | F1 | | | F2 | | | F3 | | |
|---|---|---|---|---|---|---|---|---|---|---|
| | | Vg (vg/mL) | Log (IU) | Appearance | Vg (vg/mL) | Log (IU) | Appearance | Vg | Log (IU) | Appearance |
| Freeze -thaw test | Once | 6.07E+12 | 9.38 | Colorless clear liquid | There were no significant changes in the VG/IU of the product after 5 freeze-thaw cycles | | | | | |
| | 3 cycles | 6.08E+12 | 9.30 | Colorless clear liquid | | | | | | |
| | 5 cycles | 6.09E+12 | 9.38 | Colorless clear liquid | | | | | | |
| "N/A" means not detected, "W" means week, and "M" means month. | | | | | | | | | | |

**Test Example 2: Component Screening of Formulas**

A virus solution containing rAAV particles was prepared. See Table 24 for information on excipients therein. The stability was studied under different conditions of 25 °C, 5 °C, and ≤ -60 °C. The results are shown in Table 25. The VG trends under different storage conditions were substantially consistent. Under the condition of 25 °C, IU tended to decrease, and the trends under the other three conditions were substantially the same. The quality of the formulations stored at 2-8 °C was equivalent to that at < -60 °C.

Table 24. Formulas for screening

| Excipient | F4 |
|---|---|
| L-histidine | 5 mM |
| Sodium chloride | 100 mM |
| Magnesium chloride hexahydrate | 2 mM |
| Glycerol | 0.25%(w/v) |
| BTP | 10 mM |
| Poloxamer 188 | 0.01 %(w/v) |
| Sucrose | 2.50%(w/v) |
| pH | pH adjusted to 7.5 |

Table 25. Stability study results

| Batch No. | | VG(VG/mL) | Log(IU) |
|---|---|---|---|
| Condition | Time point | | |
| T0 | | 6.20E+12 | 8.88 |
| 25 °C | 1W | 6.12E+12 | 8.55 |
| | 2W | 5.60E+12 | 7.97 |
| | 3W | 5.94E+12 | 9.38 |
| | 1M | 6.26E+12 | 9.05 |
| | 2M | 4.88E+12 | 8.88 |
| | 3M | 5.55E+12 | 8.80 |
| | 6M | 5.90E+12 | 8.22 |
| 5 °C | 1W | 6.05E+12 | 8.22 |
| | 2W | 5.42E+12 | 7.97 |
| | 3W | 5.66E+12 | 9.13 |
| | 1M | 6.75E+12 | 9.30 |
| | 2M | 4.69E+12 | 9.55 |
| | 3M | 5.50E+12 | 9.13 |
| | 6M | 5.56E+12 | 9.38 |

(continued)

| Batch No. | | VG(VG/mL) | Log(IU) |
|---|---|---|---|
| Condition | Time point | | |
| ≤-60 °C | 2W | 6.17E+12 | 8.13 |
| | 1M | 6.76E+12 | 9.30 |
| | 2M | 4.50E+12 | 9.30 |
| | 3M | 5.80E+12 | 9.13 |
| | 6M | 5.33E+12 | 9.55 |
| | 9M | 7.63E+12 | 8.80 |
| | 12M | 6.28E+12 | 9.22 |

**Test Example 3: pH Screening of Formula**

1) A formulation buffer was prepared according to the following formula: 5 mM L-histidine, 100 mM sodium chloride, 2 mM magnesium chloride hexahydrate, 0.25% (w/v) glycerol, 10 mM BTP, 2.50% (w/v) sucrose, and 0.01% (w/v) poloxamer 188, and the formulation buffer was adjusted to 6.0, 6.5, 7.0, 7.5, and 8.0 separately at 25 °C using a sodium hydroxide or hydrochloric acid solution. 2) The formulation buffer after pH adjustment was sterilized and filtered. 3) After sterilization, the virus suspension was subjected to ultrafiltration using the sterile formulation buffer to complete solvent exchange. 4) The virus stock solution obtained after ultrafiltration was sterilized and filtered. 5) The virus solution obtained after ultrafiltration was subjected to Vg determination, and the virus solution was diluted to (6-7)E12 vg/mL based on the determination result.

The virus thermal stability and freeze-thaw stability testing was performed on the virus solution after concentration adjustment, and several indexes such as virus titer, IU, appearance, and viral biological activity were mainly observed. The results are shown in Table 27 and show that in the pH range of 6-8, no significant reductions were observed in the indexes such as VG and IU of the product under the 3M accelerated condition.

Table 26. Test conditions

| pH | Formula | Stability conditions and time points | Test items |
|---|---|---|---|
| | | 25 °C/relative humidity at 60% | |
| 6 | F5 | 0/1W/2W/3W/1M/2M/3M | VG, IU |
| 6.5 | F6 | 0/1W/2W/3W1/M/2M/3M | |
| 7 | F7 | 0/1W/2W/3W1/M/2M/3M | |
| 7.5 | F8 | 0/1W/2W/3W1/M/2M/3M | |
| 8 | F9 | 0/1W/2W/3W1/M/2M/3M | |

Table 27. Study on effect of different pH on virus stability

| Time point | F5 | | F6 | | F7 | | F8 | | F9 | |
|---|---|---|---|---|---|---|---|---|---|---|
| | Vg (vg/mL) | LOG (IU) | Vg (vg/mL) | LOG (IU) | Vg (vg/mL) | LOG (IU) | Vg (vg/mL) | LOG (IU) | Vg (vg/mL) | LOG (IU) |
| T0 | 6.64E+12 | 9.97 | 6.29E+12 | 10.05 | 6.81E+12 | 10.22 | 6.62E+12 | 10.13 | 6.70E+12 | 8.55 |
| 1W | 7.64E+12 | 9.97 | 7.11E+12 | 9.8 | 7.67E+12 | 9.8 | 7.44E+12 | 9.55 | 7.42E+12 | 10.13 |
| 2W | 8.90E+12 | 9.97 | 8.31E+12 | 9.8 | 8.39E+12 | 9.88 | 8.77E+12 | 9.97 | 8.32E+12 | 9.97 |
| 3W | 8.26E+12 | 10.22 | 7.92E+12 | 9.88 | 8.12E+12 | 9.97 | 8.81E+12 | 9.97 | 8.62E+12 | 9.72 |
| 1M | 8.35E+12 | 9.8 | 8.65E+12 | 9.97 | 7.99E+12 | 9.63 | 8.65E+12 | 9.38 | 8.59E+12 | 9.8 |
| 2M | 7.16E+12 | 9.8 | 6.93E+12 | 9.97 | 7.68E+12 | 9.47 | 7.39E+12 | 9.55 | 7.28E+12 | 9.72 |
| 3M | 8.53E+12 | 9.55 | 8.58E+12 | 9.88 | 8.79E+12 | 9.72 | 8.71E+12 | 9.3 | 8.84E+12 | 9.47 |

(continued)

| Time point | F5 | | F6 | | F7 | | F8 | | F9 | |
|---|---|---|---|---|---|---|---|---|---|---|
| | Vg (vg/mL) | LOG (IU) | Vg (vg/mL) | LOG (IU) | Vg (vg/mL) | LOG (IU) | Vg (vg/mL) | LOG (IU) | Vg (vg/mL) | LOG (IU) |
| 6M | N/A | N/A | N/A | N/A | N/A | N/A | 8.62E+12 | 8.88 | N/A | N/A |
| "N/A" means not detected. | | | | | | | | | | |

**Test Example 4: Effect of Other Buffer Systems in Formulation on Virus Stability**

1) A formulation buffer was prepared according to the information of formula F10 shown in Table 28, and the pH of the formulation buffer was adjusted to 7.5 at 25 °C. 2) The formulation buffer was sterilized and filtered. 3) After sterilization, the virus suspension was subjected to ultrafiltration using the sterile formulation buffer to complete solvent exchange. 4) The virus stock solution obtained after ultrafiltration was sterilized and filtered. 5) The virus solution obtained after ultrafiltration was subjected to Vg determination, and the virus solution was diluted to 9.6E12 vg/mL based on the determination result. The virus thermal stability and freeze-thaw stability testing was performed on the virus solution after concentration adjustment, and several indexes such as virus titer, IU, appearance, and viral biological activity were mainly observed. The viral biological activity was obtained by infecting cells with the virus and detecting the protein expression by SDS-PAGE.

The results are shown in Table 29. The results for test items such as viral genome titer, AAV capsid protein purity, infectious titer, biological activity, and pH met the quality standards under the long-term (12M) and accelerated (3M) conditions, and showed no significant changes. The biological activity was evaluated by measuring AADC expression (pg/μg), and the AADC expression was 5.7 pg/μg at T0 and showed no significant change after 3M treatment under the long-term and accelerated conditions. The empty-to-full ratio of the AAV viral vector met the quality standards under the 6M long-term and 3M accelerated conditions, and there was no significant change.

Table 28. Excipients in formula F10

| Excipient | Formula | Percentage |
|---|---|---|
| L-histidine | 5 mM | 0.08%(w/v) |
| Sodium chloride | 100 mM | 0.58%(w/v) |
| Magnesium chloride hexahydrate | 2 mM | 0.04%(w/v) |
| Glycerol | 0.25%(w/v) | |
| HEPES | 10 mM | 0.24%(w/v) |
| Sucrose | 2.50%(w/v) | |
| Poloxamer 188 | 0.01%(w/v) | |
| pH | 7.5 | |

Table 29. Effect of other buffer systems in formulation on virus stability

| Time point | Genome titer (vg/mL) | | Infectious titer (log IU) | | Purity of AAV capsid protein | | Empty-to-full ratio of AAV viral vector | | pH | |
|---|---|---|---|---|---|---|---|---|---|---|
| | 5 °C | 25 °C | 5 °C | 25 °C | 5 °C | 25 °C | 5 °C | 25 °C | 5 °C | 25 °C |
| T0 | 9.60E+12 | | 9.88 | | 99.80% | | 14% | | 7.5 | |
| 2W | N/A | 8.80E+12 | N/A | N/A | N/A | N/A | N/A | N/A | N/A | 7.5 |
| 1M | N/A | 8.60E+12 | N/A | N/A | N/A | 99.30% | N/A | 12% | N/A | 7.5 |
| 2M | N/A | N/A | N/A | N/A | N/A | N/A | N/A | N/A | N/A | N/A |
| 3M | 9.10E+12 | 8.00E+12 | 9.72 | 9.72 | 99.70% | 99.10% | 14% | 12% | 7.5 | 7.5 |
| 6M | 8.70E+12 | N/A | 9.38 | N/A | 99.20% | N/A | 15% | N/A | 7.6 | N/A |

(continued)

| Time point | Genome titer (vg/mL) | | Infectious titer (log IU) | | Purity of AAV capsid protein | | Empty-to-full ratio of AAV viral vector | | pH | |
|---|---|---|---|---|---|---|---|---|---|---|
| | 5 °C | 25 °C | 5 °C | 25 °C | 5 °C | 25 °C | 5 °C | 25 °C | 5 °C | 25 °C |
| 12M | 7.70E+12 | N/A | 9.55 | N/A | 99.80% | N/A | N/A | N/A | 7.6 | N/A |
| "N/A" means not detected. | | | | | | | | | | |

## Test Example 5. Study on Effect of Temperature on Formulation pH

1. A formulation buffer at pH 7.5 was prepared at 27.2 °C according to the formula F10.
2. About 3 mL of the formulation buffer was added to a centrifuge tube. The pH value in the process of cooling from room temperature to about 2 °C was measured with a pH meter in an ice-water bath and recorded, and then the pH change in the process of heating the solution from 2 °C to 30 °C was recorded.

The results are shown in FIG. 15. The temperature had a certain effect on the pH of the buffer. The pH increased when the temperature decreased, and the pH increased to 8.08 when the temperature decreased to 2 °C. When the temperature increased, the pH decreased. For every 10 °C temperature change, the pH changed by about 0.24. When the temperature increased to 30 °C, the pH decreased to 7.42.

3. The formulation buffer was adjusted to 5 different pH values of 6.0, 6.5, 7.0, 7.5, and 8.0 at 25 °C and adjusted to pH 7.5 at 5 °C using a sodium hydroxide or hydrochloric acid solution, and the pH change of the solution at 2 °C to 30 °C was recorded.

The results are shown in FIG. 16. At 25 °C, there were cases where temperature changes brought about pH changes in buffers at 5 different pH values. When the temperature increased, the pH decreased; when the temperature decreased, the pH increased; when the pH was in the range of 6.0-8.0, for every 10 °C temperature change of the solution, the pH changed by about 0.2.

## Test Example 6: Study on Temperature Sensitivity of pH of Different Buffers

1. Buffer working solutions were prepared using different buffers, and the buffering ranges are shown in Table 30.
2. About 3 mL of each of different buffers was added to a centrifuge tube. The pH value in the process of cooling from 30 °C to about 2 °C was measured with a pH meter in an ice-water bath and recorded, and then the pH change in the process of heating the solution from 2 °C to 30 °C was recorded.

Table 30. Buffering ranges of different buffers

| Buffer system | BTP | Tris | HEPES |
|---|---|---|---|
| Buffering range | 6.3-9.5 | 7.0-9.2 | 6.8-8.2 |

The results are shown in FIG. 17. The pH values of the 3 buffer systems BTP, Tris, and HEPES all decreased with the increase of the temperature. The pH values of BTP and Tris decreased by about 0.024 as the temperature increased by 1 °C. The pH value of HEPES decreased by about 0.001 as the temperature increased by 1 °C. The stability of the HEPES buffer system was relatively high.

## Test Example 7. Study on Effect of Different pH Range on Stability

1) According to the formulas shown in Test Example 3, formulation buffers were prepared and each adjusted to 6.5, 7.0, 7.5, 8.0, and 8.5 (F11-F15) at 25 °C using a sodium hydroxide or hydrochloric acid solution.

EP 4 768 039 A1

2) After sterilization, the virus suspension was subjected to ultrafiltration using the sterile formulation buffer to complete solvent exchange.

3) The virus stock solution obtained after ultrafiltration was sterilized and filtered.

4) The virus solution obtained after ultrafiltration was subjected to Vg determination, and the virus solution was diluted to (1.1-1.4)E13 vg/mL based on the determination result. The virus thermal stability (storage at 25 °C) testing was performed on the virus solution after concentration adjustment, and several indexes such as virus particle size, titer, IU, appearance, viral biological activity, and MFI were mainly observed. The particle size of the virions was observed by dynamic light scattering (DLS), and the degradation of the virions was studied by the micro-flow imaging particle analysis method (MFI). IP (representing a viral particle containing an incomplete gene) and FP (representing a viral particle with a complete gene sequence) were detected by AUC (analytical ultracentrifuge). The results are shown in Table 32. After being stored for 1M to 2M, the particles of the samples at each pH gradient were stable in particle size, and the purity was reduced to a certain extent.

Table 31. Formulas of test stock solution and test formulation

| Formula | | | F11 | F12 | F13 | F14 | F15 |
|---|---|---|---|---|---|---|---|
| pH value | | | 6.5 | 7 | 7.5 | 8 | 8.5 |
| Stock solution | DLS | | 29.83 nm | 30.39 nm | 30 nm | 29.25 nm | 29.33 nm |
| | VG | | 1.1E13 Vg/mL | 1.1E13 Vg/mL | 1.4E13 Vg/mL | 1.2E13 Vg/mL | 1.1E13 Vg/mL |
| | Purity | | / | / | 0.97% | / | / |
| | Host DNA (HCD) | | / | / | 3 ng/1E12VG | / | / |
| | Host protein (HCP) | | / | / | <20 ng/mL | / | / |
| Formulation | DLS | | 30.06 nm | 29.45 nm | 30.25 nm | 29.08 nm | 29.84 nm |
| | MFI | ≥10 μm | 50 | 66 | 25 | 7 | 28 |
| | | ≥25 μm | 9 | 14 | 2 | 0 | 7 |
| | | ≥50 μm | 2 | 0 | 0 | 0 | 0 |
| | pH | | 6.63 | 7.08 | 7.53 | 7.87 | 8.19 |
| | Osmolality | | 317 mOsm | 320 mOsm | 327 mOsm | 324 mOsm | 326 mOsm |
| | Vg | | 9.3E12 vg/mL | 9.6E12 vg/mL | 1.0E13 vg/mL | 1.0E13 vg/mL | 9.3E12 vg/mL |
| | Purity | | 0.992 | 0.99 | 0.982 | 0.981 | 0.97 |
| | AUC | | IP:21%, 64s | IP:21%, 64s | IP:21%, 64s | IP:21%, 64s | IP:21%, 64s |
| | | | FP:78%, 98s | FP: 78%, 98s | FP: 78%, 98s | FP: 78%, 98s | FP: 78%, 98s |

Table 32. Study on storage stability at 25 °C

| pH | Storage time | DLS | | pH | VG | Log(IU) | Biological activity (ng/mL) | Purity | MFI | | |
|---|---|---|---|---|---|---|---|---|---|---|---|
| | | Size (nm) | PDI | | (VG/mL) | | | | ≥10 μm | ≥25 μm | ≥50 μm |
| 6.5 | T0 | 30.06 | 0.238 | 7 | 9.30E+12 | 11 | 323 | 0.99 | 50 | 9 | 2 |
| | 2W | 29.54 | 0.2362 | 7 | 8.60E+12 | N/A | N/A | / | / | / | / |

(continued)

| pH | Storage time | DLS Size (nm) | DLS PDI | pH | VG (VG/mL) | Log(IU) | Biological activity (ng/mL) | Purity | MFI ≥10 μm | MFI ≥25 μm | MFI ≥50 μm |
|---|---|---|---|---|---|---|---|---|---|---|---|
| | 1M | 29.59 | 0.2302 | 7 | 1.10E+13 | 11 | 265 | 0.97 | 7 | 0 | 0 |
| | 2M | 31.32 | 0.2893 | 7 | 1.10E+13 | N/A | N/A | 0.96 | 2 | 2 | 0 |
| 7 | T0 | 29.45 | 0.2296 | 7 | 9.60E+12 | 12 | 316.9 | 0.99 | 66 | 14 | 0 |
| | 2W | 28.27 | 0.1894 | 7 | 9.70E+12 | N/A | N/A | / | / | / | / |
| | 1M | 29.31 | 0.2659 | 7 | 1.10E+13 | 12 | 278.8 | 0.95 | 2 | 2 | 0 |
| | 2M | 28.84 | 0.1532 | 7 | 1.10E+13 | N/A | N/A | 0.92 | 0 | 0 | 0 |
| 7.5 | T0 | 30.25 | 0.2238 | 8 | 1.00E+13 | 12 | 311.8 | 0.98 | 25 | 2 | 0 |
| | 2W | 28.98 | 0.1773 | 8 | 1.10E+13 | N/A | N/A | / | / | / | / |
| | 1M | 30.22 | 0.1985 | 8 | 1.30E+13 | 12 | 295.8 | 0.96 | 3 | 2 | 0 |
| | 2M | 30.90 | 0.2168 | 8 | 1.20E+13 | N/A | N/A | 0.9 | 14 | 2 | 5 |
| 8 | T0 | 29.08 | 0.1876 | 8 | 1.00E+13 | 12 | 248.6 | 0.98 | 7 | 0 | 0 |
| | 2W | 28.65 | 0.1723 | 8 | 1.00E+13 | N/A | N/A | / | / | / | / |
| | 1M | 29.03 | 0.1654 | 8 | 1.20E+13 | 11 | 246 | 0.92 | 0 | 0 | 0 |
| | 2M | 29.84 | 0.1619 | 8 | 1.20E+13 | N/A | N/A | 0.89 | 46 | 0 | 0 |
| 8.5 | T0 | 29.84 | 0.1899 | 8 | 9.30E+12 | 12 | 296.1 | 0.97 | 28 | 7 | 0 |
| | 2W | 29.98 | 0.203 | 8 | 1.10E+13 | N/A | N/A | / | / | / | / |
| | 1M | 30.07 | 0.1781 | 8 | 1.20E+13 | 11 | 163.7 | 0.91 | 10 | 2 | 0 |
| | 2M | 31.57 | 0.2042 | 8 | 1.20E+13 | N/A | N/A | 0.86 | 5 | 2 | 0 |

## Test Example 8: Effect of Temperature and Formulation pH Range on Virus Stability

1. A high-temperature forced degradation experiment was performed on the stock solution to study the effect of temperature on product quality. The experimental conditions and results are shown in Table 33. It was found that different high-temperature degradation conditions had no significant effect on the purity of the stock solution of this batch of products.

Table 33. Results of high-temperature forced degradation experimental conditions and purity measurement

| | F15 | | | | |
|---|---|---|---|---|---|
| Condition | T0 | 40 °C | | 100 °C | |
| | | 30 min | 5 min | 10 min | 30 min |
| Purity | 0.995 | 0.991 | 0.976 | 0.99 | 0.958 |

2. The effect of different acidic and basic conditions on product stability was studied.

1) Based on the formula F10, sodium chloride was adjusted to 150 mM, a formulation buffer was prepared and then sterilized and filtered, and the pH of the formulation buffer was adjusted to 5, 9, and 6.8 (corresponding to F16-F18, respectively) at 25 °C using a sodium hydroxide or hydrochloric acid solution.
2) Filtration was performed using a 0.2 μm PES filter membrane (syringe filter), with the filter membrane model being KM2EKVS.
3) After sterilization, the virus suspension was subjected to ultrafiltration using the sterile formulation buffer to complete solvent exchange.
4) The virus stock solution obtained after ultrafiltration was sterilized and filtered. The virus solution obtained after ultrafiltration was subjected to Vg determination, and the virus solution was diluted to (1.1-1.2)E13 vg/mL based on the determination result.

The stability of formulations with different pH values was studied according to the conditions in Table 26, with a focus on the virus titer and purity. The results are shown in Table 34. There was no significant decrease in virus purity under the 3 pH conditions at 5 °C. Compared with the 5 °C condition, after 1M storage under 25 °C accelerated condition, the purity was reduced at pH 9.0, and the purity was affected at high pH. Under the condition of 40 °C, both the VG and the purity were reduced, and under the condition of pH 5.0, the VG was reduced more.

Table 34. Effect of different pH values of formulations on virus stability

| Batch No. | | | | F16 | F17 | F18 |
|---|---|---|---|---|---|---|
| pH | | | | 5.0 | 9.0 | 6.8 |
| T0 | | VG | | 1.00E+13 | 1.00E+13 | 1.00E+13 |
| | | Purity | | 0.995 | 0.957 | 0.962 |
| Sample storage for stability test | 5 °C | 1M | VG | 1.00E+13 | 1.00E+13 | 1.00E+13 |
| | | | Purity | 0.993 | 0.99 | 0.989 |
| | 25 °C | 1W | Purity | 0.987 | 0.986 | 0.983 |
| | | 1M | VG | 1.00E+13 | 1.00E+13 | 1.00E+13 |
| | | | Purity | 0.983 | 0.967 | 0.988 |
| | 40 °C | 1W | Purity | 0.986 | 0.94 | 0.974 |
| | | 1M | VG | 5.00E+12 | 8.00E+12 | 1.00E+13 |
| | | | Purity | 0.913 | 0.909 | 0.791 |

**Test Example 9: Effect of Formulation pH Range on Virus Stability**

According to the formula in Table 35, a formulation buffer was prepared and then sterilized and filtered. After sterilization, the virus suspension was subjected to ultrafiltration using the sterile formulation buffer to complete solvent exchange. The virus stock solution obtained after ultrafiltration was sterilized and filtered. The virus solution obtained after ultrafiltration was subjected to Vg determination, and the virus solution was diluted to 9.8E12-1.3E13 Vg/mL based on the determination result.

The virus thermal stability and freeze-thaw stability testing was performed on the virus solution after concentration adjustment, and several indexes such as virus titer and purity were mainly observed. The results are shown in Table 36. After being stored for 3 months under a long-term condition, the VG and the purity of the product were stable; under the 25 °C accelerated condition, the VG of the product was stable, the purity was reduced to a certain extent at 3M, but it met the quality standard.

Table 35. Formula to be screened

| Excipient | Concentration | Percentage% (w/v) |
|---|---|---|
| Sodium chloride | 150 mM | 0.88%(w/v) |
| Magnesium chloride hexahydrate | 2 mM | 0.04%(w/v) |
| HEPES | 10 mM | 0.24%(w/v) |
| Histidine | 5 mM | 0.08%(w/v) |
| Poloxamer 188 | 0.05 mg | 0.01%(w/v) |
| Sucrose | | 1.0%(w/v) |
| Glycerol | | 0.25%(w/v) |

Table 36. Effect of formulation pH range on virus stability

| pH | | | 5.5 | | 6 | | 6.5 | | 7 | |
|---|---|---|---|---|---|---|---|---|---|---|
| Formula | | | F19 | | F20 | | F21 | | F22 | |
| Test conditions | | | VG | Purity | VG | Purity | VG | Purity | VG | Purity |
| T0 | | | 9.8E+12 | 0.988 | 9.3E+12 | 0.992 | 1.3E+13 | 0.987 | 1.3E+13 | 0.992 |
| Stability temperature | 5 °C | 1M | 1.1E+13 | 0.988 | 9.7E+12 | 0.987 | 1.4E+13 | 0.985 | 1.3E+13 | 0.99 |
| | | 2M | 1.1E+13 | 0.99 | 9.5E+12 | 0.982 | 1.2E+13 | 0.988 | 1.2E+13 | 0.985 |
| | | 3M | 1.1E+13 | 0.99 | 9.8E+12 | 0.978 | 1.3E+13 | 0.987 | 1.5E+13 | 0.985 |
| | 25 °C | 1M | 1.2E+13 | 0.99 | 9.7E+12 | 0.986 | 1.3E+13 | 0.987 | 1.3E+13 | 0.979 |
| | | 2M | -- | 0.97 | -- | 0.98 | -- | 0.987 | -- | 0.979 |
| | | 3M | 8.9E+12 | 0.958 | 9.8E+12 | 0.967 | 1.4E+13 | 0.96 | 1.4E+13 | 0.952 |
| "--" means not detected. | | | | | | | | | | |

**Test Example 10. Effect of Formulation pH on Virus Tm Value**

According to the formula in Table 37, a formulation buffer was prepared and then sterilized and filtered. After sterilization, the virus suspension was subjected to ultrafiltration using the sterile formulation buffer to complete solvent exchange. The virus stock solution obtained after ultrafiltration was sterilized and filtered. The Tm value of the virus solution after ultrafiltration was measured. The results are shown in Table 38. Different pH conditions of the formulation had a significant effect on the Tm value of the virus.

Table 37. Formula of test formulation

| Excipient | Concentration | Percentage |
|---|---|---|
| Sodium chloride | 150 mM | 0.88%(w/v) |
| Magnesium chloride hexahydrate | 2 mM | 0.04%(w/v) |
| HEPES | 10 mM | 0.24%(w/v) |
| Histidine | 5 mM | 0.08%(w/v) |
| Poloxamer 188 | 0.05 mg | 0.01 %(w/v) |
| Sucrose | 2.50%(w/v) | |
| Glycerol | 0.25%(w/v) | |

Table 38. Effect of pH of test formulation on Tm value of virus

| Type of formulation buffer | pH | Formula | Tm(°C) |
|---|---|---|---|
| NaCl 150 mM | 5 | F22 | 72.72 |
| | 5.5 | F23 | 75.81 |
| | 6 | F24 | 77.4 |
| | 6.5 | F25 | 76.61 |
| | 6.8 | F26 | 73.51 |
| | 7 | F27 | 72.78 |
| | 7.5 | F28 | 68.43 |
| | 9 | F29 | 61.28 |

**Test Example 11. Effect of Formula on Virus Stability**

According to the formula in Table 39, a formulation buffer was prepared and then sterilized and filtered. After sterilization, the virus suspension was subjected to ultrafiltration using the sterile formulation buffer to complete solvent exchange. The virus stock solution obtained after ultrafiltration was sterilized and filtered. The virus solution obtained after ultrafiltration was subjected to Vg determination, and the virus solution was diluted to (1.2-1.4)E13 vg/mL based on the determination result.

The virus thermal stability and freeze-thaw stability testing was performed on the virus solution after concentration adjustment, and several indexes such as virus titer, purity, potency, IU, appearance, and viral biological activity were mainly observed. The results are shown in Table 40. After being stored for 1 month, 2 months, 3 months, and 6 months under a long-term condition, the VG, the purity, the *in vitro* biological activity, and the infectious titer of the product were all stable.

Table 39. Formula of test formulation

| Excipient | Concentration | Percentage |
|---|---|---|
| Sodium chloride | 150 mM | 0.88%(w/v) |
| Magnesium chloride hexahydrate | 2 mM | 0.04%(w/v) |
| HEPES | 10 mM | 0.24%(w/v) |
| Histidine | 5 mM | 0.08%(w/v) |
| Poloxamer 188 | 0.05 mg | 0.01 %(w/v) |
| Sucrose | 2.50%(w/v) | |
| Glycerol | 0.25%(w/v) | |
| pH | 6.0 | |

Table 40. Effect of formula of test formulation on virus stability

| Test items | | T0 | 5 °C | | | 25 °C | |
|---|---|---|---|---|---|---|---|
| | | | 1M | 3M | 6M | 1M | 2M |
| Appearance and description | Appearance | Colorless clear liquid | Colorless clear liquid | Colorless clear liquid | Colorless clear liquid | Colorless clear liquid | Colorless clear liquid |
| Identification | AAV capsid identification | 96% | / | / | / | / | / |
| | Gene nucleotide sequence determination | Consistent with target sequence | / | / | / | / | / |
| Purity and impurities | Purity of AAV capsid protein | 99.20% | 98.80% | 99.00% | 98.80% | 98.70% | 97.90% |
| | Empty-to-full ratio of AAV viral vector | EP: 23% FP: 74% | EP: 24% FP: 74% | EP: 24% FP: 74% | EP: 25% FP: 74% | / | / |
| | Residual amount of polysorbate 20 | <0.05 mg/mL | / | / | / | / | / |
| Potency | *In vitro* biological activity | 344.1 ng/mL | 390.9 ng/mL | 398.6 ng/mL | 431.5 ng/mL | 313.9 ng/mL | 252.9 ngmL |
| | Infectious titer (logIU) | 11.8 | 12.05 | 11.55 | 12.05 | 11.72 | 11.13 |
| Content | Genome titer | 1.4E13 vg/ml | 1.5E13 vg/mL | 1.4E13 vg/mL | 1.2E13 vg/mL | 1.5E13 vg/mL | 1.3E13 vg/mL |
| | Content of poloxamer 188 | 0.01% | / | / | / | / | / |
| General inspection | pH value | 6.6 | 6.6 | 6.6 | 6.6 | 6.6 | 6.6 |
| | Insoluble microparticle | $\geq 10\ \mu m$, 1 microparticle/m L | / | / | / | $\geq 10\ \mu m$, 1 microparticle/m L | $\geq 10\ \mu m$, 2 microparticles/m L |
| | | $\geq 25\ \mu m$, 1 microparticle/m L | | | | $\geq 25\ \mu m$, 1 microparticle/m L | $\geq 25\ \mu m$, 0 microparticles/m L |
| | | $\geq 50\ \mu m$, 1 microparticle/m L | | | | $\geq 50\ \mu m$, 1 microparticle/m L | $\geq 50\ \mu m$, 0 microparticles/m L |
| | Particle size distribution | 30 nm | 30 nm | 30 nm | 29 nm | 30 nm | 30 nm |
| | Determination of osmolarity | 400 | / | / | / | / | / |
| | Visible foreign matter | Comply with the standard | Comply with the standard | Comply with the standard | Comply with the standard | Comply with the standard | Comply with the standard |

(continued)

| Test items | | T0 | 5 °C | | | 25 °C | |
|---|---|---|---|---|---|---|---|
| | | | 1M | 3M | 6M | 1M | 2M |
| | Bacterial endotoxin | <0.4EU/ml | / | / | / | <0.4EU/ml | <0.4EU/ml |
| | Sterility test (Ch.P) | Comply with the standard | / | / | / | / | / |
| | Sterility test (USP) | Comply with the standard | / | / | / | / | / |

The following sequences are also used in the present disclosure:

>Amino acid sequence of AADC (SEQ ID NO: 1)

MNASEFRRRGKEMVDYVANYMEGIEGRQVYPDVEPGYLRPLIPAAAPQEPDTFEDIIN
DVEKIIMPGVTHWHSPYFFAYFPTASSYPAMLADMLCGAIGCIGFSWAASPACTELET
VMMDWLGKMLELPKAFLNEKAGEGGGVIQGSASEATLVALLAARTKVIHRLQAASP
ELTQAAIMEKLVAYSSDQAHSSVERAGLIGGVKLKAIPSDGNFAMRASALQEALERDK
AAGLIPFFMVATLGTTTCCSFDNLLEVGPICNKEDIWLHVDAAYAGSAFICPEFRHLLN
GVEFADSFNFNPHKWLLVNFDCSAMWVKKRTDLTGAFRLDPTYLKHSHQDSGLITD
YRHWQIPLGRRFRSLKMWFVFRMYGVKGLQAYIRKHVQLSHEFESLVRQDPRFEICV
EVILGLVCFRLKGSNKVNEALLQRINSAKKIHLVPCHLRDKFVLRFAICSRTVESAHVQ
RAWEHIKELAADVLRAERE

>Amino acid sequence of GDNF (SEQ ID NO: 2)

MKLWDVVAVCLVLLHTASAFPLPAGKRPPEAPAEDRSLGRRRAPFALSSDSNMPEDYP
DQFDDVMDFIQATIKRLKRSPDKQMAVLPRRERNRQAAAANPENSRGKGRRGQRGK
NRGCVLTAIHLNVTDLGLGYETKEELIFRYCSGSCDAAETTYDKILKNLSRNRRLVSD
KVGQACCRPIAFDDDLSFLDDNLVYHILRKHSAKRCGCI

> AADC wild-type DNA (SEQ ID NO: 3)

atgaacgcaagtgaattccgaaggagagggaaggagatggtggattacatggccaactacatggaaggcattgagggacgccaggt
ctaccctgacgtggagcccgggtacctgcggccgctgatccctgccgctgcccctcaggagccagacacgtttgaggacatcatcaa
cgacgttgagaagataatcatgcctggggtgacgcactggcacagcccctacttcttcgcctacttccccactgccagctcgtacccgg
ccatgcttgcggacatgctgtgcgggggccattggctgcatcggcttctcctgggcggcaagcccagcatgcacagagctggagactgt
gatgatggactggctcgggaagatgctggaactaccaaaggcattttgaatgagaaagctggagaaggggaggagtgatccagg
gaagtgccagtgaagccaccctggtggccctgctggccgctcggaccaaagtgatccatcggctgcaggcagcgtccccagagctc
acacaggccgctatcatggagaagctggtggcttactcatccgatcaggcacactcctcagtggaaagagctgggttaattggtggagt
gaaattaaaagccatcccctcagatggcaacttcgccatgcgtgcgtctgccctgcaggaagccctggagagagacaaagcggctgg
cctgattcctttctttatggttgccaccctggggaccacaacatgctgctcctttgacaatctcttagaagtcggtcctatctgcaacaagga
agacatatggctgcacgttgatgcagcctacgcaggcagtgcattcatctgccctgagttccggcaccttctgaatggagtggagtttgc
agattcattcaactttaatccccacaaatggctattggtgaattttgactgttctgccatgtgggtgaaaaagagaacagacttaacgggag

cctttagactggacccccacttacctgaagcacagccatcaggattcagggcttatcactgactaccggcattggcagataccactgggc
agaagatttcgctctttgaaaatgtggtttgtatttaggatgtatggagtcaaaggactgcaggcttatatccgcaagcatgtccagctgtc
ccatgagtttgagtcactggtgcgccaggatccccgctttgaaatctgtgtggaagtcattctggggcttgtctgctttcggctaaaggggtt
ccaacaaagtgaatgaagctcttctgcaaagaataaacagtgccaaaaaaatccacttggttccatgtcacctcagggacaagtttgtcc
tgcgctttgccatctgttctcgcacggtggaatctgcccatgtgcagcgggcctgggaacacatcaaagagctggcggccgacgtgct
gcgagcagagagggagtag

>AADC01 DNA (SEQ ID NO:4)

atgaatgctagcgagtttcgcagaagggggcaaggagatggtggattacgtggctaattacatggagggcatcgagggccggcaggtg
tacccagatgtggagccaggctacctgagaccactgatccctgctgccgccccacaggagccagacaccttcgaggatatcatcaatg
acgtggagaagatcatcatgcctggcgtgacccactggcatagcccatacttctttgcctactttccaacagcctcttcctatcccgctatg
ctggctgatatgctgtgcggcgctatcggctgcatcggcttctcttgggccgctagccctgcttgcacagagctggagaccgtgatgat
ggattggctgggcaagatgctggagctgcctaaggcctttctgaacgagaaggctggcgagggcggcggcgtgatccagggctctg
cctccgaggctacactggtggctctgctggctgccccggacaaaggtaatccacagactgcaggctgcctctccagagctgacccagg
ccgctatcatggagaagctggtggcttactctagcgaccaggctcattcttccgtggagcgggctggcctgatcggcggcgtgaagct
gaaggccatcccttccgacggcaatttcgccatgagagcttctgccctgcaggaggctctggagagagataaggctgccggcctgatc
cctttctttatggtggccaccctgggcacaaccacctgttgctccttcgacaacctgctggaggtgggccctatctgtaacaaggaggac
atctggctgcatgtggatgccgcttatgctggctctgcctttatctgccccgagtttagacatctgctgaacggcgtggagtttgccgaca
gctttaacttcaaccctcacaagtggctgctggtgaattttgactgctctgccatgtgggtgaagaagaggacagacctgaccggcgctt
ttaggctggatccaacctacctgaagcacagccatcaggacagcggcctgatcacagactacagacactggcagatcccactgggca
gaaggtttcggtctctgaagatgtggttcgtgtttcgcatgtatggcgtgaagggcctgcaggcttatatccggaagcacgtgcagctgt
ctcatgagttcgagtccctggtgcggcaggacccaagatttgagatctgcgtggaggtaatcctgggcctggtgtgcttcaggctgaag
ggctctaataaggtgaacgaggctctgctgcagaggatcaacagcgccaagaagatccatctggtgccttgtcatctgagggacaagt
tcgtgctgagattcgccatctgctctagaacagtggagtctgctcatgtgcagagggcttgggagcatatcaaggagctggccgctgac
gtgctgagggctgagagggagtga

>AADC02 DNA (SEQ ID NO:5)

atgaacgctagcgagtttcggaggagagaggcaaggagatggtggactacgtggccaactacatggagggcatcgagggcagacagg
tgtacccagacgtggagcctggctatctgcggccactgatccctgctgccgccccacaggagccagatacctttgaggacatcatcaa
cgacgtggagaagatcatcatgcccggcgtgacccactggcattcccccttacttctttgcctactttcctacagcttcttcctatcctgccat
gctggccgatatgctgtgcggcgctatcggctgcatcggcttctcttgggccgcttcccccgcttgcaccgagctggagaccgtgatga
tggattggctgggcaagatgctggagctgcccaaggctttcctgaatgagaaggctggcgagggcggcggcgtgatccagggctct
gcttccgaggccacactggtggccctgctggctgccaggacaaaggtaatccacagactgcaggctgcctcccccgagctgaccca
ggccgctatcatggagaagctggtggcttactcctctgaccaggctcactcttccgtggagagagctggcctgatcggcggcgtgaag
ctgaaggctatcccttctgatggcaatttcgccatgagggcttctgctctgcaggaggctctggagagggataaggctgccggcctgat
ccccttttttcatggtggccaccctgggcacaaccacctgttgcagctttgacaatctgctggaggtgggccctatctgcaacaaggagg
acatctggctgcacgtggatgctgcctacgctggcagcgccttcatctgtcccgagtttaggcacctgctgaatggcgtggagtttgctg
actcctttaatttcaaccccataagtggctgctggtgaacttcgactgctctgctatgtgggtgaagaagaggacagatctgacaggcg
cctttaggctggacccaacctacctgaagcactcccatcaggatagcggcctgatcaccgactaccggcactggcagatccctctgggg
caggcgctttagatccctgaagatgtggttcgtgtttaggatgtacggcgtgaagggcctgcaggcttatatccggaagcatgtgcagct
gtcccacgagttcgagtctctggtgcggcaggacccaaggtttgagatctgcgtggaggtaatcctgggcctggtgtgcttcagactga
agggctccaataaggtgaacgaggctctgctgcagagaatcaactccgccaagaagatccacctggtgccttgtcacctgagggataa
gttcgtgctgaggtttgccatctgctctagaaccgtggagagcgcccatgtgcagagggcttgggagcacatcaaggagctggctgcc
gacgtgctgagggctgagagagagtga

>AADC03 DNA (SEQ ID NO:6)

atgaatgcctctgagttccgcagacgcggcaaggagatggtggactacgtggctaactacatggagggcatcgagggcagacaggt

gtaccccgacgtggagcctggctatctgaggccactgatccctgctgctgcccctcaggagcccgacaccttcgaggatatcatcaat
gacgtggagaagatcatcatgccaggcgtgacccattggcactctccatacttctttgcttacttccctaccgcctcttcctatccagctatg
ctggccgatatgctgtgcggcgccatcggctgcatcggctttagctgggctgcctctccagcttgtaccgagctggagacagtgatgat
ggactggctgggcaagatgctggagctgcctaaggcctttctgaacgagaaggccggcgagggcggcggcgtgatccagggctcc
gcttctgaggccaccctggtggctctgctggctgccagaaccaaggtaatccacaggctgcaggccgctagccctgagctgacccag
gccgctatcatggagaagctggtggcttactcttccgaccaggcccattctagcgtggagagggctggcctgatcggcggcgtgaag
ctgaaggctatcccttctgacggcaacttcgccatgagagctagcgccctgcaggaggccctggagcgggacaaggctgccggcct
gatcccatttttcatggtggctaccctgggcacaacaacctgctgttctttcgacaacctgctggaggtgggccctatctgcaataaggag
gacatctggctgcatgtggatgctgcctatgctggcagcgcctttatctgcccagagttcaggcatctgctgaacggcgtggagtttgcc
gactcttttaacttcaatccccataagtggctgctggtgaactttgactgtagcgccatgtgggtgaagaagaggacagatctgaccggc
gcctttcggctggatcctacctacctgaagcactctcatcaggattctggcctgatcaccgattacagacattggcagatccctctgggcc
ggagatttcggagcctgaagatgtggttcgtgtttcggatgtatggcgtgaagggcctgcaggcctatatcagaaagcacgtgcagctg
tcccacgagtttgagagcctggtgcgccaggacccacggtttgagatctgcgtggaggtaatcctgggcctggtgtgcttcaggctgaa
gggctctaataaggtgaacgaggctctgctgcagcgcatcaactccgctaagaagatccacctggtgccttgtcacctgcgggacaag
ttcgtgctgaggtttgccatctgctccagaaccgtggagtctgcccatgtgcagagagcttgggagcatatcaaggagctggccgctga
tgtgctgagggctgagagagagtga

>AADC04 DNA (SEQ ID NO:7)

atgaacgcctctgagtttcggaggaggggcaaggagatggtggactacgtggctaactatatggagggcatcgagggcagacaggt
gtaccctgacgtggagccaggctacctgagaccactgatcccagccgccgctcctcaggagcctgacacctttgaggacatcatcaat
gatgtggagaagatcatcatgcctggcgtgacccattggcacagccccttactttttcgcctatttcccaaccgctagctcctatccagccat
gctggctgatatgctgtgcggcgcctatcggctgtatcggctttagctgggctgcctctcctgcttgtacagagctggagacagtgatgat
ggattggctgggcaagatgctggagctgcccaaggcctttctgaacgagaaggccggcgagggcggcggcgtgatccagggctct
gcttctgaggccacactggtggctctgctggccgctaggaccaaggtaatccacaggctgcaggctgccagcccagagctgacccca
ggctgccatcatggagaagctggtggcctactctagcgaccaggctcattcctctgtggagagggctggcctgatcggcggcgtgaa
gctgaaggctatcccttccgatggcaatttcgctatgagagccagcgccctgcaggaggctctggagagggacaaggctgccggcct
gatccctttcttcatggtggccacactgggcacaaccacatgctgttctttcgacaacctgctggaggtgggccccatctgcaataagga
ggacatctggctgcatgtggatgctgcctacgccggctccgcttttatctgccctgagtttcggcatctgctgaacggcgtggagtttgcc
gacagctttaacttcaatccccacaagtggctgctggtgaacttcgactgctctgctatgtgggtgaagaagaggacagatctgaccgg
cgctttcagactggatccaacctacctgaagcactctcatcaggactctggcctgatcaccgattaccggcattggcagatccctctggg
ccgccggtttaggagcctgaagatgtggttcgtgtttcggatgtatggcgtgaagggcctgcaggcctatatccggaagcatgtgcagc
tgtctcatgagtttgagtccctggtgcgccaggacccaggttcgagatctgcgtggaggtaatcctgggcctggtgtgcttcaggctga
agggcagcaataaggtgaacgaggctctgctgcagaggatcaattccgctaagaagatccacctggtgccttgtcatctgcgggataa
gtttgtgctgaggttcgccatctgtagccgcaccgtggagtctgcccacgtgcagagagcttgggagcacatcaaggagctggccgct
gatgtgctgagagctgagagggagtga

>GDNF wild-type DNA (SEQ ID NO: 8)

atgaagttatgggatgtcgtggctgtctgcctggtgctgctccacaccgcgtccgccttcccgctgcccgccggtaagagagcctcccga
ggcgcccgccgaagaccgctccctcggccgccgccgcgcgcccttcgcgctgagcagtgactcaaatatgccagaggattatcctg
atcagttcgatgatgtcatggatttattcaagccaccattaaaagactgaaaaggtcaccagataaacaaatggcagtgcttcctagaag
agagcggaatcggcaggctgcagctgccaacccagagaattccagaggaaaaggtcggagaggccagaggggcaaaaaccggg
gttgtgtcttaactgcaatacatttaaatgtcactgacttgggtctgggctatgaaaccaaggaggaactgatttttaggtactgcagcggc
tcttgcgatgcagctgagacaacgtacgacaaaatattgaaaaacttatccagaaatagaaggctggtgagtgacaaagtagggcagg
catgttgcagacccatcgcctttgatgatgacctgtcgttttagatgataacctggtttaccatattctaagaaagcattccgctaaaaggt
gtggatgtatctga

>GDNF01 DNA (SEQ ID NO:9)

atgaagctgtgggatgtggtggccgtgtgcctggtgctgctgcatacagctagcgcctttcccctgccagctggcaagagaccacctg
aggctcctgccgaggacagatctctgggcaggagacgggccccttcgctctgtctagcgattccaacatgcccgaggactatccaga
ccagttcgatgacgtgatggacttcatccaggctaccatcaagaggctgaagagaagcccagacaagcagatggctgtgctgcctag
acgcgagaggaatagacaggccgctgctgccaatcctgagaacagcaggggcaagggccggaggggccagcgcggcaagaata
gaggctgcgtgctgaccgctatccacctgaacgtgacagacctgggcctgggctacgagaccaaggaggagctgatcttccgctatt
gttccggctcttgtgatgccgctgagaccacatatgacaagatcctgaagaacctgagcagaaacaggagactggtgtctgacaaggt
gggccaggcctgttgcagaccaatcgccttcgatgatgacctgagctttctggacgataacctggtgtatcacatcctgagaaagcactc
tgccaagagatgtggctgcatctga

>GDNF02 DNA (SEQ ID NO:10)

atgaagctgtgggatgtggtggccgtgtgcctggtgctgctgcatacagcttctgcctttcctctgcccgctggcaagagagcctccaga
ggcccccagctgaggacagatccctgggcagaaggagagccccttttgctctgtccagcgactctaacatgcccgaggattatcccgac
cagttcgacgatgtgatggacttcatccaggctaccatcaagaggctgaagagaagcccagataagcagatggctgtgctgccacgc
agagagaggaacagacaggccgctgctgctaaccctgagaactctcggggcaagggcagaaggggccagagaggcaagaatcg
cggctgcgtgctgacagctatccacctgaacgtgacagatctgggcctgggctacgagacaaaggaggagctgatcttcagatactgt
tctggcagctgtgatgctgccgagacaacctacgacaagatcctgaagaacctgagcagaaatcggaggctggtgtccgataaggtg
ggccaggcctgttgcagacctatcgctttcgacgacgatctgagcttcctggacgataacctggtgtaccacatcctgcggaagcattct
gccaagagatgcggctgtatctga

>GDNF03 DNA (SEQ ID NO:11)

atgaagctgtgggatgtggtggccgtgtgcctggtgctgctgcatacagcttctgccttccctctgccagctggcaagagaccacctga
ggcccccgctgaggatagaagcctgggccgcagaagggctccttttgccctgagctccgactctaatatgcctgaggattacccagac
cagttcgatgacgtgatggactttatccaggccacaatcaagcgcctgaagaggtctccagacaagcagatggctgtgctgcctagga
gagagagaaaccggcaggctgccgctgctaatccagagaactctaggggcaagggcaggagaggccagagggggcaagaatcgc
ggctgcgtgctgaccgctatccatctgaatgtgaccgatctgggcctgggctacgagacaaaggaggagctgatcttcaggtactgca
gcggctcttgtgacgccgctgagaccacatacgacaagatcctgaagaacctgagcagaaaccgcagactggtgtctgataaggtgg
gccaggcttgttgcaggcccatcgcttttgatgacgatctgtctttcctggacgataatctggtgtaccacatcctgcggaagcactctgct
aagcggtgtggctgcatctga

>GDNF04 DNA (SEQ ID NO: 12)

atgaagctgtgggatgtggtggccgtgtgcctggtgctgctgcacaccgcttctgccttcccactgcctgccggcaagagacctcccga
ggcccctgccgaggacagaagcctgggcaggcggagagccccatttgctctgtctagcgattccaacatgcctgaggattaccccga
tcagttcgatgacgtgatggatttcatccaggccaccatcaagagactgaagagatctcctgacaagcagatggctgtgctgcctagaa
gggagagaaacaggcaggccgctgctgccaatccagagaactccaggggcaagggcagaaggggccagcgcggcaagaatag
aggctgcgtgctgacagccatccacctgaacgtgaccgacctgggcctgggctacgagaccaaggaggagctgatcttcaggtactg
tagcggctcctgtgatgctgccgagaccacatacgacaagatcctgaagaacctgtccaggaacagaaggctggtgtctgacaaggt
gggccaggcttgctgtaggccaatcgctttcgacgacgatctgtcctttctggatgacaacctggtgtaccacatcctgaggaagcattc
cgctaagagatgtggctgcatctga

>HRPDAAV01-A (SEQ ID NO:13)

cctgcaggcagctgcgcgctcgctcgctcactgaggccgcccgggcaaagcccgggcgtcgggcgacctttggtcgcccggcctc
agtgagcgagcgagcgcgcagagagggagtggccaactccatcactaggggttcctatcgatatcaagcttcgttacataacttacgg
taaatggcccgcctggctgaccgcccaacgaccccgcccattgacgtcaatagtaacgccaatagggactttccattgacgtcaatg
ggtggagtatttacggtaaactgcccacttggcagtacatcaagtgtatcatatgccaagtacgccccctattgacgtcaatgacggtaaa
tggcccgcctggcattgtgcccagtacatgaccttatgggactttcctacttggcagtacatctacgtattagtcatcgctattaccatggtc
gaggtgagccccacgttctgcttcactctccccatctcccccccctccccacccccaattttgtatttatttattttttaattattttgtgcagcg
atggggcggggggggggggggggcgcgcgccaggcggggcggggcggggcgaggggcggggcggggcgaggcggaga
ggtgcggcggcagccaatcagagcggcgcgctccgaaagtttcctttatggcgaggcggcggcggcggcggccctataaaaagc

gaagcgcgcggcgggcgggagtcgctgcgcgctgccttcgccccgtgccccgctccgccgccgcctcgcgccgcccgccccggc
tctgactgaccgcgttactcccacaggtgagcgggcgggacggcccttctcctccgggctgtaattagctgagcaagaggtaagggtt
taagggatggttggttggtggggtattaatgtttaattacctggagcacctgcctgaaatcacttttttttcaggttggggggattcgaacatcg
gccgccaccatgaacgccagcgagttcaggaggaggggcaaggagatggtggactacgtggccaactacatggagggcatcgag
ggcaggcaggtgtaccccgacgtggagcccggctacctgaggcccctgatccccgccgccgcccccccaggagcccgacaccttcg
aggacatcatcaacgacgtggagaagatcatcatgcccggcgtgacccactggcacagccccctacttcttcgcctacttccccaccgc
cagcagctaccccgccatgctggccgacatgctgtgcggcgccatcggctgcatcggcttcagctgggccgccagccccgcctgca
ccgagctggagaccgtgatgatggactggctgggcaagatgctggagctgcccaaggccttcctgaacgagaaggccggcgaggg
cggcggcgtgatccagggcagcgccagcgaggccaccctggtggccctgctggccgccaggaccaaggtgatccacaggctgca
ggccgccagccccgagctgacccaggccgccatcatggagaagctggtggcctacagcagcgaccaggcccacagcagcgtgga
gagggccggcctgatcggcggcgtgaagctgaaggccatccccagcgacggcaacttcgccatgagggccagcgccctgcagga
ggccctggagagggacaaggccgccggcctgatccccttcttcatggtggccaccctgggcaccaccacctgctgcagcttcgacaa
cctgctggaggtgggccccatctgcaacaaggaggacatctggctgcacgtggacgccgcctacgccggcagcgccttcatctgcc
ccgagttcaggcacctgctgaacggcgtggagttcgccgacagcttcaacttcaaccccccacaagtggctgctggtgaacttcgactg
cagcgccatgtgggtgaagaagaggaccgacctgaccggcgccttcaggctggaccccacctacctgaagcacagccaccaggac
agcggcctgatcaccgactacaggcactggcagatccccctgggcaggaggttcaggagcctgaagatgtggttcgtgttcaggatgt
acggcgtgaagggcctgcaggcctacatcaggaagcacgtgcagctgagccacgagttcgagagcctggtgaggcaggaccccca
ggttcgagatctgcgtggaggtgatcctgggcctggtgtgcttcaggctgaagggcagcaacaaggtgaacgaggccctgctgcaga
ggatcaacagcgccaagaagatccacctggtgccctgccacctgagggacaagttcgtgctgaggttcgccatctgcagcaggaccg
tggagagcgcccacgtgcagagggcctgggagcacatcaaggagctggccgccgacgtgctgagggccgagagggagtgactc
gaggggtggcatccctgtgaccccctccccagtgcctctcctggccctggaagttgccactccagtgcccaccagccttgtcctaataaa
attaagttgcatcattttgtctgactaggtgtccttctataatattatggggtggagggggtggtatggagcaaggggcaagttgggaag
acaacctgtagggcctgcggggtctattgggaaccaagctggagtgcagtggcacaatcttggctcactgcaatctccgcctcctgggt
tcaagcgattctcctgcctcagcctcccgagttgttgggattccaggcatgcatgaccaggctcagctaattttgtttttttggtagagacg
gggtttcaccatattggccaggctggtctccaactcctaatctcaggtgatctacccaccttggcctcccaaattgctgggattacaggcg
tgaaccactgctcccttccctgtccttcggaccgagcggcagatctaggaacccctagtgatggagttggccactccctctctgcgcgct
cgctcgctcactgaggccgggcgaccaaaggtcgcccgacgcccgggctttgcccggcggcctcagtgagcgagcgagcgcgc
agctgcctgcagg

>HRPDAAV01-B (SEQ ID NO:14)

cctgcaggcagctgcgcgctcgctcgctcactgaggccgcccgggcaaagcccgggcgtcgggcgacctttggtcgcccggcctc

agtgagcgagcgagcgcgcagagagggagtggccaactccatcactaggggttcctatcgatatcaagcttcgttacataacttacgg

taaatggcccgcctggctgaccgcccaacgaccccgcccattgacgtcaatagtaacgccaatagggactttccattgacgtcaatg

ggtggagtatttacggtaaactgcccacttggcagtacatcaagtgtatcatatgccaagtacgccccctattgacgtcaatgacggtaaa

tggcccgcctggcattgtgcccagtacatgaccttatgggactttcctacttggcagtacatctacgtattagtcatcgctattaccatggtc

gaggtgagccccacgttctgcttcactctccccatctcccccccctccccacccccaattttgtatttatttattttttaattattttgtgcagcg

atggggcgggggggggggggggggcgcgcgccaggcggggcggggcggggcgaggggcggggcggggcgaggcggaga

ggtgcggcggcagccaatcagagcggcgcgctccgaaagtttcctttttatggcgaggcggcggcggcggcggccctataaaaagc

gaagcgcgcggcgggcgggagtcgctgcgcgctgccttcgccccgtgccccgctccgccgccgcctcgcgccgcccgccccggc

tctgactgaccgcgttactcccacaggtgagcgggcgggacggcccttctcctccgggctgtaattagctgagcaagaggtaagggtt

taagggatggttggttggtggggtattaatgtttaattacctggagcacctgcctgaaatcacttttttttcaggttggggggattcgaacatcg

gccgccaccatgaacgccagcgagttcaggaggaggggcaaggagatggtggactacgtggccaactacatggagggcatcgag

ggcaggcaggtgtaccccgacgtggagcccggctacctgaggcccctgatccccgccgccgcccccaggagcccgacaccttcg

aggacatcatcaacgacgtggagaagatcatcatgcccggcgtgacccactggcacagcccctacttcttcgcctacttccccaccgc

cagcagctaccccgccatgctggccgacatgctgtgcggcgccatcggctgcatcggcttcagctgggccgccagccccgcctgca

ccgagctggagaccgtgatgatggactggctggggcaagatgctggagctgcccaaggccttcctgaacgagaaggccggcgaggg

cggcggcgtgatccagggcagcgccagcgaggccaccctggtggccctgctggccgccaggaccaaggtgatccacaggctgca

ggccgccagccccgagctgacccaggccgccatcatggagaagctggtggcctacagcagcgaccaggcccacagcagcgtgga

gagggccggcctgatcggcggcgtgaagctgaaggccatcccccagcgacggcaacttcgccatgagggccagcgccctgcagga

ggccctggagagggacaaggccgccggcctgatcccccttcttcatggtggccaccctgggcaccaccacctgctgcagcttcgacaa

cctgctggaggtgggcccccatctgcaacaaggaggacatctggctgcacgtggacgccgcctacgccggcagcgccttcatctgcc

ccgagttcaggcacctgctgaacggcgtggagttcgccgacagcttcaacttcaaccccccacaagtggctgctggtgaacttcgactg

cagcgccatgtgggtgaagaagaggaccgacctgaccggcgccttcaggctggaccccacctacctgaagcacagccaccaggac

agcggcctgatcaccgactacaggcactggcagatcccccctgggcaggaggttcaggagcctgaagatgtggttcgtgttcaggatgt

acggcgtgaagggcctgcaggcctacatcaggaagcacgtgcagctgagccacgagttcgagagcctggtgaggcaggaccccca

ggttcgagatctgcgtggaggtgatcctgggcctggtgtgcttcaggctgaagggcagcaacaaggtgaacgaggccctgctgcaga

ggatcaacagcgccaagaagatccacctggtgccctgccacctgagggacaagttcgtgctgaggttcgccatctgcagcaggaccg

tggagagcgcccacgtgcagagggcctgggagcacatcaaggagctggccgccgacgtgctgagggccgagagggagtgactc

gagtaagatacattgatgagtttggacaaaccacaactagaatgcagtgaaaaaaatgctttatttgtgaaatttgtgatgctattgctttattt

gtaaccattataagctgcaataaacaagttcggaccgagcggcagatctaggaacccctagtgatggagttggccactccctctctgcg

cgctcgctcgctcactgaggccgggcgaccaaaggtcgcccgacgcccgggctttcccgggcggcctcagtgagcgagcgagc

gcgcagctgcctgcagg

>HRPDAAV01-C (SEQ ID NO:15)

cctgcaggcagctgcgcgctcgctcgctcactgaggccgcccgggcaaagcccgggcgtcgggcgacctttggtcgcccggcctc

agtgagcgagcgagcgcgcagagagggagtggccaactccatcactaggggttcctatcgatatcaagcttcgttacataacttacgg

taaatggcccgcctggctgaccgcccaacgaccccgcccattgacgtcaatagtaacgccaatagggactttccattgacgtcaatg

ggtggagtatttacggtaaactgcccacttggcagtacatcaagtgtatcatatgccaagtacgcccccattgacgtcaatgacggtaaa

tggcccgcctggcattgtgcccagtacatgaccttatgggactttcctacttggcagtacatctacgtattagtcatcgctattaccatggtc

gaggtgagccccacgttctgcttcactctccccatctcccccccctccccacccccaattttgtatttatttattttttaattattttgtgcagcg

atggggggcggggggggggggggggcgcgcgccaggcggggcggggcggggcgaggggcggggcggggcgaggcggaga

ggtgcggcggcagccaatcagagcggcgcgctccgaaagtttccttttatggcgaggcggcggcggcggcggccctataaaaagc

gaagcgcgcggcgggcgggagtcgctgcgcgctgccttcgccccgtgccccgctccgccgccgcctcgcgccgcccgccccggc

tctgactgaccgcgttactcccacaggtgagcgggcgggacggcccttctcctccgggctgtaattagctgagcaagaggtaagggtt

taagggatggttggttggtggggtattaatgtttaattacctggagcacctgcctgaaatcactttttttcaggttggggggattcgaacatcg

gccgccaccatgaacgccagcgagttcaggaggaggggcaaggagatggtggactacgtggccaactacatggagggcatcgag

ggcaggcaggtgtaccccgacgtggagcccggctacctgaggcccctgatccccgccgccgcccccccaggagcccgacaccttcg

aggacatcatcaacgacgtggagaagatcatcatgcccggcgtgacccactggcacagcccctacttcttcgcctacttccccaccgc

cagcagctaccccgccatgctggccgacatgctgtgcggcgccatcggctgcatcggcttcagctgggccgccagccccgcctgca

ccgagctggagaccgtgatgatggactggctgggcaagatgctggagctgcccaaggccttcctgaacgagaaggccggcgaggg

cggcggcgtgatccagggcagcgccagcgaggccaccctggtggccctgctggccgccaggaccaaggtgatccacaggctgca

ggccgccagccccgagctgacccaggccgccatcatggagaagctggtggcctacagcagcgaccaggcccacagcagcgtgga

gagggccggcctgatcggcggcgtgaagctgaaggccatccccagcgacggcaacttcgccatgagggccagcgccctgcagga

ggccctggagagggacaaggccgccggcctgatccccttcttcatggtggccaccctgggcaccaccacctgctgcagcttcgacaa

cctgctggaggtgggccccatctgcaacaaggaggacatctggctgcacgtggacgccgcctacgccggcagcgccttcatctgcc

ccgagttcaggcacctgctgaacggcgtggagttcgccgacagcttcaacttcaaccccccacaagtggctgctggtgaacttcgactg

cagcgccatgtgggtgaagaagaggaccgacctgaccggcgccttcaggctggaccccacctacctgaagcacagccaccaggac

agcggcctgatcaccgactacaggcactggcagatcccccctgggcaggaggttcaggagcctgaagatgtggttcgtgttcaggatgt

acggcgtgaagggcctgcaggcctacatcaggaagcacgtgcagctgagccacgagttcgagagcctggtgaggcaggacccca

ggttcgagatctgcgtggaggtgatcctgggcctggtgtgcttcaggctgaagggcagcaacaaggtgaacgaggccctgctgcaga

ggatcaacagcgccaagaagatccacctggtgccctgccacctgagggacaagttcgtgctgaggttcgccatctgcagcaggaccg

tggagagcgcccacgtgcagagggcctgggagcacatcaaggagctggccgccgacgtgctgagggccgagagggagtgactc

gagaatcaacctctggattacaaaatttgtgaaagattgactggtattcttaactatgttgctccttttacgctatgtggatacgctgctttaat

gcctttgtatcatgctattgcttcccgtatggctttcattttctcctccttgtataaatcctggttgctgtctctttatgaggagttgtggcccgttg

tcaggcaacgtggcgtggtgtgcactgtgtttgctgacgcaacccccactggttggggcattgccaccacctgtcagctcctttccggg

actttcgctttccccctccctattgccacggcggaactcatcgccgcctgccttgcccgctgctggacaggggctcggctgttgggcact

gacaattccgtggtgttgtcggggaaatcatcgtcctttccttggctgctcgcctgtgttgccacctggattctgcgcgggacgtccttctg

ctacgtcccttcggccctcaatccagcggaccttccttcccgcggcctgctgccggctctgcggcctcttccgcgtcttcgccttcgccct

cagacgagtcggatctccctttgggccgcctccccgctaagatacattgatgagtttggacaaaccacaactagaatgcagtgaaaaaa

atgctttatttgtgaaatttgtgatgctattgctttatttgtaaccattataagctgcaataaacaagttcggaccgagcggcagatctaggaa

cccctagtgatggagttggccactccctctctgcgcgctcgctcgctcactgaggccgggcgaccaaaggtcgcccgacgcccggg

ctttgcccgggcggcctcagtgagcgagcgagcgcgcagctgcctgcagg

>HRPDAAV02-A (SEQ ID NO:16)

cctgcaggcagctgcgcgctcgctcgctcactgaggccgcccgggcaaagcccgggcgtcgggcgacctttggtcgcccggcctc

agtgagcgagcgagcgcgcagagagggagtggccaactccatcactaggggttcctatcgatatcaagcttcgttacataacttacgg

taaatggcccgcctggctgaccgcccaacgacccccgcccattgacgtcaatagtaacgccaatagggactttccattgacgtcaatg

ggtggagtatttacggtaaactgcccacttggcagtacatcaagtgtatcatatgccaagtacgccccctattgacgtcaatgacggtaaa

tggcccgcctggcattgtgcccagtacatgaccttatgggactttcctacttggcagtacatctacgtattagtcatcgctattaccatggtc

gaggtgagccccacgttctgcttcactctccccatctcccccccctccccacccccaattttgtatttatttattttttaattattttgtgcagcg

atggggcggggggggggggggggcgcgcgccaggcggggcggggcggggcgaggggcggggcggggcgaggcggaga

ggtgcggcggcagccaatcagagcggcgcgctccgaaagtttcctttatggcgaggcggcggcggcggcggccctataaaaagc

gaagcgcgcggcgggcgggagtcgctgcgcgctgccttcgccccgtgccccgctccgccgccgcctcgcgccgcccgcccccggc

tctgactgaccgcgttactcccacaggtgagcgggcgggacggcccttctcctccgggctgtaattagctgagcaagaggtaagggtt

taagggatggttggttggtggggtattaatgtttaattacctggagcacctgcctgaaatcactttttttcaggttggggggattcgaacatcg

gccgccaccatgaacgccagcgagttcaggaggaggggcaaggagatggtggactacgtggccaactacatggagggcatcgag

ggcaggcaggtgtaccccgacgtggagcccggctacctgaggcccctgatccccgccgccgccccccaggagcccgacaccttcg

aggacatcatcaacgacgtggagaagatcatcatgcccggcgtgacccactggcacagcccctacttcttcgcctacttccccaccgc

cagcagctaccccgccatgctggccgacatgctgtgcggcgccatcggctgcatcggcttcagctgggccgccagccccgcctgca

ccgagctggagaccgtgatgatggactggctgggcaagatgctggagctgcccaaggccttcctgaacgagaaggccggcgaggg

cggcggcgtgatccagggcagcgccagcgaggccaccctggtggccctgctggccgccaggaccaaggtgatccacaggctgca

ggccgccagccccgagctgacccaggccgccatcatggagaagctggtggcctacagcagcgaccaggcccacagcagcgtgga

gagggccggcctgatcggcggcgtgaagctgaaggccatccccagcgacggcaacttcgccatgagggccagcgccctgcagga

ggccctggagagggacaaggccgccggcctgatccccttcttcatggtggccaccctgggcaccaccacctgctgcagcttcgacaa

cctgctggaggtgggcccccatctgcaacaaggaggacatctggctgcacgtggacgccgcctacgccggcagcgccttcatctgcc

ccgagttcaggcacctgctgaacggcgtggagttcgccgacagcttcaacttcaaccccccacaagtggctgctggtgaacttcgactg

cagcgccatgtgggtgaagaagaggaccgacctgaccggcgccttcaggctggaccccacctacctgaagcacagccaccaggac

agcggcctgatcaccgactacaggcactggcagatcccccctgggcaggaggttcaggagcctgaagatgtggttcgtgttcaggatgt

acggcgtgaagggcctgcaggcctacatcaggaagcacgtgcagctgagccacgagttcgagagcctggtgaggcaggaccccca

ggttcgagatctgcgtggaggtgatcctgggcctggtgtgcttcaggctgaagggcagcaacaaggtgaacgaggccctgctgcaga

ggatcaacagcgccaagaagatccacctggtgccctgccacctgagggacaagttcgtgctgaggttcgccatctgcagcaggaccg

tggagagcgcccacgtgcagagggcctgggagcacatcaaggagctggccgccgacgtgctgagggccgagagggaggccacc

aacttctccctgctgaagcaggccggcgacgtggaggagaaccccggccccatgaagttatgggatgtcgtggctgtctgcctggtgc

tgctccacaccgcgtccgccttcccgctgccccgccggtaagaggcctcccgaggcgcccgccgaagaccgctccctcggccgccg

ccgcgcgcccttcgcgctgagcagtgactcaaatatgccagaggattatcctgatcagttcgatgatgtcatggatttttattcaagccacc

attaaaaagactgaaaaggtcaccagataaacaaatggcagtgcttcctagaagagagcggaatcggcaggctgcagctgccaacccagagaattccagaggaaaaggtcggagaggccagaggggcaaaaaaccggggttgtgtcttaactgcaatacatttaaatgtcactgacttgggtctgggctatgaaaccaaggaggaactgattttaggtactgcagcggctcttgcgatgcagctgagacaacgtacgacaaaatattgaaaaacttatccagaaatagaaggctggtgagtgacaaagtagggcaggcatgttgcagaccatcgcctttgatgatgacctgtcgtttttagatgataacctggtttaccatattctaagaaagcattccgctaaaaggtgtggatgtatctgactcgagaatcaacctctggattacaaaatttgtgaaagattgactggtattcttaactatgttgctcctttacgctatgtggatacgctgctttaatgcctttgtatcatgctattgcttcccgtatggctttcattttctcctccttgtataaatcctggttgctgtctctttatgaggagttgtggcccgttgtcaggcaacgtggcgtggtgtgcactgtgtttgctgacgcaacccccactggttggggcattgccaccacctgtcagctcctttccgggactttcgctttccccctccctattgccacggcggaactcatcgccgcctgccttgcccgctgctggacaggggctcggctgttgggcactgacaattccgtggtgttgtcggggaaatcatcgtcctttccttggctgctcgcctgtgttgccacctggattctgcgcgggacgtccttctgctacgtccttcggccctcaatccagcggaccttccttcccgcggcctgctgccggctctgcggcctcttccgcgtcttcgccttcgccctcagacgagtcggatctcccttggggccgcctccccgctaagatacattgatgagtttggacaaaccacaactagaatgcagtgaaaaaaatgctttatttgtgaaatttgtgatgctattgctttatttgtaaccattataagctgcaataaacaagttcggaccgagcggcagatctaggaacccctagtgatggagttggccactccctctctgcgcgctcgctcgctcactgaggccgggcgaccaaaggtcgcccgacgcccgggctttgcccgggcggcctcagtgagcgagcgagcgcgcagctgcctgcagg

>HRPDAAV02-B (SEQ ID NO:17)

cctgcaggcagctgcgcgctcgctcgctcactgaggccgcccgggcaaagcccgggcgtcgggcgacctttggtcgcccggcctc

agtgagcgagcgagcgcgcagagagggagtggccaactccatcactaggggttcctatcgatatcaagcttcgttacataacttacgg

taaatggcccgcctggctgaccgcccaacgaccccgcccattgacgtcaatagtaacgccaataagggactttccattgacgtcaatg

ggtggagtatttacggtaaactgcccacttggcagtacatcaagtgtatcatatgccaagtacgcccccattgacgtcaatgacggtaaa

tggcccgcctggcattgtgcccagtacatgaccttatgggactttcctacttggcagtacatctacgtattagtcatcgctattaccatggtc

gaggtgagccccacgttctgcttcactctccccatctccccccccctccccacccccaattttgtatttatttattttttaattattttgtgcagcg

atggggggcggggggggggggggggcgcgcgccaggcggggcggggcggggcgaggggcggggcggggcgaggcggaga

ggtgcggcggcagccaatcagagcggcgcgctccgaaagtttcctttttatggcgaggcggcggcggcggcggccctataaaaagc

gaagcgcgcggcgggcgggagtcgctgcgcgctgccttcgccccgtgccccgctccgccgccgcctcgcgccgcccgccccggc

tctgactgaccgcgttactcccacaggtgagcgggcgggacggccccttctcctccgggctgtaattagctgagcaagaggtaagggtt

taagggatggttggttggtggggtattaatgtttaattacctggagcacctgcctgaaatcactttttttcaggttggggggattcgaacatcg

gccgccaccatgaagttatgggatgtcgtggctgtctgcctggtgctgctccacaccgcgtccgccttcccgctgcccgccggtaaga

ggcctcccgaggcgcccgccgaagaccgctccctcggccgccgccgcgcgcccttcgcgctgagcagtgactcaaatatgccaga

ggattatcctgatcagttcgatgatgtcatggatttttattcaagccaccattaaaagactgaaaaggtcaccagataaacaaatggcagtg

cttcctagaagagagcggaatcggcaggctgcagctgccaacccagagaattccagaggaaaaggtcggagaggccagaggggc

aaaaaccggggttgtgtcttaactgcaatacatttaaatgtcactgacttgggtctgggctatgaaaccaaggaggaactgattttttaggta

ctgcagcggctcttgcgatgcagctgagacaacgtacgacaaaatattgaaaaacttatccagaaatagaaggctggtgagtgacaaa

gtagggcaggcatgttgcagacccatcgcctttgatgatgacctgtcgtttttagatgataacctggtttaccatattctaagaaagcattcc

gctaaaaggtgtggatgtatctgagccaccaacttctccctgctgaagcaggccggcgacgtggaggagaacccccggccccatgaac

gccagcgagttcaggaggaggggcaaggagatggtggactacgtggccaactacatggagggcatcgagggcaggcaggtgtac

cccgacgtggagcccggctacctgaggcccctgatccccgccgccgcccccaggagcccgacaccttcgaggacatcatcaacg

acgtggagaagatcatcatgcccggcgtgacccactggcacagcccctacttcttcgcctacttccccaccgccagcagctacccccgc

catgctggccgacatgctgtgcggcgccatcggctgcatcggcttcagctgggccgccagccccgcctgcaccgagctggagaccg

tgatgatggactggctgggcaagatgctggagctgcccaaggccttcctgaacgagaaggccggcgagggcggcggcgtgatcca

gggcagcgccagcgaggccaccctggtggccctgctggccgccaggaccaaggtgatccacaggctgcaggccgccagccccg

agctgacccaggccgccatcatggagaagctggtggcctacagcagcgaccaggcccacagcagcgtggagagggccggcctga

tcggcggcgtgaagctgaaggccatccccagcgacggcaacttcgccatgagggccagcgccctgcaggaggccctggagaggg

acaaggccgccggcctgatccccttcttcatggtggccaccctgggcaccaccacctgctgcagcttcgacaacctgctggaggtgg

gccccatctgcaacaaggaggacatctggctgcacgtggacgccgcctacgccggcagcgccttcatctgccccgagttcaggcac
ctgctgaacggcgtggagttcgccgacagcttcaacttcaaccccccacaagtggctgctggtgaacttcgactgcagcgccatgtggg
tgaagaagaggaccgacctgaccggcgccttcaggctggaccccacctacctgaagcacagccaccaggacagcggcctgatcac
cgactacaggcactggcagatcccccctgggcaggaggttcaggagcctgaagatgtggttcgtgttcaggatgtacggcgtgaaggg
cctgcaggcctacatcaggaagcacgtgcagctgagccacgagttcgagagcctggtgaggcaggaccccaggttcgagatctgcg
tggaggtgatcctgggcctggtgtgcttcaggctgaagggcagcaacaaggtgaacgaggccctgctgcagaggatcaacagcgcc
aagaagatccacctggtgccctgccacctgagggacaagttcgtgctgaggttcgccatctgcagcaggaccgtggagagcgccca
cgtgcagagggcctgggagcacatcaaggagctggccgccgacgtgctgagggccgagagggagtgactcgagaatcaacctctg
gattacaaaatttgtgaaagattgactggtattcttaactatgttgctccttttacgctatgtggatacgctgctttaatgcctttgtatcatgcta
ttgcttcccgtatggctttcattttctcctccttgtataaatcctggttgctgtctctttatgaggagttgtggcccgttgtcaggcaacgtggc
gtggtgtgcactgtgtttgctgacgcaaccccccactggttggggcattgccaccacctgtcagctcctttccgggactttcgctttcccct
ccctattgccacggcggaactcatcgccgcctgccttgcccgctgctggacaggggctcggctgttgggcactgacaattccgtggtgt
tgtcggggaaatcatcgtcctttccttggctgctcgcctgtgttgccacctggattctgcgcgggacgtccttctgctacgtcccttcggcc
ctcaatccagcggaccttccttcccgcggcctgctgccggctctgcggcctcttccgcgtcttcgccttcgccctcagacgagtcggatc
tccctttgggccgcctccccgctaagatacattgatgagtttggacaaaccacaactagaatgcagtgaaaaaaatgctttatttgtgaaat
ttgtgatgctattgctttatttgtaaccattataagctgcaataaacaagttcggaccgagcggcagatctaggaaccccctagtgatggagt
tggccactccctctctgcgcgctcgctcgctcactgaggccgggcgaccaaaggtcgcccgacgcccgggctttgcccgggcggcc
tcagtgagcgagcgagcgcgcagctgcctgcagg

>HRPDAAV03-A (SEQ ID NO:18)

cctgcaggcagctgcgcgctcgctcgctcactgaggccgcccgggcaaagcccgggcgtcgggcgacctttggtcgcccggcctc

agtgagcgagcgagcgcgcagagagggagtggccaactccatcactaggggttcctatcgatatcaagcttcgttacataacttacgg

taaatggcccgcctggctgaccgcccaacgacccccgcccattgacgtcaatagtaacgccaatagggactttccattgacgtcaatg

ggtggagtatttacggtaaactgcccacttggcagtacatcaagtgtatcatatgccaagtacgccccctattgacgtcaatgacggtaaa

tggcccgcctggcattgtgcccagtacatgaccttatgggactttcctacttggcagtacatctacgtattagtcatcgctattaccatggtc

gaggtgagccccacgttctgcttcactctccccatctccccccccctccccaccccaattttgtatttatttatttttaattatttttgtgcagcg

atggggggcggggggggggggggggggcgcgcgccaggcggggcggggcggggcgaggggcggggcggggcgaggcggaga

ggtgcggcggcagccaatcagagcggcgcgctccgaaagtttccttttatggcgaggcggcggcggcggcggccctataaaaagc

gaagcgcgcggcgggcgggagtcgctgcgcgctgccttcgccccgtgccccgctccgccgccgcctcgcgccgcccgccccggc

tctgactgaccgcgttactcccacaggtgagcgggcgggacggcccttctcctccgggctgtaattagctgagcaagaggtaagggtt

taagggatggttggttggtggggtattaatgtttaattacctggagcacctgcctgaaatcactttttttcaggttggggggattcgaacatcg

gccgccaccatgaacgccagcgagttcaggaggaggggcaaggagatggtggactacgtggccaactacatggagggcatcgag

ggcaggcaggtgtaccccgacgtggagcccggctacctgaggcccctgatccccgccgccgccccccaggagcccgacaccttcg

aggacatcatcaacgacgtggagaagatcatcatgcccggcgtgacccactggcacagcccctacttcttcgcctacttccccaccgc

cagcagctaccccgccatgctggccgacatgctgtgcggcgccatcggctgcatcggcttcagctgggccgccagccccgcctgca

ccgagctggagaccgtgatgatggactggctgggcaagatgctggagctgcccaaggccttcctgaacgagaaggccggcgaggg

cggcggcgtgatccagggcagcgccagcgaggccaccctggtggccctgctggccgccaggaccaaggtgatccacaggctgca

ggccgccagccccgagctgacccaggccgccatcatggagaagctggtggcctacagcagcgaccaggcccacagcagcgtgga

gagggccggcctgatcggcggcgtgaagctgaaggccatccccagcgacggcaacttcgccatgagggccagcgccctgcagga

ggccctggagagggacaaggccgccggcctgatcccccttcttcatggtggccaccctgggcaccaccacctgctgcagcttcgacaa

cctgctggaggtgggcccccatctgcaacaaggaggacatctggctgcacgtggacgccgcctacgccggcagcgccttcatctgcc

ccgagttcaggcacctgctgaacggcgtggagttcgccgacagcttcaacttcaaccccccacaagtggctgctggtgaacttcgactg

cagcgccatgtgggtgaagaagaggaccgacctgaccggcgccttcaggctggaccccacctacctgaagcacagccaccaggac

agcggcctgatcaccgactacaggcactggcagatcccccctgggcaggaggttcaggagcctgaagatgtggttcgtgttcaggatgt

acggcgtgaagggcctgcaggcctacatcaggaagcacgtgcagctgagccacgagttcgagagcctggtgaggcaggaccccca

ggttcgagatctgcgtggaggtgatcctgggcctggtgtgcttcaggctgaagggcagcaacaaggtgaacgaggccctgctgcaga

ggatcaacagcgccaagaagatccacctggtgccctgccacctgagggacaagttcgtgctgaggttcgccatctgcagcaggaccg

tggagagcgcccacgtgcagagggcctgggagcacatcaaggagctggccgccgacgtgctgagggccgagagggagtgactc

gagtaagatacattgatgagtttggacaaaccacaactagaatgcagtgaaaaaaatgctttatttgtgaaatttgtgatgctattgctttattt

gtaaccattataagctgcaataaacaagttcgttacataacttacggtaaatggcccgcctggctgaccgcccaacgacccccgcccatt

gacgtcaataatgacgtatgttcccatagtaacgtcaataggggactttccattgacgtcaatgggtggagtatttacggtaaactgcccact

tggcagtacatcaagtgtatcatatgccaagtacgccccctattgacgtcaatgacggtaaatggcccgcctggcattatgcccagtaca

tgaccttatgggactttcctacttggcagtacatctacgtattagtcatcgctattaccatggtgatgcggttttggcagtacatcaatgggc

gtggatagcggtttgactcacggggatttccaagtctccaccccattgacgtcaatgggagtttgttttgcaccaaaatcaacgggacttt

ccaaaatgtcgtaacaactccgccccattgacgcaaatgggcggtaggcgtgtacggtgggaggtctatataagcagagctgccacc

atgaagttatgggatgtcgtggctgtctgcctggtgctgctccacaccgcgtccgccttcccgctgcccgccggtaagaggcctcccga

ggcgcccgccgaagaccgctccctcggccgccgccgcgcgcccttcgcgctgagcagtgactcaaatatgccagaggattatcctg

atcagttcgatgatgtcatggatttattcaagccaccattaaaagactgaaaaggtcaccagataaacaaatggcagtgcttcctagaag

agagcggaatcggcaggctgcagctgccaacccagagaattccagaggaaaaggtcggagaggccagagggcaaaaaccggg

gttgtgtcttaactgcaatacatttaaatgtcactgacttgggtctgggctatgaaaccaaggaggaactgatttttaggtactgcagcggc

tcttgcgatgcagctgagacaacgtacgacaaaatattgaaaaacttatccagaaatagaaggctggtgagtgacaaagtagggcagg

catgttgcagacccatcgcctttgatgatgacctgtcgttttagatgataacctggtttaccatattctaagaaagcattccgctaaaaggt

gtggatgtatctgaaataaagtctgagtgggcggcagcctgtgtgtgcctggttctctctgtcccggaatgtgcaaacaatggaggtgc

ggaccgagcggcagatctaggaacccctagtgatggagttggccactccctctctgcgcgctcgctcgctcactgaggccgggcgac

caaaggtcgcccgacgcccgggctttgcccgggcggcctcagtgagcgagcgagcgcgcagctgcctgcagg

>HRPDAAV03-B (SEQ ID NO:19)

cctgcaggcagctgcgcgctcgctcgctcactgaggccgcccgggcaaagcccgggcgtcgggcgacctttggtcgcccggcctc

agtgagcgagcgagcgcgcagagagggagtggccaactccatcactaggggttcctatcgatatcaagcttcgttacataacttacgg

taaatggcccgcctggctgaccgcccaacgacccccgcccattgacgtcaatagtaacgccaatagggactttccattgacgtcaatg

ggtggagtatttacggtaaaactgcccacttggcagtacatcaagtgtatcatatgccaagtacgccccctattgacgtcaatgacggtaaa

tggcccgcctggcattgtgcccagtacatgaccttatgggactttcctacttggcagtacatctacgtattagtcatcgctattaccatggtc

gaggtgagccccacgttctgcttcactctccccatctccccccccctccccacccccaatttttgtatttatttattttttaattattttgtgcagcg

atggggggggggggggggggggcgcgcgccaggcggggcggggcggggcgaggggcggggcggggcgaggcggaga

ggtgcggcggcagccaatcagagcggcgcgctccgaaagtttcctttatggcgaggcggcggcggcggcggccctataaaaagc

gaagcgcgcggcgggcgggagtcgctgcgcgctgccttcgccccgtgccccgctccgccgccgcctcgcgccgcccgccccggc

tctgactgaccgcgttactcccacaggtgagcgggcgggacggcccttctcctccgggctgtaattagctgagcaagaggtaagggtt

taagggatggttggttggtggggtattaatgtttaattacctggagcacctgcctgaaatcacttttttttcaggttggggggattcgaacatcg

gccgccaccatgaacgccagcgagttcaggaggaggggcaaggagatggtggactacgtggccaactacatggagggcatcgag

ggcaggcaggtgtaccccgacgtggagcccggctacctgaggcccctgatccccgccgccgccccccaggagcccgacaccttcg

aggacatcatcaacgacgtggagaagatcatcatgcccggcgtgacccactggcacagccccctacttcttcgcctacttccccaccgc

cagcagctaccccgccatgctggccgacatgctgtgcggcgccatcggctgcatcggcttcagctgggccgccagccccgcctgca

ccgagctggagaccgtgatgatggactggctgggccaagatgctggagctgcccaaggccttcctgaacgagaaggccggcgaggg

cggcggcgtgatccagggcagcgccagcgaggccaccctggtggccctgctggccgccaggaccaaggtgatccacaggctgca

ggccgccagccccgagctgacccaggccgccatcatggagaagctggtggcctacagcagcgaccaggcccacagcagcgtgga

gagggccggcctgatcggcggcgtgaagctgaaggccatccccagcgacggcaacttcgccatgagggccagcgccctgcagga

ggccctggagagggacaaggccgccggcctgatcccttcttcatggtggccaccctgggcaccaccacctgctgcagcttcgacaa

cctgctggaggtgggcccccatctgcaacaaggaggacatctggctgcacgtggacgccgcctacgccggcagcgccttcatctgcc

ccgagttcaggcacctgctgaacggcgtggagttcgccgacagcttcaacttcaaccccacaagtggctgctggtgaacttcgactg

cagcgccatgtgggtgaagaagaggaccgacctgaccggcgccttcaggctggaccccacctacctgaagcacagccaccaggac

agcggcctgatcaccgactacaggcactggcagatccccctgggcaggaggttcaggagcctgaagatgtggttcgtgttcaggatgt

acggcgtgaagggcctgcaggcctacatcaggaagcacgtgcagctgagccacgagttcgagagcctggtgaggcaggacccca

ggttcgagatctgcgtggaggtgatcctgggcctggtgtgcttcaggctgaagggcagcaacaaggtgaacgaggccctgctgcaga

ggatcaacagcgccaagaagatccacctggtgccctgccacctgagggacaagttcgtgctgaggttcgccatctgcagcaggaccg

tggagagcgcccacgtgcagagggcctgggagcacatcaaggagctggccgccgacgtgctgagggccgagagggagtgactc

gagtaagatacattgatgagtttggacaaaccacaactagaatgcagtgaaaaaaatgctttatttgtgaaatttgtgatgctattgctttattt

gtaaccattataagctgcaataaacaagttcgttacataacttacggtaaatggcccgcctggctgaccgcccaacgaccccgcccatt

gacgtcaataatgacgtatgttcccatagtaacgtcaataggactttccattgacgtcaatgggtggagtatttacggtaaactgcccact

tggcagtacatcaagtgtatcatatgccaagtacgccccctattgacgtcaatgacggtaaatggcccgcctggcattatgcccagtaca

tgaccttatgggactttcctacttggcagtacatctacgtattagtcatcgctattaccatggtgatgcggttttggcagtacatcaatgggc

gtggatagcggtttgactcacggggatttccaagtctccaccccattgacgtcaatgggagtttgttttgcaccaaaatcaacgggacttt

ccaaaatgtcgtaacaactccgccccattgacgcaaatgggcggtaggcgtgtacggtgggaggtctatataagcagagctgccacc

atgaagttatgggatgtcgtggctgtctgcctggtgctgctccacaccgcgtccgccttcccgctgcccgccggtaagaggcctcccga

ggcgcccgccgaagaccgctccctcggccgccgccgcgcgcccttcgcgctgagcagtgactcaaatatgccagaggattatcctg

atcagttcgatgatgtcatggatttattcaagccaccattaaaagactgaaaaggtcaccagataaacaaatggcagtgcttcctagaag

agagcggaatcggcaggctgcagctgccaacccagagaattccagaggaaaaggtcggagaggccagaggggcaaaaaccggg

gttgtgtcttaactgcaatacatttaaatgtcactgacttgggtctgggctatgaaaccaaggaggaactgattttttaggtactgcagcggc

tcttgcgatgcagctgagacaacgtacgacaaaatattgaaaaacttatccagaaatagaaggctggtgagtgacaaagtagggcagg

catgttgcagacccatcgcctttgatgatgacctgtcgttttagatgataacctggtttaccatattctaagaaagcattccgctaaaaggt

gtggatgtatctgaaatcaacctctggattacaaaatttgtgaaagattgactggtattcttaactatgttgctcctttttacgctatgtggatac

gctgctttaatgcctttgtatcatgctattgcttcccgtatggctttcattttctcctccttgtataaatcctggttgctgtctctttatgaggagttg

tggcccgttgtcaggcaacgtggcgtggtgtgcactgtgtttgctgacgcaaccccccactggttggggcattgccaccacctgtcagct

cctttccgggactttcgctttcccctccttattgccacggcggaactcatcgccgcctgccttgcccgctgctggacagggggctcggct

gttgggcactgacaattccgtggtgttgtcggggaaatcatcgtcctttccttggctgctcgcctgtgttgccacctggattctgcgcggg

acgtccttctgctacgtcccttcggccctcaatccagcggaccttccttcccgcggcctgctgccggctctgcggcctcttccgcgtcttc

gccttcgccctcagacgagtcggatctccctttgggccgcctccccgcaataaagtctgagtgggcggcagcctgtgtgtgcctgggtt

ctctctgtcccggaatgtgcaaacaatggaggtgcggaccgagcggcagatctaggaacccctagtgatggagttggccactccctct

ctgcgcgctcgctcgctcactgaggccgggcgaccaaaggtcgcccgacgcccgggctttgcccgggcggcctcagtgagcgagc

gagcgcgcagctgcctgcagg

>HRPDAAV03-C (SEQ ID NO:20)

cctgcaggcagctgcgcgctcgctcgctcactgaggccgcccgggcaaagcccgggcgtcgggcgacctttggtcgcccggcctc

agtgagcgagcgagcgcgcagagagggagtggccaactccatcactaggggttcctatcgatatcaagcttcgttacataacttacgg

taaatggcccgcctggctgaccgcccaacgaccccgcccattgacgtcaatagtaacgccaatagggactttccattgacgtcaatg

ggtggagtatttacggtaaactgcccacttggcagtacatcaagtgtatcatatgccaagtacgccccctattgacgtcaatgacggtaaa

tggcccgcctggcattgtgcccagtacatgaccttatgggactttcctacttggcagtacatctacgtattagtcatcgctattaccatggtc

gaggtgagccccacgttctgcttcactctccccatctccccccccctccccacccccaattttgtatttatttattttttaattattttgtgcagcg

atggggcgggggggggggggggggcgcgcgccaggcggggcggggcggggcgaggggcggggcggggcgaggcggaga

ggtgcggcggcagccaatcagagcggcgcgctccgaaagtttcctttttatggcgaggcggcggcggcggcggcccctataaaaagc

gaagcgcgcggcgggcgggagtcgctgcgcgctgccttcgccccgtgccccgctccgccgccgcctcgcgccgcccgccccggc

tctgactgaccgcgttactcccacaggtgagcgggcgggacggcccttctcctccgggctgtaattagctgagcaagaggtaagggtt

taagggatggttggttggtggggtattaatgtttaattacctggagcacctgcctgaaatcacttttttttcaggttggggggattcgaacatcg

gccgccaccatgaacgccagcgagttcaggaggaggggcaaggagatggtggactacgtggccaactacatggagggcatcgag

ggcaggcaggtgtaccccgacgtggagcccggctacctgaggcccctgatccccgccgccgcccccccaggagcccgacaccttcg

aggacatcatcaacgacgtggagaagatcatcatgcccggcgtgacccactggcacagcccctacttcttcgcctacttccccaccgc

cagcagctaccccgccatgctggccgacatgctgtgcggcgccatcggctgcatcggcttcagctgggccgccagccccgcctgca

ccgagctggagaccgtgatgatggactggctgggcaagatgctggagctgcccaaggccttcctgaacgagaaggccggcgaggg

cggcggcgtgatccagggcagcgccagcgaggccaccctggtggccctgctggccgccaggaccaaggtgatccacaggctgca

ggccgccagccccgagctgacccaggccgccatcatggagaagctggtggcctacagcagcgaccaggcccacagcagcgtgga

gagggccggcctgatcggcggcgtgaagctgaaggccatccccagcgacggcaacttcgccatgagggccagcgccctgcagga

ggccctggagagggacaaggccgccggcctgatccccttcttcatggtggccaccctgggcaccaccacctgctgcagcttcgacaa

cctgctggaggtgggccccatctgcaacaaggaggacatctggctgcacgtggacgccgcctacgccggcagcgccttcatctgcc

ccgagttcaggcacctgctgaacggcgtggagttcgccgacagcttcaacttcaacccccacaagtggctgctggtgaacttcgactg

cagcgccatgtgggtgaagaagaggaccgacctgaccggcgccttcaggctggaccccacctacctgaagcacagccaccaggac

agcggcctgatcaccgactacaggcactggcagatccccctgggcaggaggttcaggagcctgaagatgtggttcgtgttcaggatgt

acggcgtgaagggcctgcaggcctacatcaggaagcacgtgcagctgagccacgagttcgagagcctggtgaggcaggaccccca

ggttcgagatctgcgtggaggtgatcctgggcctggtgtgcttcaggctgaagggcagcaacaaggtgaacgaggccctgctgcaga

ggatcaacagcgccaagaagatccacctggtgccctgccacctgagggacaagttcgtgctgaggttcgccatctgcagcaggaccg

tggagagcgcccacgtgcagagggcctgggagcacatcaaggagctggccgccgacgtgctgagggccgagagggagtgactc

gagtaagatacattgatgagtttggacaaaccacaactagaatgcagtgaaaaaaatgctttatttgtgaaatttgtgatgctattgctttattt

gtaaccattataagctgcaataaacaagttcgttacataacttacggtaaatggcccgcctggctgaccgcccaacgaccccccgcccatt

gacgtcaataatgacgtatgttcccatagtaacgtcaatagggactttccattgacgtcaatgggtggagtatttacggtaaactgcccact

tggcagtacatcaagtgtatcatatgccaagtacgccccctattgacgtcaatgacggtaaatggcccgcctggcattatgcccagtaca

tgaccttatgggactttcctacttggcagtacatctacgtattagtcatcgctattaccatggtgatgcggttttggcagtacatcaatgggc

gtggatagcggtttgactcacggggatttccaagtctccacccattgacgtcaatgggagtttgttttgcaccaaaatcaacgggacttt

ccaaaatgtcgtaacaactccgccccattgacgcaaatgggcggtaggcgtgtacggtgggaggtctatataagcagagctgtgagttt

ggggacccttgattgttctttcttttcgctattgtaaaattcatgttatatggagggggcaaagttttcaggtgttgtttagaatgggaagat

gtcccttgtatcaccatggaccctcatgataattttgtttctttcactttctactctgttgacaaccattgtctcctcttattttctttcattttctgta

acttttcgttaaactttagcttgcatttgtaacgaattttaaattcacttttgtttatttgtcagattgtaagtactttctctaatcactttttttttcaag

gcaatcagggtatattatattgtacttcagcacagttttagagaacaattgttataattaaatgataaggtagaatatttctgcatataaattctg

gctggcgtggaaatattcttattggtagaaacaactacatcctggtcatcatcctgcctttctctttatggttacaatgatatacactgtttgag

atgaggataaaatactctgagtccaaaccgggcccctctgctaaccatgttcatgccttcttcttttcctacaggccaccatgaagttatgg

gatgtcgtggctgtctgcctggtgctgctccacaccgcgtccgccttcccgctgcccgccggtaagaggcctcccgaggcgcccgcc

gaagaccgctccctcggccgccgccgcgcgcccttcgcgctgagcagtgactcaaatatgccagaggattatcctgatcagttcgatg

atgtcatggatttattcaagccaccattaaaagactgaaaaggtcaccagataaacaaatggcagtgcttcctagaagagagcggaatc

ggcaggctgcagctgccaacccagagaattccagaggaaaaggtcggagaggccagagggggcaaaaaccgggggttgtgtcttaac

tgcaatacatttaaatgtcactgacttgggtctgggctatgaaaccaaggaggaactgatttttaggtactgcagcggctcttgcgatgca

gctgagacaacgtacgacaaaatattgaaaaacttatccagaaatagaaggctggtgagtgacaaagtagggcaggcatgttgcagac

ccatcgcctttgatgatgacctgtcgttttagatgataacctggtttaccatattctaagaaagcattccgctaaaaggtgtggatgtatctg

aaataaagtctgagtgggcggcagcctgtgtgtgcctgggttctctctgtcccggaatgtgcaaacaatggaggtgcggaccgagcgg

cagatctaggaaccccctagtgatggagttggccactccctctctgcgcgctcgctcgctcactgaggccgggcgaccaaaggtcgcc

cgacgcccgggctttgcccggcggcctcagtgagcgagcgagcgcgcagctgcctgcagg

>HRPDAAV03-D (SEQ ID NO:21)

cctgcaggcagctgcgcgctcgctcgctcactgaggccgcccgggcaaagcccgggcgtcgggcgacctttggtcgcccggcctc
agtgagcgagcgagcgcgcagagagggagtggccaactccatcactaggggttcctatcgatatccgttacataacttacggtaaatg
gcccgcctggctgaccgcccaacgacccccgcccattgacgtcaataatgacgtatgttcccatagtaacgtcaatagggactttccatt
gacgtcaatgggtggagtatttacggtaaactgcccacttggcagtacatcaagtgtatcatatgccaagtacgccccctattgacgtcaa
tgacggtaaatggcccgcctggcattatgcccagtacatgaccttatgggactttcctacttggcagtacatctacgtattagtcatcgctat
taccatggtgatgcggttttggcagtacatcaatgggcgtggatagcggtttgactcacggggatttccaagtctccacccattgacgtc

aatgggagtttgttttgcaccaaaatcaacgggactttccaaaatgtcgtaacaactccgccccattgacgcaaatgggcggtaggcgtg

tacggtggggaggtctatataagcagagctgtgagtttgggggacccttgattgttctttcttttttcgctattgtaaaattcatgttatatggaggg

ggcaaagttttcagggtgttgtttagaatgggaagatgtcccttgtatcaccatggaccctcatgataattttgtttctttcactttctactctgtt

gacaaccattgtctcctcttattttctttttcattttctgtaacttttttcgttaaactttagcttgcatttgtaacgaattttttaaattcacttttgtttatttg

tcagattgtaagtactttctctaatcactttttttttcaaggcaatcagggtatattatattgtacttcagcacagttttagagaacaattgttataat

taaatgataaggtagaatatttctgcatataaaattctggctggcgtggaaatattcttattggtagaaacaactacatcctggtcatcatcctg

cctttctctttatggttacaatgatatacactgtttgagatgaggataaaatactctgagtccaaaccgggcccctctgctaaccatgttcatg

ccttcttcttttttcctacaggccaccatgaagttatgggatgtcgtggctgtctgcctggtgctgctccacaccgcgtccgccttcccgctg

cccgccggtaagaggcctcccgaggcgcccgccgaagaccgctccctcggccgccgccgcgcgcccttcgcgctgagcagtgact

caaatatgccagaggattatcctgatcagttcgatgatgtcatggattttattcaagccaccattaaaagactgaaaaggtcaccagataa

acaaatggcagtgcttcctagaagagagcggaatcggcaggctgcagctgccaacccagagaattccagaggaaaaggtcggaga

ggccagaggggcaaaaaccggggttgtgtcttaactgcaatacatttaaatgtcactgacttgggtctgggctatgaaaccaaggagga

actgattttaggtactgcagcggctcttgcgatgcagctgagacaacgtacgacaaaatattgaaaaacttatccagaaatagaaggct

ggtgagtgacaaagtagggcaggcatgttgcagacccatcgcctttgatgatgacctgtcgttttagatgataacctggtttaccatattc

taagaaagcattccgctaaaaggtgtggatgtatctgaaataaagtctgagtgggcggcagcctgtgtgtgcctgggttctctctgtccc

ggaatgtgcaaacaatggaggtgaagcttcgttacataacttacggtaaatggcccgcctggctgaccgcccaacgacccccgcccat

tgacgtcaatagtaacgccaatagggactttccattgacgtcaatgggtggagtatttacggtaaactgcccacttggcagtacatcaagt

gtatcatatgccaagtacgccccctattgacgtcaatgacggtaaatggcccgcctggcattgtgcccagtacatgaccttatgggacttt

cctacttggcagtacatctacgtattagtcatcgctattaccatggtcgaggtgagccccacgttctgcttcactctccccatctccccccc

ctccccaccccaattttgtatttatttattttttaattattttgtgcagcgatggggggcgggggggggggggggggcgcgcgccaggcgg

ggcggggcggggcgaggggcggggcggggcgaggcggagaggtgcggcggcagccaatcagagcggcgcgctccgaaagtt

tcctttatggcgaggcggcggcggcggcggccctataaaaagcgaagcgcgcggcgggcgggagtcgctgcgcgctgccttcgc

cccgtgccccgctccgccgccgcctcgcgccgcccgccccggctctgactgaccgcgttactcccacaggtgagcgggcgggacg

gcccttctcctccgggctgtaattagctgagcaagaggtaagggtttaagggatggttggttggtggggtattaatgtttaattacctggag

cacctgcctgaaatcacttttttttcaggttgggggattcgaacatcggccgccaccatgaacgccagcgagttcaggaggaggggcaa

ggagatggtggactacgtggccaactacatggagggcatcgagggcaggcaggtgtaccccgacgtggagcccggctacctgagg

cccctgatccccgccgccgccccccaggagcccgacaccttcgaggacatcatcaacgacgtggagaagatcatcatgcccggcgt

gacccactggcacagccccctacttcttcgcctacttccccaccgccagcagctaccccgccatgctggccgacatgctgtgcggcgcc

atcggctgcatcggcttcagctgggccgccagccccgcctgcaccgagctggagaccgtgatgatggactggctgggcaagatgct

ggagctgcccaaggccttcctgaacgagaaggccggcgagggcggcggcgtgatccagggcagcgccagcgaggccaccctgg

tggccctgctggccgccaggaccaaggtgatccacaggctgcaggccgccagccccgagctgacccaggccgccatcatggagaa

gctggtggcctacagcagcgaccaggcccacagcagcgtggagagggccggcctgatcggcggcgtgaagctgaaggccatccc

cagcgacggcaacttcgccatgagggccagcgccctgcaggaggccctggagagggacaaggccgccggcctgatccccttcttc

atggtggccaccctgggcaccaccacctgctgcagcttcgacaacctgctggaggtgggccccatctgcaacaaggaggacatctgg

ctgcacgtggacgccgcctacgccggcagcgccttcatctgccccgagttcaggcacctgctgaacggcgtggagttcgccgacag

cttcaacttcaaccccccacaagtggctgctggtgaacttcgactgcagcgccatgtgggtgaagaagaggaccgacctgaccggcgc

cttcaggctggaccccacctacctgaagcacagccaccaggacagcggcctgatcaccgactacaggcactggcagatcccccctgg

gcaggaggttcaggagcctgaagatgtggttcgtgttcaggatgtacggcgtgaagggcctgcaggcctacatcaggaagcacgtgc

agctgagccacgagttcgagagcctggtgaggcaggaccccaggttcgagatctgcgtggaggtgatcctgggcctggtgtgcttca

ggctgaagggcagcaacaaggtgaacgaggccctgctgcagaggatcaacagcgccaagaagatccacctggtgccctgccacct

gagggacaagttcgtgctgaggttcgccatctgcagcaggaccgtggagagcgcccacgtgcagagggcctgggagcacatcaag

gagctggccgccgacgtgctgagggccgagagggagtgactcgagtaagatacattgatgagtttggacaaaccacaactagaatgc

agtgaaaaaaatgctttatttgtgaaatttgtgatgctattgctttatttgtaaccattataagctgcaataaacaagttcggaccgagcggca

gatctaggaaccccctagtgatggagttggccactccctctctgcgcgctcgctcgctcactgaggccgggcgaccaaaggtcgcccg

acgcccgggctttgcccgggcggcctcagtgagcgagcgagcgcgcagctgcctgcagg

>HRPDAAV03-E (SEQ ID NO:22)

cctgcaggcagctgcgcgctcgctcgctcactgaggccgcccgggcaaagcccgggcgtcgggcgacctttggtcgcccggcctc

agtgagcgagcgagcgcgcagagagggagtggccaactccatcactaggggttcctatcgatatccgttacataacttacggtaaatg

gcccgcctggctgaccgcccaacgacccccgcccattgacgtcaataatgacgtatgttcccatagtaacgtcaatagggactttccatt

gacgtcaatgggtggagtatttacggtaaactgcccacttggcagtacatcaagtgtatcatatgccaagtacgccccctattgacgtcaa

tgacggtaaatggcccgcctggcattatgcccagtacatgaccttatgggactttcctacttggcagtacatctacgtattagtcatcgctat

taccatggtgatgcggttttggcagtacatcaatgggcgtggatagcggtttgactcacggggatttccaagtctccaccccattgacgtc

aatgggagtttgttttgcaccaaaatcaacgggactttccaaaatgtcgtaacaactccgccccattgacgcaaatgggcggtaggcgtg

tacggtgggaggtctatataagcagagctgccaccatgaagttatgggatgtcgtggctgtctgcctggtgctgctccacaccgcgtcc

gccttcccgctgcccgccggtaagaggcctcccgaggcgcccgccgaagaccgctccctcggccgccgccgcgcgcccttcgcgc

tgagcagtgactcaaatatgccagaggattatcctgatcagttcgatgatgtcatggattttattcaagccaccattaaaagactgaaaag

gtcaccagataaacaaatggcagtgcttcctagaagagagcggaatcggcaggctgcagctgccaacccagagaattccagaggaa

aaggtcggagaggccagagggggcaaaaaccggggttgtgtcttaactgcaatacatttaaatgtcactgacttgggtctgggctatgaa

accaaggaggaactgattttttaggtactgcagcggctcttgcgatgcagctgagacaacgtacgacaaaatattgaaaaacttatccag

aaatagaaggctggtgagtgacaaagtagggcaggcatgttgcagacccatcgcctttgatgatgacctgtcgttttagatgataacct

ggtttaccatattctaagaaagcattccgctaaaaggtgtggatgtatctgaaataaagtctgagtgggcggcagcctgtgtgtgcctgg

gttctctctgtcccggaatgtgcaaacaatggaggtgaagcttcgttacataacttacggtaaatggcccgcctggctgaccgcccaacg

accccgcccattgacgtcaatagtaacgccaatagggactttccattgacgtcaatgggtggagtatttacggtaaactgcccacttgg

cagtacatcaagtgtatcatatgccaagtacgccccctattgacgtcaatgacggtaaatggcccgcctggcattgtgcccagtacatga

ccttatgggactttcctacttggcagtacatctacgtattagtcatcgctattaccatggtcgaggtgagccccacgttctgcttcactctccc

catctccccccccctccccacccccaattttgtatttatttattttttaattattttgtgcagcgatgggggcggggggggggggggggcgcg

cgccaggcggggcggggcggggcgaggggcggggcggggcgaggcggagaggtgcggcggcagccaatcagagcggcgc

gctccgaaagtttccttttatggcgaggcggcggcggcggcggccctataaaaagcgaagcgcgcggcgggcgggagtcgctgcg

cgctgccttcgccccgtgccccgctccgccgccgcctcgcgccgcccgccccggctctgactgaccgcgttactcccacaggtgagc

gggcgggacggcccttctcctccgggctgtaattagctgagcaagaggtaagggtttaagggatggttggttggtggggtattaatgttt

aattacctggagcacctgcctgaaatcactttttttcaggttgggggattcgaacatcggccgccaccatgaacgccagcgagttcagga

ggaggggcaaggagatggtggactacgtggccaactacatggagggcatcgagggcaggcaggtgtaccccgacgtggagcccg

gctacctgaggccccctgatccccgccgccgcccccccaggagcccgacaccttcgaggacatcatcaacgacgtggagaagatcatc

atgcccggcgtgacccactggcacagcccctacttcttcgcctacttcccccaccgccagcagctacccccgccatgctggccgacatgc

tgtgcggcgccatcggctgcatcggcttcagctgggccgccagccccgcctgcaccgagctggagaccgtgatgatggactggctg

ggcaagatgctggagctgcccaaggccttcctgaacgagaaggccggcgagggcggcggcgtgatccagggcagcgccagcga

ggccaccctggtggccctgctggccgccaggaccaaggtgatccacaggctgcaggccgccagccccgagctgacccaggccgc

catcatggagaagctggtggcctacagcagcgaccaggcccacagcagcgtggagagggccggcctgatcggcggcgtgaagct

gaaggccatccccagcgacggcaacttcgccatgagggccagcgccctgcaggaggccctggagagggacaaggccgccggcc

tgatccccttcttcatggtggccaccctgggcaccaccacctgctgcagcttcgacaacctgctggaggtgggccccatctgcaacaag

gaggacatctggctgcacgtggacgccgcctacgccggcagcgccttcatctgccccgagttcaggcacctgctgaacggcgtgga

gttcgccgacagcttcaacttcaacccccacaagtggctgctggtgaacttcgactgcagcgccatgtgggtgaagaagaggaccga

cctgaccggcgccttcaggctggaccccacctacctgaagcacagccaccaggacagcggcctgatcaccgactacaggcactggc

agatccccctgggcaggaggttcaggagcctgaagatgtggttcgtgttcaggatgtacggcgtgaagggcctgcaggcctacatca

ggaagcacgtgcagctgagccacgagttcgagagcctggtgaggcaggaccccaggttcgagatctgcgtggaggtgatcctgggc

ctggtgtgcttcaggctgaagggcagcaacaaggtgaacgaggccctgctgcagaggatcaacagcgccaagaagatccacctggt

gccctgccacctgagggacaagttcgtgctgaggttcgccatctgcagcaggaccgtggagagcgcccacgtgcagagggcctgg

gagcacatcaaggagctggccgccgacgtgctgagggccgagagggagtgactcgagaatcaacctctggattacaaaatttgtgaa

agattgactggtattcttaactatgttgctccttttacgctatgtggatacgctgctttaatgcctttgtatcatgctattgcttcccgtatggcttt
cattttctcctccttgtataaatcctggttgctgtctctttatgaggagttgtggcccgttgtcaggcaacgtggcgtggtgtgcactgtgtttg
ctgacgcaacccccactggttggggcattgccaccacctgtcagctcctttccgggactttcgctttccccctccctattgccacggcgg
aactcatcgccgcctgccttgcccgctgctggacagggggctcggctgttgggcactgacaattccgtggtgttgtcggggaaatcatcg
tcctttccttggctgctcgcctgtgttgccacctggattctgcgcgggacgtccttctgctacgtcccttcggccctcaatccagcggacct
tccttcccgcggcctgctgccggctctgcggcctcttccgcgtcttcgccttcgccctcagacgagtcggatctccctttgggccgcctc
cccgctaagatacattgatgagtttggacaaaccacaactagaatgcagtgaaaaaaatgctttatttgtgaaatttgtgatgctattgcttta
tttgtaaccattataagctgcaataaacaagttcggaccgagcggcagatctaggaacccctagtgatggagttggccactccctctctg
cgcgctcgctcgctcactgaggccgggcgaccaaaggtcgcccgacgcccgggctttgcccgggcggcctcagtgagcgagcga
gcgcgcagctgcctgcagg

>HRPDAAV03-F (SEQ ID NO:23)

cctgcaggcagctgcgcgctcgctcgctcactgaggccgcccgggcaaagcccgggcgtcgggcgacctttggtcgcccggcctc

agtgagcgagcgagcgcgcagagagggagtggccaactccatcactaggggttcctatcgatatccgttacataacttacggtaaatg

gcccgcctggctgaccgcccaacgacccccgcccattgacgtcaataatgacgtatgttcccatagtaacgtcaatagggactttccatt

gacgtcaatgggtggagtatttacggtaaactgcccacttggcagtacatcaagtgtatcatatgccaagtacgccccctattgacgtcaa

tgacggtaaatggcccgcctggcattatgcccagtacatgaccttatgggactttcctacttggcagtacatctacgtattagtcatcgctat

taccatggtgatgcggttttggcagtacatcaatgggcgtggatagcggtttgactcacggggatttccaagtctccacccattgacgtc

aatgggagtttgttttgcaccaaaatcaacgggactttccaaaatgtcgtaacaactccgccccattgacgcaaatgggcggtaggcgtg

tacggtgggaggtctatataagcagagctaagaggtaagggtttaagggatggttggttggtggggtattaatgtttaattacctggagca

cctgcctgaaatcactttttttcaggttgggccaccatgaagttatgggatgtcgtggctgtctgcctggtgctgctccacaccgcgtccgc

cttcccgctgcccgccggtaagaggcctccccgaggcgcccgccgaagaccgctccctcggccgccgccgcgcgcccttcgcgctg

agcagtgactcaaatatgccagaggattatcctgatcagttcgatgatgtcatggattttattcaagccaccattaaaagactgaaaaggtc

accagataaacaaatggcagtgcttcctagaagagagcggaatcggcaggctgcagctgccaacccagagaattccagaggaaaag

gtcggagaggccagaggggcaaaaaccggggttgtgtcttaactgcaatacatttaaatgtcactgacttgggtctgggctatgaaacc

aaggaggaactgattttttaggtactgcagcggctcttgcgatgcagctgagacaacgtacgacaaaatattgaaaaacttatccagaaat

agaaggctggtgagtgacaaagtagggcaggcatgttgcagacccatcgcctttgatgatgacctgtcgttttagatgataacctggttt

accatattctaagaaagcattccgctaaaaggtgtggatgtatctgaaataaagtctgagtgggcggcagcctgtgtgtgcctgggttctc

tctgtcccggaatgtgcaaacaatggaggtgaagcttcgttacataacttacggtaaatggcccgcctggctgaccgcccaacgacccc

cgcccattgacgtcaatagtaacgccaataggactttccattgacgtcaatgggtggagtatttacggtaaactgcccacttggcagta

catcaagtgtatcatatgccaagtacgccccctattgacgtcaatgacggtaaatggcccgcctggcattgtgcccagtacatgaccttat

gggactttcctacttggcagtacatctacgtattagtcatcgctattaccatggtcgaggtgagccccacgttctgcttcactctccccatct

ccccccctccccaccccaatttgtatttatttattttttaattattttgtgcagcgatgggggcggggggggggggggggcgcgcgcc

aggcggggcggggcggggcgaggggcggggcggggcgaggcggagaggtgcggcggcagccaatcagagcggcgcgctcc

gaaagtttccttttatggcgaggcggcggcggcggcggccctataaaaagcgaagcgcgcggcgggcgggagtcgctgcgcgctg

ccttcgccccgtgccccgctccgccgccgcctcgcgccgcccgccccggctctgactgaccgcgttactcccacaggtgagcgggc

gggacggcccttctcctccgggctgtaattagctgagcaagaggtaagggtttaagggatggttggttggtggggtattaatgtttaatta

cctggagcacctgcctgaaatcactttttttcaggttgggggattcgaacatcggccgccaccatgaacgccagcgagttcaggagga

ggggcaaggagatggtggactacgtggccaactacatggagggcatcgagggcaggcaggtgtaccccgacgtggagcccggct

acctgaggcccctgatccccgccgccgcccccaggagcccgacaccttcgaggacatcatcaacgacgtggagaagatcatcatg

cccggcgtgacccactggcacagccccctacttcttcgcctacttccccaccgccagcagctaccccgccatgctggccgacatgctgt

gcggcgccatcggctgcatcggcttcagctgggccgccagccccgcctgcaccgagctggagaccgtgatgatggactggctggg

caagatgctggagctgcccaaggccttcctgaacgagaaggccggcgagggcggcggcgtgatccagggcagcgccagcgagg

ccaccctggtggccctgctggccgccaggaccaaggtgatccacaggctgcaggccgccagcccccgagctgacccaggccgccat

catggagaagctggtggcctacagcagcgaccaggcccacagcagcgtggagagggccggcctgatcggcggcgtgaagctgaa

ggccatccccagcgacggcaacttcgccatgagggccagcgccctgcaggaggccctggagagggacaaggccgccggcctgat
ccccttcttcatggtggccaccctgggcaccaccacctgctgcagcttcgacaacctgctggaggtggggccccatctgcaacaaggag
gacatctggctgcacgtggacgccgcctacgccggcagcgccttcatctgccccgagttcaggcacctgctgaacggcgtggagttc
gccgacagcttcaacttcaaccccacaagtggctgctggtgaacttcgactgcagcgccatgtgggtgaagaagaggaccgacctg
accggcgccttcaggctggaccccacctacctgaagcacagccaccaggacagcggcctgatcaccgactacaggcactggcagat
cccccctgggcaggaggttcaggagcctgaagatgtggttcgtgttcaggatgtacggcgtgaagggcctgcaggcctacatcaggaa
gcacgtgcagctgagccacgagttcgagagcctggtgaggcaggaccccaggttcgagatctgcgtggaggtgatcctgggcctgg
tgtgcttcaggctgaagggcagcaacaaggtgaacgaggccctgctgcagaggatcaacagcgccaagaagatccacctggtgccc
tgccacctgagggacaagttcgtgctgaggttcgccatctgcagcaggaccgtggagagcgcccacgtgcagagggcctgggagc
acatcaaggagctggccgccgacgtgctgagggccgagagggagtgactcgagaatcaacctctggattacaaaatttgtgaaagatt
gactggtattcttaactatgttgctccttttacgctatgtggatacgctgctttaatgcctttgtatcatgctattgcttcccgtatggctttcatttt
ctcctccttgtataaatcctggttgctgtctctttatgaggagttgtggcccgttgtcaggcaacgtggcgtggtgtgcactgtgtttgctga
cgcaacccccactggttggggcattgccaccacctgtcagctcctttccgggactttcgctttccccctccctattgccacggcggaact
catcgccgcctgccttgcccgctgctggacaggggctcggctgttgggcactgacaattccgtggtgttgtcggggaaatcatcgtcctt
tccttggctgctcgcctgtgttgccacctggattctgcgcgggacgtccttctgctacgtcccttcggccctcaatccagcggaccttcctt
cccgcggcctgctgccggctctgcggcctcttccgcgtcttcgccttcgccctcagacgagtcggatctccctttgggccgcctccccg
ctaagatacattgatgagtttggacaaaccacaactagaatgcagtgaaaaaaatgctttatttgtgaaatttgtgatgctattgctttatttgt
aaccattataagctgcaataaacaagttcggaccgagcggcagatctaggaacccctagtgatggagttggccactccctctctgcgcg
ctcgctcgctcactgaggccgggcgaccaaaggtcgcccgacgcccgggctttgcccgggcggcctcagtgagcgagcgagcgc
gcagctgcctgcagg

>HRPDAAV02-AI (SEQ ID NO:24)

EP 4 768 039 A1

cctgcaggcagctgcgcgctcgctcgctcactgaggccgcccgggcaaagcccgggcgtcgggcgacctttggtcgcccggcctc

agtgagcgagcgagcgcgcagagagggagtggccaactccatcactaggggttcctatcgatatcaagcttcgttacataacttacgg

taaatggcccgcctggctgaccgcccaacgacccccgcccattgacgtcaatagtaacgccaatagggactttccattgacgtcaatg

ggtggagtatttacggtaaactgcccacttggcagtacatcaagtgtatcatatgccaagtacgccccctattgacgtcaatgacggtaaa

tggcccgcctggcattgtgcccagtacatgaccttatgggactttcctacttggcagtacatctacgtattagtcatcgctattaccatggtc

gaggtgagccccacgttctgcttcactctccccatctcccccccctccccaccccaattttgtatttatttattttttaattattttgtgcagcg

atggggggcggggggggggggggggcgcgcgccaggcggggcggggcggggcgaggggcggggcggggcgaggcggaga

ggtgcggcggcagccaatcagagcggcgcgctccgaaagtttcctttatggcgaggcggcggcggcggcggcccctataaaaagc

gaagcgcgcggcgggcgggagtcgctgcgcgctgccttcgccccgtgccccgctccgccgccgcctcgcgccgcccgccccggc

tctgactgaccgcgttactcccacaggtgagcgggcgggacggcccttctcctccgggctgtaattagctgagcaagaggtaagggtt

taagggatggttggttggtggggtattaatgtttaattacctggagcacctgcctgaaatcactttttttcaggttggggggattcgaacatcg

gccgccaccatgaatgctagcgagtttcgcagaaggggcaaggagatggtggattacgtggctaattacatggagggcatcgagggc

cggcaggtgtacccagatgtggagccaggctacctgagaccactgatccctgctgccgcccccacaggagccagacaccttcgagga

tatcatcaatgacgtggagaagatcatcatgcctggcgtgacccactggcatagcccatacttctttgcctactttccaacagcctcttcct

atcccgctatgctggctgatatgctgtgcggcgctatcggctgcatcggcttctcttgggccgctagccctgcttgcacagagctggaga

ccgtgatgatggattggctgggcaagatgctggagctgcctaaggcctttctgaacgagaaggctggcgagggcggcggcgtgatcc

agggctctgcctccgaggctacactggtggctctgctggctgcccggacaaaggtaatccacagactgcaggctgcctctccagagct

gacccaggccgctatcatggagaagctggtggcttactctagcgaccaggctcattcttccgtggagcgggctggcctgatcggcggc

gtgaagctgaaggccatcccttccgacggcaatttcgccatgagagcttctgccctgcaggaggctctggagagagataaggctgcc

ggcctgatccctttctttatggtggccaccctgggcacaaccacctgttgctccttcgacaacctgctggaggtgggccctatctgtaaca

aggaggacatctggctgcatgtggatgccgcttatgctggctctgcctttatctgccccgagtttagacatctgctgaacggcgtggagtt

tgccgacagctttaacttcaacccctcacaagtggctgctggtgaattttgactgctctgccatgtgggtgaagaagaggacagacctgac

cggcgctttaggctggatccaacctacctgaagcacagccatcaggacagcggcctgatcacagactacagacactggcagatccc

actgggcagaaggtttcggtctctgaagatgtggttcgtgtttcgcatgtatggcgtgaagggcctgcaggcttatatccggaagcacgt
gcagctgtctcatgagttcgagtccctggtgcggcaggacccaagatttgagatctgcgtggaggtaatcctgggcctggtgtgcttca
ggctgaagggctctaataaggtgaacgaggctctgctgcagaggatcaacagcgccaagaagatccatctggtgccttgtcatctgag
ggacaagttcgtgctgagattcgccatctgctctagaacagtggagtctgctcatgtgcagagggcttgggagcatatcaaggagctgg
ccgctgacgtgctgagggctgagagggaggccaccaacttctccctgctgaagcaggccggcgacgtggaggagaacccccggcc
ccatgaagctgtgggatgtggtggccgtgtgcctggtgctgctgcacaccgcttctgccttcccactgcctgccggcaagagacctccc
gaggcccctgccgaggacagaagcctgggcaggcggagagccccatttgctctgtctagcgattccaacatgcctgaggattacccc
gatcagttcgatgacgtgatggatttcatccaggccaccatcaagagactgaagagatctcctgacaagcagatggctgtgctgcctag
aagggagagaaacaggcaggccgctgctgccaatccagagaactccaggggcaagggcagaaggggccagcgcggcaagaata
gaggctgcgtgctgacagccatccacctgaacgtgaccgacctgggcctgggctacgagaccaaggaggagctgatcttcaggtact
gtagcggctcctgtgatgctgccgagaccacatacgacaagatcctgaagaacctgtccaggaacagaaggctggtgtctgacaagg
tgggccaggcttgctgtaggccaatcgctttcgacgacgatctgtcctttctggatgacaacctggtgtaccacatcctgaggaagcattc
cgctaagagatgtggctgcatctgactcgagaatcaacctctggattacaaaatttgtgaaagattgactggtattcttaactatgttgctcc
ttttacgctatgtggatacgctgctttaatgcctttgtatcatgctattgcttcccgtatggctttcattttctcctccttgtataaatcctggttgct
gtctctttatgaggagttgtggcccgttgtcaggcaacgtggcgtggtgtgcactgtgtttgctgacgcaacccccactggttggggcatt
gccaccacctgtcagctcctttccgggactttcgctttcccctccctattgccacggcggaactcatcgccgcctgccttgcccgctgct
ggacaggggctcggctgttgggcactgacaattccgtggtgttgtcggggaaatcatcgtcctttccttggctgctcgcctgtgttgccac
ctggattctgcgcgggacgtccttctgctacgtcccttcggccctcaatccagcggaccttccttcccgcggcctgctgccggctctgcg
gcctcttccgcgtcttcgccttcgccctcagacgagtcggatctccctttgggccgcctccccgctaagatacattgatgagtttggacaa
accacaactagaatgcagtgaaaaaaatgctttatttgtgaaatttgtgatgctattgctttatttgtaaccattataagctgcaataaacaagt
tcggaccgagcggcagatctaggaacccctagtgatggagttggccactccctctctgcgcgctcgctcgctcactgaggccgggcg
accaaaggtcgcccgacgcccgggctttgcccgggcggcctcagtgagcgagcgagcgcgcagctgcctgcagg

>HRPDAAV03-DI (SEQ ID NO:25)

cctgcaggcagctgcgcgctcgctcgctcactgaggccgcccgggcaaagcccgggcgtcgggcgacctttggtcgcccggcctc

agtgagcgagcgagcgcgcagagagggagtggccaactccatcactaggggttcctatcgatatccgttacataacttacggtaaatg

gcccgcctggctgaccgcccaacgacccccgcccattgacgtcaataatgacgtatgttcccatagtaacgtcaatagggactttccatt

gacgtcaatgggtggagtatttacggtaaactgcccacttggcagtacatcaagtgtatcatatgccaagtacgcccccctattgacgtcaa

tgacggtaaatggcccgcctggcattatgcccagtacatgaccttatgggactttcctacttggcagtacatctacgtattagtcatcgctat

taccatggtgatgcggttttggcagtacatcaatgggcgtggatagcggtttgactcacggggatttccaagtctccacccattgacgtc

aatgggagtttgttttgcaccaaaatcaacgggactttccaaaatgtcgtaacaactccgccccattgacgcaaatgggcggtaggcgtg

tacggtgggaggtctatataagcagagctgtgagtttggggacccttgattgttctttcttttcgctattgtaaaattcatgttatatggaggg

ggcaaagttttcagggtgttgtttagaatgggaagatgtcccttgtatcaccatggaccctcatgataattttgtttctttcactttctactctgtt

gacaaccattgtctcctcttattttctttcattttctgtaacttttttcgttaaactttagcttgcatttgtaacgaattttttaaattcacttttgtttatttg

tcagattgtaagtactttctctaatcactttttttttcaaggcaatcagggtatattatattgtacttcagcacagttttagagaacaattgttataat

taaatgataaggtagaatatttctgcatataaattctggctggcgtggaaatattcttattggtagaaacaactacatcctggtcatcatcctg

cctttctctttatggttacaatgatatacactgtttgagatgaggataaaatactctgagtccaaaccgggcccctctgctaaccatgttcatg

ccttcttctttttcctacaggccaccatgaagctgtgggatgtggtggccgtgtgcctggtgctgctgcacaccgcttctgccttcccactg

cctgccggcaagagacctcccgaggcccctgccgaggacagaagcctgggcaggcggagagccccatttgctctgtctagcgattc

caacatgcctgaggattaccccgatcagttcgatgacgtgatggatttcatccaggccaccatcaagagactgaagagatctcctgaca

agcagatggctgtgctgcctagaagggagagaaacaggcaggccgctgctgccaatccagagaactccagggggcaagggcagaa

ggggccagcgcggcaagaatagaggctgcgtgctgacagccatccacctgaacgtgaccgacctgggcctgggctacgagaccaa

ggaggagctgatcttcaggtactgtagcggctcctgtgatgctgccgagaccacatacgacaagatcctgaagaacctgtccaggaac

agaaggctggtgtctgacaaggtgggccaggcttgctgtaggccaatcgctttcgacgacgatctgtcctttctggatgacaacctggtg

taccacatcctgaggaagcattccgctaagagatgtggctgcatctgaaataaagtctgagtgggcggcagcctgtgtgtgcctgggtt

ctctctgtcccggaatgtgcaaacaatggaggtgaagcttcgttacataacttacggtaaatggcccgcctggctgaccgcccaacgac
cccgcccattgacgtcaatagtaacgccaatagggactttccattgacgtcaatgggtggagtatttacggtaaactgcccacttggca
gtacatcaagtgtatcatatgccaagtacgccccctattgacgtcaatgacggtaaatggcccgcctggcattgtgcccagtacatgacc
ttatgggactttcctacttggcagtacatctacgtattagtcatcgctattaccatggtcgaggtgagccccacgttctgcttcactctcccca
tctcccccccctccccacccccaattttgtatttatttattttttaattattttgtgcagcgatggggcggggggggggggggggcgcgc
gccaggcggggcggggcggggcgagggggcggggcggggcgaggcggagaggtgcggcggcagccaatcagagcggcgcg
ctccgaaagtttcctttatggcgaggcggcggcggcggcggccctataaaaagcgaagcgcgcggcgggcgggagtcgctgcgc
gctgccttcgccccgtgccccgctccgccgccgcctcgcgccgcccgccccggctctgactgaccgcgttactcccacaggtgagc
gggcgggacggcccttctcctccgggctgtaattagctgagcaagaggtaagggtttaagggatggttggttggtggggtattaatgttt
aattacctggagcacctgcctgaaatcacttttttttcaggttggggggattcgaacatcggccgccaccatgaatgctagcgagtttcgcag
aaggggcaaggagatggtggattacgtggctaattacatggagggcatcgagggccggcaggtgtacccagatgtggagccaggct
acctgagaccactgatccctgctgccgccccacaggagccagacaccttcgaggatatcatcaatgacgtggagaagatcatcatgcc
tggcgtgacccactggcatagcccatacttctttgcctactttccaacagcctcttcctatcccgctatgctggctgatatgctgtgcggcg
ctatcggctgcatcggcttctcttgggccgctagccctgcttgcacagagctggagaccgtgatgatggattggctgggcaagatgctg
gagctgcctaaggcctttctgaacgagaaggctggcgagggcggcggcgtgatccagggctctgcctccgaggctacactggtggc
tctgctggctgcccggacaaaggtaatccacagactgcaggctgcctctccagagctgacccaggccgctatcatggagaagctggt
ggcttactctagcgaccaggctcattcttccgtggagcgggctggcctgatcggcggcgtgaagctgaaggccatcccttccgacggc
aatttcgccatgagagcttctgccctgcaggaggctctggagagagataaggctgccggcctgatcccttctttatggtggccaccctg
ggcacaaccacctgttgctccttcgacaacctgctggaggtgggccctatctgtaacaaggaggacatctggctgcatgtggatgccg
cttatgctggctctgcctttatctgccccgagtttagacatctgctgaacggcgtggagtttgccgacagctttaacttcaaccctcacaagt
ggctgctggtgaattttgactgctctgccatgtgggtgaagaagaggacagacctgaccggcgcttttaggctggatccaacctacctg
aagcacagccatcaggacagcggcctgatcacagactacagacactggcagatcccactgggcagaaggtttcggtctctgaagatg
tggttcgtgtttcgcatgtatggcgtgaagggcctgcaggcttatatccggaagcacgtgcagctgtctcatgagttcgagtccctggtgc
ggcaggacccaagatttgagatctgcgtggaggtaatcctgggcctggtgtgcttcaggctgaagggctctaataaggtgaacgaggc
tctgctgcagaggatcaacagcgccaagaagatccatctggtgccttgtcatctgagggacaagttcgtgctgagattcgccatctgctc
tagaacagtggagtctgctcatgtgcagagggcttgggagcatatcaaggagctggccgctgacgtgctgagggctgagagggagct
cgagtaagatacattgatgagtttggacaaaccacaactagaatgcagtgaaaaaaatgctttatttgtgaaatttgtgatgctattgctttat
ttgtaaccattataagctgcaataaacaagttcggaccgagcggcagatctaggaaccccagtgatggagttggccactccctctctgc
gcgctcgctcgctcactgaggccgggcgaccaaaggtcgcccgacgcccgggctttgcccgggcggcctcagtgagcgagcgag
cgcgcagctgcctgcagg

>5'ITR (SEQ ID NO: 26)

cctgcaggcagctgcgcgctcgctcgctcactgaggccgcccgggcaaagcccgggcgtcgggcgacctttggtcgcccggcctc
agtgagcgagcgagcgcgcagagagggagtggccaactccatcactaggggttcct

>CMV enhancer 1 (SEQ ID NO: 27)

cgttacataacttacggtaaatggcccgcctggctgaccgcccaacgacccccgcccattgacgtcaatagtaacgccaatagggactt
tccattgacgtcaatgggtggagtatttacggtaaactgcccacttggcagtacatcaagtgtatcatatgccaagtacgccccctattga
cgtcaatgacggtaaatggcccgcctggcattgtgcccagtacatgaccttatgggactttcctacttggcagtacatctacgtattagtca
tcgctattaccatg

>CBA promoter (SEQ ID NO: 28)

tcgaggtgagccccacgttctgcttcactctccccatctcccccccctccccacccccaattttgtatttatttattttttaattattttgtgcagc
gatggggggcggggggggggggggggggcgcgcgccaggcggggcggggcggggcgaggggcggggcggggcgaggcggag
aggtgcggcggcagccaatcagagcggcgcgctccgaaagtttccttttatggcgaggcggcggcggcggcggccctataaaag
cgaagcgcgcggcgggcg

>hybrid intron (SEQ ID NO: 29)

ggagtcgctgcgcgctgccttcgccccgtgccccgctccgccgccgcctcgcgccgcccgccccggctctgactgaccgcgttactc
ccacaggtgagcgggcgggacggcccttctcctccgggctgtaattagctgagcaagaggtaagggtttaagggatggttggttggtg
gggtattaatgtttaattacctggagcacctgcctgaaatcactttttttcag

>hGH poly(A) (SEQ ID NO:30)

Gggtggcatccctgtgacccctccccagtgcctctcctggccctggaagttgccactccagtgcccaccagccttgtcctaataaaatta
agttgcatcattttgtctgactaggtgtccttctataatattatggggtggagggggggtggtatggagcaaggggcaagttgggaagaca
acctgtagggcctgcggggtctattgggaaccaagctggagtgcagtggcacaatcttggctcactgcaatctccgcctcctgggttca
agcgattctcctgcctcagcctcccgagttgttgggattccaggcatgcatgaccaggctcagctaattttttgtttttttggtagagacggg
gtttcaccatattggccaggctggtctccaactcctaatctcaggtgatctacccaccttggcctcccaaattgctgggattacaggcgtg
aaccactgctcccttccctgtcctt

>3'ITR (SEQ ID NO: 31)

Aggaacccctagtgatggagttggccactccctctctgcgcgctcgctcgctcactgaggccgggcgaccaaaggtcgcccgacgc
ccgggctttgcccgggcggcctcagtgagcgagcgagcgcgcagctgcctgcagg

>SV40 poly(A) (SEQ ID NO:32)

Taagatacattgatgagtttggacaaaccacaactagaatgcagtgaaaaaaatgctttatttgtgaaatttgtgatgctattgctttatttgt
aaccattataagctgcaataaacaagtt

>HPRE (SEQ ID NO:33)

ataacaggcctattgattggaaagtttgtcaacgaattgtgggtcttttggggtttgctgcccctttacgcaatgtggatatcctgctttaatg
cctttatatgcatgtatacaagcaaaacaggcttttactttctcgccaacttacaaggcctttctcagtaaacagtatatgacccctttaccccg
ttgctcggcaacggcctggtctgtgccaagtgtttgctgacgcaaccccccactggttggggcttggccataggccatcagcgcatgcgt
ggaacctttgtgtctcctctgccgatccatactgcggaactcctagccgcttgttttgctcgcagcaggtctggagcaaacctcatcgggga
ccgacaattctgtcgtactctcccgcaagtatacatcgtttccatggctgctaggctgtgctgccaactggatcctgcgcgggacgtccttt
gtttacgtcccgtcggcgctgaatcccgcggacgacccctcccggggccgcttggggctctaccgcccgcttctccgtctgccgtacc
gtccgaccacggggcgcacctctctttacgcggactccccgtctgtgccttctcatctgccggaccgtgtgcacttcgcttcacctctgca
cgtcgcatggaggccaccgtgaacgcccaccggaacctgcccaaggtcttgcataagaggactcttggactttcagcaatgtcatc

>WPRE (SEQ ID NO:34)

Aatcaacctctggattacaaaatttgtgaaagattgactggtattcttaactatgttgctccttttacgctatgtggatacgctgctttaatgcc tttgtatcatgctattgcttcccgtatggctttcattttctcctccttgtataaatcctggttgctgtctctttatgaggagttgtggcccgttgtca ggcaacgtggcgtggtgtgcactgtgtttgctgacgcaacccccactggttggggcattgccaccacctgtcagctcctttccgggactt tcgctttccccctccctattgccacggcggaactcatcgccgcctgccttgcccgctgctggacaggggctcggctgttgggcactgac aattccgtggtgttgtcggggaaatcatcgtcctttccttggctgctcgcctgtgttgccacctggattctgcgcgggacgtccttctgctac gtcccttcggccctcaatccagcggaccttccttcccgcggcctgctgccggctctgcggcctcttccgcgtcttcgccttcgccctcag acgagtcggatctccctttgggccgcctccccgc

> CMV promoter (SEQ ID NO: 35)

Gtgatgcggttttggcagtacatcaatgggcgtggatagcggtttgactcacggggatttccaagtctccaccccattgacgtcaatggg agtttgttttgcaccaaaatcaacgggactttccaaaatgtcgtaacaactccgccccattgacgcaaatgggcggtaggcgtgtacggt gggaggtctatataagcagagct

> PA75 polyA (SEQ ID NO:36)

Aataaagtctgagtgggcggcagcctgtgtgtgcctgggttctctctgtcccggaatgtgcaaacaatggaggtgggcctacatcagg aagcacgtgcagctgagccacgagttcgagagcctggtgaggcaggaccccaggttcgagatctgcgtggaggtgatcctgggcct ggtgtgcttcaggctgaagggcagcaacaaggtgaacgaggccctgctgcagaggatcaacagcgccaagaagatccacctggtg ccctgccacctgagggacaagttcgtgctgaggttcgccatctgcagcaggaccgtggagagcgcccacgtgcagagggcctggga gcacatcaaggagctggccgccgacgtgctgagggccgagagggagtgactcgagtaagatacattgatgagtttggacaaaccac aactagaatgcagtgaaaaaaatgctttatttgtgaaatttgtgatgctattgctttatttgtaaccattataagctgcaataaacaagttcgtta

cataacttacggtaaatggcccgcctggctgaccgcccaacgacccccgcccattgacgtcaataatgacgtatgttcccatagtaacg tcaatagggactttccattgacgtcaatgggtggagtatttacggtaaactgcccacttggcagtacatcaagtgtatcatatgccaagtac gccccctattgacgtcaatgacggtaaatggcccgcctggcattatgcccagtacatgaccttatgggactttcctacttggcagtacatc tacgtattagtcatcgctattaccatggtgatgcggttttggcagtacatcaatgggcgtggatagcggtttgactcacggggatttccaag tctccaccccattgacgtcaatgggagtttgttttgcaccaaaatcaacgggactttccaaaatgtcgtaacaactccgccccattgacgc aaatgggcggtaggcgtgtacggtgggaggtctatataagcagagctGCCACCatgaagttatgggatgtcgtggctgtctgcct ggtgctgctccacaccgcgtccgccttcccgctgcccgccggtaagaggcctcccgaggcgcccgccgaagaccgctccctcggcc gccgccgcgcgcccttcgcgctgagcagtgactcaaatatgccagaggattatcctgatcagttcgatgatgtcatggatttttattcaagc caccattaaaagactgaaaaggtc

> CMV enhancer 2 (SEQ ID NO: 37)

Cgttacataacttacggtaaatggcccgcctggctgaccgcccaacgacccccgcccattgacgtcaataatgacgtatgttcccatag taacgtcaatagggactttccattgacgtcaatgggtggagtatttacggtaaactgcccacttggcagtacatcaagtgtatcatatgcca agtacgccccctattgacgtcaatgacggtaaatggcccgcctggcattatgcccagtacatgaccttatgggactttcctacttggcagt acatctacgtattagtcatcgctattaccatg

> β-globin intron (SEQ ID NO: 38)

Gtgagtttggggacccttgattgttctttcttttcgctattgtaaaattcatgttatatggaggggggcaaagttttcagggtgttgtttagaatg
ggaagatgtcccttgtatcaccatggaccctcatgataattttgtttctttcactttctactctgttgacaaccattgtctcctcttattttcttttcat
tttctgtaacttttcgttaaactttagcttgcatttgtaacgaattttttaaattcactttgtttatttgtcagattgtaagtactttctctaatcactttt
ttttcaaggcaatcaggggtatattatattgtacttcagcacagttttagagaacaattgttataattaaatgataaggtagaatatttctgcatat
aaattctggctggcgtggaaatattcttattggtagaaacaactacatcctggtcatcatcctgcctttctctttatggttacaatgatatacac
tgtttgagatgaggataaaatactctgagtccaaaccgggcccctctgctaaccatgttcatgccttcttcttttttcctacag

> MVM intron (SEQ ID NO:39)
aagaggtaagggtttaagggatggttggttggtggggtattaatgtttaattacctggagcacctgcctgaaatcactttttttcaggttgg
> MCS (SEQ ID NO:40)
atcgatatccgttaca
> AAV2 capsid (SEQ ID NO: 41)

atggctgccgatggttatcttccagattggctcgaggacactctctctgaaggaataagacagtggtggaagctcaaacctggcccacc
accaccaaagcccgcagagcggcataaggacgacagcaggggtcttgtgcttcctgggtacaagtacctcggacccttcaacggact
cgacaagggagagccggtcaacgaggcagacgccgcggccctcgagcacgacaaagcctacgaccggcagctcgacagcgga
gacaacccgtacctcaagtacaaccacgccgacgcggagtttcaggagcgcccttaaagaagatacgtctttggggggcaacctcgga
cgagcagtcttccaggcgaaaaagagggttcttgaacctctgggcctggttgaggaacctgttaagacggctccgggaaaaaagagg
ccggtagagcactctcctgtggagccagactcctcctcgggaaccggaaaggcgggccagcagcctgcaagaaaaagattgaatttt
ggtcagactggagacgcagactcagtacctgacccccagcctctcggacagccaccagcagcccctctggtctgggaactaatacg
atggctacaggcagtggcgcaccaatggcagacaataacgagggcgccgacggagtgggtaattcctcgggaaattggcattgcga
ttccacatggatgggcgacagagtcatcaccaccagcacccgaacctgggccctgcccacctacaacaaccacctctacaaacaatt
tccagccaatcaggagcctcgaacgacaatcactactttggctacagcaccccttgggggtattttgacttcaacagattccactgccact
tttcaccacgtgactggcaaagactcatcaacaacaactggggattccgacccaagagactcaacttcaagctctttaacattcaagtca
aagaggtcacgcagaatgacggtacgacgacgattgccaataaccttaccagcacggttcaggtgtttactgactcggagtaccagct
cccgtacgtcctcggctcggcgcatcaaggatgcctcccgccgttcccagcagacgtcttcatggtgccacagtatggatacctcaccc
tgaacaacgggagtcaggcagtaggacgctcttcattttactgcctggagtactttccttctcagatgctgcgtaccggaaacaactttac
cttcagctacacttttgaggacgttcctttccacagcagctacgctcacagccagagtctggaccgtctcatgaatcctctcatcgaccag
tacctgtattacttgagcagaacaaacactccaagtggaaccaccacgcagtcaaggcttcagttttctcaggccggagcgagtgacatt
cgggaccagtctaggaactggcttcctggaccctgttaccgccagcagcgagtatcaaagacatctgcggataacaacaacagtgaat
actcgtggactggagctaccaagtaccacctcaatggcagagactctctggtgaatccgggcccggccatggcaagcacaaggac

gatgaagaaagttttttcctcagagcgggggttctcatctttgggaagcaaggctcagagaaaacaaatgtggacattgaaaaggtcatg
attacagacgaagaggaaatcaggacaaccaatcccgtggctacggagcagtatggttctgtatctaccaacctccagagaggcaaca
gacaagcagctaccgcagatgtcaacacacaaggcgttcttccaggcatggtctggcaggacagagatgtgtaccttcaggggccca
tctgggcaaagattccacacacggacggacatttttcaccctctcccctcatgggtggattcggacttaaacaccctcctccacagattct
catcaagaacaccccggtacctgcgaatccttcgaccaccttcagtgcggcaaagtttgcttccttcatcacacagtactccacgggaca
ggtcagcgtggagatcgagtgggagctgcagaaggaaaacagcaaacgctggaatcccgaaattcagtacacttccaactacaacaa
gtctgttaatgtggactttactgtggacactaatggcgtgtattcagagcctcgcccccattggcaccagatacctgactcgtaatctgtaa

> AAV9 capsid (SEQ ID NO: 42)

atggctgccgatggttatcttccagattggctcgaggacaaccttagtgaaggaattcgcgagtggtgggctttgaaacctggagcccct
caacccaaggcaaatcaacaacatcaagacaacgctcgaggtcttgtgcttccgggttacaaataccttggacccggcaacggactcg
acaaggggggagccggtcaacgcagcagacgcggcggccctcgagcacgacaaggcctacgaccagcagctcaaggccggaga
caacccgtacctcaagtacaaccacgccgacgccgagttccaggagcggctcaaagaagatacgtcttttggggggcaacctcgggc
gagcagtcttccaggccaaaaagaggcttcttgaacctcttggtctggttgaggaagcggctaagacggctcctggaaagaagaggc
ctgtagagcagtctcctcaggaaccggactcctccgcgggtattggcaaatcgggtgcacagcccgctaaaaagagactcaatttcgg
tcagactggcgacacagagtcagtcccagaccctcaaccaatcggagaacctcccgcagcccctcaggtgtgggatctcttacaatg
gcttcaggtggtggcgcaccagtggcagacaataacgaaggtgccgatggagtgggtagttcctcgggaaattggcattgcgattccc
aatggctggggggacagagtcatcaccaccagcacccgaacctgggccctgcccacctacaacaatcacctctacaagcaaatctcca
acagcacatctggaggatcttcaaatgacaacgcctacttcggctacagcaccccctggggggtattttgacttcaacagattccactgcc
acttctcaccacgtgactggcagcgactcatcaacaacaactggggattccggcctaagcgactcaacttcaagctcttcaacattcagg
tcaaagaggttacggacaacaatggagtcaagaccatcgccaataaccttaccagcacggtccaggtcttcacggactcagactatca
gctcccgtacgtgctcgggtcggctcacgagggctgcctcccgccgttcccagcggacgttttcatgattcctcagtacgggtatctgac
gcttaatgatggaagccaggccgtgggtcgttcgtcctttactgcctggaatatttcccgtcgcaaatgctaagaacgggtaacaacttc
cagttcagctacgagtttgagaacgtacctttccatagcagctacgctcacagccaaagcctggaccgactaatgaatccactcatcgac
caatacttgtactatctctcaaagactattaacggttctggacagaatcaacaaacgctaaaattcagtgtggccggacccagcaacatg
gctgtccagggaagaaaactacatacctggacccagctaccgacaacaacgtgtctcaaccactgtgactcaaaacaacaacagcgaa
tttgcttggcctggagcttcttcttgggctctcaatggacgtaatagcttgatgaatcctggacctgctatggccagccacaaagaaggag
aggaccgtttctttcctttgtctggatctttaatttttggcaaacaaggaactggaagagacaacgtggatgcggacaaagtcatgataacc
aacgaagaagaaattaaaactactaacccggtagcaacggagtcctatggacaagtggccacaaaccaccagagtgcccaagcaca
ggcgcagaccggctgggttcaaaaccaaggaatacttccgggtatggtttggcaggacagagatgtgtacctgcaaggacccatttgg
gccaaaattcctcacacggacggcaactttcacccttctccgctgatgggagggtttggaatgaagcacccgcctcctcagatcctcatc
aaaaacacacctgtacctgcggatcctccaacggccttcaacaaggacaagctgaactctttcatcacccagtattctactggccaagtc
agcgtggagatcgagtgggagctgcagaaggaaaacagcaagcgctggaacccggagatccagtacacttccaactattacaagtct
aataatgttgaatttgctgttaatactgaaggtgtatatagtgaaccccgccccattggcaccagatacctgactcgtaatctgtaa

> T2A amino acid sequence (SEQ ID NO: 43)
EGRGSLLTCGDVEENPGP
> P2A amino acid sequence (SEQ ID NO: 44)
ATNFSLLKQAGDVEENPGP
> E2A amino acid sequence (SEQ ID NO: 45)
QCTNYALLKLAGDVESNPGP
> F2A amino acid sequence (SEQ ID NO: 46)
VKQTLNFDLLKLAGDVESNPGP
> T2A nucleotide sequence (SEQ ID NO: 47)
gagggcagaggcagtctgctgacatgcggtgacgtggaagagaatcccggccct
> P2A nucleotide sequence (SEQ ID NO: 48)
gccaccaacttctccctgctgaagcaggccggcgacgtggaggagaaccccggcccc
> E2A nucleotide sequence (SEQ ID NO: 49)
cagtgcaccaactacgccctgctgaagctggccggcgatgtggagagcaaccccgggccc
> F2A nucleotide sequence (SEQ ID NO: 50)
gtgaaacagactttgaattttgaccttctcaagttggcgggagacgtggagtccaaccctggacct
> Wild-type VEGFR2 D2D3 sequence:

SPFIASVSDQHGVVYITENKNKTVVIPCLGSISNLNVSLCARYPEKRFVPDGNRISWDS
KKGFTIPSYMISYAGMVFCEAKINDESYQSIMYIVVVVGYRIYDVVLSPSHGIELSVGE
KLVLNCTARTELNVGIDFNWEYPSSKHQHKKLVNRDLKTQSGSEMKKFLSTLTIDGV
TRSDQGLYTCAASSGLMTKKNSTFVRVHEK

SEQ ID NO: 119

>Aflibercept (PR1076)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKDKTHTCPPCPAPELLGGPSVFLFPPKPKDTLM
ISRTPEVTCVVVDVSHEDPEVKFNWYVDGVEVHNAKTKPREEQYNSTYRVVSVLTVL
HQDWLNGKEYKCKVSNKALPAPIEKTISKAKGQPREPQVYTLPPSRDELTKNQVSLTC
LVKGFYPSDIAVEWESNGQPENNYKTTPPVLDSDGSFFLYSKLTVDKSRWQQGNVFSC
SVMHEALHNHYTQKSLSLSPG

SEQ ID NO: 120

>OPT-302(HRAMD-C3)

YSMTPPTLNITEESHVIDTGDSLSISCRGQHPLEWAWPGAQEAPATGDKDSEDTGVVR
DCEGTDARPYCKVLLLHEVHAQDTGSYVCYYKYIKARIEGTTAASSYVFVRDFEQPFI
NKPDTLLVNRKDAMWVPCLVSIPGLNVTLRSQSSVLWPDGQEVVWDDRRGMLVSTP
LLHDALYLQCETTWGDQDFLSNPFLVHITGNELYDIQLLPRKSLELLVGEKLVLNCTV
WAEFNSGVTFDWDYPGKQAERGKWVPERRSQQTHTELSSILTIHNVSQHDLGSYVCK
ANNGIQRFRESTEVIVHEEPKSCDKTHTCPPCPAPELLGGPSVFLFPPKPKDTLMISRTP
EVTCVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLTCLVK
GFYPSDIAVEWESNGQPENNYKTTPPVLDSDGSFFLYSKLTVDKSRWQQGNVFSCSV
MHEALHNHYTQKSLSLSPGK

SEQ ID NO: 121

>Bevacizumab

Heavy chain:

EVQLVESGGGLVQPGGSLRLSCAASGYTFTNYGMNWVRQAPGKGLEWVGWINTYT
GEPTYAADFKRRFTFSLDTSKSTAYLQMNSLRAEDTAVYYCAKYPHYYGSSHWYFDV
WGQGTLVTVSSASTKGPSVFPLAPSSKSTSGGTAALGCLVKDYFPEPVTVSWNSGALT

113

SGVHTFPAVLQSSGLYSLSSVVTVPSSSLGTQTYICNVNHKPSNTKVDKKVEPKSCDK
THTCPPCPAPEAAGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTIS
KAKGQPREPQVYTLPPSREEMTKNQVSLTCLVKGFYPSDIAVEWESNGQPENNYKTT
PPVLDSDGSFFLYSKLTVDKSRWQQGNVFSCSVMHEALHNHYTQKSLSLSPGK

SEQ ID NO: 122

Light chain:

DIQMTQSPSSLSASVGDRVTITCSASQDISNYLNWYQQKPGKAPKVLIYFTSSLHSGVP
SRFSGSGSGTDFTLTISSLQPEDFATYYCQQYSTVPWTFGQGTKVEIKRTVAAPSVFIFP
PSDEQLKSGTASVVCLLNNFYPREAKVQWKVDNALQSGNSQESVTEQDSKDSTYSLS
STLTLSKADYEKHKVYACEVTHQGLSSPVTKSFNRGEC

SEQ ID NO: 123

>PR1010(R1D2-R2D3-linker-R2D2-R2D3-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEK*GGGGS*SPFIASVSDQHGVVYITENKNKTVVIP
CLGSISNLNVSLCARYPEKRFVPDGNRISWDSKKGFTIPSYMISYAGMVFCEAKINDES
YQSIMYIVVVVGYRIYDVVLSPSHGIELSVGEKLVLNCTARTELNVGIDFNWEYPSSK
HQHKKLVNRDLKTQSGSEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 53

>PR1011(R2D2-R2D3-linker-R1D2-R2D3-Fc)

SPFIASVSDQHGVVYITENKNKTVVIPCLGSISNLNVSLCARYPEKRFVPDGNRISWDS
KKGFTIPSYMISYAGMVFCEAKINDESYQSIMYIVVVVGYRIYDVVLSPSHGIELSVGE
KLVLNCTARTELNVGIDFNWEYPSSKHQHKKLVNRDLKTQSGSEMKKFLSTLTIDGV
TRSDQGLYTCAASSGLMTKKNSTFVRVHEK*GGGGS*SDTGRPFVEMYSEIPEIIHMTEG
RELVIPCRVTSPNITVTLKKFPLDTLIPDGKRIIWDSRKGFIISNATYKEIGLLTCEATVNG
HLYKTNYLTHRQTNTIIDVVLSPSHGIELSVGEKLVLNCTARTELNVGIDFNWEYPSSK
HQHKKLVNRDLKTQSGSEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 54


>PR1088(R2D2-R2D3-Fc)

SPFIASVSDQHGVVYITENKNKTVVIPCLGSISNLNVSLCARYPEKRFVPDGNRISWDS
KKGFTIPSYMISYAGMVFCEAKINDESYQSIMYIVVVVGYRIYDVVLSPSHGIELSVGE
KLVLNCTARTELNVGIDFNWEYPSSKHQHKKLVNRDLKTQSGSEMKKFLSTLTIDGV
TRSDQGLYTCAASSGLMTKKNSTFVRVHEKDKTHTCPPCPAPEAAGGPSVFLFPPKPK
DTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSREEMTKN
QVSLTCLVKGFYPSDIAVEWESNGQPENNYKTTPPVLDSDGSFFLYSKLTVDKSRWQQ
GNVFSCSVMHEALHNHYTQKSLSLSPGK

SEQ ID NO: 55

>PR1160(R2D2(C162V)-R2D3-Fc)

SPFIASVSDQHGVVYITENKNKTVVIPCLGSISNLNVSLVARYPEKRFVPDGNRISWDS
KKGFTIPSYMISYAGMVFCEAKINDESYQSIMYIVVVVGYRIYDVVLSPSHGIELSVGE
KLVLNCTARTELNVGIDFNWEYPSSKHQHKKLVNRDLKTQSGSEMKKFLSTLTIDGV
TRSDQGLYTCAASSGLMTKKNSTFVRVHEKDKTHTCPPCPAPEAAGGPSVFLFPPKPK
DTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSREEMTKN
QVSLTCLVKGFYPSDIAVEWESNGQPENNYKTTPPVLDSDGSFFLYSKLTVDKSRWQQ
GNVFSCSVMHEALHNHYTQKSLSLSPGK

SEQ ID NO: 56

>PR1090(R1D2-R2D3-linker-R2D2(V135Y)-R2D3-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGYVYITENKNKTVVIP
CLGSISNLNVSLCARYPEKRFVPDGNRISWDSKKGFTIPSYMISYAGMVFCEAKINDES
YQSIMYIVVVVGYRIYDVVLSPSHGIELSVGEKLVLNCTARTELNVGIDFNWEYPSSK
HQHKKLVNRDLKTQSGSEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 57

>PR1094(R1D2-R2D3-linker-R2D2(C162V)-R2D3-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD

QGLYTCAASSGLMTKKNSTFVRVHEKGGGGSSPFIASVSDQHGVVYITENKNKTVVIP
CLGSISNLNVSLVARYPEKRFVPDGNRISWDSKKGFTIPSYMISYAGMVFCEAKINDES
YQSIMYIVVVVGYRIYDVVLSPSHGIELSVGEKLVLNCTARTELNVGIDFNWEYPSSK
HQHKKLVNRDLKTQSGSEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 58

>PR1101(R1D2-R2D3-linker-R2D2(M197Y)-R2D3-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLCARYPEKRFVPDGNRISWDSKKGFTIPSYMISYAGYVFCEAKINDES
YQSIMYIVVVVGYRIYDVVLSPSHGIELSVGEKLVLNCTARTELNVGIDFNWEYPSSK
HQHKKLVNRDLKTQSGSEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 59

>PR1103(R1D2-R2D3-linker-R2D2(F199Q)-R2D3-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLCARYPEKRFVPDGNRISWDSKKGFTIPSYMISYAGMVQCEAKINDES
YQSIMYIVVVVGYRIYDVVLSPSHGIELSVGEKLVLNCTARTELNVGIDFNWEYPSSK
HQHKKLVNRDLKTQSGSEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 60

>PR1106(R1D2-R2D3-linker-R2D2(M213N)-R2D3-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLCARYPEKRFVPDGNRISWDSKKGFTIPSYMISYAGMVFCEAKINDES
YQSINYIVVVVGYRIYDVVLSPSHGIELSVGEKLVLNCTARTELNVGIDFNWEYPSSKH
QHKKLVNRDLKTQSGSEMKKFLSTLTIDGVTRSDQGLYTCAASSGLMTKKNSTFVRV
HEKDKTHTCPPCPAPEAAGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPA
PIEKTISKAKGQPREPQVYTLPPSREEMTKNQVSLTCLVKGFYPSDIAVEWESNGQPEN
NYKTTPPVLDSDGSFFLYSKLTVDKSRWQQGNVFSCSVMHEALHNHYTQKSLSLSPG
K

SEQ ID NO: 61

>PR1107(R1D2-R2D3-linker-R2D2(I215F)-R2D3-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLCARYPEKRFVPDGNRISWDSKKGFTIPSYMISYAGMVFCEAKINDES
YQSIMYFVVVVGYRIYDVVLSPSHGIELSVGEKLVLNCTARTELNVGIDFNWEYPSSK
HQHKKLVNRDLKTQSGSEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 62

>PR1108(R1D2-R2D3-linker-R2D2(I215H)-R2D3-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLCARYPEKRFVPDGNRISWDSKKGFTIPSYMISYAGMVFCEAKINDES
YQSIMYHVVVVGYRIYDVVLSPSHGIELSVGEKLVLNCTARTELNVGIDFNWEYPSSK
HQHKKLVNRDLKTQSGSEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP

GK

SEQ ID NO: 63

>PR1109(R1D2-R2D3-linker-R2D2(V2191)-R2D3-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLCARYPEKRFVPDGNRISWDSKKGFTIPSYMISYAGMVFCEAKINDES
YQSIMYIVVVIGYRIYDVVLSPSHGIELSVGEKLVLNCTARTELNVGIDFNWEYPSSKH
QHKKLVNRDLKTQSGSEMKKFLSTLTIDGVTRSDQGLYTCAASSGLMTKKNSTFVRV
HEKDKTHTCPPCPAPEAAGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPA
PIEKTISKAKGQPREPQVYTLPPSREEMTKNQVSLTCLVKGFYPSDIAVEWESNGQPEN
NYKTTPPVLDSDGSFFLYSKLTVDKSRWQQGNVFSCSVMHEALHNHYTQKSLSLSPG
K

SEQ ID NO: 64

>PR1110(R1D2-R2D3-linker-R2D2(Y221N)-R2D3-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLCARYPEKRFVPDGNRISWDSKKGFTIPSYMISYAGMVFCEAKINDES
YQSIMYIVVVVGNRIYDVVLSPSHGIELSVGEKLVLNCTARTELNVGIDFNWEYPSSK
HQHKKLVNRDLKTQSGSEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 65

>PR1111(R1D2-R2D3-linker-R2D2-R2D3(D257Q)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLCARYPEKRFVPDGNRISWDSKKGFTIPSYMISYAGMVFCEAKINDES
YQSIMYIVVVVGYRIYDVVLSPSHGIELSVGEKLVLNCTARTELNVGIQFNWEYPSSK
HQHKKLVNRDLKTQSGSEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK

FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 66

>PR1112(R1D2-R2D3-linker-R2D2-R2D3(D257T)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLCARYPEKRFVPDGNRISWDSKKGFTIPSYMISYAGMVFCEAKINDES
YQSIMYIVVVVGYRIYDVVLSPSHGIELSVGEKLVLNCTARTELNVGITFNWEYPSSK
HQHKKLVNRDLKTQSGSEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 67

>PR1113(R1D2-R2D3-linker-R2D2-R2D3(N274F)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLCARYPEKRFVPDGNRISWDSKKGFTIPSYMISYAGMVFCEAKINDES
YQSIMYIVVVVGYRIYDVVLSPSHGIELSVGEKLVLNCTARTELNVGIDFNWEYPSSK
HQHKKLVFRDLKTQSGSEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 68

>PR1114(R1D2-R2D3-linker-R2D2-R2D3(N274I)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLCARYPEKRFVPDGNRISWDSKKGFTIPSYMISYAGMVFCEAKINDES

YQSIMYIVVVVGYRIYDVVLSPSHGIELSVGEKLVLNCTARTELNVGIDFNWEYPSSK
HQHKKLVIRDLKTQSGSEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 69

>PR1115(R1D2-R2D3-linker-R2D2-R2D3(R275L)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLCARYPEKRFVPDGNRISWDSKKGFTIPSYMISYAGMVFCEAKINDES
YQSIMYIVVVVGYRIYDVVLSPSHGIELSVGEKLVLNCTARTELNVGIDFNWEYPSSK
HQHKKLVNLDLKTQSGSEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 70

>PR1116(R1D2-R2D3-linker-R2D2-R2D3(R275E)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLCARYPEKRFVPDGNRISWDSKKGFTIPSYMISYAGMVFCEAKINDES
YQSIMYIVVVVGYRIYDVVLSPSHGIELSVGEKLVLNCTARTELNVGIDFNWEYPSSK
HQHKKLVNEDLKTQSGSEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 71

>PR1118(R1D2-R2D3-linker-R2D2-R2D3(D276Q)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV

LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLCARYPEKRFVPDGNRISWDSKKGFTIPSYMISYAGMVFCEAKINDES
YQSIMYIVVVVGYRIYDVVLSPSHGIELSVGEKLVLNCTARTELNVGIDFNWEYPSSK
HQHKKLVNRQLKTQSGSEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 72

>PR1119(R1D2-R2D3-linker-R2D2-R2D3(L277R)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLCARYPEKRFVPDGNRISWDSKKGFTIPSYMISYAGMVFCEAKINDES
YQSIMYIVVVVGYRIYDVVLSPSHGIELSVGEKLVLNCTARTELNVGIDFNWEYPSSK
HQHKKLVNRDRKTQSGSEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 73

>PR1120(R1D2-R2D3-linker-R2D2-R2D3(K278D)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLCARYPEKRFVPDGNRISWDSKKGFTIPSYMISYAGMVFCEAKINDES
YQSIMYIVVVVGYRIYDVVLSPSHGIELSVGEKLVLNCTARTELNVGIDFNWEYPSSK
HQHKKLVNRDLDTQSGSEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 74

>PR1121(R1D2-R2D3-linker-R2D2-R2D3(K2788S)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLCARYPEKRFVPDGNRISWDSKKGFTIPSYMISYAGMVFCEAKINDES
YQSIMYIVVVVGYRIYDVVLSPSHGIELSVGEKLVLNCTARTELNVGIDFNWEYPSSK
HQHKKLVNRDLSTQSGSEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 75

>PR1122(R1D2-R2D3-linker-R2D2-R2D3(K278E)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLCARYPEKRFVPDGNRISWDSKKGFTIPSYMISYAGMVFCEAKINDES
YQSIMYIVVVVGYRIYDVVLSPSHGIELSVGEKLVLNCTARTELNVGIDFNWEYPSSK
HQHKKLVNRDLETQSGSEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 76

>PR1123(R1D2-R2D3-linker-R2D2-R2D3(Q280E)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLCARYPEKRFVPDGNRISWDSKKGFTIPSYMISYAGMVFCEAKINDES
YQSIMYIVVVVGYRIYDVVLSPSHGIELSVGEKLVLNCTARTELNVGIDFNWEYPSSK
HQHKKLVNRDLKTESGSEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE

NNYKTTPPVLDSDGSFFLYSKLTVDKSRWQQGNVFSCSVMHEALHNHYTQKSLSLSP
GK

SEQ ID NO: 77

>PR1124(R1D2-R2D3-linker-R2D2-R2D3(S283T)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLCARYPEKRFVPDGNRISWDSKKGFTIPSYMISYAGMVFCEAKINDES
YQSIMYIVVVVGYRIYDVVLSPSHGIELSVGEKLVLNCTARTELNVGIDFNWEYPSSK
HQHKKLVNRDLKTQSGTEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 78

>PR1125(R1D2-R2D3-linker-R2D2-R2D3(E284H)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLCARYPEKRFVPDGNRISWDSKKGFTIPSYMISYAGMVFCEAKINDES
YQSIMYIVVVVGYRIYDVVLSPSHGIELSVGEKLVLNCTARTELNVGIDFNWEYPSSK
HQHKKLVNRDLKTQSGSHMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 79

>PR1126(R1D2-R2D3-linker-R2D2-R2D3(F288Y)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLCARYPEKRFVPDGNRISWDSKKGFTIPSYMISYAGMVFCEAKINDES
YQSIMYIVVVVGYRIYDVVLSPSHGIELSVGEKLVLNCTARTELNVGIDFNWEYPSSK
HQHKKLVNRDLKTQSGSEMKKYLSTLTIDGVTRSDQGLYTCAASSGLMTKKNSTFVR

VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 80

>PR1127(R1D2-R2D3-linker-R2D2-R2D3(L289Y)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLCARYPEKRFVPDGNRISWDSKKGFTIPSYMISYAGMVFCEAKINDES
YQSIMYIVVVVGYRIYDVVLSPSHGIELSVGEKLVLNCTARTELNVGIDFNWEYPSSK
HQHKKLVNRDLKTQSGSEMKKFYSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 81

>PR1128(R1D2-R2D3-linker-R2D2-R2D3(L313Y)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLCARYPEKRFVPDGNRISWDSKKGFTIPSYMISYAGMVFCEAKINDES
YQSIMYIVVVVGYRIYDVVLSPSHGIELSVGEKLVLNCTARTELNVGIDFNWEYPSSK
HQHKKLVNRDLKTQSGSEMKKFLSTLTIDGVTRSDQGLYTCAASSGYMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 82

>PR1129(R1D2-R2D3-linker-R2D2-R2D3(M314S)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP

CLGSISNLNVSLCARYPEKRFVPDGNRISWDSKKGFTIPSYMISYAGMVFCEAKINDES
YQSIMYIVVVVGYRIYDVVLSPSHGIELSVGEKLVLNCTARTELNVGIDFNWEYPSSK
HQHKKLVNRDLKTQSGSEMKKFLSTLTIDGVTRSDQGLYTCAASSGLSTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 83

>PR1130(R1D2-R2D3-linker-R2D2-R2D3(M314Q)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLCARYPEKRFVPDGNRISWDSKKGFTIPSYMISYAGMVFCEAKINDES
YQSIMYIVVVVGYRIYDVVLSPSHGIELSVGEKLVLNCTARTELNVGIDFNWEYPSSK
HQHKKLVNRDLKTQSGSEMKKFLSTLTIDGVTRSDQGLYTCAASSGLQTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 84

>PR1131(R1D2-R2D3-linker-R2D2(Y221N)-R2D3(M314Q)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLCARYPEKRFVPDGNRISWDSKKGFTIPSYMISYAGMVFCEAKINDES
YQSIMYIVVVVGNRIYDVVLSPSHGIELSVGEKLVLNCTARTELNVGIDFNWEYPSSK
HQHKKLVNRDLKTQSGSEMKKFLSTLTIDGVTRSDQGLYTCAASSGLQTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 85

>PR1132(R1D2-R2D3-linker-R2D2-R2D3(L277R/L289Y)-Fc)

SDTGRPFVEMYSEIPEIIHMTEGRELVIPCRVTSPNITVTLKKFPLDTLIPDGKRIIWDSR

KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLCARYPEKRFVPDGNRISWDSKKGFTIPSYMISYAGMVFCEAKINDES
YQSIMYIVVVVGYRIYDVVLSPSHGIELSVGEKLVLNCTARTELNVGIDFNWEYPSSK
HQHKKLVNRDRKTQSGSEMKKFYSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 86

>PR1135(R1D2-R2D3-linker-R2D2(Y221S/R222K)-R2D3-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLCARYPEKRFVPDGNRISWDSKKGFTIPSYMISYAGMVFCEAKINDES
YQSIMYIVVVVGSKIYDVVLSPSHGIELSVGEKLVLNCTARTELNVGIDFNWEYPSSK
HQHKKLVNRDLKTQSGSEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 87

>PR1136(R1D2-R2D3-linker-R2D2-R2D3(L313R)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLCARYPEKRFVPDGNRISWDSKKGFTIPSYMISYAGMVFCEAKINDES
YQSIMYIVVVVGYRIYDVVLSPSHGIELSVGEKLVLNCTARTELNVGIDFNWEYPSSK
HQHKKLVNRDLKTQSGSEMKKFLSTLTIDGVTRSDQGLYTCAASSGRMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 88

>PR1139(R1D2-R2D3-linker-R2D2-R2D3(N245Q)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLCARYPEKRFVPDGNRISWDSKKGFTIPSYMISYAGMVFCEAKINDES
YQSIMYIVVVVGYRIYDVVLSPSHGIELSVGEKLVLQCTARTELNVGIDFNWEYPSSK
HQHKKLVNRDLKTQSGSEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 89

>PR1141(R1D2-R2D3-linker-R2D2(C162V)-R2D3(N274F)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLVARYPEKRFVPDGNRISWDSKKGFTIPSYMISYAGMVFCEAKINDES
YQSIMYIVVVVGYRIYDVVLSPSHGIELSVGEKLVLNCTARTELNVGIDFNWEYPSSK
HQHKKLVFRDLKTQSGSEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 90

>PR1142(R1D2-R2D3-linker-R2D2(C162V)-R2D3(N274I)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLVARYPEKRFVPDGNRISWDSKKGFTIPSYMISYAGMVFCEAKINDES
YQSIMYIVVVVGYRIYDVVLSPSHGIELSVGEKLVLNCTARTELNVGIDFNWEYPSSK
HQHKKLVIRDLKTQSGSEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP

APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 91

>PR1143(R1D2-R2D3-linker-R2D2(C162V)-R2D3(R275L)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLVARYPEKRFVPDGNRISWDSKKGFTIPSYMISYAGMVFCEAKINDES
YQSIMYIVVVVGYRIYDVVLSPSHGIELSVGEKLVLNCTARTELNVGIDFNWEYPSSK
HQHKKLVNLDLKTQSGSEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 92

>PR1144(R1D2-R2D3-linker-R2D2(C162V)-R2D3(D276Q)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLVARYPEKRFVPDGNRISWDSKKGFTIPSYMISYAGMVFCEAKINDES
YQSIMYIVVVVGYRIYDVVLSPSHGIELSVGEKLVLNCTARTELNVGIDFNWEYPSSK
HQHKKLVNRQLKTQSGSEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 93

>PR1145(R1D2-R2D3-linker-R2D2(C162V)-R2D3(S283T)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLVARYPEKRFVPDGNRISWDSKKGFTIPSYMISYAGMVFCEAKINDES
YQSIMYIVVVVGYRIYDVVLSPSHGIELSVGEKLVLNCTARTELNVGIDFNWEYPSSK

HQHKKLVNRDLKTQSGTEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 94

>PR1146(R1D2-R2D3-linker-R2D2(C162V)-R2D3(L289Y)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLVARYPEKRFVPDGNRISWDSKKGFTIPSYMISYAGMVFCEAKINDES
YQSIMYIVVVVGYRIYDVVLSPSHGIELSVGEKLVLNCTARTELNVGIDFNWEYPSSK
HQHKKLVNRDLKTQSGSEMKKFYSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 95

>PR1147(R1D2-R2D3-linker-R2D2(C162V)-R2D3(L313Y)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLVARYPEKRFVPDGNRISWDSKKGFTIPSYMISYAGMVFCEAKINDES
YQSIMYIVVVVGYRIYDVVLSPSHGIELSVGEKLVLNCTARTELNVGIDFNWEYPSSK
HQHKKLVNRDLKTQSGSEMKKFLSTLTIDGVTRSDQGLYTCAASSGYMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 96

>PR1148(R1D2-R2D3-linker-R2D2(C162V)-R2D3(L277R/L289Y)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD

QGLYTCAASSGLMTKKNSTFVRVHEKGGGGSSPFIASVSDQHGVVYITENKNKTVVIP
CLGSISNLNVSLVARYPEKRFVPDGNRISWDSKKGFTIPSYMISYAGMVFCEAKINDES
YQSIMYIVVVVGYRIYDVVLSPSHGIELSVGEKLVLNCTARTELNVGIDFNWEYPSSK
HQHKKLVNRDRKTQSGSEMKKFYSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 97

>PR1149(R1D2-R2D3-linker-R2D2(C162V/Y221S/R222K)-R2D3-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLVARYPEKRFVPDGNRISWDSKKGFTIPSYMISYAGMVFCEAKINDES
YQSIMYIVVVVGSKIYDVVLSPSHGIELSVGEKLVLNCTARTELNVGIDFNWEYPSSK
HQHKKLVNRDLKTQSGSEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 98

>PR1150(R1D2-R2D3-linker-R2D2(C162V)-R2D3(L313R)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLVARYPEKRFVPDGNRISWDSKKGFTIPSYMISYAGMVFCEAKINDES
YQSIMYIVVVVGYRIYDVVLSPSHGIELSVGEKLVLNCTARTELNVGIDFNWEYPSSK
HQHKKLVNRDLKTQSGSEMKKFLSTLTIDGVTRSDQGLYTCAASSGRMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 99

> PR1163 (heavy chain H: R2D2 (C162V)-R2D3-Fc-linker-VH-CH1; light chain L: VL-CL)

Heavy chain:

SPFIASVSDQHGVVYITENKNKTVVIPCLGSISNLNVSLVARYPEKRFVPDGNRISWDS
KKGFTIPSYMISYAGMVFCEAKINDESYQSIMYIVVVVGYRIYDVVLSPSHGIELSVGE
KLVLNCTARTELNVGIDFNWEYPSSKHQHKKLVNRDLKTQSGSEMKKFLSTLTIDGV
TRSDQGLYTCAASSGLMTKKNSTFVRVHEKDKTHTCPPCPAPEAAGGPSVFLFPPKPK
DTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSREEMTKN
QVSLTCLVKGFYPSDIAVEWESNGQPENNYKTTPPVLDSDGSFFLYSKLTVDKSRWQQ
GNVFSCSVMHEALHNHYTQKSLSLSPGGGGGSGGGGSGGGGSGGGGSEVQLVESGG
GLVQPGGSLRLSCAASGYTFTNYGMNWVRQAPGKGLEWVGWINTYTGEPTYAADF
KRRFTFSLDTSKSTAYLQMNSLRAEDTAVYYCAKYPHYYGSSHWYFDVWGQGTLVT
VSSASTKGPSVFPLAPSSKSTSGGTAALGCLVKDYFPEPVTVSWNSGALTSGVHTFPAV
LQSSGLYSLSSVVTVPSSSLGTQTYICNVNHKPSNTKVDKKVEPKSC

SEQ ID NO: 100

Light chain:

DIQMTQSPSSLSASVGDRVTITCSASQDISNYLNWYQQKPGKAPKVLIYFTSSLHSGVP
SRFSGSGSGTDFTLTISSLQPEDFATYYCQQYSTVPWTFGQGTKVEIKRTVAAPSVFIFP
PSDEQLKSGTASVVCLLNNFYPREAKVQWKVDNALQSGNSQESVTEQDSKDSTYSLS
STLTLSKADYEKHKVYACEVTHQGLSSPVTKSFNRGEC

SEQ ID NO: 101

>PR1165(R1D2-R2D3-linker-R2D2(C162I)-R2D3-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLIARYPEKRFVPDGNRISWDSKKGFTIPSYMISYAGMVFCEAKINDES
YQSIMYIVVVVGYRIYDVVLSPSHGIELSVGEKLVLNCTARTELNVGIDFNWEYPSSK
HQHKKLVNRDLKTQSGSEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 102

>PR1166(R1D2-R2D3-linker-R2D2(C162L)-R2D3-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP

CLGSISNLNVSLLARYPEKRFVPDGNRISWDSKKGFTIPSYMISYAGMVFCEAKINDES
YQSIMYIVVVVGYRIYDVVLSPSHGIELSVGEKLVLNCTARTELNVGIDFNWEYPSSK
HQHKKLVNRDLKTQSGSEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 103

>PR1167(R1D2-R2D3-R2D2(C162V)-R2D3(N274F/S283T)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKSPFIASVSDQHGVVYITENKNKTVVIPCLGSIS
NLNVSLVARYPEKRFVPDGNRISWDSKKGFTIPSYMISYAGMVFCEAKINDESYQSIM
YIVVVVGYRIYDVVLSPSHGIELSVGEKLVLNCTARTELNVGIDFNWEYPSSKHQHKK
LVFRDLKTQSGTEMKKFLSTLTIDGVTRSDQGLYTCAASSGLMTKKNSTFVRVHEKD
KTHTCPPCPAPEAAGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTI
SKAKGQPREPQVYTLPPSREEMTKNQVSLTCLVKGFYPSDIAVEWESNGQPENNYKT
TPPVLDSDGSFFLYSKLTVDKSRWQQGNVFSCSVMHEALHNHYTQKSLSLSPGK

SEQ ID NO: 104

>PR1168(R1D2-R2D3-R2D2(C162V/Y221S/R222K)-R2D3(N274F)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKSPFIASVSDQHGVVYITENKNKTVVIPCLGSIS
NLNVSLVARYPEKRFVPDGNRISWDSKKGFTIPSYMISYAGMVFCEAKINDESYQSIM
YIVVVVGSKIYDVVLSPSHGIELSVGEKLVLNCTARTELNVGIDFNWEYPSSKHQHKK
LVFRDLKTQSGSEMKKFLSTLTIDGVTRSDQGLYTCAASSGLMTKKNSTFVRVHEKD
KTHTCPPCPAPEAAGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTI
SKAKGQPREPQVYTLPPSREEMTKNQVSLTCLVKGFYPSDIAVEWESNGQPENNYKT
TPPVLDSDGSFFLYSKLTVDKSRWQQGNVFSCSVMHEALHNHYTQKSLSLSPGK

SEQ ID NO: 105

>PR1169(R1D2-R2D3-R2D2(C162V/Y221S/R222K)-R2D3(S283T)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD

QGLYTCAASSGLMTKKNSTFVRVHEKSPFIASVSDQHGVVYITENKNKTVVIPCLGSIS
NLNVSLVARYPEKRFVPDGNRISWDSKKGFTIPSYMISYAGMVFCEAKINDESYQSIM
YIVVVVGSKIYDVVLSPSHGIELSVGEKLVLNCTARTELNVGIDFNWEYPSSKHQHKK
LVNRDLKTQSGTEMKKFLSTLTIDGVTRSDQGLYTCAASSGLMTKKNSTFVRVHEKD
KTHTCPPCPAPEAAGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTI
SKAKGQPREPQVYTLPPSREEMTKNQVSLTCLVKGFYPSDIAVEWESNGQPENNYKT
TPPVLDSDGSFFLYSKLTVDKSRWQQGNVFSCSVMHEALHNHYTQKSLSLSPGK

SEQ ID NO: 106

>PR1170(R1D2-R2D3-R2D2(C162V/Y221S/R222K)-R2D3(N274F/S283T)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKSPFIASVSDQHGVVYITENKNKTVVIPCLGSIS
NLNVSLVARYPEKRFVPDGNRISWDSKKGFTIPSYMISYAGMVFCEAKINDESYQSIM
YIVVVVGSKIYDVVLSPSHGIELSVGEKLVLNCTARTELNVGIDFNWEYPSSKHQHKK
LVFRDLKTQSGTEMKKFLSTLTIDGVTRSDQGLYTCAASSGLMTKKNSTFVRVHEKD
KTHTCPPCPAPEAAGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTI
SKAKGQPREPQVYTLPPSREEMTKNQVSLTCLVKGFYPSDIAVEWESNGQPENNYKT
TPPVLDSDGSFFLYSKLTVDKSRWQQGNVFSCSVMHEALHNHYTQKSLSLSPGK

SEQ ID NO: 107

>PR1171(R1D2-R2D3-linker-R2D2(C162V)-R2D3(N274F/S283T)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLVARYPEKRFVPDGNRISWDSKKGFTIPSYMISYAGMVFCEAKINDES
YQSIMYIVVVVGYRIYDVVLSPSHGIELSVGEKLVLNCTARTELNVGIDFNWEYPSSK
HQHKKLVFRDLKTQSGTEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 108

>PR1172(R1D2-R2D3-linker-R2D2(C162V/Y221S/R222K)-R2D3(N274F)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV

LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLVARYPEKRFVPDGNRISWDSKKGFTIPSYMISYAGMVFCEAKINDES
YQSIMYIVVVVGSKIYDVVLSPSHGIELSVGEKLVLNCTARTELNVGIDFNWEYPSSK
HQHKKLVFRDLKTQSGSEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 109

>PR1173(R1D2-R2D3-linker-R2D2(C162V/Y221S/R222K)-R2D3(S283T)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLVARYPEKRFVPDGNRISWDSKKGFTIPSYMISYAGMVFCEAKINDES
YQSIMYIVVVVGSKIYDVVLSPSHGIELSVGEKLVLNCTARTELNVGIDFNWEYPSSK
HQHKKLVNRDLKTQSGTEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 110

>PR1174(R1D2-R2D3-linker-R2D2(C162V/Y221S/R222K)-R2D3(N274F/S283T)-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKGGGGSSPFIASVSDQHGVVYITENKNKTVVIP
CLGSISNLNVSLVARYPEKRFVPDGNRISWDSKKGFTIPSYMISYAGMVFCEAKINDES
YQSIMYIVVVVGSKIYDVVLSPSHGIELSVGEKLVLNCTARTELNVGIDFNWEYPSSK
HQHKKLVFRDLKTQSGTEMKKFLSTLTIDGVTRSDQGLYTCAASSGLMTKKNSTFVR
VHEKDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSP
GK

SEQ ID NO: 111

>PR1175(R2D2(C162V)-R2D3(N274F/S283T)-Fc)

SPFIASVSDQHGVVYITENKNKTVVIPCLGSISNLNVSLVARYPEKRFVPDGNRISWDS
KKGFTIPSYMISYAGMVFCEAKINDESYQSIMYIVVVVGYRIYDVVLSPSHGIELSVGE
KLVLNCTARTELNVGIDFNWEYPSSKHQHKKLVFRDLKTQSGTEMKKFLSTLTIDGVT
RSDQGLYTCAASSGLMTKKNSTFVRVHEKDKTHTCPPCPAPEAAGGPSVFLFPPKPKD
TLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSREEMTKNQ
VSLTCLVKGFYPSDIAVEWESNGQPENNYKTTPPVLDSDGSFFLYSKLTVDKSRWQQG
NVFSCSVMHEALHNHYTQKSLSLSPGK

SEQ ID NO: 112

>PR1176(R2D2(C162V/Y221S/R222K)-R2D3(N274F)-Fc)

SPFIASVSDQHGVVYITENKNKTVVIPCLGSISNLNVSLVARYPEKRFVPDGNRISWDS
KKGFTIPSYMISYAGMVFCEAKINDESYQSIMYIVVVVGSKIYDVVLSPSHGIELSVGE
KLVLNCTARTELNVGIDFNWEYPSSKHQHKKLVFRDLKTQSGSEMKKFLSTLTIDGVT
RSDQGLYTCAASSGLMTKKNSTFVRVHEKDKTHTCPPCPAPEAAGGPSVFLFPPKPKD
TLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSREEMTKNQ
VSLTCLVKGFYPSDIAVEWESNGQPENNYKTTPPVLDSDGSFFLYSKLTVDKSRWQQG
NVFSCSVMHEALHNHYTQKSLSLSPGK

SEQ ID NO: 113

>PR1177(R2D2(C162V/Y221S/R222K)-R2D3(S283T)-Fc)

SPFIASVSDQHGVVYITENKNKTVVIPCLGSISNLNVSLVARYPEKRFVPDGNRISWDS
KKGFTIPSYMISYAGMVFCEAKINDESYQSIMYIVVVVGSKIYDVVLSPSHGIELSVGE
KLVLNCTARTELNVGIDFNWEYPSSKHQHKKLVNRDLKTQSGTEMKKFLSTLTIDGV
TRSDQGLYTCAASSGLMTKKNSTFVRVHEKDKTHTCPPCPAPEAAGGPSVFLFPPKPK
DTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSREEMTKN
QVSLTCLVKGFYPSDIAVEWESNGQPENNYKTTPPVLDSDGSFFLYSKLTVDKSRWQQ
GNVFSCSVMHEALHNHYTQKSLSLSPGK

SEQ ID NO: 114

>PR1178(R2D2(C162V/Y221S/R222K)-R2D3(N274F/S283T)-Fc)

SPFIASVSDQHGVVYITENKNKTVVIPCLGSISNLNVSLVARYPEKRFVPDGNRISWDS
KKGFTIPSYMISYAGMVFCEAKINDESYQSIMYIVVVVGSKIYDVVLSPSHGIELSVGE
KLVLNCTARTELNVGIDFNWEYPSSKHQHKKLVFRDLKTQSGTEMKKFLSTLTIDGVT
RSDQGLYTCAASSGLMTKKNSTFVRVHEKDKTHTCPPCPAPEAAGGPSVFLFPPKPKD
TLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSREEMTKNQ
VSLTCLVKGFYPSDIAVEWESNGQPENNYKTTPPVLDSDGSFFLYSKLTVDKSRWQQG
NVFSCSVMHEALHNHYTQKSLSLSPGK

SEQ ID NO: 115

>PR1179(R1D2-R2D3-R2D2(C162V/Y221S/R222K)-R2D3-Fc)

SDTGRPFVEMYSEIPEIIHMTEGRELVIPCRVTSPNITVTLKKFPLDTLIPDGKRIIWDSR
KGFIISNATYKEIGLLTCEATVNGHLYKTNYLTHRQTNTIIDVVLSPSHGIELSVGEKLV
LNCTARTELNVGIDFNWEYPSSKHQHKKLVNRDLKTQSGSEMKKFLSTLTIDGVTRSD
QGLYTCAASSGLMTKKNSTFVRVHEKSPFIASVSDQHGVVYITENKNKTVVIPCLGSIS
NLNVSLVARYPEKRFVPDGNRISWDSKKGFTIPSYMISYAGMVFCEAKINDESYQSIM
YIVVVVGSKIYDVVLSPSHGIELSVGEKLVLNCTARTELNVGIDFNWEYPSSKHQHKK
LVNRDLKTQSGSEMKKFLSTLTIDGVTRSDQGLYTCAASSGLMTKKNSTFVRVHEKD
KTHTCPPCPAPEAAGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTI
SKAKGQPREPQVYTLPPSREEMTKNQVSLTCLVKGFYPSDIAVEWESNGQPENNYKT
TPPVLDSDGSFFLYSKLTVDKSRWQQGNVFSCSVMHEALHNHYTQKSLSLSPGK

SEQ ID NO: 116

> PR1186 (heavy chain H: R1D2-R2D3-R2D2 (C162V/Y221S/R222K)-R2D3 (N274F/S283T)-Fc-linker-VH-CH1; light chain L: VL-CL)

Heavy chain:

SPFIASVSDQHGVVYITENKNKTVVIPCLGSISNLNVSLVARYPEKRFVPDGNRISWDS
KKGFTIPSYMISYAGMVFCEAKINDESYQSIMYIVVVVGSKIYDVVLSPSHGIELSVGE
KLVLNCTARTELNVGIDFNWEYPSSKHQHKKLVFRDLKTQSGTEMKKFLSTLTIDGVT
RSDQGLYTCAASSGLMTKKNSTFVRVHEKDKTHTCPPCPAPEAAGGPSVFLFPPKPKD
TLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSREEMTKNQ
VSLTCLVKGFYPSDIAVEWESNGQPENNYKTTPPVLDSDGSFFLYSKLTVDKSRWQQG
NVFSCSVMHEALHNHYTQKSLSLSPGGGGGSGGGGSGGGGSGGGGSEVQLVESGGG
LVQPGGSLRLSCAASGYTFTNYGMNWVRQAPGKGLEWVGWINTYTGEPTYAADFK
RRFTFSLDTSKSTAYLQMNSLRAEDTAVYYCAKYPHYYGSSHWYFDVWGQGTLVTV
SSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSC

SEQ ID NO: 117

Light chain:

DIQMTQSPSSLSASVGDRVTITCSASQDISNYLNWYQQKPGKAPKVLIYFTSSLHSGVP
SRFSGSGSGTDFTLTISSLQPEDFATYYCQQYSTVPWTFGQGTKVEIKRTVAAPSVFIFP
PSDEQLKSGTASVVCLLNNFYPREAKVQWKVDNALQSGNSQESVTEQDSKDSTYSLS
STLTLSKADYEKHKVYACEVTHQGLSSPVTKSFNRGEC

SEQ ID NO: 118

The CMV enhancer sequence:

CGTTACATAACTTACGGTAAATGGCCCGCCTGGCTGACCGCCCAACGACCCCCGCC
CATTGACGTCAATAATGACGTATGTTCCCATAGTAACGCCAATAGGGACTTTCCATT

GACGTCAATGGGTGGAGTATTTACGGTAAACTGCCCACTTGGCAGTACATCAAGTG
TATCATATGCCAAGTACGCCCCCTATTGACGTCAATGACGGTAAATGGCCCGCCTGG
CATTATGCCCAGTACATGACCTTATGGGACTTTCCTACTTGGCAGTACATCTACGTAT
TAGTCATCGCTATTACCATG

SEQ ID NO: 124

CMV promoter sequence:

TGCTGATGCGGTTTTGGCAGTACACCAATGGGCGTGGATAGCGGTTTGACTCACGG
GGATTTCCAAGTCTCCACCCCATTGACGTCAATGGGAGTTTGTTTTGGCACCAAAA
TCAACGGGACTTTCCAAAATGTCGTAATAACCCCGCCCCGTTGACGCAAATGGGCG
GTAGGCGTGTACGGTGGGAGGTCTATATAAGCAGAGCTCGTTTAGTGAACCGTCAG
ATCGCCTGGAGAGGCCATCCACGCTGTTTTGACCTCCATAGTGGACACCGGGACC
GATCCAGCCTCCGCGTCTCAGGGGAAGCTT

SEQ ID NO: 125

5' UTR sequence

CTTGTTCTTTTTGCAGAAGCTCAGAATAAACGCTCAACTTTGG SEQ ID NO: 126
Kozak sequence: gccacc;
Codon-optimized sequence 1 of PR1145

ATGCCTCTGCTGCTGCTGTTGCCTTTGTTGTGGGCTGGCGCCCTGGCTTCTGATACC
GGCAGACCTTTCGTGGAAATGTACAGCGAGATCCCCGAGATCATCCACATGACCG
AGGGCAGAGAGCTGGTCATCCCCTGCAGAGTGACCTCTCCTAACATCACCGTGAC
TCTGAAGAAGTTCCCTCTGGACACACTGATCCCCGACGGCAAGAGAATCATCTGG
GACTCCCGGAAGGGCTTCATCATCTCCAACGCCACCTACAAAGAGATCGGCCTGC
TGACCTGCGAGGCCACCGTTAATGGCCACCTGTACAAGACCAACTATCTGACCCAC
AGACAGACCAACACCATCATCGACGTGGTGCTGAGCCCCTCTCATGGCATCGAGC
TGTCCGTGGGAGAAAAGCTGGTGCTGAACTGCACCGCCAGAACCGAGCTGAACG
TGGGCATCGACTTCAACTGGGAGTACCCCTCCAGCAAGCACCAGCACAAGAAGCT
GGTCAACCGGGACCTGAAAACCCAGTCCGGCTCCGAGATGAAGAAATTCCTGAGC
ACCCTGACCATCGACGGCGTGACCAGATCTGACCAGGGCCTGTATACCTGCGCCGC
TTCTTCTGGCCTGATGACCAAGAAAAACTCCACCTTCGTGCGGGTGCACGAGAAA
GGTGGCGGAGGCAGCTCTCCTTTTATCGCCTCTGTGTCTGATCAGCACGGCGTGGT
GTACATCACCGAGAACAAGAACAAGACCGTCGTGATCCCCTGCCTGGGCTCCATC
TCTAACCTGAACGTGTCCCTGGTGGCTAGATACCCCGAGAAGAGATTCGTGCCTGA
CGGCAACCGGATCTCCTGGGACAGCAAGAAGGGCTTTACAATCCCCTCCTACATGA
TCTCCTACGCCGGCATGGTGTTCTGCGAGGCTAAGATCAACGACGAGTCCTACCAG
TCCATCATGTACATCGTGGTGGTCGTGGGCTACAGAATCTACGATGTGGTGCTGTCC
CCTAGCCACGGAATCGAACTGAGCGTGGGCGAGAAACTGGTCCTCAACTGTACCG
CTCGGACAGAACTGAATGTGGGGATTGATTTCAATTGGGAATACCCTAGCTCCAAA
CATCAGCATAAGAAACTCGTGAACCGCGATCTCAAGACCCAGAGCGGAACAGAAA

TGAAGAAGTTTCTGTCCACACTCACCATTGACGGGGTCACCAGAAGCGATCAGGG
ACTCTATACCTGTGCTGCCTCCAGCGGACTGATGACAAAAAAGAACAGCACATTTG
TCCGCGTCCACGAGAAGGACAAAACTCACACATGCCCACCGTGCCCGGCGCCTGA
AGCCGCTGGGGGACCGTCAGTCTTCCTCTTCCCCCCAAAACCCAAGGACACCCTC
ATGATCTCCCGGACCCCTGAGGTCACATGCGTGGTGGTGGACGTGAGCCACGAAG
ACCCTGAGGTCAAGTTCAACTGGTACGTGGACGGCGTGGAGGTGCATAATGCCAA
GACAAAGCCGCGGGAGGAGCAGTACAACAGCACGTACCGGGTGGTCAGCGTCCT
CACCGTCCTGCACCAGGACTGGCTGAATGGCAAGGAGTACAAGTGCAAGGTCTCC
AACAAAGCCCTCCCAGCCCCCATCGAGAAAACCATCTCCAAAGCCAAAGGGCAG
CCCAGGGAACCACAGGTGTACACCTGCCCCCATCCCGGGAGGAGATGACCAAGA
ACCAGGTCAGCCTGACCTGCCTGGTCAAAGGCTTCTATCCCAGCGACATCGCCGT
GGAGTGGGAGAGCAATGGGCAGCCGGAGAACAACTACAAGACCACGCCTCCCGT
GCTGGACTCCGACGGCTCCTTCTTCCTCTACAGCAAGCTCACCGTGGACAAGAGC
AGGTGGCAGCAGGGGAACGTCTTCTCATGCTCCGTGATGCATGAGGCTCTGCACA
ACCACTACACGCAGAAGAGCCTCTCCCTGTCTCCGGGTAAATAA

SEQ ID NO: 128

Codon-optimized sequence 2 of PR1145

ATGCCTCTGCTGCTGCTGCTGCCTCTGCTGTGGGCCGGCGCCCTGGCCTCCGACAC
CGGCCGGCCTTTTGTTGAAATGTACTCCGAGATCCCCGAAATCATCCACATGACAG
AAGGCAGAGAGCTGGTGATCCCTTGTAGAGTGACCAGCCCAAACATCACCGTGAC
TCTGAAGAAGTTCCCCCTGGATACACTGATCCCTGATGGAAAGAGAATCATTTGGG
ATAGCAGAAAGGGCTTTATCATCAGCAACGCCACATACAAAGAGATCGGCCTGCTC
ACCTGCGAGGCCACCGTGAACGGCCACCTGTATAAGACAAACTACCTGACACATA
GACAGACCAACACCATCATTGACGTGGTGCTGTCCCCAAGCCACGGAATCGAGCT
GTCTGTGGGCGAGAAGCTGGTGCTGAACTGCACCGCCAGAACAGAGCTGAACGT
GGGCATTGACTTCAATTGGGAGTACCCTAGCTCCAAACACCAACACAAGAAGCTG
GTGAATAGAGATCTGAAAACCCAGTCTGGCAGCGAAATGAAGAAATTTCTGAGCA
CACTGACCATTGACGGCGTGACCCGCAGCGACCAGGGCCTGTACACCTGTGCCGC
CTCTTCTGGCCTTATGACCAAAAAAATTCTACCTTCGTGCGGGTGCACGAGAAGG
GCGGAGGCGGCTCTAGCCCCTTCATCGCCAGCGTCAGCGATCAGCACGGCGTGGT
CTACATCACCGAAAACAAGAACAAGACCGTGGTCATTCCTTGCCTGGGCAGCATC
AGTAATCTGAACGTGAGCCTGGTGGCCAGATACCCTGAGAAGCGGTTCGTGCCCG
ACGGCAACAGGATCAGCTGGGACAGCAAGAAGGGCTTTACGATCCCCTCTTATATG
ATCTCCTACGCCGGAATGGTGTTCTGCGAGGCTAAGATCAACGACGAGAGCTACC
AGAGCATCATGTACATCGTGGTCGTGGTCGGCTACCGGATCTACGACGTGGTGCTG
AGCCCTTCTCACGGCATCGAGCTGTCCGTCGGAGAGAAGCTGGTGTTGAATTGCA
CCGCCAGAACCGAGCTGAACGTGGGCATCGACTTTAACTGGGAATACCCCAGCTC
TAAACACCAGCACAAGAAGCTGGTGAACCGGGACCTGAAGACCCAATCTGGTAC
AGAGATGAAGAAGTTCCTAAGCACACTGACCATCGACGGCGTCACAAGAAGCGAT

CAGGGACTGTACACCTGCGCCGCTAGCAGCGGACTGATGACCAAGAAAAACAGC
ACCTTCGTGCGGGTGCACGAGAAAGACAAGACACACACCTGCCCTCCATGTCCTG
CCCCTGAAGCTGCTGGCGGACCTTCTGTGTTCCTCTTCCCACCTAAGCCTAAGGAC
ACCCTGATGATCAGCCGGACACCTGAAGTGACATGCGTGGTGGTGGATGTGTCTC
ACGAAGATCCCGAGGTGAAATTCAACTGGTACGTGGACGGCGTTGAAGTGCATAA
TGCCAAGACCAAGCCCAGAGAGGAACAGTACAACAGCACCTACAGAGTGGTGTC
CGTACTGACCGTGCTGCACCAGGACTGGCTGAACGGCAAGGAATACAAGTGCAAG
GTGTCCAACAAAGCCCTGCCTGCCCCTATCGAGAAGACCATCTCTAAGGCCAAGG
GCCAGCCTAGAGAACCCCAAGTGTACACACTGCCTCCTTCCAGAGAGGAAATGAC
CAAGAACCAGGTGTCATTGACATGTCTGGTGAAGGGGTTCTACCCAAGCGACATC
GCCGTGGAATGGGAGAGCAACGGACAGCCCGAGAACAACTACAAGACCACACCT
CCAGTGCTCGATAGCGACGGCAGCTTCTTCCTGTATAGCAAGCTGACCGTGGACAA
GTCCAGATGGCAGCAGGGCAATGTGTTCAGCTGCAGCGTGATGCACGAGGCTCTG
CACAACCACTACACCCAGAAGAGCCTGTCCCTGAGCCCCGGCAAGTGA

SEQ ID NO: 129

Codon-optimized sequence 3 of PR1145

ATGCCCCTGCTCCTGCTCCTGCCCCTGCTGTGGGCCGGCGCCCTGGCTAGCGACAC
CGGCAGACCCTTCGTGGAGATGTACAGCGAGATCCCCGAGATCATCCACATGACC
GAGGGCAGAGAGCTCGTGATCCCTTGCAGAGTGACAAGCCCCAACATCACCGTGA
CCCTGAAAAAGTTCCCCCTGGACACCCTGATCCCCGACGGCAAGAGAATCATCTG
GGACAGCAGAAAGGGCTTCATCATCAGCAACGCCACCTACAAGGAGATCGGCCTG
CTGACCTGCGAGGCCACCGTGAACGGCCACCTGTACAAGACCAACTACCTGACCC
ACAGACAGACCAACACCATCATCGACGTCGTGCTGAGCCCTAGCCACGGCATCGA
GCTGAGCGTGGGCGAAAAACTGGTCCTCAACTGTACCGCTCGGACCGAACTGAAC
GTGGGGATCGATTTCAACTGGGAGTATCCTAGCTCCAAGCATCAGCATAAGAAGCT
GGTCAACAGAGATCTCAAGACACAGAGCGGCTCCGAAATGAAAAAATTCCTCTCC
ACCCTGACAATTGACGGCGTCACACGGAGCGACCAAGGCCTGTATACCTGCGCTG
CTAGCAGCGGGCTGATGACCAAGAAGAACTCCACCTTTGTCAGAGTGCACGAAAA
GGGCGGGGGCGGCAGCAGCCCCTTCATCGCTAGCGTGAGCGATCAGCACGGCGTG
GTGTACATCACCGAGAACAAGAACAAGACCGTGGTGATCCCCTGCCTGGGCAGCA
TCAGCAACCTGAACGTGAGCCTGGTGGCTAGATACCCCGAGAAGAGATTCGTGCC
CGACGGCAACAGAATCAGCTGGGACAGCAAGAAGGGCTTCACCATCCCTAGCTAC
ATGATCAGCTACGCCGGCATGGTGTTCTGCGAGGCCAAGATCAACGACGAGAGCT
ATCAGAGCATCATGTACATCGTGGTCGTGGTGGGCTACAGAATCTACGACGTGGTG
CTGTCCCCTAGCCACGGCATTGAACTGTCCGTGGGGGGAGAAGCTGGTGCTGAACT
GTACCGCCCGGACAGAGCTGAACGTGGGCATCGACTTTAACTGGGAGTACCCTAG
CAGCAAACATCAGCACAAGAAGCTGGTGAACCGGGACCTGAAGACACAGAGCGG
CACAGAAATGAAGAAATTCCTGAGCACACTGACAATTGATGGCGTGACAAGAAGC
GACCAAGGCCTGTACCTGCCGCTAGCAGCGGCCTCATGACAAAGAAGAACA

GCACATTTGTGCGGGTGCACGAGAAAGACAAAACACATACCTGTCCTCCCTGTCC
CGCCCCTGAAGCCGCTGGGGGGCCTAGCGTGTTCCTGTTCCCCCCCAAGCCCAAG
GACACACTGATGATCAGCAGAACCCCCGAGGTGACCTGCGTGGTCGTGGACGTGA
GCCACGAGGACCCCGAGGTGAAGTTCAACTGGTACGTGGACGGCGTGGAGGTGC
ACAACGCCAAGACCAAGCCTAGAGAGGAGCAGTACAACAGCACCTACAGAGTGG
TGAGCGTGCTGACCGTGCTGCACCAAGACTGGCTGAACGGCAAGGAGTACAAGT
GCAAGGTGAGCAACAAGGCCCTGCCCGCCCCCATCGAGAAGACCATCAGCAAGG
CCAAGGGGCAGCCTAGAGAGCCCCAAGTGTACACCCTGCCCCCTAGCAGAGAGG
AGATGACCAAAAACCAAGTGAGCCTGACCTGCCTGGTGAAGGGCTTCTACCCTAG
CGACATCGCCGTGGAGTGGGAGAGCAACGGGCAGCCCGAGAACAACTACAAGAC
CACCCCCCCCGTGCTGGACAGCGACGGCAGCTTCTTCCTGTACAGCAAGCTGACC
GTGGACAAGAGCAGATGGCAGCAAGGCAACGTGTTCAGCTGCAGCGTGATGCAC
GAGGCCCTGCACAACCACTACACACAGAAGAGCCTGAGCCTGAGCCCCGGCAAG
TGA

SEQ ID NO: 130

Codon-optimized sequence 1 of PR1174

ATGCCTCTGCTGCTGCTGTTGCCTTTGTTGTGGGCTGGCGCCCTGGCTTCTGATACC
GGCAGACCTTTCGTGGAAATGTACAGCGAGATCCCCGAGATCATCCACATGACCG
AGGGCAGAGAGCTGGTCATCCCCTGCAGAGTGACCTCTCCTAACATCACCGTGAC
TCTGAAGAAGTTCCCTCTGGACACACTGATCCCCGACGGCAAGAGAATCATCTGG
GACTCCCGGAAGGGCTTCATCATCTCCAACGCCACCTACAAAGAGATCGGCCTGC
TGACCTGCGAGGCCACCGTTAATGGCCACCTGTACAAGACCAACTATCTGACCCAC
AGACAGACCAACACCATCATCGACGTGGTGCTGAGCCCCTCTCATGGCATCGAGC
TGTCCGTGGGAGAAAAGCTGGTGCTGAACTGCACCGCCAGAACCGAGCTGAACG
TGGGCATCGACTTCAACTGGGAGTACCCCTCCAGCAAGCACCAGCACAAGAAGCT
GGTCAACCGGGACCTGAAAACCCAGTCCGGCTCCGAGATGAAGAAATTCCTGAGC
ACCCTGACCATCGACGGCGTGACCAGATCTGACCAGGGCCTGTATACCTGCGCCGC
TTCTTCTGGCCTGATGACCAAGAAAAACTCCACCTTCGTGCGGGTGCACGAGAAA
GGTGGCGGAGGCAGCTCTCCTTTTATCGCCTCTGTGTCTGATCAGCACGGCGTGGT
GTACATCACCGAGAACAAGAACAAGACCGTCGTGATCCCCTGCCTGGGCTCCATC
TCTAACCTGAACGTGTCCCTGGTGGCTAGATACCCCGAGAAGAGATTCGTGCCTGA
CGGCAACCGGATCTCCTGGGACAGCAAGAAGGGCTTTACAATCCCCTCCTACATGA
TCTCCTACGCCGGCATGGTGTTCTGCGAGGCTAAGATCAACGACGAGTCCTACCAG
TCCATCATGTACATCGTGGTGGTCGTGGGCTCCAAGATCTACGATGTGGTGCTGTCC
CCTAGCCACGGAATCGAACTGAGCGTGGGCGAGAAACTGGTCCTCAACTGTACCG
CTCGGACAGAACTGAATGTGGGGATTGATTTCAATTGGGAATACCCTAGCTCCAAA
CATCAGCATAAGAAACTCGTGTTCCGCGATCTCAAGACCCAGAGCGGAACAGAAA
TGAAGAAGTTTCTGTCCACACTCACCATTGACGGGGTCACCAGAAGCGATCAGGG
ACTCTATACCTGTGCTGCCTCCAGCGGACTGATGACAAAAAAGAACAGCACATTTG

TCCGCGTCCACGAGAAGGACAAAACTCACACATGCCCACCGTGCCCGGCGCCTGA
AGCCGCTGGGGGACCGTCAGTCTTCCTCTTCCCCCCAAAACCCAAGGACACCCTC
ATGATCTCCCGGACCCCTGAGGTCACATGCGTGGTGGTGGACGTGAGCCACGAAG
ACCCTGAGGTCAAGTTCAACTGGTACGTGGACGGCGTGGAGGTGCATAATGCCAA
GACAAAGCCGCGGGAGGAGCAGTACAACAGCACGTACCGGGTGGTCAGCGTCCT
CACCGTCCTGCACCAGGACTGGCTGAATGGCAAGGAGTACAAGTGCAAGGTCTCC
AACAAAGCCCTCCCAGCCCCCATCGAGAAAACCATCTCCAAAGCCAAAGGGCAG
CCCAGGGAACCACAGGTGTACACCCTGCCCCCATCCCGGGAGGAGATGACCAAGA
ACCAGGTCAGCCTGACCTGCCTGGTCAAAGGCTTCTATCCCAGCGACATCGCCGT
GGAGTGGGAGAGCAATGGGCAGCCGGAGAACAACTACAAGACCACGCCTCCCGT
GCTGGACTCCGACGGCTCCTTCTTCCTCTACAGCAAGCTCACCGTGGACAAGAGC
AGGTGGCAGCAGGGGAACGTCTTCTCATGCTCCGTGATGCATGAGGCTCTGCACA
ACCACTACACGCAGAAGAGCCTCTCCCTGTCTCCGGGTAAATAA

SEQ ID NO: 131

Codon-optimized sequence 2 of PR1174

ATGCCTCTGCTGCTGCTCCTGCCTCTGCTGTGGGCCGGCGCCCTGGCCAGCGATAC
CGGCAGACCATTCGTGGAAATGTACAGCGAAATCCCCGAGATCATCCACATGACCG
AGGGCAGAGAACTGGTGATCCCCTGCAGAGTGACCAGCCCAAACATCACCGTGAC
CCTCAAGAAGTTTCCCCTGGACACCCTGATCCCTGATGGAAAAGAATCATCTGGG
ACAGCAGAAAGGGCTTTATCATTTCTAATGCCACATACAAAGAGATAGGACTGTTG
ACCTGTGAAGCTACAGTTAACGGCCACCTGTACAAGACCAACTACCTGACCCACC
GGCAGACAAACACCATCATCGACGTGGTGCTGAGCCCTAGCCACGGAATCGAGCT
GTCCGTGGGAGAAAAGCTGGTGCTGAATTGCACCGCCAGAACCGAGCTGAACGT
AGGCATCGACTTCAACTGGGAGTATCCTTCAAGCAAACATCAGCACAAAAAACTG
GTGAACAGAGACCTGAAGACCCAGAGTGGCAGCGAGATGAAAAAGTTCCTGAGC
ACACTGACAATCGACGGAGTGACTCGGAGCGATCAGGGCCTGTATACATGTGCTG
CTTCTAGCGGCCTGATGACAAAGAAGAACAGCACATTCGTGCGGGTGCACGAGAA
GGGGGGCGGCGGATCTTCCCCATTCATCGCCAGCGTGTCCGACCAGCATGGCGTG
GTTTACATCACAGAAAACAAGAACAAGACCGTGGTGATCCCCTGCCTGGGCTCCA
TCAGCAACCTGAATGTGTCGCTGGTGGCCAGATACCCCGAAAAGCGGTTCGTCCC
CGACGGCAACAGAATCAGCTGGGACAGCAAAAAGGGCTTCACCATCCCAAGCTAT
ATGATCAGCTACGCCGGAATGGTGTTCTGCGAGGCCAAAATCAACGACGAGTCCT
ACCAGAGCATCATGTACATCGTGGTGGTTGTCGGCAGCAAGATCTACGACGTCGTG
CTGAGCCCATCTCACGGCATTGAGCTGTCTGTGGGCGAGAAGCTGGTGCTGAATT
GCACCGCCCGGACAGAACTCAATGTGGGCATCGATTTCAACTGGGAGTACCCTAG
CTCTAAGCACCAGCACAAGAAGCTGGTCTTTAGAGATCTGAAAACCCAATCTGGC
ACCGAGATGAAAAAGTTCCTGAGCACCCTGACCATCGACGGCGTGACCAGAAGC
GACCAGGGCCTGTACACCTGCGCCGCCTCTAGCGGCCTGATGACCAAGAAAAACA
GCACCTTCGTGCGCGTTCACGAGAAGATAAGACACACACCTGTCCTCCTTGTCCT

GCCCCTGAGGCCGCTGGCGGACCTTCTGTGTTCCTGTTCCCTCCAAAGCCCAAGG
ACACCCTGATGATCAGCAGAACACCCGAAGTGACCTGCGTGGTGGTGGACGTCTC
TCACGAGGACCCCGAGGTGAAATTTAACTGGTACGTGGACGGCGTCGAGGTGCAT
AATGCCAAGACAAAGCCTCGGGAAGAGCAGTACAACAGCACATACAGAGTGGTC
AGCGTGCTGACAGTGCTGCACCAGGATTGGCTGAACGGCAAGGAATACAAGTGCA
AGGTGAGCAACAAGGCCCTGCCTGCTCCTATCGAGAAGACCATTAGCAAGGCCAA
GGGCCAGCCTAGGGAGCCTCAGGTGTACACCTTACCACCTAGCAGAGAGGAAATG
ACGAAGAACCAGGTGTCCCTGACATGCCTGGTGAAGGGTTTCTACCCTTCTGATAT
CGCCGTGGAATGGGAATCCAACGGCCAACCTGAGAACAACTACAAGACCACCCCT
CCCGTGCTCGATAGCGACGGCTCCTTCTTCCTGTACAGCAAGCTGACCGTGGATAA
GAGCCGGTGGCAGCAGGGCAATGTGTTTAGCTGCTCTGTGATGCACGAAGCCCTG
CACAACCACTACACCCAAAAGAGCCTGAGCCTGAGCCCCGGCAAGTGA

SEQ ID NO: 132

Codon-optimized sequence 3 of PR1174

ATGCCCCTGCTCCTGCTCCTGCCCCTGCTGTGGGCCGGCGCCCTGGCTAGCGACAC
CGGCAGACCCTTCGTGGAGATGTACAGCGAGATCCCCGAGATCATCCACATGACC
GAGGGCAGAGAGCTCGTGATCCCTTGCAGAGTGACAAGCCCCAACATCACCGTGA
CCCTGAAAAAGTTCCCCCTGGACACCCTGATCCCCGACGGCAAGAGAATCATCTG
GGACAGCAGAAAGGGCTTCATCATCAGCAACGCCACCTACAAGGAGATCGGCCTG
CTGACCTGCGAGGCCACCGTGAACGGCCACCTGTACAAGACCAACTACCTGACCC
ACAGACAGACCAACACCATCATCGACGTCGTGCTGAGCCCTAGCCACGGCATCGA
GCTGTCCGTGGGCGAAAAGCTCGTCCTGAACTGCACCGCTAGAACCGAACTCAAC
GTGGGGATTGATTTCAACTGGGAGTATCCTAGCTCCAAGCACCAACACAAGAAGC
TGGTGAACCGGGATCTGAAGACACAGAGCGGGAGCGAGATGAAGAAGTTCCTGA
GCACCCTGACAATCGACGGGGTGACAAGATCCGACCAAGGCCTGTACACCTGCGC
TGCTAGCAGCGGCCTGATGACAAAGAAAAATAGCACATTTGTGAGAGTGCACGAG
AAGGGCGGGGGCGGCAGCAGCCCCTTCATCGCTAGCGTGAGCGATCAGCACGGC
GTGGTGTACATCACCGAGAACAAGAACAAGACCGTGGTGATCCCCTGCCTGGGCA
GCATCAGCAACCTGAACGTGAGCCTGGTGGCTAGATACCCCGAGAAGAGATTCGT
GCCCGACGGCAACAGAATCAGCTGGGACAGCAAGAAGGGCTTCACCATCCCTAGC
TACATGATCAGCTACGCCGGCATGGTGTTCTGCGAGGCCAAGATCAACGACGAGA
GCTATCAGAGCATCATGTACATCGTGGTCGTGGTGGGCAGCAAGATCTACGACGTG
GTGCTGAGCCCCTCCCACGGCATTGAGCTCAGCGTGGGCGAGAAGCTGGTCCTCA
ACTGCACAGCCCGGACCGAGCTGAACGTGGGCATCGATTTTAACTGGGAGTACCC
TAGCAGCAAGCATCAGCATAAAAAGCTGGTGTTCCGGGACCTGAAGACCCAAAGC
GGCACCGAAATGAAGAAGTTTCTCTCCACCCTGACCATCGACGGCGTGACAAGAA
GCGACCAAGGCCTGTACACATGCGCCGCTAGCTCCGGCCTCATGACAAAGAAGAA
TTCCACCTTTGTCAGAGTCCATGAAAAAGATAAGACACACACCTGTCCCCCTTGTC
CCGCCCCCGAAGCCGCTGGGGGCCCTAGCGTGTTCCTGTTCCCCCCCAAGCCCAA

GGATACCCTGATGATCAGCAGAACCCCCGAGGTGACCTGCGTGGTCGTGGACGTG
AGCCACGAGGACCCCGAGGTGAAGTTCAACTGGTACGTGGACGGCGTGGAGGTG
CACAACGCCAAGACCAAGCCTAGAGAGGAGCAGTACAACAGCACCTACAGAGTG
GTGAGCGTGCTGACCGTGCTGCACCAAGACTGGCTGAACGGCAAGGAGTACAAG
TGCAAGGTGAGCAACAAGGCCCTGCCCGCCCCCATCGAGAAGACCATCAGCAAG
GCCAAGGGGCAGCCTAGAGAGCCCCAAGTGTACACCTGCCCCCTAGCAGAGAG
GAGATGACCAAGAACCAAGTGAGCCTGACCTGCCTGGTGAAGGGCTTCTACCCTA
GCGACATCGCCGTGGAGTGGGAGAGCAACGGGCAGCCCGAGAACAACTACAAGA
CCACCCCCCCCGTGCTGGACAGCGACGGCAGCTTCTTCCTGTACAGCAAGCTGAC
CGTGGACAAGAGCAGATGGCAGCAAGGCAACGTGTTCAGCTGCAGCGTGATGCA
CGAGGCCCTGCACAACCACTACACACAGAAGAGCCTGAGCCTGAGCCCCGGCAA
GTGA

SEQ ID NO: 133

Codon-optimized sequence 1 of PR1163

ATGCCTCTGCTGCTGCTGTTGCCTTTGTTGTGGGCTGGCGCTCTGGCCTCTCCTTTT
ATCGCCTCTGTGTCTGATCAGCACGGCGTGGTGTACATCACCGAGAACAAGAACA
AGACCGTCGTGATCCCCTGCCTGGGCTCCATCTCTAACCTGAACGTGTCCCTGGTG
GCTAGATACCCCGAGAAGAGATTCGTGCCTGACGGCAACCGGATCTCCTGGGACA
GCAAGAAGGGCTTTACAATCCCCTCCTACATGATCTCCTACGCCGGCATGGTGTTC
TGCGAGGCTAAGATCAACGACGAGTCCTACCAGTCCATCATGTACATCGTGGTGGT
CGTGGGCTACAGAATCTACGATGTGGTGCTGTCCCCTAGCCACGGAATCGAACTGA
GCGTGGGCGAGAAACTGGTCCTCAACTGTACCGCTCGGACAGAACTGAATGTGGG
GATTGATTTCAATTGGGAATACCCTAGCTCCAAACATCAGCATAAGAAACTCGTGA
ACCGCGATCTCAAGACCCAGAGCGGAAGCGAAATGAAGAAGTTTCTGTCCACACT
CACCATTGACGGGGTCACCAGAAGCGATCAGGGACTCTATACCTGTGCTGCCTCCA
GCGGACTGATGACAAAAAAGAACAGCACATTTGTCCGCGTCCACGAGAAGGACA
AAACTCACACATGCCCACCGTGCCCGGCGCCTGAAGCCGCTGGGGGACCGTCAGT
CTTCCTCTTCCCCCCAAAACCCAAGGACACCCTCATGATCTCCCGGACCCCTGAGG
TCACATGCGTGGTGGTGGACGTGAGCCACGAAGACCCTGAGGTCAAGTTCAACTG
GTACGTGGACGGCGTGGAGGTGCATAATGCCAAGACAAAGCCGCGGGAGGAGCA
GTACAACAGCACGTACCGGGTGGTCAGCGTCCTCACCGTCCTGCACCAGGACTGG
CTGAATGGCAAGGAGTACAAGTGCAAGGTCTCCAACAAAGCCCTCCCAGCCCCCA
TCGAGAAAACCATCTCCAAAGCCAAAGGGCAGCCCAGGGAACCACAGGTGTACA
CCCTGCCCCCATCCCGGGAGGAGATGACCAAGAACCAGGTCAGCCTGACCTGCCT
GGTCAAAGGCTTCTATCCCAGCGACATCGCCGTGGAGTGGGAGAGCAATGGGCAG
CCGGAGAACAACTACAAGACCACGCCTCCCGTGCTGGACTCCGACGGCTCCTTCT
TCCTCTACAGCAAGCTCACCGTGGACAAGAGCAGGTGGCAGCAGGGGAACGTCT
TCTCATGCTCCGTGATGCATGAGGCTCTGCACAACCACTACACGCAGAAGAGCCTC
TCCCTGTCTCCGGGTGGCGGAGGCGGCTCCGGTGGAGGCGGTTCAGGCGGAGGT

GGCTCTGGCGGTGGCGGATCGGAGGTGCAGCTGGTGGAGAGCGGGGGGGGACTG
GTGCAGCCAGGAGGAAGCCTGAGACTGAGCTGTGCCGCCAGCGGGTACACATTTA
CCAACTACGGAATGAACTGGGTGAGGCAGGCTCCTGGCAAGGGGCTGGAGTGGG
TGGGGTGGATTAACACCTACACCGGAGAGCCCACATACGCTGCTGATTTTAAGCGG
AGATTCACCTTCAGCCTGGACACCAGCAAGAGCACCGCTTACCTGCAGATGAACA
GCCTGAGAGCCGAGGACACAGCCGTGTACTACTGCGCCAAATACCCCCATTACTAC
GGCAGCAGCCACTGGTACTTCGACGTGTGGGGCCAGGGAACACTGGTGACCGTG
AGCAGCGCTAGCACCAAGGGCCCATCGGTCTTCCCCCTGGCACCCTCCTCCAAGA
GCACCTCTGGGGGCACAGCGGCCCTGGGCTGCCTGGTCAAGGACTACTTCCCCGA
ACCGGTGACGGTGTCGTGGAACTCAGGCGCCCTGACCAGCGGCGTGCACACCTTC
CCGGCTGTCCTACAGTCCTCAGGACTCTACTCCCTCAGCAGCGTGGTGACCGTGCC
CTCCAGCAGCTTGGGCACCCAGACCTACATCTGCAACGTGAATCACAAGCCCAGC
AACACCAAGGTGGACAAGAAAGTTGAGCCCAAATCTTGTGAGGGCAGAGGAAGT
CTTCTAACATGCGGTGACGTGGAGGAGAATCCCGGCCCTATGCCTCTGCTGCTGCT
GTTGCCTTTGTTGTGGGCTGGCGCTCTGGCCGACATTCAGATGACCCAGAGCCCCA
GCAGCCTGAGCGCCAGCGTGGGAGACAGAGTGACCATCACCTGCAGCGCCAGCC
AGGACATCAGCAACTACCTGAACTGGTATCAGCAGAAACCCGGCAAAGCCCCCAA
AGTGCTGATCTACTTCACAAGTTCACTGCACAGCGGAGTGCCCAGCAGATTTTCAG
GAAGCGGCAGCGGCACCGACTTCACCCTGACCATCAGCAGCCTGCAGCCCGAGGA
TTTCGCCACCTACTACTGCCAGCAGTACTCTACCGTGCCATGGACCTTCGGCCAGG
GGACCAAGGTGGAAATCAAGCGTACGGTGGCTGCACCATCTGTCTTCATCTTCCCG
CCATCTGATGAGCAGTTGAAATCTGGAACTGCCTCTGTTGTGTGCCTGCTGAATAA
CTTCTATCCCAGAGAGGCCAAAGTACAGTGGAAGGTGGATAACGCCCTCCAATCG
GGTAACTCCCAGGAGAGTGTCACAGAGCAGGACAGCAAGGACAGCACCTACAGC
CTCAGCAGCACCCTGACGCTGAGCAAAGCAGACTACGAGAAACACAAAGTCTAC
GCCTGCGAAGTCACCCATCAGGGCCTGAGTTCGCCCGTCACAAAGAGCTTCAACA
GGGGAGAGTGTTAA

<div align="right">SEQ ID NO: 134</div>

Codon-optimized sequence 2 of PR1163

ATGCCTCTGCTTCTGCTGCTGCCTCTGCTTTGGGCTGGCGCCCTGGCCAGCCCCTT
CATCGCCTCTGTTAGTGACCAGCATGGCGTGGTGTATATCACGGAGAACAAGAATA
AGACAGTAGTGATTCCTTGCCTGGGCAGCATCTCTAATCTGAACGTGTCGCTGGTT
GCCAGATACCCTGAGAAGAGATTCGTGCCCGACGGCAACCGGATCAGCTGGGACA
GCAAGAAGGGCTTTACCATCCCCTCTTACATGATCAGCTATGCCGGAATGGTGTTCT
GCGAAGCCAAGATCAACGACGAGTCCTATCAGTCTATCATGTACATCGTGGTCGTA
GTGGGCTACAGAATCTACGACGTGGTGCTGAGCCCATCTCACGGCATCGAGCTGA
GCGTGGGAGAGAAGCTGGTGCTGAACTGCACAGCTAGAACCGAGCTGAACGTGG
GCATAGACTTCAACTGGGAGTACCCCTCCAGCAAGCACCAGCACAAGAAACTGGT
CAACAGAGATCTGAAGACCCAGAGCGGCTCTGAGATGAAGAAGTTCCTGTCCACC

CTGACAATCGACGGCGTGACCAGAAGCGATCAGGGACTGTACACTTGTGCTGCGA
GCTCTGGCCTGATGACCAAGAAAAACAGCACCTTCGTGCGGGTGCACGAGAAGG
ACAAGACCCACACATGCCCTCCTTGTCCTGCCCCTGAGGCCGCTGGCGGCCCTAG
CGTGTTCCTGTTCCCCCCTAAACCCAAGGACACCCTCATGATCTCCCGGACACCTG
AAGTGACTTGCGTGGTCGTGGACGTCAGCCACGAAGATCCAGAGGTGAAGTTCAA
CTGGTATGTGGATGGCGTGGAAGTGCATAATGCGAAGACAAAGCCTCGGGAAGAG
CAGTACAACTCCACGTACAGAGTGGTGAGCGTGCTGACCGTGCTGCATCAAGACT
GGCTGAACGGCAAGGAATACAAGTGCAAGGTGTCCAACAAGGCCCTGCCCGCTC
CTATCGAAAAAACCATCTCTAAGGCCAAGGGCCAGCCTAGAGAGCCTCAGGTGTAT
ACCCTGCCTCCTAGCCGGGAAGAAATGACAAAGAACCAGGTGTCTCTGACCTGCC
TGGTGAAGGGCTTCTACCCCAGCGACATCGCCGTGGAGTGGGAGTCCAACGGCCA
ACCTGAAAACAATTACAAGACCACCCCTCCCGTGCTGGACAGCGACGGCAGCTTC
TTCCTGTATTCCAAGCTGACCGTCGACAAGAGCAGATGGCAGCAGGGCAACGTGT
TTAGCTGTAGCGTGATGCACGAGGCCCTGCACAACCACTACACACAAAAGTCTCT
GTCCCTGAGCCCTGGAGGCGGCGGCGGAAGCGGAGGGGGCGGCTCCGGCGGCGG
AGGCAGCGGCGGCGGCGGTTCCGAAGTGCAGCTGGTGGAAAGCGGAGGCGGTCT
GGTTCAGCCGGGCGGCAGCCTGAGACTGTCTTGCGCCGCCAGCGGCTACACATTC
ACCAACTACGGCATGAACTGGGTCAGACAGGCCCCTGGCAAGGGCCTGGAATGG
GTGGGATGGATCAACACCTACACCGGCGAACCAACATACGCCGCTGATTTTAAGCG
GAGGTTCACCTTTTCTCTGGATACCTCCAAAAGCACCGCCTACCTGCAAATGAACA
GCCTGCGGGCCGAGGACACCGCCGTTTACTACTGCGCCAAATATCCTCACTACTAC
GGCAGCTCTCACTGGTACTTCGACGTTTGGGGCCAGGGCACACTGGTGACCGTGT
CCTCGGCCAGCACAAAGGGCCCTAGCGTGTTCCCACTGGCACCATCTAGCAAGAG
CACCAGTGGAGGCACCGCCGCCCTGGGATGCCTGGTGAAAGATTACTTCCCCGAG
CCTGTCACCGTGTCTTGGAACTCCGGTGCTCTGACGAGCGGAGTGCACACCTTCC
CAGCCGTGCTTCAGTCCAGCGGTCTGTACAGCCTGTCTTCTGTTGTGACCGTGCCT
TCCAGCTCCCTGGGCACGCAGACCTACATCTGCAACGTGAACCACAAGCCTAGCA
ATACCAAGGTGGATAAGAAGGTGGAACCCAAAAGCTGCGAGGGAAGAGGCTCCC
TCCTGACATGCGGCGACGTGGAAGAGAACCCCGGCCCTATGCCCCTGCTGCTGCT
GCTGCCTCTGCTGTGGGCCGGCGCCCTGGCCGATATCCAGATGACCCAGTCTCCTT
CCTCCTTATCCGCCTCTGTGGGCGACAGAGTGACCATCACCTGTTCTGCCAGCCAG
GATATCAGCAACTACCTGAACTGGTACCAGCAGAAGCCCGGCAAAGCCCCAAAGG
TGCTGATTTACTTCACAAGCAGCCTGCACAGCGGAGTGCCTAGCCGCTTCAGCGG
CTCTGGCAGCGGGACCGACTTTACACTGACCATTAGCAGCCTGCAGCCTGAGGATT
TCGCCACATACTACTGTCAGCAATACAGCACAGTCCCTTGGACCTTTGGACAAGGA
ACAAAGGTGGAAATCAAGCGGACCGTGGCCGCTCCCAGCGTGTTCATCTTCCCTC
CTAGCGACGAGCAACTGAAATCTGGCACCGCCAGCGTCGTGTGCCTGCTGAACAA
CTTCTACCCGAGAGAGGCTAAGGTGCAGTGGAAGGTGGACAACGCCCTGCAGAG
CGGGAATAGCCAGGAGAGCGTGACCGAGCAGGACTCCAAAGACAGCACATACTC

TCTGAGCAGCACCCTTACACTGAGCAAGGCCGACTACGAGAAACACAAAGTGTAC
GCTTGTGAGGTGACCCACCAGGGCCTGAGCTCTCCCGTGACCAAAAGCTTTAATA
GAGGCGAGTGCTGA

SEQ ID NO: 135

Codon-optimized sequence 3 of PR1163

ATGCCTCTGCTGCTGCTTCTGCCTCTTCTTTGGGCTGGCGCTCTGGCTAGCCCTTTT
ATCGCCTCCGTGTCCGATCAGCACGGCGTGGTGTACATCACCGAGAACAAGAACA
AGACCGTGGTCATCCCCTGCCTGGGCAGCATCAGCAACCTGAATGTGTCCCTGGTG
GCCAGATATCCCGAGAAGAGATTCGTGCCCGACGGCAACAGAATCAGCTGGGACA
GCAAGAAGGGCTTCACAATCCCCAGCTACATGATCAGCTACGCCGGCATGGTGTTC
TGCGAGGCCAAGATCAACGACGAGAGCTACCAGAGCATCATGTACATCGTGGTGG
TCGTGGGCTACAGAATCTACGACGTGGTGCTGAGCCCTAGCCACGGCATTGAACT
GTCTGTGGGCGAGAAGCTGGTGCTGAACTGTACCGCCAGAACCGAGCTGAACGT
GGGCATCGACTTCAACTGGGAGTACCCCAGCAGCAAGCACCAGCACAAGAAACT
GGTCAACCGGGACCTGAAAACCCAGAGCGGCAGCGAGATGAAGAAGTTCCTGAG
CACCCTGACCATCGACGGCGTGACCAGATCTGACCAGGGCCTGTACACATGTGCC
GCCAGCTCTGGCCTGATGACCAAGAAAAACAGCACCTTCGTGCGGGTGCACGAG
AAGGACAAGACCCACACCTGTCCTCCATGTCCTGCTCCAGAAGCTGCTGGCGGCC
CTTCCGTGTTTCTGTTCCCTCCAAAGCCTAAGGACACCCTGATGATCAGCAGAACC
CCTGAAGTGACCTGCGTGGTGGTGGATGTGTCCCACGAGGATCCCGAAGTGAAGT
TCAATTGGTACGTGGACGGCGTGGAAGTGCACAACGCCAAGACCAAGCCTAGAG
AGGAACAGTACAACTCCACCTACAGAGTGGTGTCCGTGCTGACCGTGCTGCACCA
GGATTGGCTGAACGGCAAAGAGTACAAGTGCAAGGTGTCCAACAAGGCCCTGCC
TGCTCCTATCGAGAAAACCATCAGCAAGGCCAAGGGCCAGCCTAGGGAACCCCAG
GTTTACACACTGCCTCCAAGCCGGGAAGAGATGACAAAGAACCAGGTGTCCCTGA
CCTGCCTGGTCAAGGGCTTCTACCCTTCCGATATCGCCGTGGAATGGGAGAGCAAT
GGCCAGCCTGAGAACAACTACAAGACAACCCCTCCTGTGCTGGACAGCGACGGC
TCATTCTTCCTGTACAGCAAGCTGACAGTGGACAAGAGCAGATGGCAGCAGGGCA
ACGTGTTCAGCTGCAGCGTGATGCACGAGGCCCTGCACAACCACTACACCCAGAA
GTCCCTGTCTCTGTCTCCTGGCGGAGGCGGAGGATCTGGTGGCGGAGGAAGCGGT
GGCGGCGGATCAGGCGGTGGTGGTTCTGAAGTTCAGCTGGTGGAAAGCGGCGGT
GGCCTTGTTCAACCTGGCGGATCTCTGAGACTGAGCTGTGCCGCCTCTGGCTACAC
CTTCACCAACTACGGCATGAATTGGGTCCGACAGGCCCCTGGCAAAGGCCTGGAA
TGGGTCGGATGGATCAACACCTACACCGGCGAGCCAACATACGCCGCCGACTTCA
AGCGGAGATTCACCTTCAGCCTGGACACCAGCAAGAGCACCGCCTACCTGCAGAT
GAACTCCCTGAGAGCCGAGGACACCGCCGTGTACTACTGCGCCAAGTATCCCCAC
TACTACGGCAGCAGCCACTGGTACTTTGACGTGTGGGGACAGGGCACCCTGGTCA
CAGTTAGCAGCGCCTCTACAAAGGGCCCCAGCGTTTTCCCACTGGCTCCTAGCAG
CAAGTCTACCTCCGGTGGAACAGCTGCCCTGGGCTGTCTCGTGAAGGACTACTTTC

CTGAGCCTGTGACCGTGTCCTGGAACTCTGGCGCACTGACATCTGGCGTGCACAC
CTTTCCAGCTGTGCTGCAGTCTAGCGGCCTGTACTCTCTGAGCAGCGTCGTGACAG
TGCCTAGCAGCTCTCTGGGCACCCAGACCTACATCTGCAATGTGAACCACAAGCCT
AGCAACACCAAGGTCGACAAGAAGGTGGAACCCAAGAGCTGCGAAGGCAGAGG
CAGCCTGCTTACATGTGGCGACGTGGAAGAGAACCCCGGACCTATGCCACTTCTCC
TGCTGTTGCCATTGCTGTGGGCAGGCGCACTGGCCGACATTCAGATGACACAGAG
CCCAAGCAGCCTGAGCGCCTCTGTGGGAGATAGAGTGACCATCACCTGTTCCGCC
AGCCAGGACATCTCCAACTACCTGAACTGGTATCAGCAAAAGCCCGGCAAGGCCC
CTAAGGTGCTGATCTACTTTACCAGCAGCCTGCACAGCGGCGTGCCCAGCAGATTT
TCTGGCTCTGGCAGCGGCACCGACTTCACCCTGACAATATCTAGCCTGCAGCCAGA
GGACTTCGCCACCTACTACTGCCAGCAGTACAGCACCGTGCCTTGGACATTTGGCC
AGGGCACCAAGGTGGAAATCAAGCGGACAGTCGCCGCTCCTAGCGTGTTCATCTT
TCCACCTAGCGACGAGCAGCTGAAGTCTGGCACAGCCTCTGTCGTGTGCCTGCTG
AACAACTTCTACCCCAGAGAAGCCAAGGTGCAGTGGAAGGTGGACAATGCCCTG
CAGTCCGGCAACAGCCAAGAGAGCGTGACAGAGCAGGACTCCAAGGACAGCACC
TACAGCCTGTCTAGCACACTGACCCTGAGCAAGGCCGACTATGAGAAGCACAAAG
TGTACGCCTGCGAAGTGACCCACCAGGGGCTTTCTAGCCCAGTGACCAAGAGCTT
CAACCGGGGCGAGTGTTGA

SEQ ID NO: 136

3'-UTR sequence:

GTCTAGAAACCAGCCTCAAGAACACCCGAATGGAGTCTCTAAGCTACATAATACCA
ACTTACACTTTACAAAATGTTGTCCCCCAAAATGTAGCCATTCGTATCTGCTCCTAA
TAAAAAGAAAGTTTCTTCAC

SEQ ID NO: 137

WPRE sequence:

AATCAACCTCTGGATTACAAAATTTGTGAAAGATTGACTGGTATTCTTAACTATGTT
GCTCCTTTTACGCTATGTGGATACGCTGCTTTAATGCCTTTGTATCATGCTATTGCTT
CCCGTATGGCTTTCATTTTCTCCTCCTTGTATAAATCCTGGTTGCTGTCTCTTTATGA
GGAGTTGTGGCCCGTTGTCAGGCAACGTGGCGTGGTGTGCACTGTGTTTGCTGAC
GCAACCCCCACTGGTTGGGGCATTGCCACCACCTGTCAGCTCCTTTCCGGGACTTT
CGCTTTCCCCCTCCCTATTGCCACGGCGGAACTCATCGCCGCCTGCCTTGCCCGCT
GCTGGACAGGGGCTCGGCTGTTGGGCACTGACAATTCCGTGGTGTTGTCGGGGAA
ATCATCGTCCTTTCCTTGGCTGCTCGCCTGTGTTGCCACCTGGATTCTGCGCGGGAC
GTCCTTCTGCTACGTCCCTTCGGCCCTCAATCCAGCGGACCTTCCTTCCCGCGGCC
TGCTGCCGGCTCTGCGGCCTCTTCCGCGTCTTCGCCTTCGCCCTCAGACGAGTCGG
ATCTCCCTTTGGGCCGCCTCCCCGC

SEQ ID NO: 138

SV40polyA sequence:

CCTAGGTAAGATACATTGATGAGTTTGGACAAACCACAACTAGAATGCAGTGAAA

AAAATGCTTTATTTGTGAAATTTGTGATGCTATTGCTTTATTTGTAACCATTATAAGC
TGCAATAAACAAGTTATCGATAGATCT

<div align="right">SEQ ID NO: 139</div>

Capsid protein sequence:

MAADGYLPDWLEDTLSEGIRQWWKLKPGPPPPKPAERHKDDSRGLVLPGYKYLGPF
NGLDKGEPVNEADAAALEHDKAYDRQLDSGDNPYLKYNHADAEFQERLKEDTSFG
GNLGRAVFQAKKRVLEPLGLVEEPVKTAPGKKRPVEHSPVEPDSSSGTGKAGQQPAR
KRLNFGQTGDADSVPDPQPLGQPPAAPSGLGTNTMATGSGAPMADNNEGADGVGNS
SGNWHCDSTWMGDRVITTSTRTWALPTYNNHLYKQISSQSGASNDNHYFGYSTPWG
YFDFNRFHCHFSPRDWQRLINNNWGFRPKRLNFKLFNIQVKEVTQNDGTTTIANNLT
STVQVFTDSEYQLPYVLGSAHQGCLPPFPADVFMVPQYGYLTLNNGSQAVGRSSFYC
LEYFPSQMLRTGNNFTFSYTFEDVPFHSSYAHSQSLDRLMNPLIDQYLYYLSRTNTPSG
TTTQSRLQFSQAGASDIRDQSRNWLPGPCYRQQRVSKTSADNNNSEYSWTGATKYHL
NGRDSLVNPGPAMASHKDDEEKFFPQSGVLIFGKQGSEKTNVDIEKVMITDEEEIRTT
NPVATEQYGSVSTNLRGNLALGDTTRPARQAATADVNTQGVLPGMVWQDRDVYL
QGPIWAKIPHTDGHFHPSPLMGGFGLKHPPPQILIKNTPVPANPSTTFSAAKFASFITQY
STGQVSVEIEWELQKENSKRWNPEIQYTSNYNKSINVDFTVDTNGVYSEPRPIGTRYL
TRNL

<div align="right">SEQ ID NO: 127</div>

Codon-optimized sequence of HRPDAAV02-AI:

cctgcaggcagctgcgcgctcgctcgctcactgaggccgcccgggcaaagcccgggcgtcgggcgacctttggtcgcccggcctc
agtgagcgagcgagcgcgcagagagggagtggccaactccatcactaggggttcctatcgatatcaagcttCGTTACATAA
CTTACGGTAAATGGCCCGCCTGGCTGACCGCCCAACGACCCCCGCCCATTGACGTC
AATAGTAACGCCAATAGGGACTTTCCATTGACGTCAATGGGTGGAGTATTTACGGT
AAACTGCCCACTTGGCAGTACATCAAGTGTATCATATGCCAAGTACGCCCCCTATTG
ACGTCAATGACGGTAAATGGCCCGCCTGGCATTGTGCCCAGTACATGACCTTATGG
GACTTTCCTACTTGGCAGTACATCTACGTATTAGTCATCGCTATTACCATGGTCGAG
GTGAGCCCCACGTTCTGCTTCACTCTCCCCATCTCCCCCCCCTCCCCACCCCCAATT
TTGTATTTATTTATTTTTTAATTATTTTGTGCAGCGATGGGGGCGGGGGGGGGGGGG
GGGCGCGCGCCAGGCGGGGCGGGGCGGGGCGAGGGGCGGGGCGGGGCGAGGCG
GAGAGGTGCGGCGGCAGCCAATCAGAGCGGCGCGCTCCGAAAGTTTCCTTTTATG
GCGAGGCGGCGGCGGCGGCGGCCCTATAAAAAGCGAAGCGCGCGGCGGGCGGGA
GTCGCTGCGCGCTGCCTTCGCCCCGTGCCCCGCTCCGCCGCCGCCTCGCGCCGCCC
GCCCCGGCTCTGACTGACCGCGTTACTCCCACAGGTGAGCGGGCGGGACGGCCCT
TCTCCTCCGGGCTGTAATTAGCTGAGCAAGAGGTAAGGGTTTAAGGGATGGTTGGT
TGGTGGGGTATTAATGTTTAATTACCTGGAGCACCTGCCTGAAATCACTTTTTTCA
GGTTGGgggattcgaacatcggccGCCACCATGAACGCCAGCGAGTTTCGGCGGAGAGGCA
AAGAAATGGTGGACTACGTGGCCAACTACATGGAAGGCATCGAGGGCAGACAGGT
GTACCCCGATGTGGAACCTGGCTACCTGAGGCCTCTGATTCCTGCCGCTGCTCCCC

EP 4 768 039 A1

AAGAGCCTGACACCTTCGAGGACATCATCAACGACGTGGAAAAGATCATCATGCC
CGGCGTGACCCACTGGCACAGCCCCTACTTTTTCGCCTACTTTCCCACCGCCAGCA
GCTACCCTGCTATGCTGGCTGATATGCTGTGTGGCGCCATCGGCTGCATCGGCTTTT
CTTGGGCTGCCTCTCCTGCCTGCACCGAGCTGGAAACCGTGATGATGGATTGGCTG
GGCAAGATGCTGGAACTGCCCAAGGCCTTCCTGAACGAGAAAGCTGGCGAAGGC
GGCGGAGTGATTCAGGGATCTGCCTCTGAAGCCACACTGGTGGCTCTGCTGGCCG
CCAGAACAAAAGTGATCCACAGACTGCAGGCCGCCTCTCCAGAACTGACACAGG
CCGCCATCATGGAAAAGCTGGTGGCCTACAGCAGCGATCAGGCCCACAGCTCTGT
GGAAAGAGCCGGACTGATTGGCGGCGTGAAGCTGAAGGCCATTCCTAGCGACGG
CAACTTCGCCATGAGAGCCTCTGCTCTGCAAGAGGCCCTGGAAAGAGATAAGGCC
GCTGGACTGATCCCCTTCTTCATGGTGGCTACCCTGGGCACCACCACCTGTTGCAG
CTTCGACAACCTGCTGGAAGTGGGCCCCATCTGCAACAAAGAGGACATCTGGCTG
CACGTGGACGCCGCCTATGCCGGCTCTGCCTTTATCTGCCCCGAGTTCAGACATCT
GCTGAACGGCGTGGAATTCGCCGACAGCTTCAACTTCAACCCTCACAAGTGGCTG
CTGGTCAACTTCGACTGCAGCGCTATGTGGGTCAAGAAGCGGACCGATCTGACCG
GCGCCTTCAGACTGGATCCCACCTACCTGAAGCACTCCCACCAGGATAGCGGCCT
GATCACCGACTACCGGCACTGGCAGATTCCTCTGGGCAGAAGATTCGGAGCCTG
AAGATGTGGTTCGTGTTCCGGATGTATGGCGTGAAGGGCCTCCAGGCCTACATCAG
AAAACATGTGCAGCTGAGCCACGAGTTCGAGAGCCTTGTCAGACAGGACCCCAGA
TTCGAGATCTGCGTGGAAGTGATCCTGGGCCTCGTGTGCTTTAGACTGAAGGGCA
GCAACAAAGTGAACGAGGCCCTGCTGCAGCGGATCAACAGCGCCAAAAAGATCC
ACCTGGTGCCTTGCCACCTGAGAGACAAGTTCGTGCTGAGATTCGCCATCTGCAG
CCGGACAGTGGAAAGCGCCCATGTGCAGAGAGCCTGGGAGCACATCAAAGAACT
GGCCGCCGATGTGCTGAGGGCCGAGAGAGAAgccaccaacttctccctgctgaagcaggccggcgacg
tggaggagaacccccggccccATGAAGCTGTGGGACGTGGTGGCCGTGTGCCTGGTGCTGCTG
CACACCGCCAGCGCCTTCCCCCTGCCCGCCGGCAAGCGCCCCCCCGAGGCCCCCG
CCGAGGACCGCAGCCTGGGCCGCCGCCGCGCCCCCTTCGCCCTGAGCAGCGACA
GCAACATGCCCGAGGACTACCCCGACCAGTTCGACGACGTGATGGACTTCATCCA
GGCCACCATCAAGCGCCTGAAGCGCAGCCCCGACAAGCAGATGGCCGTGCTGCCC
CGCCGCGAGCGCAACCGCCAGGCCGCCGCCGCCAACCCCGAGAACAGCCGCGGC
AAGGGCCGCCGCGGCCAGCGCGGCAAGAACCGCGGCTGCGTGCTGACCGCCATC
CACCTGAACGTGACCGACCTGGGCCTGGGCTACGAGACCAAGGAGGAGCTGATCT
TCCGCTACTGCAGCGGCAGCTGCGACGCCGCCGAGACCACCTACGACAAGATCCT
GAAGAACCTGAGCCGCAACCGCCGCCTGGTGAGCGACAAGGTGGGCCAGGCCTG
CTGCCGCCCCATCGCCTTCGACGACGACCTGAGCTTCCTGGACGACAACCTGGTG
TACCACATCCTGCGCAAGCACAGCGCCAAGCGCTGCGGCTGCATCTAActcgagaatcaa
cctctggattacaaaatttgtgaaagattgactggtattcttaactatgttgctccttttacgctatgtggatacgctgctttaatgcctttgtatc
atgctattgcttcccgtatggctttcatttttctcctccttgtataaatcctggttgctgtctctttatgaggagttgtggcccgttgtcaggcaac
gtggcgtggtgtgcactgtgtttgctgacgcaaccccactggttggggcattgccaccacctgtcagctcctttccgggactttcgcttt

cccccctccctattgccacggcggaactcatcgccgcctgccttgcccgctgctggacaggggctcggctgttgggcactgacaattcc

gtggtgttgtcggggaaatcatcgtcctttccttggctgctcgcctgtgttgccacctggattctgcgcgggacgtccttctgctacgtccc

ttcggccctcaatccagcggaccttccttcccgcggcctgctgccggctctgcggcctcttccgcgtcttcgccttcgccctcagacgag

tcggatctcccttgggccgcctccccgctaagatacattgatgagtttggacaaaccacaactagaatgcagtgaaaaaaatgctttattt

gtgaaatttgtgatgctattgctttatttgtaaccattataagctgcaataaacaagttCGGACCGAGCGGCagatctaggaacc

cctagtgatggagttggccactccctctctgcgcgctcgctcgctcactgaggccgggcgaccaaaggtcgcccgacgcccgggcttt

gcccgggcggcctcagtgagcgagcgagcgcgcagctgcctgcagg

<div align="right">SEQ ID NO: 140</div>

Codon-optimized sequence of HRPDAAV03-DI:

cctgcaggcagctgcgcgctcgctcgctcactgaggccgcccgggcaaagcccgggcgtcgggcgacctttggtcgcccggcctc

agtgagcgagcgagcgcgcagagagggagtggccaactccatcactagggggttcctatcgatatccgttacataacttacggtaaatg

gcccgcctggctgaccgcccaacgacccccgcccattgacgtcaataatgacgtatgttcccatagtaacgtcaatagggactttccatt

gacgtcaatgggtggagtatttacggtaaactgcccacttggcagtacatcaagtgtatcatatgccaagtacgccccctattgacgtcaa

tgacggtaaatggcccgcctggcattatgcccagtacatgaccttatgggactttcctacttggcagtacatctacgtattagtcatcgctat

taccatggtgatgcggttttggcagtacatcaatgggcgtggatagcggtttgactcacggggatttccaagtctccaccccattgacgtc

aatgggagtttgttttgcaccaaaatcaacgggactttccaaaatgtcgtaacaactccgccccattgacgcaaatgggcggtaggcgtg

tacggtgggaggtctatataagcagagctgtgagtttgggacccttgattgttctttcttttcgctattgtaaaattcatgttatatggaggg

ggcaaagttttcagggtgttgtttagaatgggaagatgtcccttgtatcaccatggaccctcatgataattttgtttctttcactttctactctgtt

gacaaccattgtctcctcttatttttctttttcatttttctgtaacttttttcgttaaactttagcttgcatttgtaacgaatttttaaattcacttttgtttatttg

tcagattgtaagtactttctctaatcactttttttttcaaggcaatcagggtatattatattgtacttcagcacagttttagagaacaattgttataat

taaatgataaggtagaatatttctgcatataaaattctggctggcgtggaaatattcttattggtagaaacaactacatcctggtcatcatcctg

cctttctcttatggttacaatgatatacactgtttgagatgaggataaaatactctgagtccaaaccgggcccctctgctaaccatgttcatg

ccttcttcttttttcctacagGCCACCATGAAGCTGTGGGACGTGGTGGCCGTGTGCCTGGTGCT

GCTGCACACCGCCAGCGCCTTCCCCCTGCCCGCCGGCAAGCGCCCCCCCGAGGCC

CCCGCCGAGGACCGCAGCCTGGGCCGCCGCCGCGCCCCCTTCGCCCTGAGCAGCG

ACAGCAACATGCCCGAGGACTACCCCGACCAGTTCGACGACGTGATGGACTTCAT

CCAGGCCACCATCAAGCGCCTGAAGCGCAGCCCCGACAAGCAGATGGCCGTGCTG

CCCCGCCGCGAGCGCAACCGCCAGGCCGCCGCCGCCAACCCCGAGAACAGCCGC

GGCAAGGGCCGCCGCGGCCAGCGCGGCAAGAACCGCGGCTGCGTGCTGACCGCC

ATCCACCTGAACGTGACCGACCTGGGCCTGGGCTACGAGACCAAGGAGGAGCTGA

TCTTCCGCTACTGCAGCGGCAGCTGCGACGCCGCCGAGACCACCTACGACAAGAT

CCTGAAGAACCTGAGCCGCAACCGCCGCCTGGTGAGCGACAAGGTGGGCCAGGC

CTGCTGCCGCCCCATCGCCTTCGACGACGACCTGAGCTTCCTGGACGACAACCTG

GTGTACCACATCCTGCGCAAGCACAGCGCCAAGCGCTGCGGCTGCATCTAAaataaag

tctgagtgggcggcagcctgtgtgtgcctgggttctctctgtcccggaatgtgcaaacaatggaggtgaagcttCGTTACATAA

CTTACGGTAAATGGCCCGCCTGGCTGACCGCCCAACGACCCCCGCCCATTGACGTC

AATAGTAACGCCAATAGGGACTTTCCATTGACGTCAATGGGTGGAGTATTTACGGT

AAACTGCCCACTTGGCAGTACATCAAGTGTATCATATGCCAAGTACGCCCCCTATTG

ACGTCAATGACGGTAAATGGCCCGCCTGGCATTGTGCCCAGTACATGACCTTATGG

GACTTTCCTACTTGGCAGTACATCTACGTATTAGTCATCGCTATTACCATGGTCGAG

GTGAGCCCCACGTTCTGCTTCACTCTCCCCATCTCCCCCCCCTCCCCACCCCCAATT

TTGTATTTATTTATTTTTTAATTATTTTGTGCAGCGATGGGGGCGGGGGGGGGGGGG

<div align="center">158</div>

```
GGGCGCGCGCCAGGCGGGGCGGGGCGGGGCGAGGGGCGGGGCGGGGCGAGGCG
GAGAGGTGCGGCGGCAGCCAATCAGAGCGGCGCGCTCCGAAAGTTTCCTTTTATG
GCGAGGCGGCGGCGGCGGCGGCCCTATAAAAAGCGAAGCGCGCGGCGGGCGGGA
GTCGCTGCGCGCTGCCTTCGCCCCGTGCCCCGCTCCGCCGCCGCCTCGCGCCGCCC
GCCCCGGCTCTGACTGACCGCGTTACTCCCACAGGTGAGCGGGCGGGACGGCCCT
TCTCCTCCGGGCTGTAATTAGCTGAGCAAGAGGTAAGGGTTTAAGGGATGGTTGGT
TGGTGGGGTATTAATGTTTAATTACCTGGAGCACCTGCCTGAAATCACTTTTTTCA
GGTTGGgggattcgaacatcggccGCCACCATGAACGCCAGCGAGTTTCGGCGGAGAGGCA
AAGAAATGGTGGACTACGTGGCCAACTACATGGAAGGCATCGAGGGCAGACAGGT
GTACCCCGATGTGGAACCTGGCTACCTGAGGCCTCTGATTCCTGCCGCTGCTCCCC
AAGAGCCTGACACCTTCGAGGACATCATCAACGACGTGGAAAAGATCATCATGCC
CGGCGTGACCCACTGGCACAGCCCCTACTTTTTCGCCTACTTTCCCACCGCCAGCA
GCTACCCTGCTATGCTGGCTGATATGCTGTGTGGCGCCATCGGCTGCATCGGCTTTT
CTTGGGCTGCCTCTCCTGCCTGCACCGAGCTGGAAACCGTGATGATGGATTGGCTG
GGCAAGATGCTGGAACTGCCCAAGGCCTTCCTGAACGAGAAAGCTGGCGAAGGC
GGCGGAGTGATTCAGGGATCTGCCTCTGAAGCCACACTGGTGGCTCTGCTGGCCG
CCAGAACAAAAGTGATCCACAGACTGCAGGCCGCCTCTCCAGAACTGACACAGG
CCGCCATCATGGAAAAGCTGGTGGCCTACAGCAGCGATCAGGCCCACAGCTCTGT
GGAAAGAGCCGGACTGATTGGCGGCGTGAAGCTGAAGGCCATTCCTAGCGACGG
CAACTTCGCCATGAGAGCCTCTGCTCTGCAAGAGGCCCTGGAAAGAGATAAGGCC
GCTGGACTGATCCCCTTCTTCATGGTGGCTACCCTGGGCACCACCACCTGTTGCAG
CTTCGACAACCTGCTGGAAGTGGGCCCCATCTGCAACAAAGAGGACATCTGGCTG
CACGTGGACGCCGCCTATGCCGGCTCTGCCTTTATCTGCCCCGAGTTCAGACATCT
GCTGAACGGCGTGGAATTCGCCGACAGCTTCAACTTCAACCCTCACAAGTGGCTG
CTGGTCAACTTCGACTGCAGCGCTATGTGGGTCAAGAAGCGGACCGATCTGACCG
GCGCCTTCAGACTGGATCCCACCTACCTGAAGCACTCCCACCAGGATAGCGGCCT
GATCACCGACTACCGGCACTGGCAGATTCCTCTGGGCAGAAGATTCGGAGCCTG
AAGATGTGGTTCGTGTTCCGGATGTATGGCGTGAAGGGCCTCCAGGCCTACATCAG
AAAACATGTGCAGCTGAGCCACGAGTTCGAGAGCCTTGTCAGACAGGACCCCAGA
TTCGAGATCTGCGTGGAAGTGATCCTGGGCCTCGTGTGCTTTAGACTGAAGGGCA
GCAACAAAGTGAACGAGGCCCTGCTGCAGCGGATCAACAGCGCCAAAAAGATCC
ACCTGGTGCCTTGCCACCTGAGAGACAAGTTCGTGCTGAGATTCGCCATCTGCAG
CCGGACAGTGGAAAGCGCCCATGTGCAGAGAGCCTGGGAGCACATCAAAGAACT
GGCCGCCGATGTGCTGAGGGCCGAGAGAGAATGActcgagtaagatacattgatgagtttggacaaac
cacaactagaatgcagtgaaaaaaatgctttatttgtgaaatttgtgatgctattgctttatttgtaaccattataagctgcaataaacaagttC
GGACCGAGCGGCagatctaggaaccccctagtgatggagttggccactccctctctgcgcgctcgctcgctcactgaggccg
ggcgaccaaaggtcgcccgacgcccgggctttgcccgggcggcctcagtgagcgagcgagcgcgcagctgcctgcagg
```

SEQ ID NO: 141

## Claims

1. A pharmaceutical composition, comprising recombinant adeno-associated virus (rAAV) particles and excipients, wherein the excipient comprises Hepes; preferably, the Hepes is at a concentration of about 1 mM to about 100 mM, preferably about 5 mM to about 50 mM, and preferably about 5 mM to about 25 mM.

2. The pharmaceutical composition according to claim 1, wherein the rAAV comprises a nucleic acid molecule, the nucleic acid molecule comprises a polynucleotide encoding a VEGF-binding molecule, and the VEGF-binding molecule comprises a first VEGF-binding domain and a second VEGF-binding domain, wherein

the first VEGF-binding domain comprises immunoglobulin-like domain 2 of VEGFR2 (R2D2) and immunoglobulin-like domain 3 of VEGFR2 (R2D3);
the second VEGF-binding domain comprises a VEGF Trap or comprises an anti-VEGF antibody or an antigen-binding fragment thereof;
preferably, the VEGF Trap comprises immunoglobulin-like domain 2 of VEGFR1 (R1D2) and/or immunoglobulin-like domain 3 of VEGFR2 (R2D3).

3. The pharmaceutical composition according to claim 2, wherein:

the first VEGF-binding domain comprises an amino acid mutation at any one or more positions selected from the group consisting of the following: position 162, position 221, position 222, position 274, position 276, position 277, position 280, position 284, position 288, position 289, and position 313; the position of the amino acid mutation is relative to a natural sequence position of the amino acid sequence set forth in SEQ ID NO: 51; or
the first VEGF-binding domain comprises an amino acid mutation at any one or more positions selected from the group consisting of the following: position 135, position 197, position 199, position 213, position 215, position 219, position 257, position 275, position 277, position 278, position 283, position 289, position 313, and position 314; the position of the amino acid mutation is relative to a natural sequence position of the amino acid sequence set forth in SEQ ID NO: 51; preferably, the first VEGF-binding domain comprises an amino acid mutation at position 283;
preferably, the first VEGF-binding domain comprises any one or more sets of amino acid mutations selected from the group consisting of the following:

- 162V or 162I;
- 162V/274F; 162V/274I; 162I/274F; or 162I/274I;
- 221S/222K;
- 277R/289Y;
- 162V/221S/222K;
- 162V/276Q;
- 162V/289Y;
- 162V/277R/289Y;
- 162V/313R; and
- 162V/221S/222K/274F;

more preferably, the first VEGF-binding domain comprises any one or more sets of amino acid mutations selected from the group consisting of the following:

- 162V; and
- 162V/221S/222K/274F; or

preferably, the first VEGF-binding domain comprises any one or more sets of amino acid mutations selected from the group consisting of the following:

- 135Y;
- 197Y;
- 199Q or 199T;
- 213N;
- 215F or 215H;
- 219I;
- 257Q or 257T;
- 275L or 275E;
- 277R;
- 278D, 278S, or 278E;
- 283T;
- 289Y;
- 313Y; and

- 314S or 314Q;

more preferably, the first VEGF-binding domain comprises an amino acid mutation 283T.

4. The VEGF-binding molecule according to any one of claims 2 to 3, wherein:

the first VEGF-binding domain comprises an amino acid mutation at any one or more positions selected from the group consisting of the following: position 162, position 221,
position 222, position 274, position 276, position 277, position 280, position 284, position 288, position 289, and position 313; and
the first VEGF-binding domain comprises an amino acid mutation at any one or more positions selected from the group consisting of the following: position 135, position 197,
position 199, position 213, position 215, position 219, position 257, position 275, position 277, position 278, position 283, position 289, position 313, and position 314;
preferably, the first VEGF-binding domain comprises:

(i) any one or more sets of amino acid mutations selected from the group consisting of the following:

- 162A, 162V, 162S, 162I, or 162L;
- 221S;
- 222K;
- 274F or 274I;
- 276Q;
- 277R;
- 280E;
- 284H;
- 288Y;
- 289Y; and
- 313R; and

(ii) any one or more sets of amino acid mutations selected from the group consisting of the following:

- 135Y;
- 197Y;
- 199Q or 199T;
- 213N;
- 215F or 215H;
- 219I;
- 257Q or 257T;
- 275L or 275E;
- 277R;
- 278D, 278S, or 278E;
- 283T;
- 289Y;
- 313Y; and
- 314S or 314Q;

preferably, the first VEGF-binding domain comprises any one or more sets of amino acid mutations selected from the group consisting of the following:

- 162V/275L;
- 162V/283T;
- 162V/313Y;
- 162V/274F/283T;
- 162V/221S/222K/283T; and
- 162V/221S/222K/274F/283T;

more preferably, the first VEGF-binding domain comprises any one or more sets of amino acid mutations selected from the group consisting of the following:

- 162V/283T; and
- 162V/221S/222K/274F/283T.

**5.** The pharmaceutical composition according to any one of claims 2 to 4, wherein:

the VEGF-binding molecule further comprises an Fc; preferably, the Fc is an Fc of human IgG1, human IgG2, human IgG3, or human IgG4; preferably,
the VEGF-binding molecule comprises, from N-terminus to C-terminus, any one selected from the group consisting of the following:

R2D2-R2D3-Fc;
R1D2-R2D3-R2D2-R2D3-Fc;
R1D2-R2D3-linker-R2D2-R2D3-Fc;
R2D2-R2D3-R1D2-R2D3-Fc; and
R2D2-R2D3-linker-R1D2-R2D3-Fc; or
the VEGF-binding molecule comprises a heavy chain and a light chain, wherein the heavy chain comprises, from N-terminus to C-terminus: R2D2-R2D3-Fc-linker-VH-CH1, and the light chain comprises, from N-terminus to C-terminus: VL-CL;
preferably, the VH and the VL are the VH and VL of bevacizumab or the VH and VL of ranibizumab;
preferably, the linker is a peptide linker;
more preferably, the linker is selected from the group consisting of:

(GS)a(GGS)b(GGGS)c(GGGGS)d(GGGGG)e, wherein a, b, c, d, and e are independently integers greater than or equal to 0; or
the linker is selected from the group consisting of: $(EAAAK)_3$, $(EAAAR)_3$, $(EGGGK)_3$, $(EGGGR)_3$, $(DAAAR)_3$, $(DAAAK)_3$, $(DGGGR)_3$, and $(DGGGK)_3$; or
the linker is $(G_xS)_y$, wherein x is selected from the group consisting of integers of 1-5, and y is selected from the group consisting of integers of 1-6.

**6.** The pharmaceutical composition according to any one of claims 2 to 5, wherein:

the VEGF-binding molecule comprises the amino acid sequence set forth in any one of SEQ ID NOs: 55-56 and SEQ ID NOs: 112-115 or an amino acid sequence having at least 90% sequence identity thereto; or
the VEGF-binding molecule consists of the amino acid sequence set forth in any one of SEQ ID NOs: 55-56 and SEQ ID NOs: 112-115 or an amino acid sequence having at least 90% sequence identity thereto.
preferably,

1) the VEGF-binding molecule comprises the amino acid sequence set forth in any one of SEQ ID NOs: 53-54, SEQ ID NOs: 57-99, SEQ ID NOs: 102-111, and SEQ ID NO: 116, or an amino acid sequence having at least 90% sequence identity thereto;
2) the heavy chain of the VEGF-binding molecule comprises the amino acid sequence set forth in SEQ ID NO: 100 or an amino acid sequence having at least 90% sequence identity thereto, and the light chain of the VEGF-binding molecule comprises the amino acid sequence set forth in SEQ ID NO: 101 or an amino acid sequence having at least 90% sequence identity thereto; or
3) the heavy chain of the VEGF-binding molecule comprises the amino acid sequence set forth in SEQ ID NO: 117 or an amino acid sequence having at least 90% sequence identity thereto, and the light chain of the VEGF-binding molecule comprises the amino acid sequence set forth in SEQ ID NO: 118 or an amino acid sequence having at least 90% sequence identity thereto.

**7.** The pharmaceutical composition according to any one of claims 2 to 6, wherein the nucleic acid molecule comprises a nucleic acid sequence having at least 70% identity to the nucleic acid sequence set forth in any one of SEQ ID NOs: 128-136;
preferably, the nucleic acid molecule further comprises any one or any combination of the following: a Kozak sequence, a 5'-UTR and a 3'-UTR, a WPRE sequence, a polyA sequence, and an intron, wherein the Kozak sequence is preferably located between the intron and the polynucleotide encoding the VEGF-binding molecule, and the polyA sequence is preferably an sv40 polyA sequence.

8. The pharmaceutical composition according to claim 1, wherein the rAAV comprises a nucleic acid molecule, and the nucleic acid molecule comprises a first polynucleotide and a second polynucleotide, wherein the first polynucleotide encodes aromatic L-amino acid decarboxylase (AADC) protein, and the second polynucleotide encodes glial cell line-derived neurotrophic factor (GDNF) protein;

preferably, the AADC protein comprises the amino acid sequence set forth in SEQ ID NO: 1, and/or the GDNF protein comprises the amino acid sequence set forth in SEQ ID NO: 2;
preferably, the first polynucleotide comprises a sequence having at least 75% identity to SEQ ID NO: 3 or a sequence having at least 95% identity to any one of SEQ ID NOs: 4-7, and/or the second polynucleotide comprises a sequence having at least 70% identity to SEQ ID NO: 8 or a sequence having at least 95% identity to any one of SEQ ID NOs: 9-12.

9. The pharmaceutical composition according to claim 8, wherein the nucleic acid molecule further comprises any one or any combination of a 5' inverted terminal repeat (5' ITR), a 3' inverted terminal repeat (3' ITR), an intron, a post-transcriptional regulatory element, a polyadenylation signal (polyA), and a multiple cloning site (MCS);

the 5' ITR and/or 3' ITR are/is derived from AAV1, AAV2, AAV3, AAV4, AAV5, AAV6, AAV7, AAV8, AAV9, AAV9.47, AAV9(hu14), AAV10, AAV11, AAV12, AAV13, AAVrh8, AAVrh10, AAV-DJ, or AAV-DJ8, preferably from AAV2 or AAV9;
a promoter is selected from the group consisting of CMV, CAG, CBA, CBh, EFS, EF1, PGK, SV40, Ubi and RSV promoters, or any combination thereof;
an enhancer is selected from the group consisting of Ubi, CMV and RSV enhancers, or any combination thereof;
the intron is selected from the group consisting of MVM, SV40, $\beta$ globin, EF-1$\alpha$ and hybrid introns, or any combination thereof;
the polyA is selected from the group consisting of PA75 polyA, SV40 polyA, hGH polyA, BGH polyA, rbGlob polyA, or any combination thereof; and/or the post-transcriptional regulatory element is selected from the group consisting of WPRE, HPRE, or a combination thereof;
preferably, the nucleic acid molecule comprises, from 5' end to 3' end:

(1) CMV enhancer-CBA promoter-hybrid intron-first polynucleotide-third polynucleotide-second polynucleotide encoding GDNF-WPRE-SV40 polyA;
(2) CMV enhancer-CBA promoter-hybrid intron-second polynucleotide encoding GDNF-third polynucleotide-first polynucleotide-WPRE-SV40 polyA;
(3) CMV enhancer-CBA promoter-hybrid intron-first polynucleotide-SV40 polyA-CMV enhancer-CMV promoter-second polynucleotide encoding GDNF-PA75 polyA;
(4) CMV enhancer-CBA promoter-hybrid intron-first polynucleotide-SV40 polyA-CMV enhancer-CMV promoter-second polynucleotide encoding GDNF-WPRE-PA75 polyA;
(5) CMV enhancer-CBA promoter-hybrid intron-first polynucleotide-SV40 polyA-CMV enhancer-CMV promoter-$\beta$ globin intron-second polynucleotide encoding GDNF-PA75 polyA;
(6) CMV enhancer-CMV promoter-$\beta$ globin intron-second polynucleotide encoding GDNF-PA75 polyA-CMV enhancer-CBA promoter-hybrid intron-first polynucleotide-SV40 polyA;
(7) CMV enhancer-CMV promoter-second polynucleotide encoding GDNF-PA75 polyA-CMV enhancer-CBA promoter-hybrid intron-first polynucleotide-WPRE-SV40 polyA; or
(8) CMV enhancer-CMV promoter-MVM intron-second polynucleotide encoding GDNF-PA75 polyA-CMV enhancer-CBA promoter-hybrid intron-first polynucleotide-WPRE-SV40 polyA.

10. The pharmaceutical composition according to claim 8 or 9, wherein the nucleic acid molecule comprises the sequence set forth in any one of SEQ ID NOs: 13-25 and 140-141 or a sequence having at least 95% sequence identity to any one of SEQ ID NOs: 13-25 and 140-141.

11. The pharmaceutical composition according to any one of claims 1 to 10, further comprising an inorganic salt, wherein the inorganic salt is preferably a sodium salt and/or a magnesium salt; the sodium salt is preferably sodium chloride, and the magnesium salt is preferably magnesium chloride, more preferably magnesium chloride hexahydrate.

12. The pharmaceutical composition according to any one of claims 1 to 11, wherein the sodium salt is at a concentration of about 10 mM to about 500 mM, preferably about 10 mM to about 200 mM, and more preferably about 100 mM to about 150 mM; and/or the magnesium salt is at a concentration of about 0.1 mM to about 50 mM, preferably about 1 mM to about 10 mM, and more preferably about 1 mM to about 5 mM.

**13.** The pharmaceutical composition according to any one of claims 1 to 12, further comprising one or more of a saccharide, an alcohol, and a sugar alcohol, wherein

preferably, the saccharide is selected from the group consisting of sucrose, mannose, and/or trehalose, and more preferably, the saccharide is sucrose;
preferably, the alcohol is glycerol;
preferably, the pharmaceutical composition comprises a saccharide and an alcohol, wherein:

the saccharide is at a concentration of about 0.1% (w/v) to about 10% (w/v), preferably about 1% (w/v) to about 10% (w/v), and preferably about 1% (w/v) to about 5% (w/v); and/or
the alcohol is at a concentration of about 0.01% (w/v) to about 10% (w/v), preferably about 0.05% (w/v) to about 5% (w/v), and preferably about 0.1% (w/v) to about 0.5% (w/v).

**14.** The pharmaceutical composition according to any one of claims 1 to 13, further comprising a surfactant, wherein

preferably, the surfactant is poloxamer 188 and/or polysorbate 80, and more preferably, the surfactant is poloxamer 188;
preferably, the surfactant is at a concentration of about 0.001% (w/v) to about 1% (w/v), preferably about 0.001% (w/v) to about 0.05% (w/v), and preferably about 0.01% (w/v) to about 0.02% (w/v).

**15.** The pharmaceutical composition according to any one of claims 1 to 14, further comprising an amino acid, wherein the amino acid is preferably histidine;
preferably, the amino acid is at a concentration of about 1 mM to about 50 mM, preferably about 1 mM to about 10 mM, and more preferably about 1 mM to about 5 mM.

**16.** The pharmaceutical composition according to any one of claims 1 to 15, wherein the pharmaceutical composition has a pH of about 4.0 to about 10 and preferably about 6.0 to about 7.5.

**17.** The pharmaceutical composition according to any one of claims 1 to 16, wherein the recombinant adeno-associated virus (rAAV) particle has a titer of about $1 \times 10^8$ Vg/mL to about $1 \times 10^{14}$ Vg/mL, preferably about $1 \times 10^{11}$ Vg/mL to about $9 \times 10^{13}$ Vg/mL, and preferably about $1 \times 10^{12}$ Vg/mL to about $9 \times 10^{13}$ Vg/mL.

**18.** A pharmaceutical composition, comprising recombinant adeno-associated virus (rAAV) particles and:

(a) about 1 mM to about 100 mM Hepes,

about 10 mM to about 500 mM sodium salt and about 1 mM to about 50 mM magnesium salt,
0.1% (w/v) to about 10% (w/v) saccharide,
about 0.01% (w/v) to about 10% (w/v) alcohol,
1 mM to about 50 mM amino acid, and
about 0.001% (w/v) to about 0.05% (w/v) surfactant selected from the group consisting of poloxamer 188 and/or polysorbate 80, the pharmaceutical composition having a pH of about 4.0 to about 10.0;

(c) about 5 mM to about 50 mM Hepes,

about 10 mM to about 200 mM sodium chloride and about 1 mM to about 10 mM magnesium chloride hexahydrate,
about 1% (w/v) to about 10% (w/v) saccharide selected from the group consisting of sucrose and/or trehalose,
about 0.05% (w/v) to about 5% (w/v) glycerol,
about 1 mM to about 10 mM histidine, and
about 0.001% (w/v) to about 0.05% (w/v) surfactant selected from the group consisting of poloxamer 188 and/or polysorbate 80, the pharmaceutical composition having a pH of about 4.0 to about 10.0, preferably about 6.0 to about 8.0;

(e) about 5 mM to about 30 mM Hepes,

about 50 mM to about 200 mM sodium chloride and about 1 mM to about 5 mM magnesium chloride hexahydrate,
about 1% (w/v) to about 5% (w/v) sucrose,
about 0.1% (w/v) to about 0.5% (w/v) glycerol,
about 1 mM to about 5 mM histidine, and
about 0.001% (w/v) to about 0.02% (w/v) poloxamer 188, the pharmaceutical composition having a pH of about 6.0 to about 8.0, preferably about 6.0 to about 7.5;

(g) about 5 mM to about 20 mM Hepes,

about 100 mM to about 150 mM sodium chloride and about 1 mM to about 5 mM magnesium chloride hexahydrate,
about 1% (w/v) to about 5% (w/v) sucrose,
about 0.1% (w/v) to about 0.5% (w/v) glycerol,
about 1 mM to about 5 mM histidine, and
about 0.01% (w/v) to about 0.02% (w/v) poloxamer 188, the pharmaceutical composition having a pH of about 6.0 to about 8.0, preferably about 6.0 to about 7.5;
preferably, the recombinant adeno-associated virus (rAAV) particle has a titer of about $1 \times 10^8$ Vg/mL to about $1 \times 10^{14}$ Vg/mL, preferably about $1 \times 10^{11}$ Vg/mL to about $9 \times 10^{13}$ Vg/mL, and preferably about $1 \times 10^{12}$ Vg/mL to about $9 \times 10^{13}$ Vg/mL;
preferably, the pharmaceutical composition comprises:

(1) about $1 \times 10^{11}$ Vg/mL to about $9 \times 10^{13}$ Vg/mL said recombinant adeno-associated virus (rAAV) particle,

about 10 mM Hepes,
about 100 mM sodium chloride and about 2 mM magnesium chloride hexahydrate,
about 2.5% (w/v) sucrose,
about 0.25% (w/v) glycerol,
about 5 mM histidine, and
about 0.01% (w/v) poloxamer 188, the pharmaceutical composition having a pH of about 6.5 to about 8.0, preferably about 7.5;

(2) about $1 \times 10^{11}$ Vg/mL to about $9 \times 10^{13}$ Vg/mL said recombinant adeno-associated virus (rAAV) particle,

about 10 mM Hepes,
about 100 mM sodium chloride and about 2 mM magnesium chloride hexahydrate,
about 2.5% (w/v) sucrose,
about 0.25% (w/v) glycerol,
about 5 mM histidine, and
about 0.01% (w/v) polysorbate 80, the pharmaceutical composition having a pH of about 6.5 to about 8.0, preferably about 7.5;

(3) about $1 \times 10^{11}$ Vg/mL to about $9 \times 10^{13}$ Vg/mL said recombinant adeno-associated virus (rAAV) particle,

about 10 mM Hepes,
about 150 mM sodium chloride and about 2 mM magnesium chloride hexahydrate,
about 2.5% (w/v) sucrose,
about 0.25% (w/v) glycerol,
about 5 mM histidine, and
about 0.01% (w/v) poloxamer 188, the pharmaceutical composition having a pH of about 5.0 to about 7.0, preferably about 6.0;

(4) about $1 \times 10^{11}$ Vg/mL to about $9 \times 10^{13}$ Vg/mL said recombinant adeno-associated virus (rAAV) particle,

about 10 mM Hepes,

about 150 mM sodium chloride and about 2 mM magnesium chloride hexahydrate,
about 2.5% (w/v) sucrose,
about 0.25% (w/v) glycerol,
about 5 mM histidine, and
about 0.01% (w/v) polysorbate 80, the pharmaceutical composition having a pH of about 5.0 to about 7.0, preferably about 6.0.

19. The pharmaceutical composition according to any one of claims 1 to 18, wherein the recombinant adeno-associated virus (rAAV) particle comprises a nucleic acid molecule and an AAV capsid;
preferably, the AAV capsid is selected from the group consisting of AAV1, AAV2, AAV3, AAV4, AAV5, AAV6, AAV7, AAV8, AAV9, AAV9.47, AAV9(hu14), AAV10, AAV11, AAV12, AAV13, AAVPHP.B, AAVrh74, AAVrh8, AAVrh10, AAV-DJ, and AAV-DJ8, and more preferably, the AAV capsid is AAV2 or AAV9.

20. A pharmaceutical composition, comprising recombinant adeno-associated virus (rAAV) particles and excipients, wherein the recombinant adeno-associated virus (rAAV) particle comprises a nucleic acid molecule and an AAV9 capsid, and the excipient comprises any one of the following:

(a) about 1 mM to about 100 mM Hepes,

about 10 mM to about 500 mM sodium salt and about 1 mM to about 50 mM magnesium salt, 0.1% (w/v) to about 10% (w/v) saccharide,
about 0.01% (w/v) to about 10% (w/v) alcohol,
1 mM to about 50 mM amino acid, and
about 0.001% (w/v) to about 0.05% (w/v) surfactant selected from the group consisting of poloxamer 188 and/or polysorbate 80, the pharmaceutical composition having a pH of about 4.0 to about 10.0;

(c) about 5 mM to about 50 mM Hepes,

about 10 mM to about 200 mM sodium chloride and about 1 mM to about 10 mM magnesium chloride,
about 1% (w/v) to about 10% (w/v) saccharide selected from the group consisting of sucrose and/or trehalose,
about 0.05% (w/v) to about 5% (w/v) glycerol,
about 1 mM to about 10 mM histidine, and
about 0.001% (w/v) to about 0.05% (w/v) surfactant selected from the group consisting of poloxamer 188 and/or polysorbate 80, the pharmaceutical composition having a pH of about 4.0 to about 10.0, preferably about 6.0 to about 8.0;

(e) about 10 mM to about 30 mM Hepes,

about 50 mM to about 200 mM sodium chloride and about 1 mM to about 5 mM magnesium chloride hexahydrate,
about 1% (w/v) to about 5% (w/v) sucrose,
about 0.1% (w/v) to about 0.5% (w/v) glycerol,
about 1 mM to about 5 mM histidine, and
about 0.001% (w/v) to about 0.02% (w/v) poloxamer 188, the pharmaceutical composition having a pH of about 6.0 to about 8.0, preferably about 6.0 to about 7.5;

(h) about 5 mM to about 20 mM Hepes,

about 80 mM to about 120 mM sodium chloride and about 1 mM to about 5 mM magnesium chloride hexahydrate,
about 1% (w/v) to about 5% (w/v) sucrose,
about 0.1% (w/v) to about 0.5% (w/v) glycerol,
about 1 mM to about 5 mM histidine, and
about 0.01% (w/v) to about 0.02% (w/v) poloxamer 188, the pharmaceutical composition having a pH of about 6.0 to about 8.0, preferably about 6.0 to about 7.5;
preferably, the recombinant adeno-associated virus (rAAV) particle has a titer of about $1 \times 10^8$ Vg/mL to about $1 \times 10^{14}$ Vg/mL, preferably about $1 \times 10^{11}$ Vg/mL to about $9 \times 10^{13}$ Vg/mL, and preferably about $1 \times$

$10^{12}$ Vg/mL to about $9 \times 10^{13}$ Vg/mL;
preferably, the excipient comprises any one of the following:

(1) about $1 \times 10^{11}$ Vg/mL to about $9 \times 10^{13}$ Vg/mL said recombinant adeno-associated virus (rAAV) particle,

about 10 mM Hepes,
about 100 mM sodium chloride and about 2 mM magnesium chloride hexahydrate,
about 2.5% (w/v) sucrose,
about 0.25% (w/v) glycerol,
about 5 mM histidine, and
about 0.01% (w/v) poloxamer 188, the pharmaceutical composition having a pH of about 6.5 to about 8.0, preferably about 7.5;

(3) about $1 \times 10^{11}$ Vg/mL to about $9 \times 10^{13}$ Vg/mL said recombinant adeno-associated virus (rAAV) particle,

about 10 mM Hepes,
about 100 mM sodium chloride and about 2 mM magnesium chloride hexahydrate,
about 2.5% (w/v) sucrose,
about 0.25% (w/v) glycerol,
about 5 mM histidine, and
about 0.01% (w/v) polysorbate 80, the pharmaceutical composition having a pH of about 6.5 to about 8.0, preferably about 7.5;
preferably, the pharmaceutical composition comprises the nucleic acid molecule according to any one of claims 2 to 10; or
the pharmaceutical composition comprises recombinant adeno-associated virus (rAAV) particles and excipients wherein the recombinant adeno-associated virus (rAAV) particle comprises a nucleic acid molecule and an AAV2 capsid, and the excipient comprises any one of the following:

(a) about 1 mM to about 100 mM Hepes,

about 10 mM to about 500 mM sodium salt and about 1 mM to about 50 mM magnesium salt, 0.1% (w/v) to about 10% (w/v) saccharide,
about 0.01% (w/v) to about 10% (w/v) alcohol,
1 mM to about 50 mM amino acid, and
about 0.001% (w/v) to about 0.05% (w/v) surfactant selected from the group consisting of poloxamer 188 and/or polysorbate 80, the pharmaceutical composition having a pH of about 4.0 to about 10.0;

(c) about 5 mM to about 50 mM Hepes,

about 10 mM to about 200 mM sodium chloride and about 1 mM to about 10 mM magnesium chloride,
about 1% (w/v) to about 10% (w/v) saccharide selected from the group consisting of sucrose and/or trehalose,
about 0.05% (w/v) to about 5% (w/v) glycerol,
about 1 mM to about 10 mM histidine, and
about 0.001% (w/v) to about 0.05% (w/v) surfactant selected from the group consisting of poloxamer 188 and/or polysorbate 80, the pharmaceutical composition having a pH of about 4.0 to about 10.0, preferably about 6.0 to about 8.0;

(e) about 10 mM to about 30 mM Hepes,

about 50 mM to about 200 mM sodium chloride and about 1 mM to about 5 mM magnesium chloride hexahydrate,
about 1% (w/v) to about 5% (w/v) sucrose,
about 0.1% (w/v) to about 0.5% (w/v) glycerol,

about 1 mM to about 5 mM histidine, and
about 0.001% (w/v) to about 0.02% (w/v) poloxamer 188, the pharmaceutical composition having a pH of about 6.0 to about 8.0, preferably about 6.0 to about 7.5;

(i) about 5 mM to about 20 mM Hepes,

about 120 mM to about 180 mM sodium chloride and about 1 mM to about 5 mM magnesium chloride hexahydrate,
about 1% (w/v) to about 5% (w/v) sucrose,
about 0.1% (w/v) to about 0.5% (w/v) glycerol,
about 1 mM to about 5 mM histidine, and
about 0.01% (w/v) to about 0.02% (w/v) poloxamer 188, the pharmaceutical composition having a pH of about 6.0 to about 8.0, preferably about 6.0 to about 7.5;
preferably, the recombinant adeno-associated virus (rAAV) particle has a titer of about $1 \times 10^8$ Vg/mL to about $1 \times 10^{14}$ Vg/mL, preferably about $1 \times 10^{11}$ Vg/mL to about $9 \times 10^{13}$ Vg/mL, and preferably about $1 \times 10^{12}$ Vg/mL to about $9 \times 10^{13}$ Vg/mL;
preferably, the excipient comprises any one of the following:

(4) about $1 \times 10^{11}$ Vg/mL to about $9 \times 10^{13}$ Vg/mL said recombinant adeno-associated virus (rAAV) particle,

about 10 mM Hepes,
about 150 mM sodium chloride and about 2 mM magnesium chloride hexahydrate,
about 2.5% (w/v) sucrose,
about 0.25% (w/v) glycerol,
about 5 mM histidine, and
about 0.01% (w/v) poloxamer 188, the pharmaceutical composition having a pH of about 5.0 to about 7.0, preferably about 6.0;

(6) about $1 \times 10^{11}$ Vg/mL to about $9 \times 10^{13}$ Vg/mL said recombinant adeno-associated virus (rAAV) particle,

about 10 mM Hepes,
about 150 mM sodium chloride and about 2 mM magnesium chloride hexahydrate,
about 2.5% (w/v) sucrose,
about 0.25% (w/v) glycerol,
about 5 mM histidine, and
about 0.01% (w/v) polysorbate 80, the pharmaceutical composition having a pH of about 5.0 to about 7.0, preferably about 6.0.

21. A freeze-dried formulation, wherein the freeze-dried formulation is obtained by lyophilizing the pharmaceutical composition according to any one of claims 1 to 20, or the freeze-dried formulation, after being reconstituted, is capable of forming the pharmaceutical composition according to any one of claims 1 to 20.

22. A reconstituted solution, wherein the reconstituted solution is prepared by reconstituting the freeze-dried formulation according to claim 21.

23. An article of manufacture, comprising a container, wherein the container contains the pharmaceutical composition according to any one of claims 1 to 20, the freeze-dried formulation according to claim 27, or the reconstituted solution according to claim 22.

24. A method for treating or preventing a disease, comprising administering to a subject in need thereof a therapeutically or prophylactically effective amount of the pharmaceutical composition according to any one of claims 1 to 20, the freeze-dried formulation according to claim 21, the reconstituted solution according to claim 22, or the article of manufacture according to claim 23, wherein preferably, the disease is selected from the group consisting of a disease associated with abnormal angiogenesis and a central nervous system disease or symptom.

25. The method according to claim 24, wherein the disease is a disease associated with abnormal angiogenesis;

preferably, the disease associated with abnormal angiogenesis is an angiogenic ocular disease or cancer.

26. The method according to claim 24, wherein the disease is a central nervous system disease or symptom; preferably, the central nervous system disease or symptom is a neurodegenerative disease or symptom, more preferably dyskinesia and sleep disorder, and most preferably Parkinson's disease.

FIG. 1A

FIG. 1B

DAPI/hAADC

AAV2-02A          AAV9-02A

FIG. 2A

N = 4 mice/group

FIG. 2B

FIG. 3A

FIG. 3B

FIG. 4A

FIG. 4B

FIG. 5A

FIG. 5B

FIG. 5C

TH+ staining on sections to mark the survival protection for dopaminergic neuron axonal ends projecting to the striatum

Left    Right

FIG. 5D

FIG. 6A

FIG. 6B

FIG. 6C

FIG. 6D

FIG. 6E

FIG. 6F

VEGFR1 D2

VEGFR2 D3

VEGFR2 D2

VEGFR2 D3

VEGFR2 D2

VEGFR2 D3

VEGFR1 D2

VEGFR2 D3

VEGFR2 D2

VEGFR2 D3

Bevacizumab Fab

FIG. 7

| CMV enhancer | CMV promoter | 5'UTR | Kozak sequence | VEGF-binding molecule sequence | 3'UTR | WPRE | sv40polyA |
|---|---|---|---|---|---|---|---|

FIG. 8

FIG. 9

FIG. 10

| PBS | Aflibercept (2.5 mg/mL) | OPT302 (10 mg/mL) | PR1145 (3.75 mg/mL) | PR1145 (1.5 mg/mL) |

FIG. 11A

FIG. 11B

FIG. 12

FIG. 13

FIG. 14

| | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 |
|---|---|---|---|---|---|---|---|---|---|---|---|---|---|---|---|---|---|---|---|---|---|---|---|---|---|---|---|---|---|
| pH (cooling) | 8.08 | 8.07 | 8.05 | 8.03 | 8 | 7.98 | 7.95 | 7.93 | 7.91 | 7.88 | 7.85 | 7.83 | 7.8 | 7.77 | 7.74 | 7.72 | 7.7 | | | | | | | | | | | | |
| pH (heating) | 8.08 | 8.06 | 8.04 | 8.02 | 7.99 | 7.97 | 7.94 | 7.92 | 7.9 | 7.88 | 7.85 | 7.83 | 7.81 | 7.77 | 7.74 | 7.73 | 7.7 | 7.68 | 7.66 | 7.63 | 7.61 | 7.59 | 7.56 | 7.54 | 7.51 | 7.49 | 7.46 | 7.44 | 7.42 |

Temperature (°C)

pH (cooling) — pH (heating)

FIG. 15

FIG. 16

185

Study on effect of temperature on pH of buffer-BTP

Study on effect of temperature on pH of buffer-Tris

Study on effect of temperature on pH of buffer-Hepes

FIG. 17

## INTERNATIONAL SEARCH REPORT

| International application No. |
| --- |
| **PCT/CN2024/114158** |

| A. | CLASSIFICATION OF SUBJECT MATTER |
| --- | --- |

A61K 38/17(2006.01)i; C12N 15/86(2006.01)i; C07K 19/00(2006.01)i; C07K 16/22(2006.01)i; C07K 14/005(2006.01)i; A61P 25/28(2006.01)i

According to International Patent Classification (IPC) or to both national classification and IPC

| B. | FIELDS SEARCHED |
| --- | --- |

Minimum documentation searched (classification system followed by classification symbols)

IPC: A61K, C12N, C07K, A61P

Documentation searched other than minimum documentation to the extent that such documents are included in the fields searched

Electronic data base consulted during the international search (name of data base and, where practicable, search terms used)

CJFD, CNTXT, DWPI, ENTXT, ENTXTC, VEN, WPABS, WPABSC, CNKI, PUBMED, GENBANK: 抗VEGF, 免疫球蛋白样结构域, 突变, 腺相关病毒, 芳香族L-氨基酸脱羧酶, 神经胶质细胞系来源的神经营养因子, AADC, AAV, C162, GDNF, Hepes, R2D2, R2D3, VEGF Trap, VEGFR, VEGFR1, VEGFR2, KDR, Flt1, variant, mutant, buffer, SEQ ID NOs: 1-25, 53-55, 57, 89-99, 101-118, 128-136 and 140-141

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | CN 115554418 A (SICHUAN ZHISHAN WEIXIN BIOLOGICAL TECHNOLOGY CO., LTD.) 03 January 2023 (2023-01-03)<br>claims 1-6, and description, paragraphs 11-14 and 32 | 1, 11-14, 16, 18, 20, 21-24 |
| X | CN 114728049 A (REGENERATIVE BIOLOGICS, INC.) 08 July 2022 (2022-07-08)<br>claims 1-20, 67-75, 109-111 and 121-133 | 1, 11-16, 18, 20, 21-24 |
| PY | WO 2023155918 A1 (SHANGHAI REGENELEAD THERAPIES CO., LTD.) 24 August 2023 (2023-08-24)<br>claims 1-34 | 2-7, 21-26 |
| Y | CN 115554418 A (SICHUAN ZHISHAN WEIXIN BIOLOGICAL TECHNOLOGY CO., LTD.) 03 January 2023 (2023-01-03)<br>claims 1-6, and description, paragraphs 11-14 and 32 | 2-17, 19, 21-26 |
| Y | CN 114728049 A (REGENERATIVE BIOLOGICS, INC.) 08 July 2022 (2022-07-08)<br>claims 1-20, 67-75, 109-111 and 121-133 | 2-17, 19, 21-26 |

☑ Further documents are listed in the continuation of Box C. ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **27 November 2024** | **09 December 2024** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/114158** |

### C. DOCUMENTS CONSIDERED TO BE RELEVANT

| Category* | Citation of document, with indication, where appropriate, of the relevant passages | Relevant to claim No. |
| --- | --- | --- |
| Y | CN 101838329 A (GENOR BIOPHARMA CO., LTD.) 22 September 2010 (2010-09-22) claims 1-10, and description, paragraphs 17-18, 23 and 41-45, and embodiments 1-3 | 2, 5-6, 9, 11-17, 19, 21-26 |
| Y | US 2021163991 A1 (REGENXBIO INC.) 03 June 2021 (2021-06-03) claims 1 and 124-129 | 8-17, 21-26 |
| A | CN 101501065 A (TROPHOGEN INC.) 05 August 2009 (2009-08-05) claims 1-28 | 1-26 |
| A | CN 113861291 A (SUZHOU ALPHAMAB CO., LTD.) 31 December 2021 (2021-12-31) claims 1-10 | 1-26 |

Form PCT/ISA/210 (second sheet) (July 2022)

## INTERNATIONAL SEARCH REPORT

| International application No. |
| --- |
| **PCT/CN2024/114158** |

**Box No. I          Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑ forming part of the international application as filed.

    b.  ☐ furnished subsequent to the international filing date for the purposes of international search (Rule 13*ter*.1(a)).

          ☐ accompanied by a statement to the effect that the sequence listing does not go beyond the disclosure in the international application as filed.

2.  ☐ With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

## INTERNATIONAL SEARCH REPORT

International application No.

**PCT/CN2024/114158**

| Box No. II | Observations where certain claims were found unsearchable (Continuation of item 2 of first sheet) |

This international search report has not been established in respect of certain claims under Article 17(2)(a) for the following reasons:

1. ☑ Claims Nos.: **24-26**
   because they relate to subject matter not required to be searched by this Authority, namely:

   Claims 24-26 set forth a method for treating or preventing diseases, and thus do not comply with PCT Rule 39.1(iv). The present report is provided on the basis of amending said claims to be pharmaceutical use claims.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

| Box No. III | Observations where unity of invention is lacking (Continuation of item 3 of first sheet) |

This International Searching Authority found multiple inventions in this international application, as follows:

Invention 1: claims 1-2, 8-10, 18 and 20, and claims 3-7, 11-17, 19 and 21-26 (all in part) relate to a pharmaceutical composition, a lyophilized preparation, a reconstitution solution, a product, and a method for treating or preventing diseases, and relate to the technical solution of a first VEGF-binding domain of a VEGF-binding molecule having an amino acid mutation at position 162 thereof, wherein the site of the amino acid mutation is a natural order site relative to the amino acid sequence as shown in SEQ ID NO: 51.

Invention 2: claims 1-2, 8-10, 18 and 20, and claims 3-7, 11-17, 19 and 21-26 (all in part) relate to a pharmaceutical composition, a lyophilized preparation, a reconstitution solution, a product, and a method for treating or preventing diseases, and relate to the technical solution of a first VEGF-binding domain of a VEGF-binding molecule having an amino acid mutation at position 221 thereof, wherein the site of the amino acid mutation is a natural order site relative to the amino acid sequence as shown in SEQ ID NO: 51.

...

Invention 22: claims 1, 2, 8-10, 18 and 20, and claims 3-7, 11-17, 19 and 21-26 (all in part) relate to a pharmaceutical composition, a lyophilized preparation, a reconstitution solution, a product, and a method for treating or preventing diseases, and relate to the technical solution of a first VEGF-binding domain of a VEGF-binding molecule having an amino acid mutation at position 314 thereof, wherein the site of the amino acid mutation is a natural order site relative to the amino acid sequence as shown in SEQ ID NO: 51.

That is, according to different mutation sites of the first VEGF-binding domain of the VEGF-binding molecule that are involved, claims 1-26 of the present application can be divided into 22 groups of inventions. The VEGF-binding molecules involved in the above 22 inventions are all VEGF-binding molecule mutants and there is a mutation site in the first VEGF-binding domain; however, the 22 VEGF-binding molecule mutants do not have a common structural feature due to the different mutation sites. In addition, the prior art also discloses variants of VEGF-binding molecules. For example, D1 (CN 101501065 A, 05 August 2009) discloses a VEGF-binding molecule variant, and compared with the amino acid sequence as shown in SEQ ID NO: 51 of the present application, the amino acid sequence as shown in SEQ ID NO: 66 has an amino acid site mutation at position 208, that is, the functional definition of the first VEGF-binding domain having a mutation is also not a special technical feature of the 22 inventions. Therefore, the 22 inventions comprised in claims 1-26 do not have a special technical feature that defines a contribution which the inventions make over the prior art as defined in PCT Rule 13.2, and thus the present application lacks unity of invention (PCT Rule 13.1).

In view of the fact that the applicant does not pay the additional search fee according to the requirement in a notification for paying additional fees and protest fees, where applicable, that is issued on 14 October 2024, the examiner only provides a search report for invention 1.

Form PCT/ISA/210 (continuation of first sheet) (July 2022)

## INTERNATIONAL SEARCH REPORT

| International application No. |
|---|
| **PCT/CN2024/114158** |

**Box No. III     Observations where unity of invention is lacking (Continuation of item 3 of first sheet)**

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims: it is covered by claims Nos.: **claims 1-2, 8-10, 18 and 20, and claims 3-7, 11-17, 19 and 21-26 (all in part)**

**Remark on Protest**     ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

**INTERNATIONAL SEARCH REPORT**
Information on patent family members

International application No.

**PCT/CN2024/114158**

| Patent document cited in search report | | | Publication date (day/month/year) | Patent family member(s) | | | Publication date (day/month/year) |
|---|---|---|---|---|---|---|---|
| CN | 115554418 | A | 03 January 2023 | None | | | |
| CN | 114728049 | A | 08 July 2022 | EP | 4041292 | A1 | 17 August 2022 |
| | | | | TW | 202126319 | A | 16 July 2021 |
| | | | | KR | 20220081365 | A | 15 June 2022 |
| | | | | WO | 2021071835 | A1 | 15 April 2021 |
| | | | | IL | 291930 | A | 01 June 2022 |
| | | | | MX | 2022004146 | A | 19 September 2022 |
| | | | | AR | 120171 | A1 | 02 February 2022 |
| | | | | AU | 2020362119 | A1 | 26 May 2022 |
| | | | | CA | 3156984 | A1 | 15 April 2021 |
| | | | | US | 2024108669 | A1 | 04 April 2024 |
| | | | | JP | 2022552262 | A | 15 December 2022 |
| | | | | BR | 112022006718 | A2 | 12 July 2022 |
| WO | 2023155918 | A1 | 24 August 2023 | KR | 20240150793 | A | 16 October 2024 |
| | | | | TW | 202342526 | A | 01 November 2023 |
| | | | | AU | 2023220237 | A1 | 15 August 2024 |
| CN | 101838329 | A | 22 September 2010 | US | 2010331250 | A1 | 30 December 2010 |
| | | | | US | 8926972 | B2 | 06 January 2015 |
| | | | | JP | 2012520661 | A | 10 September 2012 |
| | | | | BRPI | 1006368 | A2 | 25 October 2016 |
| | | | | ZA | 201106638 | B | 30 May 2012 |
| | | | | WO | 2010105573 | A1 | 23 September 2010 |
| | | | | RU | 2011141522 | A | 27 April 2013 |
| US | 2021163991 | A1 | 03 June 2021 | JP | 2024084803 | A | 25 June 2024 |
| | | | | AU | 2019319976 | A1 | 28 January 2021 |
| | | | | KR | 20210043580 | A | 21 April 2021 |
| | | | | WO | 2020033842 | A1 | 13 February 2020 |
| | | | | IL | 280653 | A | 25 March 2021 |
| | | | | JP | 2021533757 | A | 09 December 2021 |
| | | | | EP | 3833745 | A1 | 16 June 2021 |
| | | | | CA | 3108113 | A1 | 13 February 2020 |
| CN | 101501065 | A | 05 August 2009 | None | | | |
| CN | 113861291 | A | 31 December 2021 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202311073344
- CN 202311077883
- WO 2022247917 A
- WO 2018232055 A
- WO 2005033321 A
- WO 2015168666 A
- WO 2015121501 A
- WO 2015038958 A
- WO 2016065001 A
- WO 2016130589 A
- WO 2016049230 A
- WO 2016134375 A
- WO 2017100671 A
- WO 2017083722 A
- WO 2017015102 A
- WO 2017058892 A
- WO 2017066764 A
- US 9546112 B
- US 7198951 B
- US 9233131 B
- US 6156303 A
- US 9624274 B
- US 9475845 B
- US 8734809 B
- US 20130224836 A
- US 20140359799 A
- US 20150315612 A
- US 20150376240 A
- US 20150159173 A
- US 20150376607 A
- US 20150238550 A
- US 20160369298 A
- US 20160361439 A
- US 20170145405 A
- US 20160017005 A
- WO 2023093905 A
- WO 2023155918 A
- WO 2016005324 A
- WO 2016005004 A
- WO 2016091391 A
- WO 2019036513 A
- WO 2020074642 A
- WO 200028004 A
- WO 200123001 A
- WO 2004112727 A
- WO 2005005610 A
- WO 2005072364 A
- WO 2013123503 A
- WO 2015191508 A
- US 20130195801 A
- WO 0028061 A
- WO 9961601 A
- WO 9811244 A
- WO 2012145601 A
- WO 2017197355 A
- WO 2018022905 A
- WO 2017218974 A

**Non-patent literature cited in the description**

- **BOYE et al.** *Mol Ther.*, March 2013, vol. 21 (3), 509-19
- **TRAPANI et al.** *Prog Retin Eye Res.*, November 2014, vol. 43, 108-28
- **MACLAREN et al.** *Lancet*, 29 March 2014, vol. 383 (9923), 1129-37
- **MAGUIRE et al.** *N Engl J Med.*, 22 May 2008, vol. 358 (21), 2240-8
- **SIMONELLI et al.** *Mol Ther.*, March 2010, vol. 18 (3), 643-50
- **NATHWANI et al.** *N Engl J Med.*, 20 November 2014, vol. 371 (21), 1994-2004
- **NPULICHERLA et al.** *MolecularTherapy*, 2011, vol. 19 (6), 1070-1078
- **SRIVISTAVA et al.** *J. Virology*, 1983, vol. 45, 555
- **CHIORINI et al.** *J. Virology*, 1998, vol. 71, 6823
- **CHIORINI et al.** *J. Virology*, 1999, vol. 73, 1309
- **BANTEL-SCHAAL et al.** *J. Virology*, 1999, vol. 73, 939
- **XIAO et al.** *J. Virology*, 1999, vol. 73, 3994
- **MURAMATSU et al.** *Virology*, 1996, vol. 221, 208
- **SHADE et al.** *J. Virol.*, 1986, vol. 58, 921
- **GAO**. *Proc. Nat. Acad. Sci. USA*, 2002, vol. 99, 11854
- **MORIS et al.** *Virology*, 2004, vol. 33, 375-383
- **VAN VLIET et al.** *Mol. Ther.*, 2006, vol. 14, 809
- **PADRON et al.** *J. Virol.*, 2005, vol. 79, 5047
- **SHEN et al.** *Mol. Ther.*, 2007, vol. 15, 1955

- **GRAINGER et al.** *Mol. Ther.*, 2005, vol. 11, S337
- **ZOLOTUKHIN et al.** *Gene Ther.*, 1999, vol. 6, 973
- Computer Methods for Macromolecular Sequence Analysis. Methods in Enzymology. Academic Press, Inc, 1996, vol. 266
- **SMITH-WATERMAN**. *Meth. Mol. Biol*, 1997, vol. 70, 173-187
- *J. Mol. Biol.*, 1970, vol. 48, 443-453
- Antibodies: A Laboratory Manual and Molecular Cloning: A Laboratory Manual. Cold Spring Harbor Laboratory
- **MARTIN LOCK et al.** Rapid, Simple, and Versatile Manufacturing of Recombinant Adeno-Associated Viral Vectors at Scale.. *HUMAN GENE THERAPY*, 2010, vol. 21, 1259-1271